# EUROPEAN PATENT SPECIFICATION

(11) **EP 2 870 143 B1**
(45) Date of publication and mention of the grant of the patent: **17.05.2017**
(21) Application number: 13737175.3
(22) Date of filing: 03.07.2013
(51) Int. Cl.: C07D 249/14, C07D 403/12, A61K 31/4196, A61P 31/14

(54) **TRIAZOLE COMPOUNDS AS ANTIVIRALS**
TRIAZOLE VERBINDUNGEN ALS ANTIVIRALE
COMPOSÉS TRIAZOLIQUES COMME ANTIVIRAUX

(30) Priority: 06.07.2012 US 201261668547 P
(43) Date of publication of application: 13.05.2015
(73) Proprietor: F. Hoffmann-La Roche AG, 4070 Basel (CH)
(72) Inventor: BILOTTA, Joseph Anthony, Nutley, New Jersey 07110 (US); CHEN, Zhi, Livingston, New Jersey 07039 (US); CHIN, Elbert, San Mateo, California 94403 (US); DING, Qingjie, Bridgewater, New Jersey 08807 (US); ERICKSON, Shawn David, Leonia, New Jersey 07605 (US); GABRIEL, Stephen Deems, Morristown, New Jersey 07960 (US); KLUMPP, Klaus, Palo Alto, California 94306 (US); MA, Han, Passaic, New Jersey 07055 (US); MERTZ, Eric, Fair Lawn, New Jersey 07410 (US); PLANCHER, Jean-Marc, F-68220 Hagenthal-le-Bas (FR); WEIKERT, Robert James, Clifton, New Jersey 07012 (US)
(74) Representative: Halbig, Dirk
(86) International application number: PCT/EP2013/063980
(87) International publication number: WO 2014/006066

(56) References cited:
- WO-A1-01/46212
- WO-A1-97/36859
- WO-A1-02/057240
- WO-A1-02/094814
- WO-A1-2005/012263
- WO-A1-2013/103384
- WO-A2-2004/032875
- WO-A2-2004/046120
- WO-A2-2007/030680
- WO-A2-2010/108187
- LIN R ET AL: "1-Acyl-1H-[1,2,4]triazole-3,5-diamine Analogues as Novel and Potent Anticancer Cyclin-Dependent Kinase Inhibitors: Synthesis and Evaluation of Biological Activities", JOURNAL OF MEDICINAL CHEMISTRY, AMERICAN CHEMICAL SOCIETY, US, vol. 48, no. 13, 3 June 2005 (2005-06-03), pages 4208-4211, XP002523423, ISSN: 0022-2623, DOI: 10.1021/JM050267E
- MALERICH J P ET AL: "Diamino-1,2,4-triazole derivatives are selective inhibitors of TYK2 and JAK1 over JAK2 and JAK3", BIOORGANIC & MEDICINAL CHEMISTRY LETTERS, PERGAMON, vol. 20, no. 24, 15 December 2010 (2010-12-15), pages 7454-7457, XP027501463, ISSN: 0960-894X, DOI: 10.1016/J.BMCL.2010.10.026 [retrieved on 2010-11-16]
- BLANK B ET AL: "SYNTHESIS OF 1,2,4-TRIAZOLES AS POTENTIAL HYPOGLYCEMIC AGENTS", JOURNAL OF MEDICINAL CHEMISTRY, AMERICAN CHEMICAL SOCIETY, US, vol. 15, no. 6, 1 January 1972 (1972-01-01), pages 694-696, XP001106940, ISSN: 0022-2623, DOI: 10.1021/JM00276A040
- FROMM E ET AL: "SPALTUNG DER DISULFIDE. SYNTHESE VON TRIAZOLEN", JUSTUS LIEBIGS ANNALEN DER CHEMIE, VERLAG CHEMIE GMBH, WEINHEIM; DE, vol. 437, no. 2, 1 January 1924 (1924-01-01), pages 106-124, XP009000177, ISSN: 0075-4617, DOI: 10.1002/JLAC.19244370108
- SCOTT J POLLACK ET AL: "A comparative study of fragment screening methods on the p38Î+- kinase: new methods, new insights", JOURNAL OF COMPUTER-AIDED MOLECULAR DESIGN, KLUWER ACADEMIC PUBLISHERS, DO, vol. 25, no. 7, 6 July 2011 (2011-07-06), pages 677-687, XP019939180, ISSN: 1573-4951, DOI: 10.1007/S10822-011-9454-9
- DAVIDSON ET AL: "Action of hydrazine hydrate on isodithiobiurets", JOURNAL OF THE CHEMICAL SOCIETY, SECTION C: ORGANIC CHEMISTRY, CHEMICAL SOCIETY. LETCHWORTH, GB, vol. 22, 1 January 1967 (1967-01-01), pages 2471-2472, XP009171763, ISSN: 0022-4952
- GLASS D ET AL: "4-(4-GUANIDINOBENZOYL)-2-IMIDAZOLONES AND RELATED COMPOUNDS: PHOSPHODIESTERASE INHIBITORS AND NOVEL CARDIOTONICS WITH COMBINED HISTAMINE H2 RECEPTOR AGONIST AND PDE III INHIBITOR ACTIVITY", ARCHIV DER PHARMAZIE, WILEY VERLAG, WEINHEIM, vol. 328, no. 10, 1 January 1995 (1995-01-01), pages 709-719, XP009002222, ISSN: 0365-6233

## Description

### FIELD OF THE INVENTION

The present invention provides compounds of Formula I useful as inhibitors of hepatitis C virus (HCV), as inhibitors of HCV infection, and for the prevention and treatment of hepatitis C infection.

Hepatitis C virus (HCV) infection is a major health problem that affects 170 million people worldwide and 3-4 million people in the United States (Armstrong, G.L., et al., Ann. Intern. Med. 2006, 144:705-714; Lauer, G.M., et al., N. Eng. J. Med. 2001, 345:41-52). HCV infection leads to chronic liver disease, such as cirrhosis and hepatocellular carcinoma in a substantial number of infected individuals. Chronic HCV infection associated liver cirrhosis and hepatocellular carcinoma are also the leading cause of liver transplantation in the United States. Current treatments for HCV infection include immunotherapy with pegylated interferon-α in combination with the nucleoside-analog ribavirin. Pegylated interferon-α in combination with ribavirin and one of the two recently approved HCV NS3 protease inhibitors Incivek or Victrelis is the current standard of care for the treatment of genotype 1 HCV infected patients, the most difficult to treat patient population. However, current HCV treatments are compromised by suboptimal sustained virological response rates and associated with severe side effects, as well as resistance to the protease inhibitors. Therefore there is a clear need for improved antiviral drugs with better efficacy, safety, and resistance profiles.

The infection of human hepatocytes by HCV, also known as HCV entry, is mediated by the functional interactions of virally-encoded envelope glycoproteins E1 and E2 and host cell co-receptors, followed by a receptor-mediated endocytosis processes. This HCV entry step is a putative target for therapeutic intervention. Several virally-encoded enzymes are also putative targets for therapeutic intervention, including a metalloprotease (NS2-3), a serine protease (NS3, amino acid residues 1-180), a helicase (NS3, full length), an NS3 protease cofactor (NS4A), a membrane protein (NS4B), a zinc metalloprotein (NS5A) and an RNA-dependent RNA polymerase (NS5B).

Systems have been developed to study the biology of HCV entry into host cells. Pseudotyping systems where the E1 and E2 glycoproteins are used to functionally replace the glycoproteins of retroviruses have been developed (Bartosch, B., Dubuisson, J. and Cosset, F.-L. J. Exp. Med. 2003, 197:633-642; Hsu, M. et al. Proc. Natl. Acad. Sci. USA. 2003, 100:7271-7276). These systems yield HCV pseudoparticles that bind to and enter host cells in a manner which is believed to be analogous to the natural virus, thus making them a convenient tool to study the viral entry steps as well as to identify inhibitors blocking this process.
There is a clear and long-felt need to develop effective therapeutics for treatment of HCV infection. Specifically, there is a need to develop compounds that selectively inhibit HCV viral entry and replication and that are useful for treating HCV-infected patients and protecting liver transplant patients from HCV re-infection. This application discloses novel compounds that are effective in prevention of HCV infection. Additionally, the disclosed compounds provide advantages for pharmaceutical uses, for example, with respect to their mechanism of action, binding, prevention of infection, inhibition efficacy, and target selectivity.

WO 02/094814 A1 discloses to 3,5-diamino-1,2,4-triazoles as kinase inhibitors, the production thereof and the use of the same as a medicament for treating various diseases, including hepatitis B and C, virus infections. More specifically, WO 02/094814 A1 discloses a Markush formula having a 5-amino-1-aryl or heteroaryl-3-heteroarylamino-1,2,4-triazole core.

WO 01/46212 A1 discloses a composition for and a method of treating a Flaviviridae (including BVDV and HCV) infection in animals and especially in humans, using β-D or β-L nucleosides or pharmaceutically acceptable salts thereof.

WO 2010/108187 A2 discloses various compounds, and pharmaceutically acceptable salts and prodrugs thereof, which are useful as inhibitors of IMPDH.

### SUMMARY OF THE INVENTION

The application provides compound of formula I 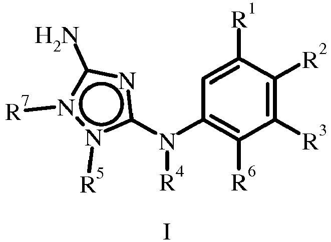 for use in the treatment of Hepatitis C Virus (HCV) infection, wherein:
R¹ is H, halo, lower haloalkyl, lower haloalkyl lower alkyl, trifluoromethyl sulfonyl, lower alkyl, alkoxy, nitro, carboxyl, lower alkyl sulfonamido, trifluoromethyl sulfinyl, cycloalkyl, SF₅, or - NHNH₂;
R² is H, halo, cyano, nitro, trifluoromethyl sulfonyl, lower halo alkyl, phenyl ethynyl, lower alkyl sulfonyl, lower alkyl amino sulfonyl, lower alkyl amido, amino sulfonyl, sulfonamido, benzyl oxy, cycloalkyl, heterocycloalkyl sulfonyl, aryl carbonyl, lower alkyl urea, lower alkyl ester, phenyl amido, heterocycloalkyl carbonyl, lower alkyl sulfonyl phenyl amido, lower alkyl cyano, cyano lower alkyl, or tetrazolyl lower alkyl;
R³ is H, halo, lower alkyl, lower alkenyl, lower haloalkyl, cyano, lower haloalkyl oxy, cyano cycloalkyl, lower haloalkyl lower alkyl, SF₅, or trifluoromethyl sulfanyl;
   or R² and R³ together form an aryl ring system;
R⁴ is H, lower alkyl, cyano lower alkyl, or phenyl lower alkyl;
R⁵ is absent, H, lower alkyl, or cycloalkyl;
R⁶ is H or halo;
R⁷ is absent, H, loweralkyl carbonyl, or lower haloalkyl phenyl sulfonyl;
with the proviso that when R⁵ is absent, R⁷ is not absent; and
with the proviso that when R⁷ is absent, R⁵ is not absent;
or a pharmaceutically acceptable salt thereof.

The application provides a composition comprising a compound of Formula I and a pharmaceutically acceptable excipient.

### DETAILED DESCRIPTION OF THE INVENTION

### Definitions

The phrase "a" or "an" entity as used herein refers to one or more of that entity; for example, a compound refers to one or more compounds or at least one compound. As such, the terms "a" (or "an"), "one or more", and "at least one" can be used interchangeably herein.

As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least". When used in the context of a process, the term "comprising" means that the process includes at least the recited steps, but may include additional steps. When used in the context of a compound or composition, the term "comprising" means that the compound or composition includes at least the recited features or components, but may also include additional features or components.

As used herein, unless specifically indicated otherwise, the word "or" is used in the "inclusive" sense of "and/or" and not the "exclusive" sense of "either/or".

The term "independently" is used herein to indicate that a variable is applied in any one instance without regard to the presence or absence of a variable having that same or a different definition within the same compound. Thus, in a compound in which R" appears twice and is defined as "independently carbon or nitrogen", both R"s can be carbon, both R"s can be nitrogen, or one R" can be carbon and the other nitrogen.

When any variable occurs more than one time in any moiety or formula depicting and describing compounds employed or claimed in the present invention, its definition on each occurrence is independent of its definition at every other occurrence. Also, combinations of substituents and/or variables are permissible only if such compounds result in stable compounds.

The symbols "*" at the end of a bond or "------" drawn through a bond each refer to the point of attachment of a functional group or other chemical moiety to the rest of the molecule of which it is a part. Thus, for example:

MeC(=O)OR⁴

wherein R⁴ = 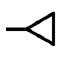 or 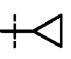 ⇒ MeC(=O)O

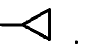

A bond drawn into ring system (as opposed to connected at a distinct vertex) indicates that the bond may be attached to any of the suitable ring atoms.

The term "optional" or "optionally" as used herein means that a subsequently described event or circumstance may, but need not, occur, and that the description includes instances where the event or circumstance occurs and instances in which it does not. For example, "optionally substituted" means that the optionally substituted moiety may incorporate a hydrogen atom or a substituent.

If a substituent is designated to be "absent", the substituent is not present.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

Certain compounds may exhibit tautomerism. Tautomeric compounds can exist as two or more interconvertable species. Prototropic tautomers result from the migration of a covalently bonded hydrogen atom between two atoms. Tautomers generally exist in equilibrium and attempts to isolate an individual tautomers usually produce a mixture whose chemical and physical properties are consistent with a mixture of compounds. The position of the equilibrium is dependent on chemical features within the molecule. For example, in many aliphatic aldehydes and ketones, such as acetaldehyde, the keto form predominates while; in phenols, the enol form predominates. Common prototropic tautomers include keto/enol (-C(=O)-CH-⇆-C(-OH)=CH-), amide/imidic acid (-C(=O)-NH-⇆-C(-OH)=N-) and amidine (-C(=NR)-NH-⇆-C(-NHR)=N-) tautomers. The latter two are particularly common in heteroaryl and heterocyclic rings and the present invention encompasses all tautomeric forms of the compounds.

The symbol 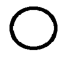 inside a ring system as used herein in Formula I: 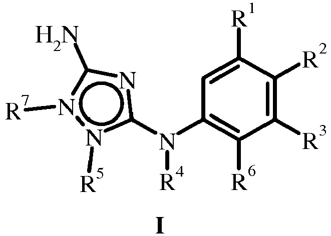 indicates that there may be double or single bonds between the various atoms of the ring system. For example, when R⁷ is defined as absent, R⁵ must not be absent and Formula I is Formula I':

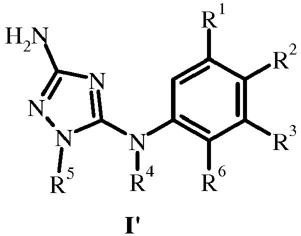

For example, when R⁷ is not defined as absent, R⁵ must be absent and Formula I is Formula I":

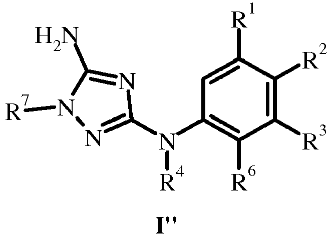

Technical and scientific terms used herein have the meaning commonly understood by one of skill in the art to which the present invention pertains, unless otherwise defined. Reference is made herein to various methodologies and materials known to those of skill in the art. Standard reference works setting forth the general principles of pharmacology include Goodman and Gilman's The Pharmacological Basis of Therapeutics, 10th Ed., McGraw Hill Companies Inc., New York (2001). Any suitable materials and/or methods known to those of skill can be utilized in carrying out the present invention. However, preferred materials and methods are described. Materials, reagents and the like to which reference are made in the following description and examples are obtainable from commercial sources, unless otherwise noted.

The definitions described herein may be appended to form chemically-relevant combinations, such as "heteroalkylaryl," "haloalkylheteroaryl," "arylalkylheterocyclyl," "alkylcarbonyl," "alkoxyalkyl," and the like. When the term "alkyl" is used as a suffix following another term, as in "phenylalkyl," or "hydroxyalkyl," this is intended to refer to an alkyl group, as defined above, being substituted with one to two substituents selected from the other specifically-named group. Thus, for example, "phenylalkyl" refers to an alkyl group having one to two phenyl substituents, and thus includes benzyl, phenylethyl, and biphenyl. An "alkylaminoalkyl" is an alkyl group having one to two alkylamino substituents. "Hydroxyalkyl" includes 2-hydroxyethyl, 2-hydroxypropyl, 1-(hydroxymethyl)-2-methylpropyl, 2-hydroxybutyl, 2,3-dihydroxybutyl, 2-(hydroxymethyl), 3-hydroxypropyl, and so forth. Accordingly, as used herein, the term "hydroxyalkyl" is used to define a subset of heteroalkyl groups defined below. The term -(ar)alkyl refers to either an unsubstituted alkyl or an aralkyl group. The term (hetero)aryl or (het)aryl refers to either an aryl or a heteroaryl group.

The term "carbonyl" or "acyl" as used herein denotes a group of formula -C(=O)R wherein R is hydrogen or lower alkyl as defined herein.

The term "ester" as used herein denotes a group of formula -C(=O)OR wherein R is lower alkyl as defined herein.

The term "alkyl" as used herein denotes an unbranched or branched chain, saturated, monovalent hydrocarbon residue containing 1 to 10 carbon atoms. The term "lower alkyl" denotes a straight or branched chain hydrocarbon residue containing 1 to 6 carbon atoms. "C₁₋₁₀ alkyl" as used herein refers to an alkyl composed of 1 to 10 carbons. Examples of alkyl groups include, but are not limited to, lower alkyl groups include methyl, ethyl, propyl, *i*-propyl, *n-*butyl, *i*-butyl, *t*-butyl or pentyl, isopentyl, neopentyl, hexyl, heptyl, and octyl.

When the term "alkyl" is used as a suffix following another term, as in "phenylalkyl," or "hydroxyalkyl," this is intended to refer to an alkyl group, as defined above, being substituted with one to two substituents selected from the other specifically-named group. Thus, for example, "phenylalkyl" denotes the radical R'R"-, wherein R' is a phenyl radical, and R" is an alkylene radical as defined herein with the understanding that the attachment point of the phenylalkyl moiety will be on the alkylene radical. Examples of arylalkyl radicals include, but are not limited to, benzyl, phenylethyl, 3-phenylpropyl. The terms "arylalkyl" or "aralkyl" are interpreted similarly except R' is an aryl radical. The terms "(het)arylalkyl" or "(het)aralkyl" are interpreted similarly except R' is optionally an aryl or a heteroaryl radical.

The terms "haloalkyl" or "halo lower alkyl" or "lower haloalkyl" refers to a straight or branched chain hydrocarbon residue containing 1 to 6 carbon atoms wherein one or more carbon atoms are substituted with one or more halogen atoms.

The term "alkylene" or "alkylenyl" as used herein denotes a divalent saturated linear hydrocarbon radical of 1 to 10 carbon atoms (*e*.*g*., (CH₂)ₙ)or a branched saturated divalent hydrocarbon radical of 2 to 10 carbon atoms (*e*.*g*., -CHMe- or -CH₂CH(*i*-Pr)CH₂-), unless otherwise indicated. Except in the case of methylene, the open valences of an alkylene group are not attached to the same atom. Examples of alkylene radicals include, but are not limited to, methylene, ethylene, propylene, 2-methyl-propylene, 1,1-dimethyl-ethylene, butylene, 2-ethylbutylene.

The term "alkoxy" as used herein means an -O-alkyl group, wherein alkyl is as defined above such as methoxy, ethoxy, *n*-propyloxy, *i*-propyloxy, *n*-butyloxy, *i*-butyloxy, *t*-butyloxy, pentyloxy, hexyloxy, including their isomers. "Lower alkoxy" as used herein denotes an alkoxy group with a "lower alkyl" group as previously defined. "C₁₋₁₀ alkoxy" as used herein refers to an-O-alkyl wherein alkyl is C₁₋₁₀.

The terms "haloalkoxy" or "halo lower alkoxy" or "lower haloalkoxy" refers to a lower alkoxy group, wherein one or more carbon atoms are substituted with one or more halogen atoms.

The term "hydroxyalkyl" as used herein denotes an alkyl radical as herein defined wherein one to three hydrogen atoms on different carbon atoms is/are replaced by hydroxyl groups.

The term "sulfinyl" as used herein denotes a -SO- group.

The term "sulfonyl" as used herein denotes a -SO₂- group.

The terms "alkylsulfonyl" and "arylsulfonyl" as used herein refers to a group of formula -S(=O)₂R wherein R is alkyl or aryl respectively and alkyl and aryl are as defined herein. The term "heteroalkylsulfonyl" as used herein refers herein denotes a group of formula -S(=O)₂R wherein R is "heteroalkyl" as defined herein.

The term "lower alkyl sulfonylamido" as used herein refers to a group of formula - S(=O)₂NR₂ wherein each R is independently hydrogen or C₁₋₃ alkyl, and lower alkyl is as defined herein.

The term "trifluoromethyl sulfonyl" as used herein refers to a group of formula - S(=O)₂CF₃.

The term "trifluoromethyl sulfinyl" as used herein refers to a group of formula -S(=O)CF₃.

The term "trifluoromethyl sulfanyl" as used herein refers to a group of formula -SCF₃.

The term "nitro" as used herein refers to a group of formula -N⁺(=O)O⁻.

The term "carboxyl" as used herein refers to a group of formula -C(=O)R₂ wherein each R is independently hydrogen or C₁₋₃ alkyl, and lower alkyl is as defined herein.

The term "cycloalkyl" denotes a monovalent saturated monocyclic or bicyclic hydrocarbon group of 3 to 10 ring carbon atoms. In particular embodiments cycloalkyl denotes a monovalent saturated monocyclic hydrocarbon group of 3 to 8 ring carbon atoms. Bicyclic means consisting of two saturated carbocycles having one or more carbon atoms in common. Particular cycloalkyl groups are monocyclic. Examples for monocyclic cycloalkyl are cyclopropyl, cyclobutanyl, cyclopentyl, cyclohexyl or cycloheptyl. Examples for bicyclic cycloalkyl are bicyclo[2.2.1]heptanyl, or bicyclo[2.2.2]octanyl.

The term "amino" as used herein denotes a group of the formula -NR'R" wherein R' and R" are independently hydrogen, alkyl, alkoxy, cycloalkyl, heterocycloalkyl, aryl or heteroaryl. Alternatively, R' and R", together with the nitrogen to which they are attached, can form a heterocycloalkyl. The term "primary amino" denotes a group wherein both R' and R" are hydrogen. The term "secondary amino" denotes a group wherein R' is hydrogen and R" is not. The term "tertiary amino" denotes a group wherein both R' and R" are not hydrogen. Particular secondary and tertiary amines are methylamine, ethylamine, propylamine, isopropylamine, phenylamine, benzylamine dimethylamine, diethylamine, dipropylamine and diisopropylamine.

The term "amido" as used herein denotes a group of the formula -C(=O)NR'R" or - NR'C(=O)R" wherein R' and R" are independently hydrogen, alkyl, alkoxy, cycloalkyl, heterocycloalkyl, aryl or heteroaryl.

The term "heteroaryl" denotes a monovalent aromatic heterocyclic mono- or bicyclic ring system of 5 to 12 ring atoms, comprising 1, 2, 3 or 4 heteroatoms selected from N, O and S, the remaining ring atoms being carbon. Examples of heteroaryl moieties include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, azepinyl, diazepinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl, or quinoxalinyl.

The term "heterocycloalkyl" denotes a monovalent saturated or partly unsaturated mono- or bicyclic ring system of 3 to 9 ring atoms, comprising 1, 2, or 3 ring heteroatoms selected from N, O and S, the remaining ring atoms being carbon. In particular embodiments, heterocycloalkyl is a monovalent saturated monocyclic ring system of 4 to 7 ring atoms, comprising 1, 2, or 3 ring heteroatoms selected from N, O and S, the remaining ring atoms being carbon. Examples for monocyclic saturated heterocycloalkyl are aziridinyl, oxiranyl, azetidinyl, oxetanyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocycloalkyl are dihydrofuryl, imidazolinyl, dihydro-oxazolyl, tetrahydro-pyridinyl, or dihydropyranyl.

### Inhibitors of HCV Entry

The application provides a compound of formula I 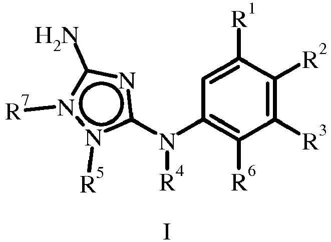 for use in the treatment of a HCV infection, wherein:
R¹ is H, halo, lower haloalkyl, lower haloalkyl lower alkyl, trifluoromethyl sulfonyl, lower alkyl, alkoxy, nitro, carboxyl, lower alkyl sulfonamido, trifluoromethyl sulfinyl, cycloalkyl, SF₅, or - NHNH₂;
R² is H, halo, cyano, nitro, trifluoromethyl sulfonyl, lower halo alkyl, phenyl ethynyl, lower alkyl sulfonyl, lower alkyl amino sulfonyl, lower alkyl amido, amino sulfonyl, sulfonamido, benzyl oxy, cycloalkyl, heterocycloalkyl sulfonyl, aryl carbonyl, lower alkyl urea, lower alkyl ester, phenyl amido, heterocycloalkyl carbonyl, lower alkyl sulfonyl phenyl amido, lower alkyl cyano, cyano lower alkyl, or tetrazolyl lower alkyl;
R³ is H, halo, lower alkyl, lower alkenyl, lower haloalkyl, cyano, lower haloalkyl oxy, cyano cycloalkyl, lower haloalkyl lower alkyl, SF₅, or trifluoromethyl sulfanyl;
   or R² and R³ together form an aryl ring system;
R⁴ is H, lower alkyl, cyano lower alkyl, or phenyl lower alkyl;
R⁵ is absent, H, lower alkyl, or cycloalkyl;
R⁶ is H or halo;
R⁷ is absent, H, loweralkyl carbonyl, or lower haloalkyl phenyl sulfonyl;
with the proviso that when R⁵ is absent, R⁷ is not absent; and
with the proviso that when R⁷ is absent, R⁵ is not absent;
or a pharmaceutically acceptable salt thereof.

The application provides a compound of Formula I, wherein R⁵ is H.

The application provides a compound of Formula I, wherein R⁴ is H.

The application provides a compound of Formula I, wherein R⁴ is H, and R⁵ is H.

The application provides a compound of Formula I, wherein R⁶ is H.

The application provides a compound of Formula I, wherein R⁶ is H, R⁴ is H, and R⁵ is H.

The application provides any of the above compounds of Formula I, wherein R¹ is halo.

The application provides any of the above compounds of Formula I, wherein R² is halo.

The application provides any of the above compounds of Formula I, wherein R³ is halo.

The application provides any of the above compounds of Formula I, wherein R³ is lower haloalkyl.

The application provides a compound of Formula I, wherein R¹ is H.

The application provides a compound of Formula I, wherein R¹ is H, R⁶ is H, R⁴ is H, and R⁵ is H.

The application provides a compound of Formula I, wherein R² is H.

The application provides a compound of Formula I, wherein R² is H, R⁶ is H, R⁴ is H, and R⁵ is H.

The application provides a compound of Formula I, wherein R³ is H.

The application provides a compound of Formula I, wherein R³ is H, R⁶ is H, R⁴ is H, and R⁵ is H.

The application provides a compound of Formula I, wherein R⁷ is absent and R⁵ is H.

The application provides a compound of Formula I, wherein R⁷ is absent and R⁵ is lower alkyl.

The application provides a compound of Formula I, wherein R⁷ is absent and R⁵ is cycloalkyl.

The application provides a compound of Formula I, wherein R⁵ is absent and R⁷ is H.

The application provides a compound of Formula I, wherein R⁵ is absent and R⁷ is lower alkyl.

The application provides a compound of Formula I, wherein R⁵ is absent and R⁷ is carbonyl.

The application provides a compound of Formula I, wherein R⁵ is absent and R⁷ is lower haloalkyl phenyl sulfonyl.

The application provides a compound of Formula I, wherein

The application provides a compound for use in the treatment of HCV infection, selected from the group consisting of:
*N*³-Naphthalen-2-yl-1*H*-[1,2,4]triazole-3,5-diamine;
4-(5-Amino-1*H*-[1,2,4]triazol-3-ylamino)-*N*-methyl-benzenesulfonamide;
4-(5-Amino-1*H*-[1,2,4]triazol-3-ylamino)-benzenesulfonamide;
3-(5-Amino-1*H*-[1,2,4]triazol-3-ylamino)-benzoic acid;
*N*³-(3-Chloro-phenyl)-1*H*-[1,2,4]triazole-3,5-diamine;

The application provides a compound selected from the group consisting of:
*N*³-(4-Bromo-3,5-dichloro-phenyl)-1*H*-[1,2,4]triazole-3,5-diamine;
4-(5-Amino-1*H*-[1,2,4]triazol-3-ylamino)-2,6-dichloro-benzonitrile;
*N*³-(3,5-Dichloro-4-iodo-phenyl)-1*H*-[1,2,4]triazole-3,5-diamine;
*N*³-(3-Trifluoromethanesulfonyl-phenyl)-1*H*-[1,2,4]triazole-3,5-diamine;
*N*³ -(4-Bromo-3-chloro-5-fluoro-phenyl)-1*H*-[1,2,4]triazole-3,5-diamine;
*N*³-(3-*tert*-Butyl-phenyl)-1*H*-[1,2,4]triazole-3,5-diamine;
*N*³-(3-Chloro-4-trifluoromethanesulfonyl-phenyl)-1*H*-[1,2,4]triazole-3,5-diamine;
*N*³-(3-Chloro-4-nitro-phenyl)-1*H*-[1,2,4]triazole-3,5-diamine;
*N*³-(3-Chloro-4-ethynyl-phenyl)-1*H*-[1,2,4]triazole-3,5-diamine;
*N*³-(3-Chloro-4-trifluoromethyl-phenyl)-1*H*-[1,2,4]triazole-3,5-diamine;
*N*³-(3,4,5-Trichloro-phenyl)-1*H*-[1,2,4]triazole-3,5-diamine;
4-(5-Amino-1*H*-[1,2,4]triazol-3-ylamino)-2-chloro-benzonitrile;
*N*³-(4-Chloro-3-nitro-phenyl)-1*H*-[1,2,4]triazole-3,5-diamine;
*N*³-(3-Chloro-4-phenylethynyl-phenyl)-1*H*-[1,2,4]triazole-3,5-diamine;
*N*³-(3-Bromo-phenyl)-1*H*-[1,2,4]triazole-3,5-diamine;
4-(5-Amino-1*H*-[1,2,4]triazol-3-ylamino)-2-*tert*-buty*l*-benzonitrile;
*N*³*-*(4-Trifluoromethanesulfonyl-phenyl)-1*H*-[1,2,4]triazole-3,5-diamine;
*N*³-(3,5-Di-*tert*-butyl-phenyl)-1*H*-[1,2,4]triazole-3,5-diamine;
*N*³-(4-Methanesulfonyl-phenyl)-1*H*-[1,2,4]triazole-3,5-diamine;
*N*³*-*(3-Methanesulfonyl-phenyl)-1*H*-[1,2,4]triazole-3,5-diamine;
[4-(5-Amino-1*H*-[1,2,4] triazol-3-ylamino)-phenyl]-morpholin-4-yl-methanone;
4-(5-Amino-1*H*-[1,2,4]triazol-3-ylamino)-*N*,*N*-dimethyl-benzamide;
4-(5-Amino-1*H*-[1,2,4]triazol-3-ylamino)-*N,N*-dimethyl-benzenesulfonamide;
*N*³-Methyl-*N*³-(3,4,5-trichloro-phenyl)-1*H-*[1,2,4]triazole-3,5-diamine;
*N*³-(3,5-Dichloro-phenyl)-*N*³-methyl-1*H*-[1,2,4]triazole-3,5-diamine;
*N*³-Benzyl-*N*³-(3,5-dichloro-phenyl)-1*H*-[1,2,4]triazole-3,5-diamine;
[(5-Amino-1*H*-[1,2,4]triazol-3-yl)-(3,5-dichloro-phenyl)-amino]-acetonitrile;
*N³-*(3-Trifluoromethanesulfinyl-5-trifluoromethylsulfanyl-phenyl)-1*H*-[1,2,4]triazole-3,5-diamine;
*N*-[3-(5-Amino-1*H*-[1,2,4]triazol-3-ylamino)-phenyl]-methanesulfonamide;
4-(5-Amino-1*H*-[1,2,4]triazol-3-ylamino)-2-trifluoromethanesulfonyl-benzonitrile;
4-(5-Amino-1*H*-[1,2,4]triazol-3-ylamino)-2-cyclopropyl-benzonitrile;
*N³-*(4-Benzyloxy-3-chloro-phenyl)-1*H*-[1,2,4]triazole-3,5-diamine;
*N*³-(3,5-Dichloro-4-methyl-phenyl)-1*H*-[1,2,4]triazole-3,5-diamine
*N³*-(3,5-Dichloro-4-cyclopropyl-phenyl)-1H-[1,2,4]triazole-3,5-diamine
*N*³-(4-Bromo-3-chloro-phenyl)-1*H*-[1,2,4]triazole-3,5-diamine;
*N*³-(3,5-Dichloro-phenyl)-1*H*-[1,2,4]triazole-3,5-diamine;
*N*³-(4-Bromo-3-chloro-5-trifluoromethyl-phenyl)-1*H*-[1,2,4]triazole-3,5-diamine;
4-(5-Amino-1*H*-[1,2,4]triazol-3-ylamino)-2-chloro-6-trifluoromethyl-benzonitrile;
*N*³-[3,5-Dichloro-4-(morpholine-4-sulfonyl)-phenyl]-1*H*-[1,2,4]triazole-3,5-diamine;
*N*³-(4-Bromo-3-fluoro-5-trifluoromethyl-phenyl)-1*H*-[1,2,4]triazole-3,5-diamine;
*N*³-(4-Bromo-3,5-difluoro-phenyl)-1*H*-[1,2,4]triazole-3,5-diamine;
4-(5-Amino-1*H*-[1,2,4]triazol-3-ylamino)-benzonitrile;
3-(5-Amino-1*H*-[1,2,4]triazol-3-ylamino)-5-chloro-benzonitrile;
*N*³-(2,3-Dichloro-phenyl)-1*H*-[1,2,4]triazole-3,5-diamine;
*N*³-(3-Chloro-5-trifluoromethyl-phenyl)-1*H*-[1,2,4]triazole-3,5-diamine;
*N*³-(4-Chloro-3-trifluoromethyl-phenyl)-1*H-*[1,2,4]triazole-3,5-diamine;
*N*³-(3-Trifluoromethoxy-phenyl)-1*H*-[1,2,4]triazole-3,5-diamine;
*N*³-(2-Fluoro-3-trifluoromethyl-phenyl)-1*H*-[1,2,4]triazole-3,5-diamine;
*N*³-(2-Fluoro-5-trifluoromethyl-phenyl)-1*H*-[1,2,4]triazole-3,5-diamine;
*N*³-[3-(2,2,2-Trifluoro-ethyl)-phenyl]-1*H*-[1,2,4]triazole-3,5-diamine;
*N*³-(3-Isopropyl-phenyl)-1*H-*[1,2,4]triazole-3,5-diamine;
[3-(5-Amino-1*H*-[1,2,4]triazol-3-ylamino)-phenyl]-phenyl-methanone;
1-[3-(5-Amino-1*H*-[1,2,4]triazol-3-ylamino)-phenyl]-cyclopropanecarbonitrile;
*N*³-(3-Chloro-4-fluoro-5-trifluoromethyl-phenyl)-1*H*-[1,2,4]triazole-3,5-diamine;
*N*³-(3-Chloro-5-fluoro-phenyl)-1*H*-[1,2,4]triazole-3,5-diamine;
1-[4-(5-Amino-1*H-*[1,2,4]triazol-3-ylamino)-2-chloro-phenyl]-3-methyl-urea;
4-(5-Amino-1*H*-[1,2,4]triazol-3-ylamino)-2,6-dichloro-benzoic acid methyl ester;
4-(5-Amino-1*H*-[1,2,4]triazol-3-ylamino)-2,6-dichloro-*N*-phenyl-benzamide;
[4-(5-Amino-1*H*-[1,2,4]triazol-3-ylamino)-2,6-dichloro-phenyl]-pyrrolidin-1-yl-methanone;
4-(5-Amino-1*H*-[1,2,4]triazol-3-ylamino)-2,6-dichloro-*N*-(3-methanesulfonyl-phenyl)-benzamide;
*N*³-(3,5-Dichloro-4-vinyl-phenyl)-1*H*-[1,2,4]triazole-3,5-diamine;
4-(5-Amino-2*H*-[1,2,4]triazol-3-ylamino)-2-fluoro-6-trifluoromethyl-benzonitrile;
*N*³-(3-Fluoro-5-trifluoromethyl-phenyl)-1*H*-[1,2,4]triazole-3,5-diamine;
4-(5-Amino-2*H*-[1,2,4]triazol-3-ylamino)-2,6-difluoro-benzonitrile;
N*3*-(3-pentafluorosulfur-phenyl)-1*H*-[1,2,4]triazole-3,5-diamine;
1-Methyl-N*5*-(3-pentafluorosulfur-phenyl)-1H-[1,2,4]triazole-3,5-diamine;
Cyclohexyl-N*5*-(3-pentafluorosulfur-phenyl)-1H-[1,2,4]triazole-3,5-diamine;
N*3*-(3-Fluoro-5-pentafluorosulfur-phenyl)-1H-[1,2,4]triazole-3,5-diamine;
N*5*-(3-Fluoro-5-pentafluorosulfur-phenyl)-1-methyl-1H-[1,2,4]triazole-3,5-diamine;
N*3*-(4-pentafluorosulfur-phenyl)-1H-[1,2,4]triazole-3,5-diamine;
*N*³-(3-Chloro-4-methyl-phenyl)-1*H*-[1,2,4]triazole-3,5-diamine;
*N*³-(3-Chloro-4-methanesulfonyl-phenyl)-1*H*-[1,2,4]triazole-3,5-diamine;
4-(5-Amino-1*H*-[1,2,4]triazol-3-ylamino)-2-chloro-*N,N*-dimethyl-benzamide;
*N*³-[3,5-Dichloro-4-(3,3-dimethyl-but-1-ynyl)-phenyl]-1*H*-[1,2,4]triazole-3,5-diamine;
*N*³-[3-Chloro-4-(3,3-dimethyl-but-1-ynyl)-phenyl]-1*H*-[1,2,4]triazole-3,5-diamine;
N*3*-(4-Bromo-3-pentafluorosulfur-phenyl)-1H-[1,2,4]triazole-3,5-diamine;
N*3*-(3-Bromo-5-pentafluorosulfur-phenyl)-1H-[1,2,4]triazole-3,5-diamine;
N*3*-(4-Bromo-3-fluoro-5-pentafluorosulfur-phenyl)-1H-[1,2,4]triazole-3,5-diamine;
[4-(5-Amino-1*H*-[1,2,4]triazol-3-ylamino)-2-chloro-6-trifluoromethyl-phenyl]-acetonitrile;
4-(5-Amino-1H-[1,2,4]triazol-3-ylamino)-2-pentafluorosulfur-benzonitrile;
4-(5-Amino-1H-[1,2,4]triazol-3-ylamino)-2-fluoro-6-pentafluorosulfur-benzonitrile;
4-(5-Amino-1H-[1,2,4]triazol-3-ylamino)-2-hydrazino-6-pentafluorosulfur-benzonitrile;
4-(1-Acetyl-5-amino-1H-[1,2,4]triazol-3-ylamino)-2,6-dichloro-benzonitrile;
4-[5-Amino-1-(4-trifluoromethyl-benzenesulfonyl)-1H-[1,2,4]triazol-3-ylamino]-2,6-dichloro-benzonitrile; and
*N*³-[3-Chloro-4-(1*H*-tetrazol-5-ylmethyl)-5-trifluoromethyl-phenyl]-1*H*-[1,2,4]triazole-3,5-diamine.

The application describes a method for preventing a Hepatitis C Virus (HCV) infection comprising administering to a patient in need thereof a therapeutically effective amount of a compound of Formula I.

The application describes the above method, further comprising administering to a patient in need thereof a therapeutically effective amount of an immune system suppressant.

The application describes a method for treating a Hepatitis C Virus (HCV) infection comprising administering to a patient in need thereof a therapeutically effective amount of a compound of Formula I.

The application describes any of the above methods, further comprising administering a combination of antiviral agents that inhibits replication of HCV.

The application describes any of the above methods, further comprising administering an immune system modulator or an antiviral agent that inhibits replication of HCV, or a combination thereof.

The application describes the above method, wherein the immune system modulator is an interferon or a chemically derivatized interferon.

The application describes any of the above methods, further comprising administering an immune system modulator or an antiviral agent that inhibits replication of HCV, or a combination thereof, wherein the antiviral agent is selected from the group consisting of a HCV protease inhibitor, a HCV polymerase inhibitor, a HCV helicase inhibitor, a HCV NS5A inhibitor, or any combination thereof.

The application provides a composition comprising a compound of Formula I and a pharmaceutically acceptable excipient.

The application provides the use of the compound of Formula I in the preparation of a medicament for the prevention of HCV.

The application provides the use of the compound of Formula I in the preparation of a medicament for the treatment of HCV.

The application provides any compound, composition, method or use as described herein.

### Compounds

Examples of representative compounds encompassed by the present invention and within the scope of the invention are provided in the following Table. These examples and preparations which follow are provided to enable those skilled in the art to more clearly understand and to practice the present invention. They should not be considered as limiting the scope of the invention, but merely as being illustrative and representative thereof.

In general, the nomenclature used in this Application is based on AUTONOMTM v.4.0, a Beilstein Institute computerized system for the generation of IUPAC systematic nomenclature. If there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

TABLE I depicts examples of compounds according to generic Formula I:

**TABLE I.**

| # | Nomenclature | Structure |
|---|---|---|
| I-1 | 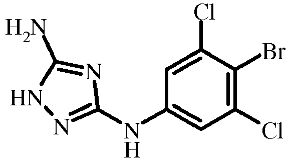 | *N*³-(4-Bromo-3,5-dichloro-phenyl)-1*H-*[1,2,4]triazole-3,5-diamine |
| I-2 | 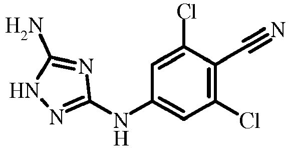 | 4-(5-Amino-1*H*-[1,2,4]triazol-3-ylamino)-2,6-dichloro-benzonitrile |
| I-3 | 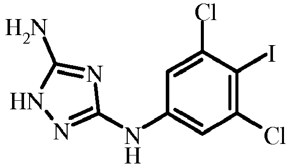 | *N*³-(3,5-Dichloro-4-iodo-phenyl)-1*H-*[1,2,4]triazole-3,5-diamine |
| I-4 | 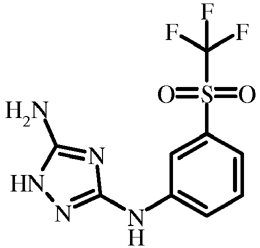 | *N*³-(3-Trifluoromethanesulfonyl-phenyl)-1*H-*[1,2,4]triazole-3,5-diamine |
| I-5 | 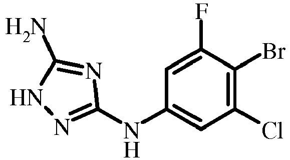 | *N*³-(4-Bromo-3-chloro-5-fluoro-phenyl)-1*H-*[1,2,4]triazole-3,5-diamine |
| I-6 | 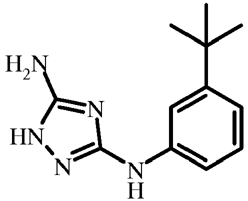 | *N*³-(3-*tert*-Butyl-phenyl)-1*H*-[1,2,4]triazole-3,5-diamine |
| I-7 | 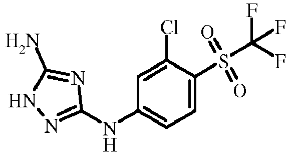 | *N*³-(3-Chloro-4-trifluoromethanesulfonyl-phenyl)-1*H*-[1,2,4]triazole-3,5-diamine |
| I-8 | 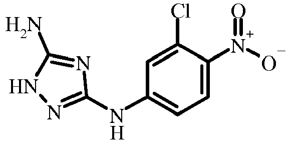 | *N*³-(3-Chloro-4-nitro-phenyl)-1*H-*[1,2,4]triazole-3,5-diamine |
| I-9 | 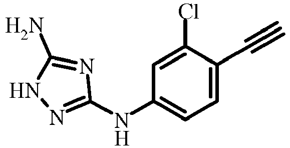 | *N*³-(3-Chloro-4-ethynyl-phenyl)-1*H-*[1,2,4]triazole-3,5-diamine |
| I-10 | 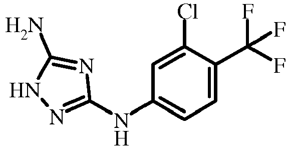 | *N*³-(3-Chloro-4-trifluoromethyl-phenyl)-1*H-*[1,2,4]triazole-3,5-diamine |
| I-11 | 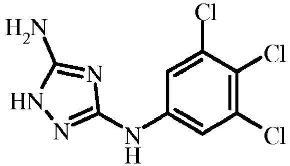 | *N*³-(3,4,5-Trichloro-phenyl)-1*H-*[1,2,4]triazole-3,5-diamine |
| I-12 | 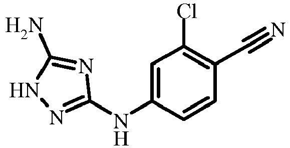 | 4-(5-Amino-1*H*-[1,2,4]triazol-3-ylamino)-2-chloro-benzonitrile |
| I-13 | 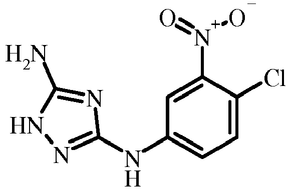 | *N*³-(4-Chloro-3-nitro-phenyl)-1*H-*[1,2,4]triazole-3,5-diamine |
| I-14 | 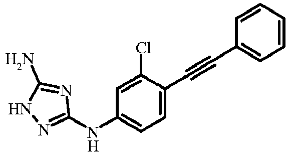 | *N*³-(3-Chloro-4-phenylethynyl-phenyl)-1*H-*[1,2,4]triazole-3,5-diamine |
| I-15 | 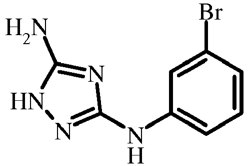 | *N*³-(3-Bromo-phenyl)-1*H*-[1,2,4]triazole-3,5-diamine |
| I-16 | 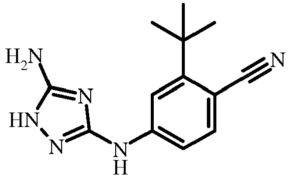 | 4-(5-Amino-1*H*-[1,2,4]triazol-3-ylamino)-2-*tert*-butyl-benzonitrile |
| I-17 | 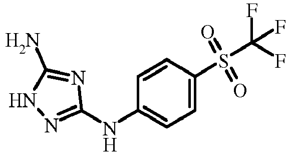 | *N*³*-*(4-Trifluoromethanesulfonyl-phenyl)-1*H-*[1,2,4]triazole-3,5-diamine |
| I-18 | 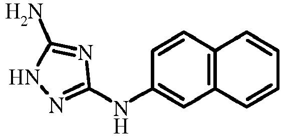 | *N*³-Naphthalen-2-yl-1*H*-[1,2,4]triazole-3,5-diamine |
| I-19 | 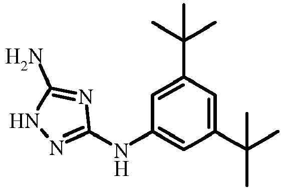 | *N*³-(3,5-Di-*tert*-butyl-phenyl)-1*H-*[1,2,4]triazole-3,5-diamine |
| I-20 | 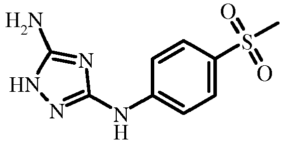 | *N*³-(4-Methanesulfonyl-phenyl)-1*H-*[1,2,4]triazole-3,5-diamine |
| I-21 | 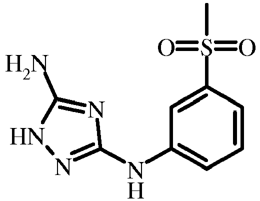 | *N*³-(3-Methanesulfonyl-phenyl)-1*H-*[1,2,4]triazole-3,5-diamine |
| I-22 | 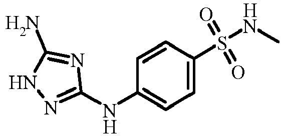 | 4-(5-Amino-1*H*-[1,2,4]triazol-3-ylamino)-*N-*methyl-benzenesulfonamide |
| I-23 | 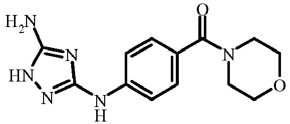 | [4-(5-Amino-1*H*-[1,2,4]triazol-3-ylamino)-phenyl]-morpholin-4-yl-methanone |
| I-24 | 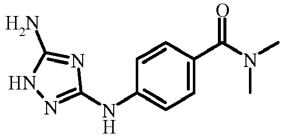 | 4-(5-Amino-1*H*-[1,2,4]triazol-3-ylamino)-*N,N*-dimethyl-benzamide |
| I-25 | 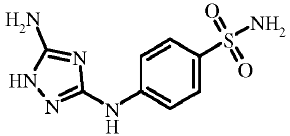 | 4-(5-Amino-1*H*-[1,2,4]triazol-3-ylamino)-benzenesulfonamide |
| I-26 | 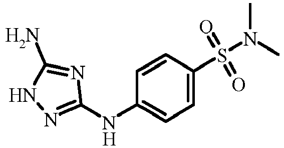 | 4-(5-Amino-1*H*-[1,2,4]triazol-3-ylamino)-*N,N*-dimethyl-benzenesulfonamide |
| I-27 | 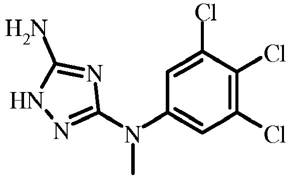 | *N*³-Methyl-*N*³-(3,4,5-trichloro-phenyl)-1*H-*[1,2,4]triazole-3,5-diamine |
| I-28 | 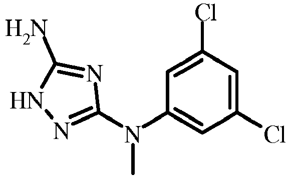 | *N*³-(3,5-Dichloro-phenyl)-*N*³-methyl-1*H-*[1,2,4]triazole-3,5-diamine |
| I-29 | 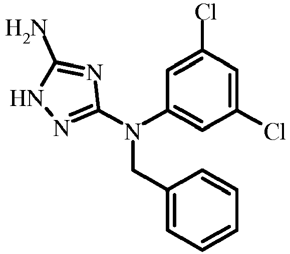 | *N*³-Benzyl-*N*³-(3,5-dichloro-phenyl)-1*H-*[1,2,4]triazole-3,5-diamine |
| I-30 | 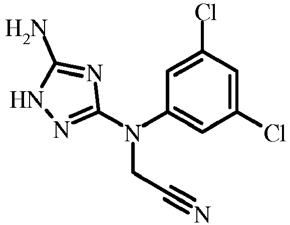 | [(5-Amino-1*H*-[1,2,4]triazol-3-yl)-(3,5-dichloro-phenyl)-amino]-acetonitrile |
| I-31 | 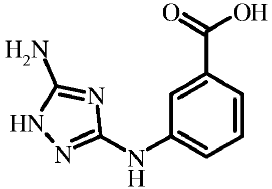 | 3-(5-Amino-1*H*-[1,2,4]triazol-3-ylamino)-benzoic acid |
| I-32 | 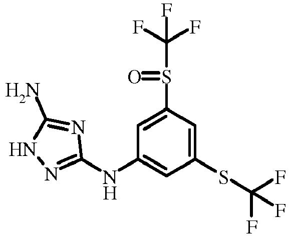 | *N*³*-*(3-Trifluoromethanesulfinyl-5-trifluoromethylsulfanyl-phenyl)-1*H-*[1,2,4]triazole-3,5-diamine |
| I-33 | 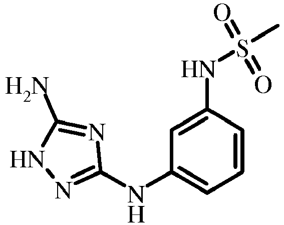 | *N*-[3-(5-Amino-1*H*-[1,2,4]triazol-3-ylamino)-phenyl]-methanesulfonamide |
| I-34 | 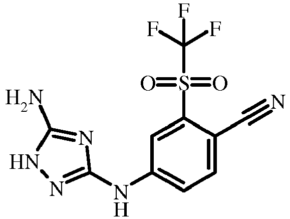 | 4-(5-Amino-1*H*-[1,2,4]triazol-3-ylamino)-2-trifluoromethanesulfonyl-benzonitrile |
| I-35 | 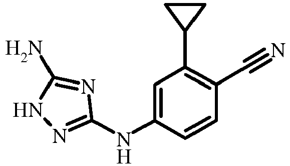 | 4-(5-Amino-1*H*-[1,2,4]triazol-3-ylamino)-2-cyclopropyl-benzonitrile |
| I-36 | 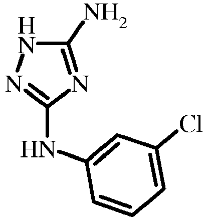 | *N*³-(3-Chloro-phenyl)-1*H*-[1,2,4]triazole-3,5-diamine |
| I-37 | 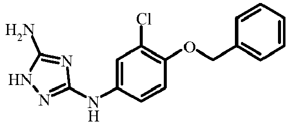 | *N*³*-*(4-Benzyloxy-3-chloro-phenyl)-1*H-*[1,2,4]triazole-3,5-diamine |
| I-38 | 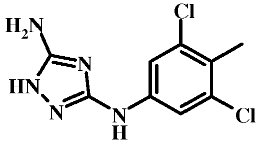 | *N*³-(3,5-Dichloro-4-methyl-phenyl)-1*H-*[1,2,4]triazole-3,5-diamine |
| I-39 | 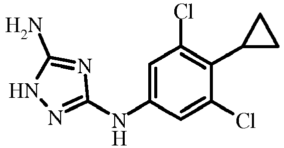 | *N*³-(3,5-Dichloro-4-cyclopropyl-phenyl)-1*H-*[1,2,4]triazole-3,5-diamine |
| I-40 | 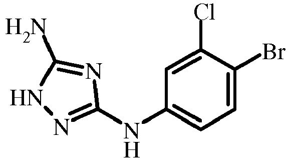 | *N*³-(4-Bromo-3-chloro-phenyl)-1*H-*[1,2,4]triazole-3,5-diamine |
| I-41 | 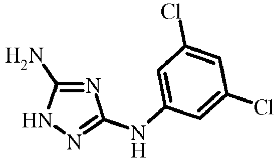 | *N*³-(3,5-Dichloro-phenyl)-1*H*-[1,2,4]triazole-3,5-diamine |
| I-42 | 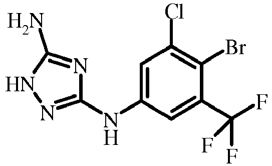 | *N*³*-*(4-Bromo-3-chloro-5-trifluoromethyl-phenyl)-1*H*-[1,2,4]triazole-3,5-diamine |
| I-43 | 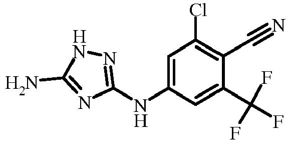 | 4-(5-Amino-1*H*-[1,2,4]triazol-3-ylamino)-2-chloro-6-trifluoromethyl-benzonitrile |
| I-44 | 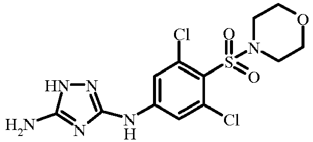 | *N*³*-*[3,5-Dichloro-4-(morpholine-4-sulfonyl)-phenyl]-1*H*-[1,2,4]triazole-3,5-diamine |
| I-45 | 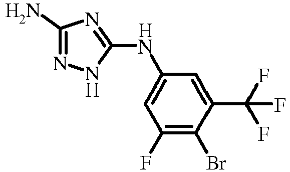 | *N*⁵-(4-Bromo-3-fluoro-5-trifluoromethylphenyl)-1*H*-[1,2,4]triazole-3,5-diamine |
| I-46 | 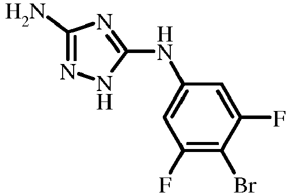 | *N*⁵-(4-Bromo-3,5-difluoro-phenyl)-1*H-*[1,2,4]triazole-3,5-diamine |
| I-47 | 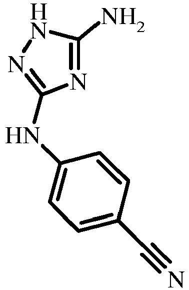 | 4-(5-Amino-1*H*-[1,2,4]triazol-3-ylamino)-benzonitrile |
| I-48 | 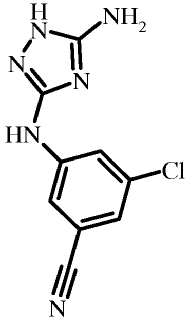 | 3-(5-Amino-1*H*-[1,2,4]triazol-3-ylamino)-5-chloro-benzonitrile |
| I-49 | 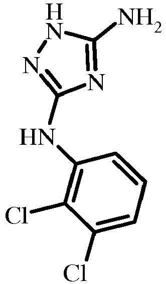 | *N*³-(2,3-Dichloro-phenyl)-1*H*-[1,2,4]triazole-3,5-diamine |
| I-50 | 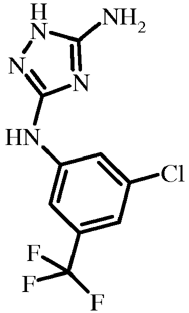 | *N*³-(3-Chloro-5-trifluoromethyl-phenyl)-1*H-*[1,2,4]triazole-3,5-diamine |
| I-51 | 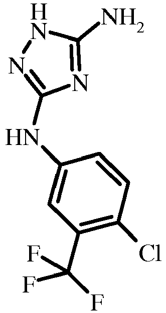 | *N*³-(4-Chloro-3-trifluoromethyl-phenyl)-1*H-*[1,2,4]triazole-3,5-diamine |
| I-52 | 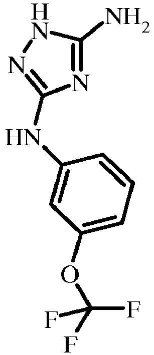 | *N*³-(3-Trifluoromethoxy-phenyl)-1*H-*[1,2,4]triazole-3,5-diamine |
| I-53 | 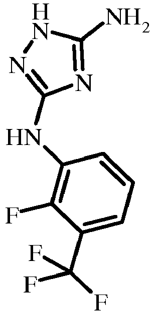 | *N*³-(2-Fluoro-3-trifluoromethyl-phenyl)-1*H-*[1,2,4]triazole-3,5-diamine |
| I-54 | 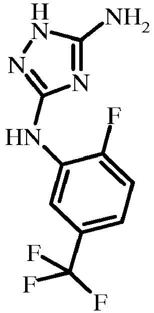 | *N*³-(2-Fluoro-5-trifluoromethyl-phenyl)-1*H-*[1,2,4]triazole-3,5-diamine |
| I-55 | 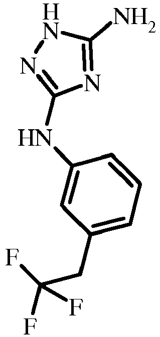 | *N*³-[3-(2,2,2-Trifluoro-ethyl)-phenyl]-1*H-*[1,2,4]triazole-3,5-diamine |
| I-56 | 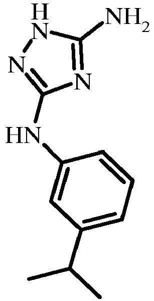 | *N*³*-*(3-Isopropyl-phenyl)-1*H*-[1,2,4]triazole-3,5-diamine |
| I-57 | 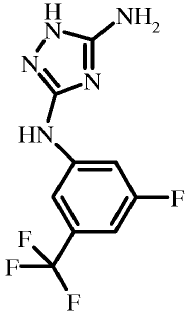 | *N*³-(3-Fluoro-5-trifluoromethyl-phenyl)-1*H-*[1,2,4]triazole-3,5-diamine |
| I-58 | 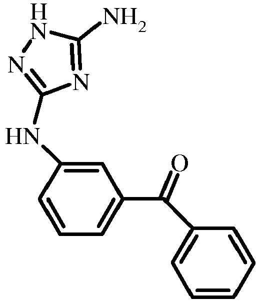 | [3-(5-Amino-1*H*-[1,2,4]triazol-3-ylamino)-phenyl]-phenyl-methanone |
| I-59 | 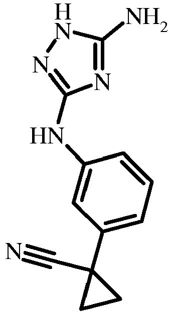 | 1-[3-(5-Amino-1*H*-[1,2,4]triazol-3-ylamino)-phenyl]-cyclopropanecarbonitrile |
| I-60 | 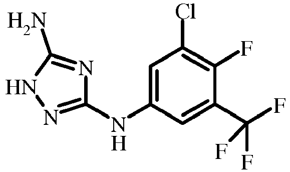 | *N*³-(3-Chloro-4-fluoro-5-trifluoromethylphenyl)-1*H*-[1,2,4]triazole-3,5-diamine |
| I-61 | 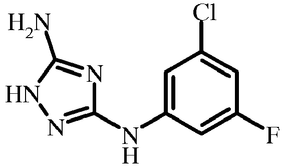 | *N*³-(3-Chloro-5-fluoro-phenyl)-1*H-*[1,2,4]triazole-3,5-diamine |
| I-62 | 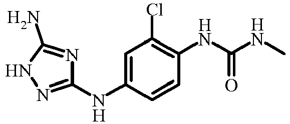 | 1-[4-(5-Amino-1*H*-[1,2,4]triazol-3-ylamino)-2-chloro-phenyl]-3-methyl-urea |
| I-63 | 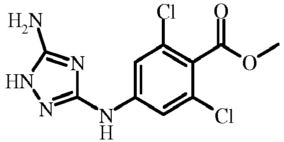 | 4-(5-Amino-1*H*-[1,2,4]triazol-3-ylamino)-2,6-dichloro-benzoic acid methyl ester |
| I-64 | 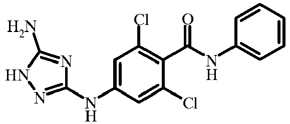 | 4-(5-Amino-1*H*-[1,2,4]triazol-3-ylamino)-2,6-dichloro-*N*-phenyl-benzamide |
| I-65 | 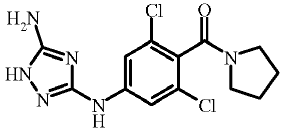 | [4-(5-Amino-1*H*-[1,2,4]triazol-3-ylamino)-2,6-dichloro-phenyl]-pyrrolidin-1-yl-methanone |
| I-66 | 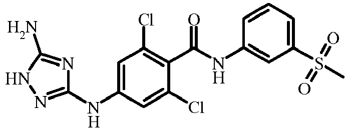 | 4-(5-Amino-1*H*-[1,2,4]triazol-3-ylamino)-2,6-dichloro-*N*-(3-methanesulfonyl-phenyl)-benzamide |
| I-67 | 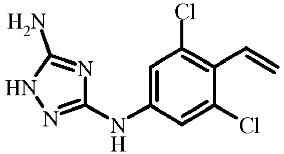 | *N*³-(3,5-Dichloro-4-vinyl-phenyl)-1*H-*[1,2,4]triazole-3,5-diamine |
| I-68 | 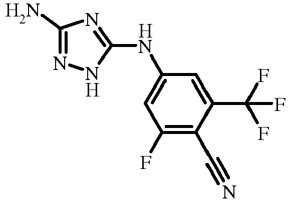 | 4-(5-Amino-2*H*-[1,2,4]triazol-3-ylamino)-2-fluoro-6-trifluoromethyl-benzonitrile |
| I-69 | 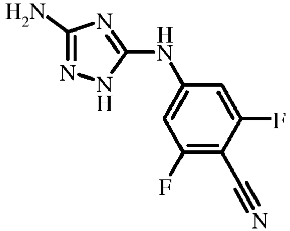 | 4-(5-Amino-2*H*-[1,2,4]triazol-3-ylamino)-2,6-difluoro-benzonitrile |
| I-70 | 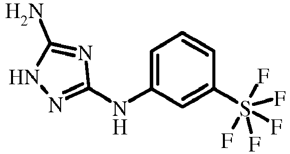 | N*3*-(3-pentafluorosulfur-phenyl)-1H-[1,2,4]triazole-3,5-diamine |
| I-71 | 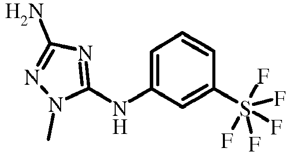 | 1-Methyl-N*5*-(3-pentafluorosulfur-phenyl)-1H-[1,2,4]triazole-3,5-diamine |
| I-72 | 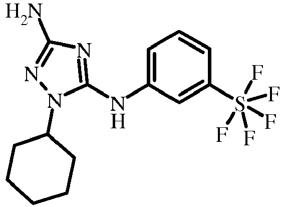 | Cyclohexyl-N*5 *-(3-pentafluorosulfur-phenyl)-1H-[1,2,4]triazole-3,5-diamine |
| I-73 | 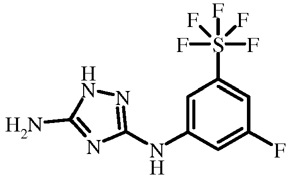 | N*3 *-(3-Fluoro-5-pentafluorosulfur-phenyl)-1H-[1,2,4]triazole-3,5-diamine |
| I-74 | 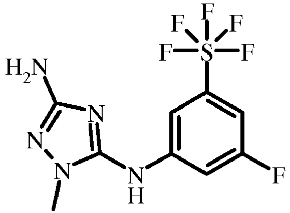 | N*5 *-(3-Fluoro-5-pentafluorosulfur-phenyl)-1-methyl-1H-[1,2,4]triazole-3,5-diamine |
| I-75 | 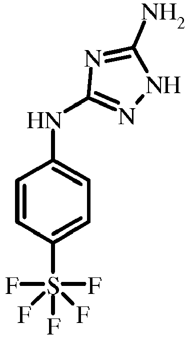 | N*3*-(4-pentafluorosulfur-phenyl)-1H-[1,2,4]triazole-3,5-diamine |
| I-76 | 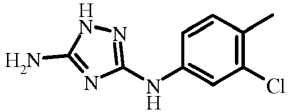 | *N*³*-*(3-Chloro-4-methyl-phenyl)-1*H-*[1,2,4]triazole-3,5-diamine |
| I-77 | 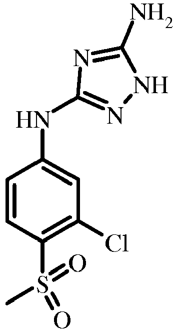 | *N*³*-*(3-Chloro-4-methanesulfonyl-phenyl)-1*H-*[1,2,4]triazole-3,5-diamine |
| I-78 | 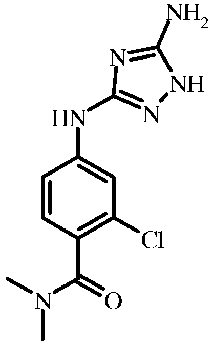 | 4-(5-Amino-1*H*-[1,2,4]triazol-3-ylamino)-2-chloro-*N,N*-dimethyl-benzamide |
| I-79 | 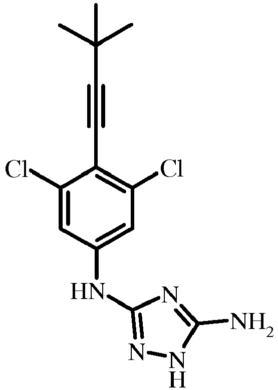 | *N*³-[3,5-Dichloro-4-(3,3-dimethyl-but-1-ynyl)-phenyl]-1*H*-[1,2,4]triazole-3,5-diamine |
| I-80 | 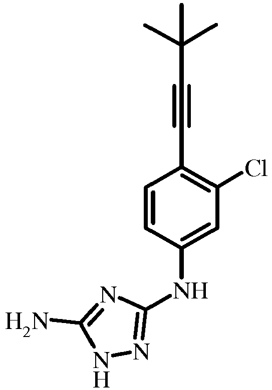 | *N*³-[3-Chloro-4-(3,3-dimethyl-but-1-ynyl)-phenyl]-1*H*-[1,2,4]triazole-3,5-diamine |
| I-81 | 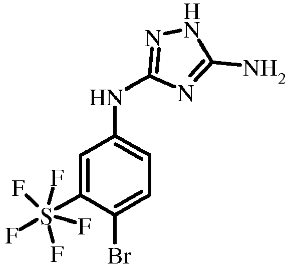 | N*3*-(4-Bromo-3-pentafluorosulfur-phenyl)-1H-[1,2,4]triazole-3,5-diamine |
| I-82 | 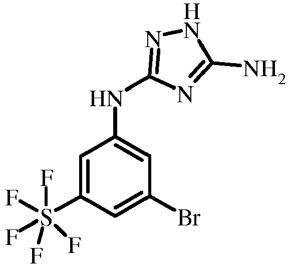 | N*3*-(3-Bromo-5-pentafluorosulfur-phenyl)-1H-[1,2,4]triazole-3,5-diamine |
| I-83 | 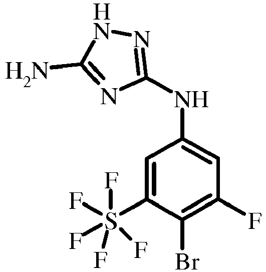 | N*3*-(4-Bromo-3-fluoro-5-pentafluorosulfur-phenyl)-1H-[1,2,4]triazole-3,5-diamine |
| I-84 | 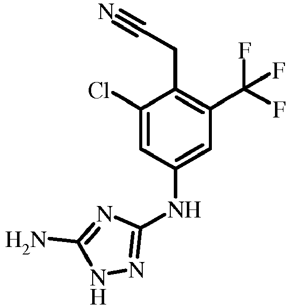 | 2-(4-(5-amino-1H-1,2,4-triazol-3-ylamino)-2-chloro-6-(trifluoromethyl)phenyl)acetonitrile |
| I-85 | 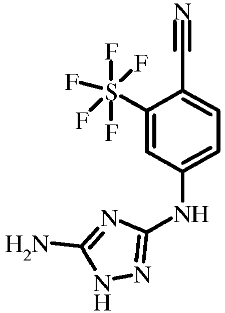 | 4-(5-Amino-1H-[1,2,4]triazol-3-ylamino)-2-pentafluorosulfur-benzonitrile |
| I-86 | 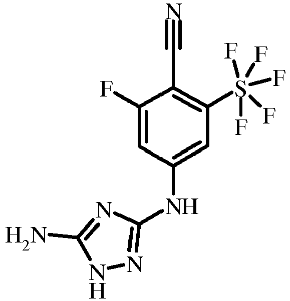 | 4-(5-Amino-1H-[1,2,4]triazol-3-ylamino)-2-fluoro-6-pentafluorosulfur-benzonitrile |
| I-87 | 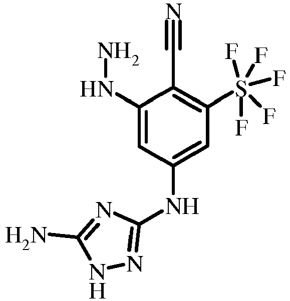 | 4-(5-Amino-1H-[1,2,4]triazol-3-ylamino)-2-hydrazino-6-pentafluorosulfur-benzonitrile |
| I-88 | 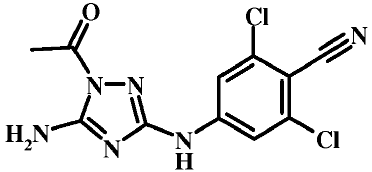 | 4-(1-Acetyl-5-amino-1H-[1,2,4]triazol-3-ylamino)-2,6-dichloro-benzonitrile |
| I-89 | 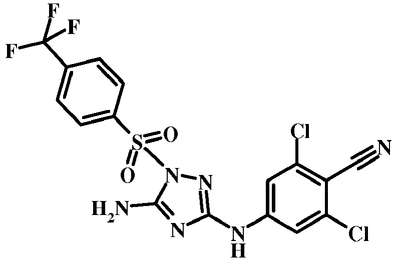 | 4-[5-Amino-1-(4-trifluoromethyl-benzenesulfonyl)-1H-[1,2,4]triazol-3-ylamino]-2,6-dichloro-benzonitrile |
| I-90 | 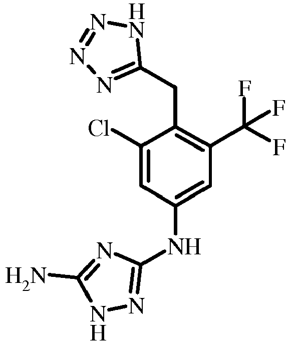 | *N*³-[3-Chloro-4-(1*H*-tetrazol-5-ylmethyl)-5-trifluoromethyl-phenyl]-1*H*-[1,2,4]triazole-3,5-diamine |

### Synthesis

### General Schemes

The following schemes depict general methods for obtaining compounds of Formula I.

### Procedure 1

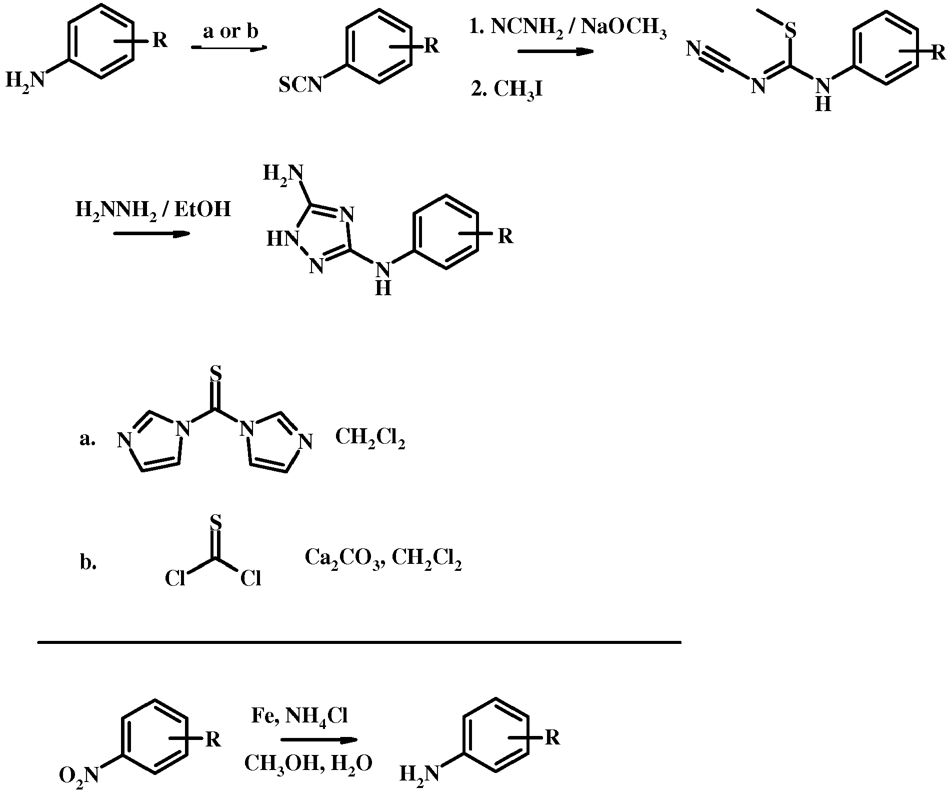

### Procedure 2

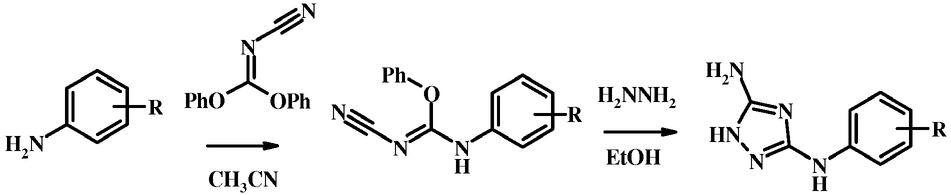

### Procedure 3

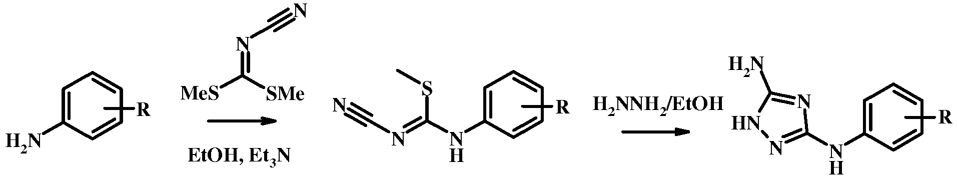

### Procedure 4

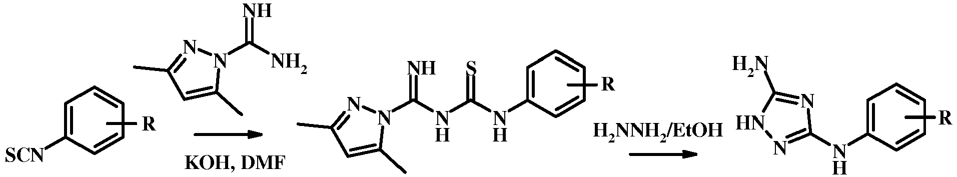

### Procedure 5

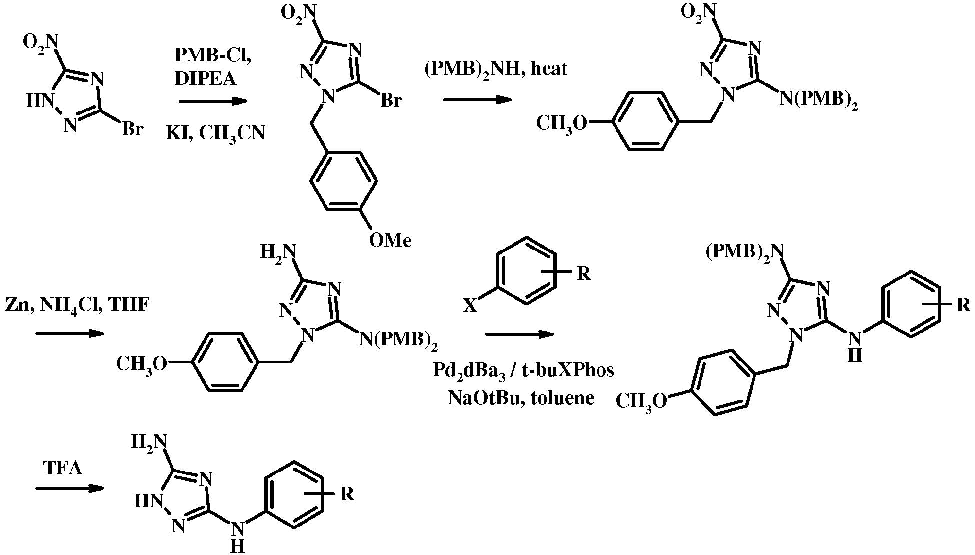

In the above Procedures 1-5, R can be R¹, R², R³ or R⁶, wherein R¹ can be H, halo, lower haloalkyl, lower haloalkyl lower alkyl, trifluoromethyl sulfonyl, lower alkyl, alkoxy, nitro, carboxyl, lower alkyl sulfonamido, trifluoromethyl sulfinyl, cycloalkyl, SF₅, and -NHNH₂;
R² can be H, halo, cyano, nitro, trifluoromethyl sulfonyl, lower halo alkyl, phenyl ethynyl, lower alkyl sulfonyl, lower alkyl amino sulfonyl, lower alkyl amido, morpholinyl methanone, amino sulfonyl, benzyl oxy, cycloalkyl, morpholinyl sulfonyl, phenyl methanone, lower alkyl urea, lower alkyl ester, phenyl amido, pyrrolidinyl methanone, lower alkyl sulfonyl phenyl amido, vinyl, lower alkyl cyano, cyano lower alkyl, tetrazolyl lower alkyl, and tetrahydro pyrimidinyl; R³ can be H, halo, lower alkyl, lower haloalkyl, cyano, lower haloalkyl oxy, cyano cycloalkyl, lower haloalkyl lower alkyl, SF₅, or trifluoromethyl sulfanyl; or R² and R³ together can form an aryl ring system; and R⁶ can be H or halo.

### Dosage and Administration:

The compounds of the present invention may be formulated in a wide variety of oral administration dosage forms and carriers. Oral administration can be in the form of tablets, coated tablets, dragees, hard and soft gelatin capsules, solutions, emulsions, syrups, or suspensions. Compounds of the present invention are efficacious when administered by other routes of administration including continuous (intravenous drip) topical parenteral, intramuscular, intravenous, subcutaneous, transdermal (which may include a penetration enhancement agent), buccal, nasal, inhalation and suppository administration, among other routes of administration. The preferred manner of administration is generally oral using a convenient daily dosing regimen which can be adjusted according to the degree of affliction and the patient's response to the active ingredient.

A compound or compounds of the present invention, as well as their pharmaceutically useable salts, together with one or more conventional excipients, carriers, or diluents, may be placed into the form of pharmaceutical compositions and unit dosages. The pharmaceutical compositions and unit dosage forms may be comprised of conventional ingredients in conventional proportions, with or without additional active compounds or principles, and the unit dosage forms may contain any suitable effective amount of the active ingredient commensurate with the intended daily dosage range to be employed. The pharmaceutical compositions may be employed as solids, such as tablets or filled capsules, semisolids, powders, sustained release formulations, or liquids such as solutions, suspensions, emulsions, elixirs, or filled capsules for oral use; or in the form of suppositories for rectal or vaginal administration; or in the form of sterile injectable solutions for parenteral use. A typical preparation will contain from about 5% to about 95% active compound or compounds (w/w). The term "preparation" or "dosage form" is intended to include both solid and liquid formulations of the active compound and one skilled in the art will appreciate that an active ingredient can exist in different preparations depending on the target organ or tissue and on the desired dose and pharmacokinetic parameters.

The term "excipient" as used herein refers to a compound that is useful in preparing a pharmaceutical composition, generally safe, non-toxic and neither biologically nor otherwise undesirable, and includes excipients that are acceptable for veterinary use as well as human pharmaceutical use. The compounds of this invention can be administered alone but will generally be administered in admixture with one or more suitable pharmaceutical excipients, diluents or carriers selected with regard to the intended route of administration and standard pharmaceutical practice.

"Pharmaceutically acceptable" means that which is useful in preparing a pharmaceutical composition that is generally safe, non-toxic, and neither biologically nor otherwise undesirable and includes that which is acceptable for veterinary as well as human pharmaceutical use.

A "pharmaceutically acceptable salt" form of an active ingredient may also initially confer a desirable pharmacokinetic property on the active ingredient which were absent in the non-salt form, and may even positively affect the pharmacodynamics of the active ingredient with respect to its therapeutic activity in the body. The phrase "pharmaceutically acceptable salt" of a compound means a salt that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, tromethamine, N-methylglucamine, and the like.

Solid form preparations include powders, tablets, pills, capsules, cachets, suppositories, and dispersible granules. A solid carrier may be one or more substances which may also act as diluents, flavoring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. Suitable carriers include but are not limited to magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. Solid form preparations may contain, in addition to the active component, colorants, flavors, stabilizers, buffers, artificial and natural sweeteners, dispersants, thickeners, solubilizing agents, and the like.

Liquid formulations also are suitable for oral administration include liquid formulation including emulsions, syrups, elixirs, aqueous solutions, aqueous suspensions. These include solid form preparations which are intended to be converted to liquid form preparations shortly before use. Emulsions may be prepared in solutions, for example, in aqueous propylene glycol solutions or may contain emulsifying agents such as lecithin, sorbitan monooleate, or acacia. Aqueous solutions can be prepared by dissolving the active component in water and adding suitable colorants, flavors, stabilizing, and thickening agents. Aqueous suspensions can be prepared by dispersing the finely divided active component in water with viscous material, such as natural or synthetic gums, resins, methylcellulose, sodium carboxymethylcellulose, and other well-known suspending agents.

The compounds of the present invention may be formulated for parenteral administration (*e.g*., by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, for example solutions in aqueous polyethylene glycol. Examples of oily or nonaqueous carriers, diluents, solvents or vehicles include propylene glycol, polyethylene glycol, vegetable oils (*e.g*., olive oil), and injectable organic esters (*e.g*., ethyl oleate), and may contain formulatory agents such as preserving, wetting, emulsifying or suspending, stabilizing and/or dispersing agents. Alternatively, the active ingredient may be in powder form, obtained by aseptic isolation of sterile solid or by lyophilisation from solution for constitution before use with a suitable vehicle, e.g., sterile, pyrogen-free water.

The compounds of the present invention may be formulated for topical administration to the epidermis as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also containing one or more emulsifying agents, stabilizing agents, dispersing agents, suspending agents, thickening agents, or coloring agents. Formulations suitable for topical administration in the mouth include lozenges comprising active agents in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

The compounds of the present invention may be formulated for administration as suppositories. A low melting wax, such as a mixture of fatty acid glycerides or cocoa butter is first melted and the active component is dispersed homogeneously, for example, by stirring. The molten homogeneous mixture is then poured into convenient sized molds, allowed to cool, and to solidify.

The compounds of the present invention may be formulated for vaginal administration. Pessaries, tampons, creams, gels, pastes, foams or sprays containing in addition to the active ingredient such carriers as are known in the art to be appropriate.

The compounds of the present invention may be formulated for nasal administration. The solutions or suspensions are applied directly to the nasal cavity by conventional means, for example, with a dropper, pipette or spray. The formulations may be provided in a single or multidose form. In the latter case of a dropper or pipette, this may be achieved by the patient administering an appropriate, predetermined volume of the solution or suspension. In the case of a spray, this may be achieved for example by means of a metering atomizing spray pump.

The compounds of the present invention may be formulated for aerosol administration, particularly to the respiratory tract and including intranasal administration. The compound will generally have a small particle size for example of the order of five (5) microns or less. Such a particle size may be obtained by means known in the art, for example by micronization. The active ingredient is provided in a pressurized pack with a suitable propellant such as a chlorofluorocarbon (CFC), for example, dichlorodifluoromethane, trichlorofluoromethane, or dichlorotetrafluoroethane, or carbon dioxide or other suitable gas. The aerosol may conveniently also contain a surfactant such as lecithin. The dose of drug may be controlled by a metered valve. Alternatively the active ingredients may be provided in a form of a dry powder, for example a powder mix of the compound in a suitable powder base such as lactose, starch, starch derivatives such as hydroxypropylmethyl cellulose and polyvinylpyrrolidine (PVP). The powder carrier will form a gel in the nasal cavity. The powder composition may be presented in unit dose form for example in capsules or cartridges of *e.g.,* gelatin or blister packs from which the powder may be administered by means of an inhaler.

When desired, formulations can be prepared with enteric coatings adapted for sustained or controlled release administration of the active ingredient. For example, the compounds of the present invention can be formulated in transdermal or subcutaneous drug delivery devices. These delivery systems are advantageous when sustained release of the compound is necessary and when patient compliance with a treatment regimen is crucial. Compounds in transdermal delivery systems are frequently attached to a skin-adhesive solid support. The compound of interest can also be combined with a penetration enhancer, e.g., Azone (1-dodecylaza-cycloheptan-2-one). Sustained release delivery systems are inserted subcutaneously into to the subdermal layer by surgery or injection. The subdermal implants encapsulate the compound in a lipid soluble membrane, e.g., silicone rubber, or a biodegradable polymer, e.g., polylactic acid.

Suitable formulations along with pharmaceutical carriers, diluents and excipients are described in Remington: The Science and Practice of Pharmacy 1995, edited by E. W. Martin, Mack Publishing Company, 19th edition, Easton, Pennsylvania. A skilled formulation scientist may modify the formulations within the teachings of the specification to provide numerous formulations for a particular route of administration without rendering the compositions of the present invention unstable or compromising their therapeutic activity.

The modification of the present compounds to render them more soluble in water or other vehicle, for example, may be easily accomplished by minor modifications (salt formulation, esterification, etc.), which are well within the ordinary skill in the art. It is also well within the ordinary skill of the art to modify the route of administration and dosage regimen of a particular compound in order to manage the pharmacokinetics of the present compounds for maximum beneficial effect in patients.

The term "therapeutically effective amount" as used herein means an amount required to reduce symptoms of the disease in an individual. The dose will be adjusted to the individual requirements in each particular case. That dosage can vary within wide limits depending upon numerous factors such as the severity of the disease to be treated, the age and general health condition of the patient, other medicaments with which the patient is being treated, the route and form of administration and the preferences and experience of the medical practitioner involved. For oral administration, a daily dosage of between about 0.01 and about 1000 mg/kg body weight per day should be appropriate in monotherapy and/or in combination therapy. A preferred daily dosage is between about 0.1 and about 500 mg/kg body weight, more preferred 0.1 and about 100 mg/kg body weight and most preferred 1.0 and about 10 mg/kg body weight per day. Thus, for administration to a 70 kg person, the dosage range would be about 7 mg to 0.7 g per day. The daily dosage can be administered as a single dosage or in divided dosages, typically between 1 and 5 dosages per day. Generally, treatment is initiated with smaller dosages which are less than the optimum dose of the compound. Thereafter, the dosage is increased by small increments until the optimum effect for the individual patient is reached. One of ordinary skill in treating diseases described herein will be able, without undue experimentation and in reliance on personal knowledge, experience and the disclosures of this application, to ascertain a therapeutically effective amount of the compounds of the present invention for a given disease and patient.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

### Indications and Method of Treatment

### Indications

The application provides a method for preventing a Hepatitis C Virus (HCV) infection comprising administering to a patient in need thereof a therapeutically effective amount of a compound of Formula I.

The application provides the above method, further comprising administering to a patient in need thereof a therapeutically effective amount of an immune system suppressant.

The application provides a method for treating a Hepatitis C Virus (HCV) infection comprising administering to a patient in need thereof a therapeutically effective amount of a compound of Formula I.

The application provides any of the above methods, further comprising administering an immune system modulator or an antiviral agent that inhibits replication of HCV, or a combination thereof.

The application provides the above method, wherein the immune system modulator is an interferon or a chemically derivatized interferon.

The application provides any of the above methods, further comprising administering an immune system modulator or an antiviral agent that inhibits replication of HCV, or a combination thereof, wherein the antiviral agent is selected from the group consisting of a HCV protease inhibitor, a HCV polymerase inhibitor, a HCV helicase inhibitor, a HCV NS5A inhibitor, or any combination thereof.

### Combination Therapy

The compounds of the invention and their isomeric forms and pharmaceutically acceptable salts thereof are useful in treating and preventing HCV infection alone or when used in combination with other compounds targeting viral or cellular elements or functions involved in the HCV lifecycle. Classes of compounds useful in the invention include, without limitation, all classes of HCV antivirals.

For combination therapies, mechanistic classes of agents that can be useful when combined with the compounds of the invention include, for example, nucleoside and non-nucleoside inhibitors of the HCV polymerase, protease inhibitors, helicase inhibitors, NS4B inhibitors, NS5A inhibitors and medicinal agents that functionally inhibit the internal ribosomal entry site (IRES) and other medicaments that inhibit HCV cell attachment or virus entry, HCV RNA translation, HCV RNA transcription, replication or HCV maturation, assembly or virus release. Specific compounds in these classes and useful in the invention include, but are not limited to, macrocyclic, heterocyclic and linear HCV protease inhibitors such as telaprevir (VX-950), boceprevir (SCH-503034), narlaprevir (SCH-9005 18), ITMN- 191 (R-7227), TMC-435350 (a.k.a. TMC-435), MK- 7009, BI-201335, BI-2061 (ciluprevir), BMS-650032, ACH-1625, ACH-1095 (HCV NS4A protease co-factor inhibitor), VX-500, VX-8 13, PHX-1766, PHX2054, IDX- 136, IDX-3 16, ABT-450 EP-0 13420 (and congeners) and VBY-376; the Nucleosidic HCV polymerase (replicase) inhibitors useful in the invention include, but are not limited to, R7128, PSI-785 1, IDX-184, IDX-102, R1479, UNX-08 189, PSI-6130, PSI-938 and PSI-879 and various other nucleoside and nucleotide analogs and HCV inhibitors including (but not limited to) those derived as 2'-C-methyl modified nucleos(t)ides, 4'-aza modified nucleos(t)ides, and 7'-deaza modified nucleos(t)ides. Non-nucleosidic HCV polymerase (replicase) inhibitors useful in the invention, include, but are not limited to, HCV-796, HCV-371, VCH-759, VCH-916, VCH- 222, ANA-598, MK-3281, ABT-333, ABT-072, PF-00868554, BI-207127, GS-9190, A- 837093, JKT-109, GL-59728 and GL-60667.

In addition, compounds of the invention can be used in combination with cyclophyllin and immunophyllin antagonists (e.g., without limitation, DEBIO compounds, NM-811 as well as cyclosporine and its derivatives), kinase inhibitors, inhibitors of heat shock proteins (e.g., HSP90 and HSP70), other immunomodulatory agents that can include, without limitation, interferons (-alpha, -beta, -omega, -gamma, -lambda or synthetic) such as Intron A, Roferon-A, Canferon-A300, Advaferon, Infergen, Humoferon, Sumiferon MP, Alfaferone, IFN-β, Feron and the like; polyethylene glycol derivatized (pegylated) interferon compounds, such as PEG interferon-α-2a (Pegasys), PEG interferon-α-2b (PEGIntron), pegylated IFN-α -con1 and the like; long acting formulations and derivatizations of interferon compounds such as the albumin-fused interferon, Albuferon, Locteron, and the like; interferons with various types of controlled delivery systems (e.g., ITCA-638, omega-interferon delivered by the DUROS subcutaneous delivery system); compounds that stimulate the synthesis of interferon in cells, such as resiquimod and the like; interleukins; compounds that enhance the development of type 1 helper T cell response, such as SCV-07 and the like; TOLL-like receptor agonists such as CpG-10101 (actilon), isotorabine, ANA773 and the like; thymosin α-1; ANA-245 and ANA-246; histamine dihydrochloride; propagermanium; tetrachlorodecaoxide; ampligen; IMP-321; KRN-7000; antibodies, such as civacir, XTL-6865 and the like and prophylactic and therapeutic vaccines such as InnoVac C, HCV E1E2/MF59 and the like. In addition, any of the above-described methods involving administering an NS5A inhibitor, a Type I interferon receptor agonist (e.g., an IFN-α) and a Type II interferon receptor agonist (e.g., an IFN-γ) can be augmented by administration of an effective amount of a TNF-α antagonist. Exemplary, non-limiting TNF-α antagonists that are suitable for use in such combination therapies include ENBREL, REMICADE, and HUMIRA.

In addition, compounds of the invention can be used in combination with antiprotozoans and other antivirals thought to be effective in the treatment of HCV infection such as, without limitation, the prodrug nitazoxanide. Nitazoxanide can be used as an agent in combination with the compounds disclosed in this invention as well as in combination with other agents useful in treating HCV infection such as peginterferon α-2a and ribavirin.

Compounds of the invention can also be used with alternative forms of interferons and pegylated interferons, ribavirin or its analogs (e.g., tarabavarin, levoviron), microRNA, small interfering RNA compounds (e.g., SIRPLEX-140-N and the like), nucleotide or nucleoside analogs, immunoglobulins, hepatoprotectants, anti-inflammatory agents and other inhibitors of NS5A. Inhibitors of other targets in the HCV lifecycle include NS3 helicase inhibitors; NS4A co-factor inhibitors; antisense oligonucleotide inhibitors, such as ISIS-14803, AVI-4065 and the like; vector-encoded short hairpin RNA (shRNA); HCV specific ribozymes such as heptazyme, RPI, 13919 and the like; entry inhibitors such as HepeX-C, HuMax-HepC and the like; alpha glucosidase inhibitors such as celgosivir, UT-231B and the like; KPE-02003002 and BIVN 401 and IMPDH inhibitors. Other illustrative HCV inhibitor compounds include those disclosed in the following publications: U.S. Pat. Nos. 5,807,876; 6,498,178; 6,344,465; and 6,054,472; PCT Patent Application Publication Nos. WO97/40028; WO98/4038 1; WO00/56331, WO02/04425; WO03/007945; WO03/010141; WO03/000254; WO01/32153; WO00/06529; WO00/18231; WO00/10573; WO00/13708; WO01/85172; WO03/037893; WO03/037894; WO03/037895; WO02/100851; WO02/100846; WO99/01582; WO00/09543; WO02/18369; WO98/17679, WO00/056331; WO98/22496; WO99/07734; WO05/073216, WO05/073195 and WO08/021927.

Additionally, combinations of, for example, ribavirin and interferon, may be administered as multiple combination therapy with at least one of the compounds of the invention. The present invention is not limited to the aforementioned classes or compounds and contemplates known and new compounds and combinations of biologically active agents. It is intended that combination therapies of the present invention include any chemically compatible combination of a compound of this inventive group with other compounds of the inventive group or other compounds outside of the inventive group, as long as the combination does not eliminate the anti-viral activity of the compound of this inventive group or the anti-viral activity of the pharmaceutical composition itself.

Combination therapy can be sequential, that is treatment with one agent first and then a second agent (for example, where each treatment comprises a different compound of the invention or where one treatment comprises a compound of the invention and the other comprises one or more biologically active agents) or it can be treatment with both agents at the same time (concurrently). Sequential therapy can include a reasonable time after the completion of the first therapy before beginning the second therapy. Treatment with both agents at the same time can be in the same daily dose or in separate doses. Combination therapy need not be limited to two agents and may include three or more agents. The dosages for both concurrent and sequential combination therapy will depend on absorption, distribution, metabolism and excretion rates of the components of the combination therapy as well as other factors known to one of skill in the art. Dosage values will also vary with the severity of the condition to be alleviated. It is to be further understood that for any particular subject, specific dosage regimens and schedules may be adjusted over time according to the individual's need and the judgment of the one skilled in the art administering or supervising the administration of the combination therapy.

The application describes a method for preventing a Hepatitis C Virus (HCV) infection comprising administering to a patient in need thereof a therapeutically effective amount of a compound of Formula I.

The application describes the above method, further comprising administering to a patient in need thereof a therapeutically effective amount of an immune system suppressant.

The application describes a method for treating a Hepatitis C Virus (HCV) infection comprising administering to a patient in need thereof a therapeutically effective amount of a compound of Formula I.

The application describes any of the above methods, further comprising administering an immune system modulator or an antiviral agent that inhibits replication of HCV, or a combination thereof.

The application describes the above method, wherein the immune system modulator is an interferon or a chemically derivatized interferon.

The application describes any of the above methods, further comprising administering an immune system modulator or an antiviral agent that inhibits replication of HCV, or a combination thereof, wherein the antiviral agent is selected from the group consisting of a HCV protease inhibitor, a HCV polymerase inhibitor, a HCV helicase inhibitor, a HCV NS5A inhibitor, or any combination thereof.

### EXAMPLES

### Abbreviations

Commonly used abbreviations include: acetyl (Ac), azo-*bis*-isobutyrylnitrile (AIBN), atmospheres (Atm), 9-borabicyclo[3.3.1]nonane (9-BBN or BBN), 2,2'-bis(diphenylphosphino)-1,1'-binaphthyl (BINAP), *tert*-butoxycarbonyl (Boc), di-*tert*-butyl pyrocarbonate or boc anhydride (BOC₂O), benzyl (Bn), butyl (Bu), Chemical Abstracts Registration Number (CASRN), benzyloxycarbonyl (CBZ or Z), carbonyl diimidazole (CDI), 1,4-diazabicyclo[2.2.2]octane (DABCO), diethylaminosulfur trifluoride (DAST), dibenzylideneacetone (dba), 1,5-diazabicyclo[4.3.0]non-5-ene (DBN), 1,8-diazabicyclo[5.4.0]undec-7-ene (DBU), N,N'-dicyclohexylcarbodiimide (DCC), 1,2-dichloroethane (DCE), dichloromethane (DCM), 2,3-Dichloro-5,6-dicyano-1,4-benzoquinone (DDQ), diethyl azodicarboxylate (DEAD), di-*iso*-propylazodicarboxylate (DIAD), di-*iso-*butylaluminumhydride (DIBAL or DIBAL-H), di-iso-propylethylamine (DIPEA), N,N-dimethyl acetamide (DMA), 4-N,N-dimethylaminopyridine (DMAP), N,N-dimethylformamide (DMF), dimethyl sulfoxide (DMSO), 1,1'-*bis*-(diphenylphosphino)ethane (dppe), *1,1'-bis-*(diphenylphosphino)ferrocene (dppf), 1-(3-dimethylaminopropyl)-3-ethylcarbodiimide hydrochloride (EDCI), 2-ethoxy-l-ethoxycarbonyl-1,2-dihydroquinoline (EEDQ), ethyl (Et), ethyl acetate (EtOAc), ethanol (EtOH), 2-ethoxy-2*H*-quinofine-1-carboxylic acid ethyl ester (EEDQ), diethyl ether (Et₂O), ethyl isopropyl ether (EtOiPr), O-(7-azabenzotriazole-1-yl)-N, N,N'N'-tetramethyluronium hexafluorophosphate acetic acid (HATU), acetic acid (HOAc), 1-N-hydroxybenzotriazole (HOBt), high pressure liquid chromatography (HPLC), *iso*-propanol (IPA), isopropylmagnesium chloride (iPrMgCl), hexamethyl disilazane (HMDS), liquid chromatography mass spectrometry (LCMS), lithium hexamethyl disilazane (LiHMDS), meta-chloroperoxybenzoic acid (m-CPBA), methanol (MeOH), melting point (mp), MeSO₂- (mesyl or Ms), methyl (Me), acetonitrile (MeCN), m-chloroperbenzoic acid (MCPBA), mass spectrum (ms), methyl *t*-butyl ether (MTBE), methyl tetrahydrofuran (MeTHF), N-bromosuccinimide (NBS), n-Butyllithium (nBuLi), N-carboxyanhydride (NCA), N-chlorosuccinimide (NCS), N-methylmorpholine (NMM), N-methylpyrrolidone (NMP), pyridinium chlorochromate (PCC), Dichloro-((bis-diphenylphosphino)ferrocenyl) palladium(II) (Pd(dppf)Cl₂), palladium(II) acetate (Pd(OAc)₂), tris(dibenzylideneacetone)dipalladium(0) (Pd₂(dba)₃), pyridinium dichromate (PDC), phenyl (Ph), propyl (Pr), *iso*-propyl (*i*-Pr), pounds per square inch (psi), pyridine (pyr), 1,2,3,4,5-Pentaphenyl-1'-(di-tert-butylphosphino)ferrocene (Q-Phos), room temperature (ambient temperature, rt or RT), sec-Butyllithium (sBuLi), *tert*-butyldimethylsilyl or *t*-BuMe₂Si (TBDMS), tetra-n-butylammonium fluoride (TBAF), triethylamine (TEA or Et₃N), 2,2,6,6-tetramethylpiperidine 1-oxyl (TEMPO), triflate or CF₃SO₂- (Tf), trifluoroacetic acid (TFA), 1,1'-*bis-*2,2,6,6-tetramethylheptane-2,6-dione (TMHD), O-benzotriazol-1-yl-N,N,N',N'-tetramethyluronium tetrafluoroborate (TBTU), thin layer chromatography (TLC), tetrahydrofuran (THF), trimethylsilyl or Me₃Si (TMS), *p*-toluenesulfonic acid monohydrate (TsOH or pTsOH), 4-Me-C₆H₄SO₂ or tosyl (Ts), and N-urethane-N-carboxyanhydride (UNCA). Conventional nomenclature including the prefixes *normal (n), iso* (*i-*)*, secondary* (*sec-*)*, tertiary* (*tert-*) and *neo* have their customary meaning when used with an alkyl moiety. (J. Rigaudy and D. P. Klesney, Nomenclature in Organic Chemistry, IUPAC 1979 Pergamon Press, Oxford.).

### General Conditions

Compounds of the invention can be made by a variety of methods depicted in the illustrative synthetic reactions described below in the Examples section.

The starting materials and reagents used in preparing these compounds generally are either available from commercial suppliers, such as Aldrich Chemical Co., or are prepared by methods known to those skilled in the art following procedures set forth in references such as Fieser and Fieser's Reagents for Organic Synthesis; Wiley & Sons: New York, 1991, Volumes 1-15; Rodd's Chemistry of Carbon Compounds, Elsevier Science Publishers, 1989, Volumes 1-5 and Supplementals; and Organic Reactions, Wiley & Sons: New York, 1991, Volumes 1-40. It should be appreciated that the synthetic reaction schemes shown in the Examples section are merely illustrative of some methods by which the compounds of the invention can be synthesized, and various modifications to these synthetic reaction schemes can be made and will be suggested to one skilled in the art having referred to the disclosure contained in this application.

The starting materials and the intermediates of the synthetic reaction schemes can be isolated and purified if desired using conventional techniques, including but not limited to, filtration, distillation, crystallization, chromatography, and the like. Such materials can be characterized using conventional means, including physical constants and spectral data.

Unless specified to the contrary, the reactions described herein are typically conducted under an inert atmosphere at atmospheric pressure at a reaction temperature range of from about -78 °C to about 150 °C, often from about 0 °C to about 125 °C, and more often and conveniently at about room (or ambient) temperature, e.g., about 20 °C.

Various substituents on the compounds of the invention can be present in the starting compounds, added to any one of the intermediates or added after formation of the final products by known methods of substitution or conversion reactions. If the substituents themselves are reactive, then the substituents can themselves be protected according to the techniques known in the art. A variety of protecting groups are known in the art, and can be employed. Examples of many of the possible groups can be found in "Protective Groups in Organic Synthesis" by Green et al., John Wiley and Sons, 1999. For example, nitro groups can be added by nitration and the nitro group can be converted to other groups, such as amino by reduction, and halogen by diazotization of the amino group and replacement of the diazo group with halogen. Acyl groups can be added by Friedel-Crafts acylation. The acyl groups can then be transformed to the corresponding alkyl groups by various methods, including the Wolff-Kishner reduction and Clemmenson reduction. Amino groups can be alkylated to form mono- and di-alkylamino groups; and mercapto and hydroxy groups can be alkylated to form corresponding ethers. Primary alcohols can be oxidized by oxidizing agents known in the art to form carboxylic acids or aldehydes, and secondary alcohols can be oxidized to form ketones. Thus, substitution or alteration reactions can be employed to provide a variety of substituents throughout the molecule of the starting material, intermediates, or the final product, including isolated products.

### Preparative Examples

### Example 1

### N*3*-(4-Bromo-3,5-dichloro-phenyl)-1H-[1,2,4]triazole-3,5-diamine (Compound 1)

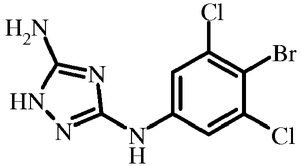

### (Z)-methyl N-4-bromo-3,5-dichlorophenyl-N'-cyanocarbamimidothioate

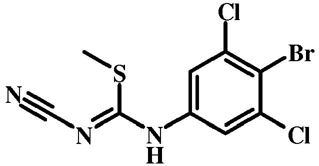

A solution of sodium methoxide (2.6 ml, 1.3 mmol, Eq: 1.23) was added to cyanamide (50 mg, 1.19 mmol, Eq: 1.12) and stirred at room temperature for 15 minutes. 2-bromo-1,3-dichloro-5-isothiocyanatobenzene (300 mg, 1.06 mmol, Eq: 1.00) was added to the reaction mixture and stirred for 1 hr. Iodomethane (331 mg, 146 µl, 2.33 mmol, Eq: 2.2) was added and the pale yellow solution was stirred overnight at room temperature. The resulting suspension was filtered and air dried to give 154 mg (43%) of desired product as a light brown solid.

### N*3*-(4-Bromo-3,5-dichloro-phenyl)-1H-[1,2,4]triazole-3,5-diamine (Compound 1)

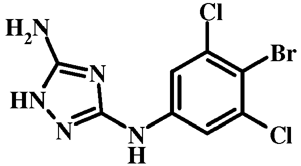

A solution of (Z)-methyl N-4-bromo-3,5-dichlorophenyl-N'-cyanocarbamimidothioate (154 mg, 454 µmol, Eq: 1.00) and hydrazine (153 mg, 150 µl, 4.78 mmol, Eq: 10.5) in ethanol (5 mL) was heated at 65°C. After 3 hr, LCMS ok, no sm. Cooled to rt and stirred solution over weekend. The reaction mixture was concentrated and chromatographed (11 g Supelco, 0 to 10% MeOH/CH2C12) to give 80 mg (55%) of desired product as an off-white solid.
¹H NMR (300MHz, DMSO) δ: 11.35 (s, 1H), 9.33 (s, 1H), 7.75 (s, 2H), 6.05 (s, 2H) ppm

### Example 2

### 4-(5-Amino-1H-[1,2,4]triazol-3-ylamino)-2,6-dichloro-benzonitrile (Compound 2)

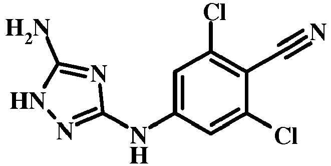

### 2,6-dichloro-4-nitrobenzonitrile

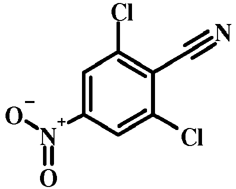

A solution of copper(i) cyanide (2.6 g, 29.0 mmol, Eq: 2) in DMSO (5 mL) was heated at 60o for 1 hr. Tert-butyl nitrite (5.98 g, 6.9 ml, 58.0 mmol, Eq: 4.00) and a solution 2,6-dichloro-4-nitroaniline (3 g, 14.5 mmol, Eq: 1.00) in DMSO (5 mL) was added and the reaction was stirred for 3 hr. The reaction mixture was poured into ice water and extracted with ethyl acetate 3x. The organic extract was washed with brine and dried over sodium sulfate. Chromatography (200g Analogix, 100% hex to 5% EtOAc/hex) gave 515 mg (16%) of desired product as a light brown solid.

### 4-amino-2,6-dichlorobenzonitrile

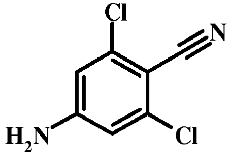

A solution of 2,6-dichloro-4-nitrobenzonitrile (2.46 g, 11.3 mmol, Eq: 1.00), iron (3.17 g, 56.7 mmol, Eq: 5) and ammonium chloride (6.06 g, 113 mmol, Eq: 10) in methanol (30 mL)/water (10 mL) was heated at reflux o/n. TLC shows incomplete reaction. Continued heating at 100 deg for 6hr, then 60 deg overnight. The reaction mixture was filtered over Celite. The filtrate was suspended in ethyl acetate to give insoluble solid. The slurry was concentrated to dryness, suspended in water, filtered, and rinsed with water. The solid was transferred to a round bottom flask, suspended in benzene, and concentrated. An additional portion of benzene was added and concentrated once more to give 1.37 g (65%) of desired product as a light brown solid.

### 2,6-dichloro-4-isothiocyanatobenzonitrile

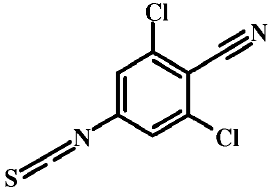

A suspension of 4-amino-2,6-dichlorobenzonitrile (500 mg, 2.67 mmol, Eq: 1.00), thiophosgene (1.35 g, 900 µl, 11.7 mmol, Eq: 4.39), triethylamine (875 mg, 1.2 ml, 8.64 mmol, Eq: 3.23) in benzene (30 ml) was heated at reflux overnight. The brown reaction mixture was concentrated and chromatographed (80 g Analogix, 0 to 5% ethyl acetate/hexane) to give 436 mg (71%) of desired product as a light brown solid.

### (Z)-methyl N'-cyano-N-(3,5-dichloro-4-cyanophenyl)carbamimidothioate

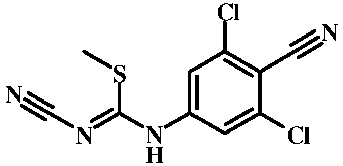

Sodium methoxide (0.5M in methanol) (5.7 ml, 2.85 mmol, Eq: 1.22) was added to cyanamide (108 mg, 2.58 mmol, Eq: 1.1). After 15 minutes, the solution was added to a solution of 2,6-dichloro-4-isothiocyanatobenzonitrile (537 mg, 2.34 mmol, Eq: 1.00) in methanol (5 mL). After 1 hr, methyl iodide (704 mg, 310 µl, 4.96 mmol, Eq: 2.11) was added and the reaction was stirred overnight at room temperature. The resulting suspension was filtered and dried to give 43 mg of desired product as a gray solid. The filtrate was concentrated and chromatographed (40 g Analogix, 50% EtOAc/hex to 75% EtOAc/hex) to give 191 mg of desired product as a yellow solid. The solids were combined to give 234 mg (35%) of desired product.

### 4-(5-Amino-1H-[1,2,4]triazol-3-ylamino)-2,6-dichloro-benzonitrile (Compound 2)

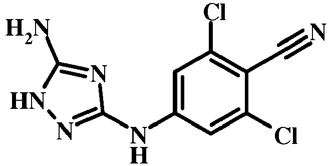

A solution of (Z)-methyl N'-cyano-N-(3,5-dichloro-4-cyanophenyl)carbamimidothioate (234 mg, 821 µmol, Eq: 1.00) and hydrazine (263 mg, 258 µl, 8.21 mmol, Eq: 10) in ethanol (10 mL) was heated at 65°C o/n. The reaction mixture was concentrated and chromatographed (23 Supelco, 100% DCM to 5% to 10% MeOH/DCM to give 156 mg (71%) of desired product as an off-white solid.
¹H NMR (300MHz, DMSO) δ: 11.55 (s, 1H), 9.99 (s, 1H), 7.74 (s, 2H), 6.14 (s, 2H) ppm

### Example 3

### N*3*-(3,5-Dichloro-4-iodo-phenyl)-1H-[1,2,4]triazole-3,5-diamine (Compound 3)

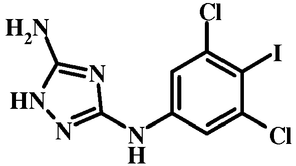

### 3,5-dichloro-4-iodoaniline

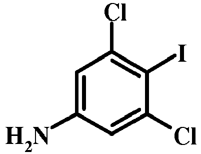

A solution of 1,3-dichloro-2-iodo-5-nitrobenzene (1 g, 3.15 mmol, Eq: 1.00), iron (878 mg, 15.7 mmol, Eq: 5) and ammonium chloride (1.68 g, 31.5 mmol, Eq: 10) in methanol (10 mL)/water (5 mL) was heated at reflux o/n. The reaction mixture was filtered over Celite, diluted with ethyl acetate, washed with brine 3x, dried over sodium sulfate, and chromatographed (60g Analogix, 100% hex to 20%EtOAc/hex) to give 255 mg (28%) of desired product as a white solid.

### 1,3-dichloro-2-iodo-5-isothiocyanatobenzene

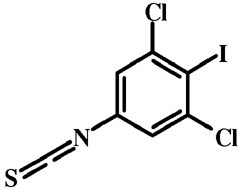

To a suspension of calcium carbonate (318 mg, 3.18 mmol, Eq: 2.5) and thiophosgene (165 mg, 110 µl, 1.44 mmol, Eq: 1.13) in dichloromethane (10.0 ml)/water (10.0 ml) at 0, was added 3,5-dichloro-4-iodoaniline (366 mg, 1.27 mmol, Eq: 1.00) The reaction was gradually warmed to room temperature and stirred overnight. Added 3 mL IN HCl. Separated organic layer, dried over sodium sulfate, and chromatographed (25 g Analogix, 100% hex to 10% EtOAc/hex) to give 271 mg (65%) of desired product as a white solid.

### (Z)-methyl N'-cyano-N-(3,5-dichloro-4-iodophenyl)carbamimidothioate

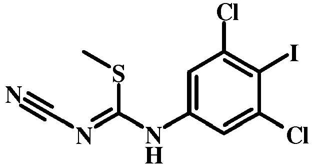

Sodium methoxide (0.5M in methanol) (2 ml, 1.00 mmol, Eq: 1.22) was added to cyanamide (40 mg, 951 µmol, Eq: 1.16). After 15 minutes, the solution was added to a solution of 1,3-dichloro-2-iodo-5-isothiocyanatobenzene (271 mg, 821 µmol, Eq: 1.00) in methanol (3 mL). After 1 hr, methyl iodide (250 mg, 110 µl, 1.76 mmol, Eq: 2.14) was added and the reaction was stirred overnight at room temperature. The reaction mixture was concentrated and chromatographed (24 g Analogix, 20% EtOAc/hex to 50% EtOAc/hex) to give 121 mg (38%) of desired product as a white solid.

### N*3*-(3,5-Dichloro-4-iodo-phenyl)-1H-[1,2,4]triazole-3,5-diamine (Compound 3)

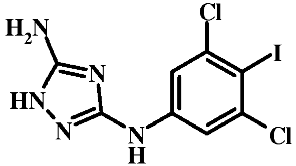

A solution of (Z)-methyl N'-cyano-N-(3,5-dichloro-4-iodophenyl)carbamimidothioate (121 mg, 313 µmol, Eq: 1.00) and hydrazine (102 mg, 100 µl, 3.19 mmol, Eq: 10.2) in ethanol (5 mL) was heated at 65°C o/n. The reaction mixture was concentrated and chromatographed (11g Supelco, 100%DCM to 10% MeOH/DCM) to give 43 mg (37%) of desired product as a white solid.
¹H NMR (300MHz, DMSO) δ: 11.30 (s, 1H), 9.23 (s, 1H), 7.71 (s, 2H), 5.98 (s, 2H) ppm

### Example 4

### N*3*-(3-Trifluoromethanesulfonyl-phenyl)-1H-[1,2,4]triazole-3,5-diamine (Compound 4)

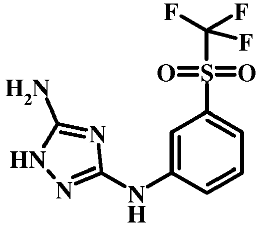

### 1-isothiocyanato-3-(trifluoromethylsulfonyl)benzene

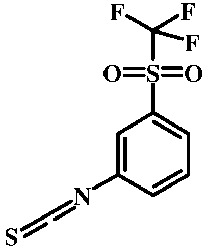

To a suspension of calcium carbonate (1.1 g, 11.0 mmol, Eq: 2.5) and thiophosgene (563 mg, 375 µl, 4.89 mmol, Eq: 1.11) in dichloromethane (10.0 ml)/water (10.0 ml) at 0, was added 3-(trifluoromethylsulfonyl)aniline (.99 g, 4.4 mmol, Eq: 1.00) The reaction was gradually warmed to room temperature and stirred overnight. Added 15 mL IN HCl slowly. Separated organic layer and dried over sodium sulfate to give 1.068g (91%) of desired product as a light brown oil.

### (Z)-methyl N'-cyano-N-(3-(trifluoromethylsulfonyl)phenyl)carbamimidothioate

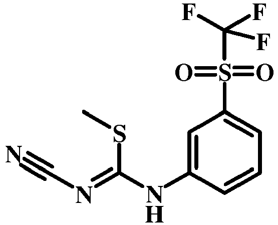

A solution of sodium methoxide (2.69 ml, 1.35 mmol, Eq: 1.2) was added to cyanamide (51.9 mg, 1.23 mmol, Eq: 1.1) and stirred at room temperature for 15 minutes. 1-isothiocyanato-3-(trifluoromethylsulfonyl)benzene (300 mg, 1.12 mmol, Eq: 1.00) was added to the reaction mixture and stirred for 1 hr. Iodomethane (319 mg, 140 µl, 2.25 mmol, Eq: 2) was added and the suspension was stirred overnight at room temperature. The reaction mixture was concentrated and chromatographed (24 g Analogix, 25 to 40% EtOAc/hex) 151 mg (42%) of desired product as a yellow solid.

### N*3*-(3-Trifluoromethanesulfonyl-phenyl)-1H-[1,2,4]triazole-3,5-diamine (Compound 4)

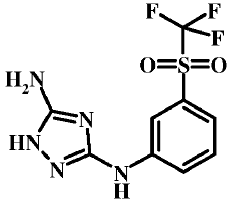

A suspension of (Z)-methyl N'-cyano-N-(3-(trifluoromethylsulfonyl)phenyl)carbamimidothioate (151 mg, 467 µmol, Eq: 1.00) and hydrazine (153 mg, 150 µl, 4.78 mmol, Eq: 10.2) in ethanol (5 mL) was heated at 65°C o/n. The resulting suspension was filtered and rinsed with cold MeOH to give 58 mg (94%) of desired product as a white solid.
¹H NMR (300MHz, DMSO) δ: 11.41 (s, 1H), 9.49 (s, 1H), 8.48 (m, 1H), 7.90 (dd, J = 9Hz, 2Hz, 1H), 7.62 (t, J = 8 Hz ,1H), 7.44-7.41 (m, 1H), 6.04 (s, 2H) ppm

### Example 5

### N*3*-(4-Bromo-3-chloro-5-fluoro-phenyl)-1H-[1,2,4]triazole-3,5-diamine (Compound 5, Intermediate 5)

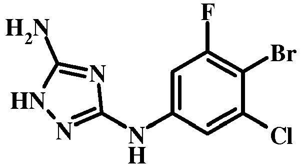

### 4-bromo-3-chloro-5-fluoroaniline

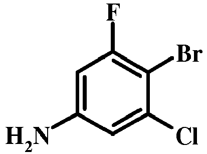

A solution of N-(4-bromo-3-chloro-5-fluorophenyl)acetamide see Example 24 from biaryl patent (2.93 g, 11.0 mmol, Eq: 1.00) and HCl (44.4 g, 37 ml, 222 mmol, Eq: 20.2) in ethanol (35 ml) was heated at reflux o/n. The reaction mixture was concentrated, dissolved in EtOAc, washed with NaOH solution (8.8g in 20 mL H2O) and dried over sodium sulfate to give 2.37g (96%) of desired product as a pale yellow solid.

### 2-bromo-1-chloro-3-fluoro-5-isothiocyanatobenzene

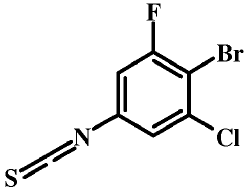

To a suspension of calcium carbonate (2.64 g, 26.4 mmol, Eq: 2.5) and thiophosgene (1.46 g, 975 µl, 12.7 mmol, Eq: 1.2) in dichloromethane (10.0 ml)/water (10.0 ml) at 0, was added 4-bromo-3-chloro-5-fluoroaniline (2.37 g, 10.6 mmol, Eq: 1.00) The reaction was gradually warmed to room temperature and stirred overnight. Added 26 mL IN HCl slowly. Separated organic layer and dried over sodium sulfate to give 2.46 g (87%) of desired product as a pale yellow solid.

### N-((4-bromo-3-chloro-5-fluorophenylamino)(methylthio)methyl)cyanamide

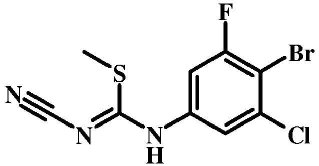

To a solution of 2-bromo-1-chloro-3-fluoro-5-isothiocyanatobenzene (2.46 g, 9.23 mmol, Eq: 1.00) in MeOH (20 mL) was added to sodium hydrogen cyanamide (627 mg, 9.79 mmol, Eq: 1.06). After 30 minutes, methyl iodide (2.62 g, 1.15 ml, 18.5 mmol, Eq: 2) was added and the reaction was stirred overnight at room temperature. The resulting suspension was filtered solid and dried with house vacuum. 2.19g (74%) of desired product as a white solid.

### N*3*-(4-Bromo-3-chloro-5-fluoro-phenyl)-1H-[1,2,4]triazole-3,5-diamine (Compound 5)

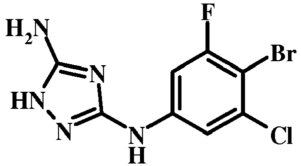

To a solution of N-((4-bromo-3-chloro-5-fluorophenylamino)(methylthio)methyl)cyanamide (2.19 g, 6.75 mmol, Eq: 1.00) in ethanol (30 mL) was added hydrazine (2.16 g, 2.12 ml, 67.5 mmol, Eq: 10) . The reaction mixture was heated at 60 deg o/n. The reaction mixture was concentrated, suspended in Et2O and filtered to give 1.03 g of desired product as a white solid. The filtrate precipitated upon standing and was filtered to give 919 mg of desired product as a white solid. The solids were combined to give 1.95 g (94%) total product.
MS m/z 306, 308 [M+H]

### Example 6

### N*3*-(3-tert-Butyl-phenyl)-1H-[1,2,4]triazole-3,5-diamine (Compound 6)

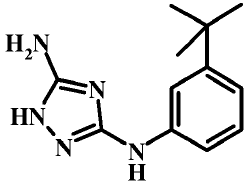

### (Z)-methyl N-3-tert-butylphenyl-N'-cyanocarbamimidothioate

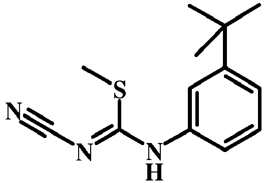

A solution of sodium methoxide (3.76 ml, 1.88 mmol, Eq: 1.2) was added to cyanamide (72.5 mg, 1.73 mmol, Eq: 1.1) and stirred at room temperature for 15 minutes. 1-tert-butyl-3-isothiocyanatobenzene (300 mg, 1.57 mmol, Eq: 1.00) was added to the reaction mixture and stirred for 1 hr. Iodomethane (499 mg, 220 µl, 3.52 mmol, Eq: 2.24) was added and the yellow solution was stirred overnight at room temperature. The reaction mixture was concentrated and chromatographed (24g Analogix, 20 to 30% EtOAc/Hex) to give 241 mg (62%) of desired product as a white solid.

### N*3*-(3-tert-Butyl-phenyl)-1H-[1,2,4]triazole-3,5-diamine (Compound 6)

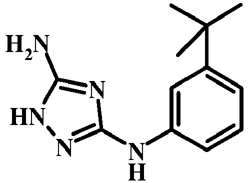

A solution of (Z)-methyl N-3-tert-butylphenyl-N'-cyanocarbamimidothioate (241 mg, 974 µmol, Eq: 1.00) and hydrazine (317 mg, 310 µl, 9.88 mmol, Eq: 10.1) in ethanol (5 mL) was heated at 65°C o/n. The reaction mixture was concentrated and chromatographed (0 to 10% MeOH/CH2Cl2, 11 g Supelco) to give 103 mg (46%) of desired product as a white solid.
¹H NMR (300MHz, DMSO) δ: 11.10 (s, 1H), 8.45 (s, 1H), 7.52 (s, 1H), 7.33 (dd, J =8 Hz,1 Hz, 1H), 7.06 (t, J = 8 Hz ,1H), 6.76-6.73 (m, 1H), 5.83 (s, 2H), 1.25 (s 9H) ppm

### Example 7

### N*3*-(3-Chloro-4-trifluoromethanesulfonyl-phenyl)-1H-[1,2,4]triazole-3,5-diamine (Compound 7)

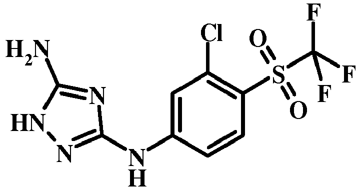

### N-(3-chloro-4-(trifluoromethylthio)phenyl)acetamide

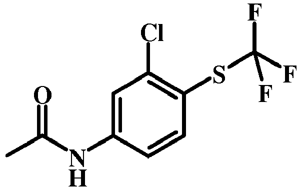

To a solution of 3-chloro-4-(trifluoromethylthio)aniline (1.5 g, 6.59 mmol, Eq: 1.00) and triethylamine (1.00 g, 1.38 mL, 9.88 mmol, Eq: 1.5) in dichloromethane (50 mL) at 0°C, was added acetyl chloride (569 mg, 515 µL, 7.25 mmol, Eq: 1.1). The reaction was gradually warmed to room temperature and stirred o/n. TLC indicated some starting material remained. Added 515 □L acetyl chloride and 1.4 mL triethylamine and stirred for 8 hours. Diluted with CH2Cl2, washed with IN HCl, brine, dried over sodium sulfate, and chromatographed (80g Analogix, 100% hex to 20% EtOAc/hex to 40% EtOAc/hex) gave 1.51 g (65%) of desired product as a light yellow solid.

### N-(3-chloro-4-(trifluoromethylsulfonyl)phenyl)acetamide

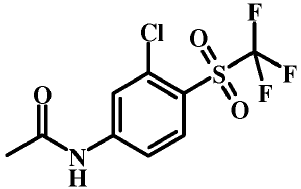

To a solution of N-(3-chloro-4-(trifluoromethylthio)phenyl)acetamide (.75 g, 2.78 mmol, Eq: 1.00) in dichloromethane (30 mL) at 0°C, was added mCPBA (1.88 g, 8.39 mmol, Eq: 3.02). The reaction was gradually warmed to room temperature and stirred o/n. The reaction mixture was quenched with NaHCO3 solution and the organic layer was separated, washed with brine, dried over sodium sulfate, and chromatographed (80 g Analogix, 20 to 40% EtOAc/Hex) to give 623 mg (74%) of desired product as a white solid.

### 3-chloro-4-(trifluoromethylsulfonyl)aniline

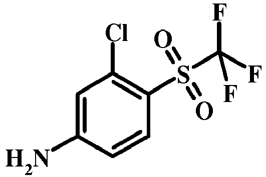

A solution of N-(3-chloro-4-(trifluoromethylsulfonyl)phenyl)acetamide (501 mg, 1.66 mmol, Eq: 1.00) and concentrated HCl (2.00 ml) and water (2 mL) was heated at reflux. The reaction was cooled to 0°C and carefully neutralized with NaOH (10 mL 2N). The resulting suspension was concentrated to dryness and chromatographed (40 g Thomson, 100% hex to 20%EtOAc/hex) to give 384 mg (89%) of desired product as a white solid.

### 2-chloro-4-isothiocyanato-1-(trifluoromethylsulfonyl)benzene

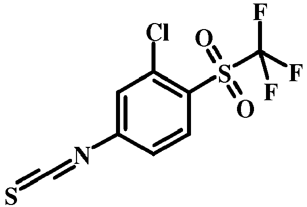

To a suspension of calcium carbonate (370 mg, 3.7 mmol, Eq: 2.5) and thiophosgene (187 mg, 125 µl, 1.63 mmol, Eq: 1.1) in dichloromethane (10.0 ml)/water (10.0 ml) at 0, was added 3-chloro-4-(trifluoromethylsulfonyl)aniline (384 mg, 1.48 mmol, Eq: 1.00) The reaction was gradually warmed to room temperature and stirred overnight. Added 12 mL IN HCl slowly. Separated organic layer and dried over sodium sulfate to give 319 mg (72%) of desired product as an orange oil.

### (Z)-methyl N-3-chloro-4-(trifluoromethylsulfonyl)phenyl-N'-cyanocarbamimidothioate

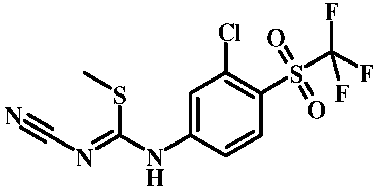

Sodium methoxide (0.5M in methanol) (2.54 ml, 1.27 mmol, Eq: 1.2) was added to cyanamide (48.9 mg, 1.16 mmol, Eq: 1.1). After 15 minutes, a solution of 2-chloro-4-isothiocyanato-1-(trifluoromethylsulfonyl)benzene (319 mg, 1.06 mmol, Eq: 1.00) in methanol (5 mL) was added. After 1 hr, methyl iodide (300 mg, 132 µl, 2.11 mmol, Eq: 2) was added and the reaction was stirred overnight at room temperature. The reaction mixture was concentrated and chromatographed (24g Analogix, 20% EtOAc/hex to 75% EtOAc/hex) to give 144 mg (38%) of desired product as a pale yellow oil.

### N*3*-(3-Chloro-4-trifluoromethanesulfonyl-phenyl)-1H-[1,2,4]triazole-3,5-diamine (Compound 7)

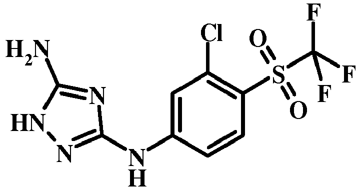

A solution of (Z)-methyl N-3-chloro-4-(trifluoromethylsulfonyl)phenyl-N'-cyanocarbamimidothioate (144 mg, 403 µmol, Eq: 1.00) and hydrazine (153 mg, 150 µl, 4.78 mmol, Eq: 11.9) in ethanol (5 mL) was heated at 65°C o/n. The reaction mixture was concentrated and chromatographed (0 to 10% MeOH/CH2Cl2, Supelco 11 g) to give 25 mg (18%) of desired product as an off-white solid.
¹H NMR (300MHz, DMSO) δ: 11.43 (s, 1H), 9.71 (s, 1H), 7.94 (d, J = 2 Hz, 1H), 7.73-7.61 (m, 2H), 6.03 (s, 2H) ppm

### Example 8

### N*3*-(3-Chloro-4-nitro-phenyl)-1H-[1,2,4]triazole-3,5-diamine (Compound 8)

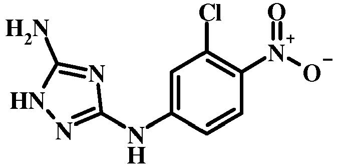

### 2-chloro-4-isothiocyanato-1-nitrobenzene

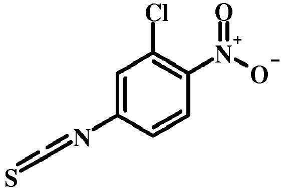

To a suspension of calcium carbonate (1.45 g, 14.5 mmol, Eq: 2.5) and thiophosgene (735 mg, 490 *l, 6.39 mmol, Eq: 1.1) in dichloromethane (10.0 ml)/water (10.0 ml) at 0 deg, was added 3-chloro-4-nitroaniline ( 1 g, 5.79 mmol, Eq: 1.00) The reaction was gradually warmed to room temperature and stirred overnight. Added 15 mL IN HCl slowly. Separated organic layer, dried over sodium sulfate, and chromatographed (40 g Analogix, 100% hex to 5% ethyl acetate/hexane) to give 1.004 g (81%) of desired product as a light yellow oil.

### (Z)-methyl N-3-chloro-4-nitrophenyl-N'-cyanocarbamimidothioate

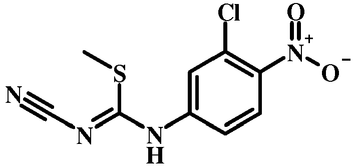

A solution of sodium methoxide (3.35 ml, 1.68 mmol, Eq: 1.2) was added to cyanamide (64.6 mg, 1.54 mmol, Eq: 1.1) and stirred at room temperature for 15 minutes. 2-chloro-4-isothiocyanato-1-nitrobenzene (300 mg, 1.4 mmol, Eq: 1.00) was added to the reaction mixture and stirred for 1 hr. Iodomethane (397 mg, 175 µl, 2.8 mmol, Eq: 2) was added and the suspension was stirred overnight at room temperature. The resulting yellow suspension was filtered to give 120 mg (32%) of desired product as a yellow solid

### N*3*-(3-Chloro-4-nitro-phenyl)-1H-[1,2,4]triazole-3,5-diamine (Compound 8)

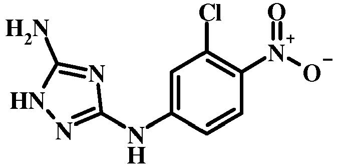

A suspension of (Z)-methyl N-3-chloro-4-nitrophenyl-N'-cyanocarbamimidothioate (120 mg, 443 µmol, Eq: 1.00) and hydrazine (143 mg, 140 µl, 4.46 mmol, Eq: 10.1) in ethanol (5 mL) was heated at 65°C o/n. The resulting suspension was filtered solid to give 91 mg (81%) of desired product as a red solid.
¹H NMR (300MHz, DMSO) δ: 11.53 (s, 1H), 9.92 (s, 1H), 8.07 (d, J =9 Hz, 1H), 7.93 (d, J = 2 Hz ,1H), 7.45 (dd, J = 9 Hz, 3 Hz, 1H), 6.12 (s, 2H) ppm

### Example 9

### N*3*-(3-Chloro-4-ethynyl-phenyl)-1H-[1,2,4]triazole-3,5-diamine (Compound 9)

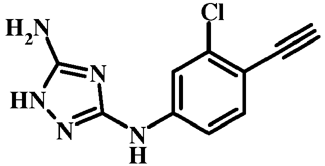

### ((2-chloro-4-nitrophenyl)ethynyl)trimethylsilane

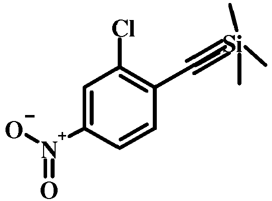

2-chloro-1-iodo-4-nitrobenzene (2 g, 7.06 mmol, Eq: 1.00), trimethylsilylacetylene (840 mg, 1.2 ml, 8.55 mmol, Eq: 1.21), copper(I) iodide (254 mg, 1.33 mmol, Eq: 0.189) and bis(triphenylphosphine)palladium (II) chloride (500 mg, 712 µmol, Eq: 0.101) were placed in a round-bottom flask. THF/diisopropylamine (20 mL/ 4 mL) was added to the flask and the reaction mixture was heated at 60 overnight. The reaction was concentrated and chromatographed (150g Analogix, 100% hex to 3% EtOAc/hex) to give 1.167 g (65%) of desired product as a brown solid.

### 3-chloro-4-((trimethylsilyl)ethynyl)aniline

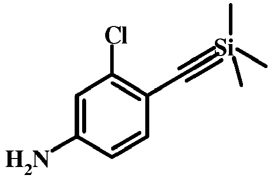

A solution of ((2-chloro-4-nitrophenyl)ethynyl)trimethylsilane (865 mg, 3.41 mmol, Eq: 1.00), iron (966 mg, 17.3 mmol, Eq: 5.07) and ammonium chloride (1.82 g, 34.1 mmol, Eq: 10) in methanol/water (30 mL/15 mL) was heated at 70°C o/n. The reaction mixture was filtered over Celite, diluted with ethyl acetate, washed with brine 3x, and dried over sodium sulfate to give 644 mg (84%) of desired product as an orange oil.

### (2-chloro-4-isothiocyanatophenyl)ethynyl)trimethylsilane

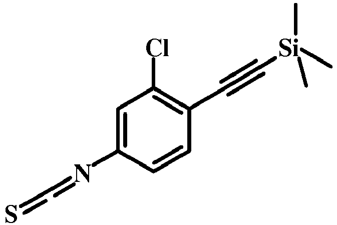

To a suspension of calcium carbonate (968 mg, 9.67 mmol, Eq: 2.5) and thiophosgene (495 mg, 330 µl, 4.31 mmol, Eq: 1.11) in dichloromethane (10.0 ml)/water (10.0 ml) at 0, was added 3-chloro-4-((trimethylsilyl)ethynyl)aniline (866 mg, 3.87 mmol, Eq: 1.00) The reaction was gradually warmed to room temperature and stirred overnight. Added 10 mL IN HCl. Separated organic layer and dried over sodium sulfate. Chromatographed (120 g Analogix, 100% hex) to give 584 mg (57%) of colorless oil, containing a ~3.5:1 mix of prod and de-silylated product. The mixture was carried onto the next step.

### (Z)-methyl N-3-chloro-4-ethynylphenyl-N'-cyanocarbamimidothioate

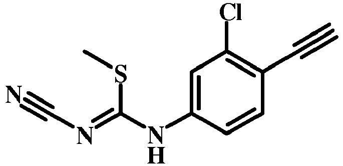

Sodium methoxide (0.5M in methanol) (3 ml, 1.5 mmol, Eq: 1.33) was added to cyanamide (57 mg, 1.36 mmol, Eq: 1.2). After 15 minutes, a solution of ((2-chloro-4-isothiocyanatophenyl)ethynyl)trimethylsilane (300 mg, 1.13 mmol, Eq: 1.00) in methanol (5 mL) was added to the reaction mixture. After 1 hr, methyl iodide (340 mg, 150 µl, 2.4 mmol, Eq: 2.13) was added and the reaction was stirred overnight at room temperature. The resulting suspension was filtered to give 57 mg of desired product as a white solid. The filtrate was concentrated and chromatographed (40 g Analogix, 20% EtOAc/hex to 40% EtOAc/hex) to give an additional 103 mg of desired product as a white solid. The products were combined for a total of 160 mg (57%)

### N*3*-(3-Chloro-4-ethynyl-phenyl)-1H-[1,2,4]triazole-3,5-diamine (Compound 9)

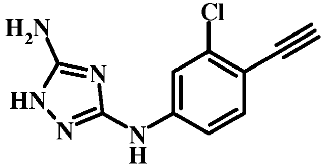

A solution of (Z)-methyl N-3-chloro-4-ethynylphenyl-N'-cyanocarbamimidothioate (160 mg, 641 µmol, Eq: 1.00) and hydrazine (204 mg, 200 µl, 6.37 mmol, Eq: 9.95) in ethanol (5 mL) was heated at 70o o/n. The reaction mixture was concentrated and chromatographed (11g Supelco, 100%DCM to 10% MeOH/DCM) to give 108 mg white solid, containing desired product and impurities. Further purification by preparative plate chromatography (10% MeOH/DCM) gave 71 mg of product with impurities. Further purification by SFC gave 48 mg (32%) of desired product as a white solid
MS m/z 234 [M+H]

### Example 10

### N*3*-(3-Chloro-4-trifluoromethyl-phenyl)-1H-[1,2,4]triazole-3,5-diamine (Compound 10)

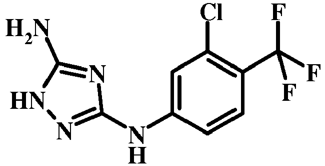

### 2-chloro-4-isothiocyanato-1-(trifluoromethyl)benzene

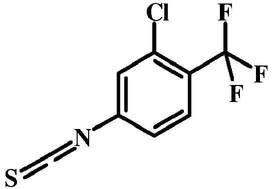

To a suspension of calcium carbonate (1.04 g, 10.4 mmol, Eq: 2.03) and thiophosgene (645 mg, 430 µl, 5.61 mmol, Eq: 1.1) in dichloromethane (13.2 g, 10.0 ml, 155 mmol, Eq: 25.3)/water (10.0 g, 10.0 ml, 555 mmol, Eq: 90.2) at 0, was added 3-chloro-4-(trifluoromethyl)aniline (1 g, 5.11 mmol, Eq: 1.00) The reaction was gradually warmed to room temperature and stirred overnight. Added 10 mL IN HCl. Separated organic layer, dried over sodium sulfate, and chromatographed (60 g Analogix, 100% hex) to give 962 mg (79%) of desired product as colorless oil.

### (Z)-methyl N-3-chloro-4-(trifluoromethyl)phenyl-N'-cyanocarbamimidothioate

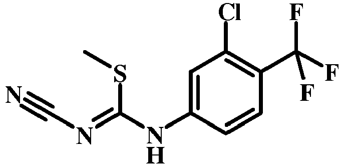

Sodium methoxide (0.5M in methanol) (4.1 ml, 2.05 mmol, Eq: 1.22) was added to cyanamide (78 mg, 1.86 mmol, Eq: 1.1). After 15 minutes, the solution was added to a solution of 2-chloro-4-isothiocyanato-1-(trifluoromethyl)benzene (400 mg, 1.68 mmol, Eq: 1.00) in methanol (5 mL). After 1 hr, methyl iodide (478 mg, 211 µl, 3.37 mmol, Eq: 2) was added and the reaction was stirred overnight at room temperature. The reaction mixture was concentrated and chromatographed (40 g Analogix, 20 to 40% EtOAc/hex) to give 294 mg (60%) of desired product as an off white solid.

### N*3*-(3-Chloro-4-trifluoromethyl-phenyl)-1H-[1,2,4]triazole-3,5-diamine (Compound 10)

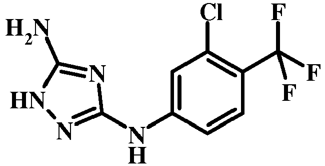

A solution of (Z)-methyl N-3-chloro-4-(trifluoromethyl)phenyl-N'-cyanocarbamimidothioate (294 mg, 1.00 mmol, Eq: 1.00) and hydrazine (332 mg, 325 µl, 10.4 mmol, Eq: 10.3) in ethanol (10 mL)was heated at 70o o/n. The reaction mixture was concentrated and chromatographed (23 g Supelco, 100% DCM to 10% MeOH/DCM) to give 187 mg (67%) of desired product as a white solid. MS m/z 278 [M+H]

### Example 11

### N*3*-(3,4,5-Trichloro-phenyl)-1H-[1,2,4]triazole-3,5-diamine (Compound 11)

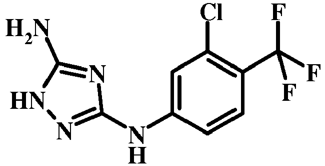

### 1,2,3-trichloro-5-isothiocyanatobenzene

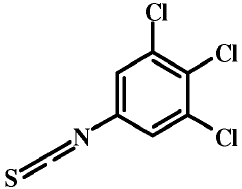

To a suspension of calcium carbonate (1.27 g, 12.7 mmol, Eq: 2.5) and thiophosgene (750 mg, 500 µl, 6.52 mmol, Eq: 1.28) in dichloromethane (13.2 g, 10.0 ml, 155 mmol, Eq: 25.3)/water (10.0 g, 10.0 ml, 555 mmol, Eq: 90.2) at 0, was added 3,4,5-trichloroaniline (1 g, 5.09 mmol, Eq: 1.00) The reaction was gradually warmed to room temperature and stirred overnight. Added 13 mL IN HCl. Separated organic layer and dried over sodium sulfate to give 1.12 g (92%) of desired product as an off-white solid.

### (Z)-methyl N'-cyano-N-(3,4,5-trichlorophenyl)carbamimidothioate

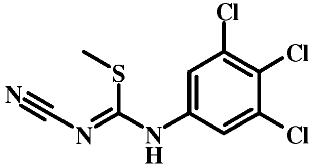

Sodium methoxide (0.5M in methanol) (3.1 ml, 1.55 mmol, Eq: 1.23) was added to cyanamide (60 mg, 1.43 mmol, Eq: 1.13). After 15 minutes, the solution was added to a solution of 1,2,3-trichloro-5-isothiocyanatobenzene (300 mg, 1.26 mmol, Eq: 1.00) in methanol (5 mL). After 1 hr, methyl iodide (357 mg, 157 µl, 2.52 mmol, Eq: 2) was added and the reaction was stirred overnight at room temperature. The resulting gray suspension was filtered to give 110 mg of desired product as an off-white solid. NMR ok (Juno 4367-72cr). Filtrate concentrated and chromatographed (40 g Analogix, 25% to 50% EtOAc/hex) to give an additional 107 mg of desired product as a white solid. The solids were combined to give a total 217 mg (59%) of product.

### N*3*-(3,4,5-Trichloro-phenyl)-1H-[1,2,4]triazole-3,5-diamine (Compound 11)

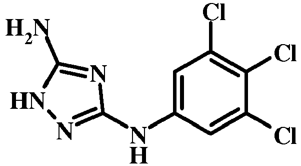

A solution of (Z)-methyl N'-cyano-N-(3,4,5-trichlorophenyl)carbamimidothioate (110 mg, 373 µmol, Eq: 1.00) and hydrazine (123 mg, 120 µl, 3.82 mmol, Eq: 10.2) in ethanol (5 mL) was heated at 70° o/n. The reaction was concentrated to dryness. Triturated in methanol/DCM and filtered to give 25 mg (24%) of desired product as an off-white solid.
MS m/z 278, 280 [M+H]

### Example 12

### 4-(5-Amino-1H-[1,2,4]triazol-3-ylamino)-2-chloro-benzonitrile (Compound 12)

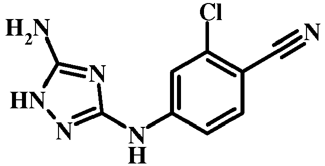

### 2-chloro-4-isothiocyanatobenzonitrile

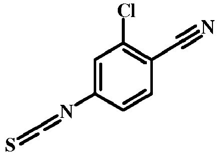

2/12 10:50 a.m. To a suspension of calcium carbonate (1.65 g, 16.5 mmol, Eq: 2.52) and thiophosgene (900 mg, 600 µL, 7.83 mmol, Eq: 1.19) in dichloromethane (13.2 g, 10 mL, 155 mmol, Eq: 23.7)/water (10.0 g, 10 mL, 555 mmol, Eq: 84.7) at 0, was added 4-amino-2-chlorobenzonitrile (1 g, 6.55 mmol, Eq: 1.00). The reaction was gradually warmed to room temperature. Added IN HCl slowly. Separated organic layer, dried over sodium sulfate, and chromatographed (40 g Analogix, 5 to 10% ethyl acetate/hexane) to give 851 mg (67%) of desired product as a white solid.

### (Z)-methyl N-3-chloro-4-cyanophenyl-N'-cyanocarbamimidothioate

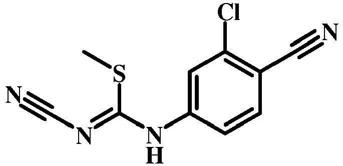

A solution of sodium methoxide (2.5 ml, 1.25 mmol, Eq: 1.22) was added to cyanamide (48 mg, 1.14 mmol, Eq: 1.11) and stirred at room temperature for 15 minutes. 2-chloro-4-isothiocyanatobenzonitrile (200 mg, 1.03 mmol, Eq: 1.00) was added to the reaction mixture and stirred for 1 hr. Iodomethane (318 mg, 140 µl, 2.24 mmol, Eq: 2.18) was added and the reaction was stirred overnight at room temperature. The white slurry was filtered to give 109 mg (42%) of desired product as a white solid.

### 4-(5-Amino-1H-[1,2,4]triazol-3-ylamino)-2-chloro-benzonitrile (compound 12)

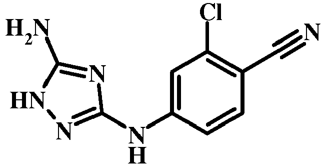

A solution of (Z)-methyl N-3-chloro-4-cyanophenyl-N'-cyanocarbamimidothioate (109 mg, 435 µmol, Eq: 1.00) and hydrazine (143 mg, 140 µL, 4.46 mmol, Eq: 10.3) in ethanol (3 mL) was heated at 65°C for 2 hr. The reaction mixture was cooled to room temperature and filtered to give 75 mg (75%) of desired product as a white solid.
MS m/z 235 [M+H]

### Example 13

### N*3*-(4-Chloro-3-nitro-phenyl)-1H-[1,2,4]triazole-3,5-diamine (compound 13)

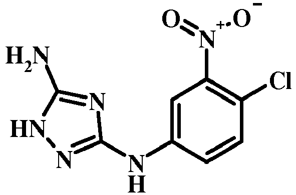

### (Z)-methyl N-4-chloro-3-nitrophenyl-N'-cyanocarbamimidothioate

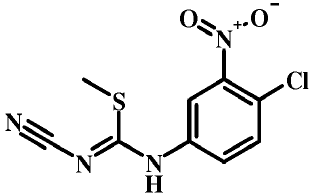

A solution of sodium methoxide (3.4 ml, 1.7 mmol, Eq: 1.22) was added to cyanamide (65 mg, 1.55 mmol, Eq: 1.11) and stirred at room temperature for 15 minutes. 1-chloro-4-isothiocyanato-2-nitrobenzene (300 mg, 1.4 mmol, Eq: 1.00) was added to the reaction mixture and stirred for 1 hr. Iodomethane (436 mg, 192 µl, 3.08 mmol, Eq: 2.2) was added and the yellow solution was stirred overnight at room temperature. The resulting suspension was filtered to give 163 mg (43%) of desired product as a light brown solid.

### N*3*-(4-Chloro-3-nitro-phenyl)-1H-[1,2,4]triazole-3,5-diamine (compound 13)

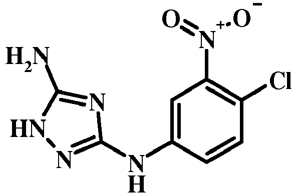

A solution of (Z)-methyl N-4-chloro-3-nitrophenyl-N'-cyanocarbamimidothioate (163 mg, 602 µmol, Eq: 1.00) and hydrazine (194 mg, 190 µl, 6.05 mmol, Eq: 10.1) in ethanol (5 mL) was heated at 65°C. After 3h, LCMS ok. Cooled to rt and stirred yellow slurry over weekend. The suspension was filtered and rinsed with cold methanol to give 89 mg (58%) of desired product as a yellow solid.
¹H NMR (300MHz, DMSO) δ: 11.38 (s, 1H), 9.46 (s, 1H), 8.34 (d, J = 3 Hz, 1H), 7.64-7.60 (m, 2H ), 6.04 (s, 2H) ppm

### Example 14

### N*3*-(3-Chloro-4-phenylethynyl-phenyl)-1H-[1,2,4]triazote-3,5-diamine (compound 14)

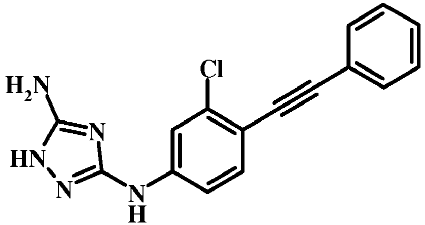

### 2-chloro-4-nitro-1-(phenylethynyl)benzene

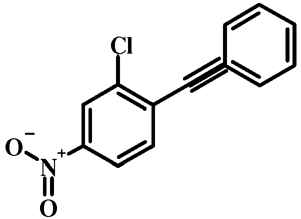

Bromo-2-chloro-4-nitrobenzene (2 g, 8.46 mmol, Eq: 1.00), ethynylbenzene (1.86 g, 2 ml, 18.2 mmol, Eq: 2.15), copper(I) iodide (332 mg, 1.74 mmol, Eq: 0.206) and bis(triphenylphosphine)palladium (II) chloride (602 mg, 858 µmol, Eq: 0.101) were placed in a round-bottom flask. THF (20 mL)/diisopropylamine (4 mL) was added to the flask and the reaction mixture was heated at 60 overnight. The reaction was concentrated and chromatographed (150 Analogix, 100% hex to 3% EtOAc/hex) to give 1.24 g (57%) of desired product as a brown solid.

### 3-chloro-4-(phenylethynyl)aniline

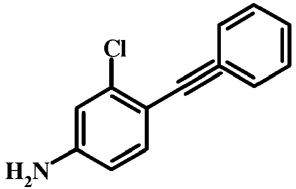

A solution of 2-chloro-4-nitro-1-(phenylethynyl)benzene (2.133 g, 8.28 mmol, Eq: 1.00), iron (2.31 g, 41.4 mmol, Eq: 5) and ammonium chloride (4.43 g, 82.8 mmol, Eq: 10) in methanol (10 mL)/water (5 mL) was heated at reflux o/n. The reaction mixture was filtered over Celite, diluted with ethyl acetate, washed with brine 3x, dried over sodium sulfate, and chromatographed (150g Analogix, 20% EtOAc/hex to 24% EtOAc/hex) to give 1.72 g (91%) of desired product as a brown solid.

### 2-chloro-4-isothiocyanato-1-(phenylethynyl)benzene

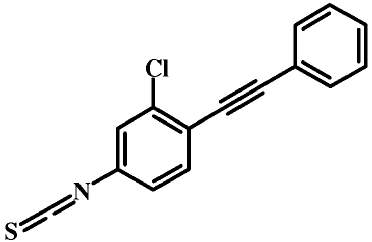

To a suspension of calcium carbonate (1.4 g, 14.0 mmol, Eq: 2.17) and thiophosgene (780 mg, 520 µl, 6.78 mmol, Eq: 1.05) in dichloromethane (13.2 g, 10.0 ml, 155 mmol, Eq: 25.3)/water (10.0 g, 10.0 ml, 555 mmol, Eq: 90.2) at 0, was added 3-chloro-4-(phenylethynyl)aniline (1.47 g, 6.46 mmol, Eq: 1.00) The reaction was gradually warmed to room temperature and stirred overnight. Added 14 mL IN HCl. Separated organic layer and dried over sodium sulfate. Chromatographed (120 g Analogix, 100% hex) to give 1.41 g (81%) of desired product as a white solid.

### (Z)-methyl N-3-chloro-4-(phenylethynyl)phenyl-N'-cyanocarbamimidothioate

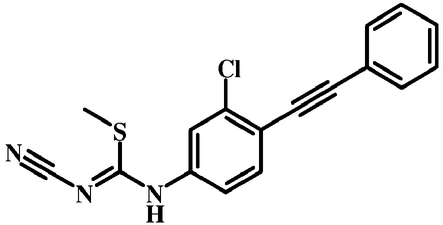

Sodium methoxide (0.5M in methanol) (2.8 ml, 1.4 mmol, Eq: 1.26) was added to cyanamide (51.4 mg, 1.22 mmol, Eq: 1.1). After 15 minutes, the solution was added to a solution of 2-chloro-4-isothiocyanato-1-(phenylethynyl)benzene (300 mg, 1.11 mmol, Eq: 1.00) in methanol (5 mL). After 1 hr, methyl iodide (318 mg, 140 µl, 2.24 mmol, Eq: 2.01) was added and the reaction was stirred overnight at room temperature. The resulting suspension was filtered to give 133 mg (37%) of desired product as a white solid.

### N*3*-(3-Chloro-4-phenylethynyl-phenyl)-1H-[1,2,4]triazole-3,5-diamine (compound 14)

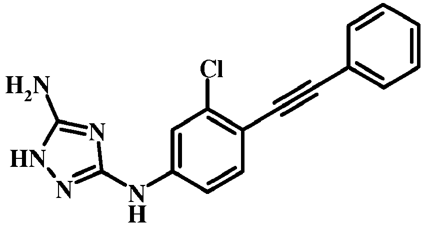

A solution of (Z)-methyl N-3-chloro-4-(phenylethynyl)phenyl-N'-cyanocarbamimidothioate (133 mg, 408 µmol, Eq: 1.00) and hydrazine (133 mg, 130 µl, 4.14 mmol, Eq: 10.1) in ethanol (5 mL) was heated at 70o o/n. The reaction mixture was concentrated and chromatographed (11 g Supelco, 100% DCM to 10% MeOH/DCM) to give 89 mg white solid, containing desired product and impurities. The solid was triturated with methanol and filtered to give 25 mg (20%) of desired product as a white solid.
MS m/z 310 [M+H]

### Example 15

### N*3*-(3-Bromo-phenyl)-1H-[1,2,4]triazole-3,5-diamine (Compound 15)

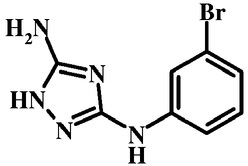

### (Z)-phenyl N-3-bromophenyl-N'-cyanocarbamimidate

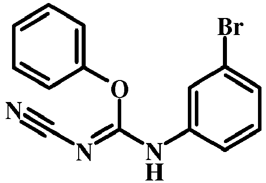

A solution of 3-bromoaniline (198 mg, 125 µl, 1.15 mmol, Eq: 1.00) and diphenyl cyanocarbonimidate (277 mg, 1.16 mmol, Eq: 1.01) in acetonitrile (5 mL) was heated at 50 overnight. The reaction mixture was concentrated and chromatographed (40 g Analogix, 100% hex to 30% EtOAc/hex) to give 119 mg (33%) of desired product as a white solid.

### N*3*-(3-Bromo-phenyl)-1H-[1,2,4]triazole-3,5-diamine (Compound 15)

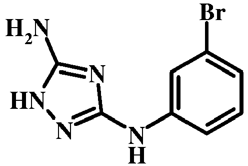

A solution of (Z)-phenyl N-3-bromophenyl-N'-cyanocarbamimidate (119 mg, 376 µmol, Eq: 1.00) and hydrazine (123 mg, 120 µl, 3.82 mmol, Eq: 10.2) in ethanol (5 mL) was heated at 70o o/n. The reaction mixture was concentrated and chromatographed (11 g Supelco, 100% DCM to 10% MeOH/DCM) to give 82 mg (86%) of desired product as a white solid.
MS m/z 254, 256 [M+H]

### Example 16

### 4-(5-Amino-1H-[1,2,4]triazol-3-ylamino)-2-tert-butyl-benzonitrile (Compound 16)

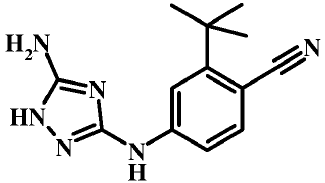

### 2-tert-butyl-4-nitrobenzonitrile

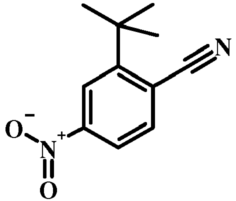

A solution of copper(i) cyanide (677 mg, 7.56 mmol, Eq: 2) in DMSO (5 mL) was heated at 60o for 1 hr. Tert-butyl nitrite (1.56 g, 1.8 ml, 15.1 mmol, Eq: 4) and a solution 2-tert-butyl-4-nitroaniline (734 mg, 3.78 mmol, Eq: 1.00) in DMSO (8 mL) was added and the reaction was stirred for 3 hr. The reaction mixture was poured into ice water and extracted with ethyl acetate 3x. The organic extract was washed with brine, dried over sodium sulfate, and chromatographed (40g Analogix, 100% hex to 10%EtOAc/hex) to give 331 mg (43%) of an orange solid, containing desired product and impurities.

### 4-amino-2-tert-butylbenzonitrile

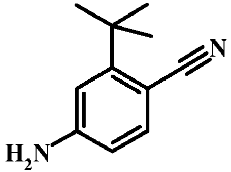

A solution of 2-tert-butyl-4-nitrobenzonitrile (803 mg, 3.93 mmol, Eq: 1.00), iron (1.38 g, 24.7 mmol, Eq: 6.28) and ammonium chloride (2.65 g, 49.5 mmol, Eq: 12.6) in methanol(10 mL)/water (5 mL) was heated at 60oC o/n. The reaction mixture was filtered over Celite, diluted with ethyl acetate, washed with brine 3x, dried over sodium sulfate to give 739 mg of desired product as a brown oil.

### 2-tert-butyl-4-isothiocyanatobenzonitrile

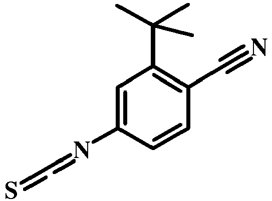

To a suspension of calcium carbonate (1.06 g, 10.6 mmol, Eq: 2.5) and thiophosgene (540 mg, 360 µl, 4.7 mmol, Eq: 1.11) in dichloromethane (15 mL)/water (15 mL) at 0, was added 4-amino-2-tert-butylbenzonitrile (739 mg, 4.24 mmol, Eq: 1.00) The reaction was gradually warmed to room temperature and stirred overnight. Added 10 mL IN HCl. Separated organic layer, dried over sodium sulfate, and chromatographed (40 g Analogix, 100% hex to 10% EtOAc/hex) to give 663 mg (72%) of desired product as a pale yellow oil.

### (Z)-methyl N-3-tert-butyl-4-cyanophenyl-N'-cyanocarbamimidothioate

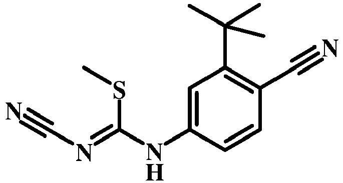

Sodium methoxide (0.5M in methanol) (7.4 ml, 3.7 mmol, Eq: 1.21) was added to cyanamide (148 mg, 3.52 mmol, Eq: 1.15). After 15 minutes, the solution was added to a solution of 2-tert-butyl-4-isothiocyanatobenzonitrile (663 mg, 3.07 mmol, Eq: 1.00) in methanol (5 mL). After 1 hr, methyl iodide (885 mg, 390 µl, 6.24 mmol, Eq: 2.03) was added and the reaction was stirred overnight at room temperature. The reaction mixture was concentrated and chromatographed (40 g Analogix, 10% EtOAc/hex to 25% to 50% EtOAc/hex) to give 274 mg (33%) of desired product as a light yellow solid.

### 4-(5-Amino-1H-[1,2,4]triazol-3-ylamino)-2-tert-butyl-benzonitrile (Compound 16)

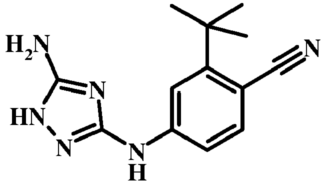

A solution of (Z)-methyl N-3-tert-butyl-4-cyanophenyl-N'-cyanocarbamimidothioate (274 mg, 1.01 mmol, Eq: 1.00) and hydrazine (327 mg, 320 µl, 10.2 mmol, Eq: 10.1) in ethanol (10 mL) was heated at 65°C o/n. The reaction mixture was concentrated and chromatographed (23 Supelco, 100%DCM to 5% to 10% MeOH/DCM to give 124 mg (48%) of desired product as a white solid.
¹H NMR (300MHz, DMSO) δ: 11.21 (s, 1H), 8.98 (s, 1H), 8.01 (d, J = 2 Hz, 1H), 7.55 (dd, J = 9 Hz,3 Hz, 1H ), 7.33 (d, J = 9 Hz ,1H), 5.93 (s, 2H), 1.40 (s, 9H) ppm

### Example 17

### N*3*-(4-Trifluoromethanesulfonyl-phenyl)-1H-[1,2,4]triazole-3,5-diamine (Compound 17)

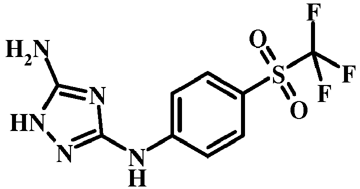

### 1-isothiocyanato-4-(trifluoromethylsulfonyl)benzene

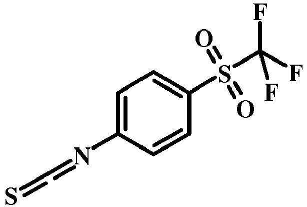

To a suspension of calcium carbonate (940 mg, 9.39 mmol, Eq: 2.52) and thiophosgene (480 mg, 320 µl, 4.17 mmol, Eq: 1.12) in dichloromethane (13.2 g, 10.0 ml, 155 mmol, Eq: 41.7)/water (10.0 g, 10.0 ml, 555 mmol, Eq: 149) at 0, was added 4-(trifluoromethylsulfonyl)aniline (840 mg, 3.73 mmol, Eq: 1.00). The reaction was gradually warmed to room temperature and stirred overnight. Separated organic layer and dried over sodium sulfate. Chromatography (40 g Analogix, 0 to 10% ethyl acetate/hexane) gave 698 mg (70%) of desired product as a white solid.

### (Z)-methyl N'-cyano-N-(4-(trifluoromethylsulfonyl)phenyl)carbamimidothioate

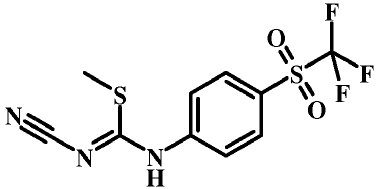

A solution of sodium methoxide (2.7 ml, 1.35 mmol, Eq: 1.2) was added to cyanamide (55 mg, 1.31 mmol, Eq: 1.17) and stirred at room temperature for 15 minutes. 1-isothiocyanato-4-(trifluoromethylsulfonyl)benzene (300 mg, 1.12 mmol, Eq: 1.00) was added to the reaction mixture and stirred for 1 hr. Iodomethane (363 mg, 160 µl, 2.56 mmol, Eq: 2.28) was added and the solution was stirred overnight at room temperature. The reaction mixture was concentrated and chromatographed (24g Analogix, 20 to 50% EtOAc/hex) to give 223 mg (61 %) of desired product as a white solid.

### N*3*-(4-Trifluoromethanesulfonyl-phenyl)-1H-[1,2,4]triazole-3,5-diamine (Compound 17)

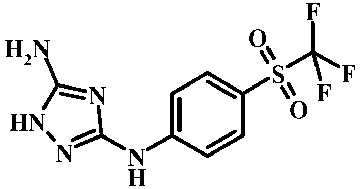

A solution of (Z)-methyl N'-cyano-N-(4-(trifluoromethylsulfonyl)phenyl)carbamimidothioate (223 mg, 690 µmol, Eq: 1.00) and hydrazine (225 mg, 220 µl, 7.01 mmol, Eq: 10.2) in ethanol (5 mL) was heated at 65°C o/n. The reaction mixture was concentrated and chromatographed (0 to 10% MeOH/CH2Cl2, Supelco 11 g) to give 169 mg (80%) of desired product as a light yellow foam. ¹H NMR (300MHz, DMSO) δ: 11.50 (bs, 1H), 10.00 (s, 1H), 7.82 (dd, J = 22 Hz, 9 Hz, 4H), 6.09 (s, 2H) ppm

### Example 18

### N*3*-Naphthalen-2-yl-1H-[1,2,4]triazole-3,5-diamine (Compound 18)

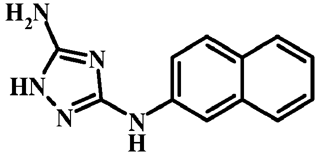

### (Z)-methyl N'-cyano-N-(naphthalen-2-yl)carbamimidothioate

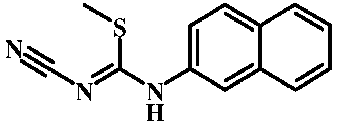

A solution of sodium methoxide (425 mg, 450µl, 1.97 mmol, Eq: 1.22) was added to cyanamide (74.9 mg, 1.78 mmol, Eq: 1.1) in MeOH (3 mL) and stirred at room temperature for 15 minutes. 2-isothiocyanatonaphthalene (300 mg, 1.62 mmol, Eq: 1.00) was added to the reaction mixture and stirred for 1 hr. Iodomethane (460 mg, 203 µl, 3.24 mmol, Eq: 2) was added and the suspension was stirred overnight at room temperature. The resulting white suspension was filtered and dried to give 232 mg (59%) of desired product as a white solid.

### N*3*-Naphthalen-2-yl-1H-[1,2,4]triazole-3,5-diamine (Compound 18)

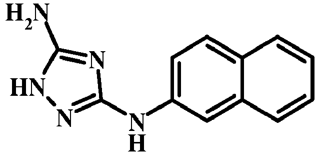

A solution of (Z)-methyl N'-cyano-N-(naphthalen-2-yl)carbamimidothioate (232 mg, 961 µmol, Eq: 1.00) and hydrazine (308 mg, 302 µl, 9.61 mmol, Eq: 10) in ethanol (10 mL) was heated at 65°C o/n. The reaction mixture was concentrated and chromatographed (0 to 10% MeOH/CH2Cl2, Supelco 11 g) to give 162 mg (75%) of desired product as an off-white solid. ¹H NMR (300MHz, DMSO) δ: 11.17 (s, 1H), 8.85 (s, 1H), 8.11 (s, 1H), 7.70-7.65 (m, 2H), 7.60 (d, J = 8 Hz, 1H), 7.47 (dd, J = 9 Hz, 2 Hz, 1H), 7.36 (m, 1H), 7.21 (m, 1H), 5.87 (s, 2H) ppm

### Example 19

### N*3*-(3,5-Di-tert-butyl-phenyl)-1H-[1,2,4]triazole-3,5-diamine (Compound 19)

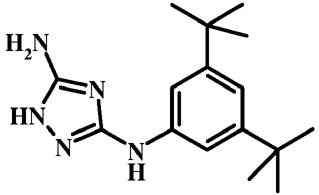

### 1,3-di-tert-butyl-5-isothiocyanatobenzene

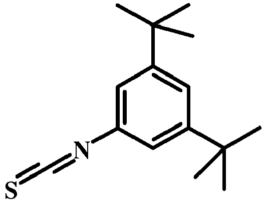

To a suspension of calcium carbonate (1.22 g, 12.2 mmol, Eq: 2.5) and thiophosgene (616 mg, 411 µl, 5.36 mmol, Eq: 1.1) in dichloromethane (13.2 g, 10.0 ml, 155 mmol, Eq: 25.3)/water (10.0 g, 10.0 ml, 555 mmol, Eq: 90.2) at 0, was added 3,5-di-tert-butylaniline (1 g, 4.87 mmol, Eq: 1.00) The reaction was gradually warmed to room temperature and stirred overnight. Added 12 mL IN HCl slowly. Separated organic layer and dried over sodium sulfate. 1.014 g (84%) of desired product as a light yellow solid.

### (Z)-methyl N'-cyano-N-(3,5-di-tert-butylphenyl)carbamimidothioate

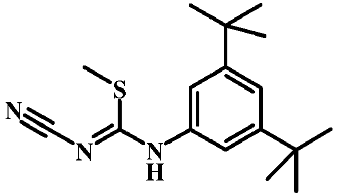

A solution of sodium methoxide (2.91 ml, 1.46 mmol, Eq: 1.2) was added to cyanamide (56.1 mg, 1.33 mmol, Eq: 1.1) and stirred at room temperature for 15 minutes. 1,3-di-tert-butyl-5-isothiocyanatobenzene (300 mg, 1.21 mmol, Eq: 1.00) was added to the reaction mixture and stirred for 1 hr. Iodomethane (344 mg, 152 µl, 2.43 mmol, Eq: 2) was added and the suspension was stirred overnight at room temperature. The resulting suspension was filtered. The filtrate was concentrated and chromatographed (24 g Analogix, 10 to 15% EtOAc/hex) to give 169 mg (46%) of desired product as a white solid.

### N*3*-(3,5-Di-tert-butyl-phenyl)-1H-[1,2,4]triazole-3,5-diamine (Compound 19)

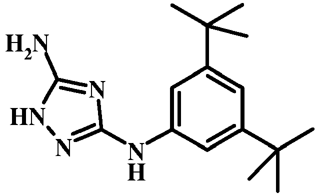

A solution of (Z)-methyl N'-cyano-N-(3,5-di-tert-butylphenyl)carbamimidothioate (255 mg, 840 µmol, Eq: 1.00) and hydrazine (269 mg, 264 µl, 8.4 mmol, Eq: 10) in ethanol (5 mL) was heated at 65°C o/n. The reaction mixture was concentrated and chromatographed (0 to 10% MeOH/CH2Cl2, Supelco 11 g) to give 35 mg (15%) of desired product as a white solid.
¹H NMR (300MHz, DMSO) δ: 11.04 (s, 1H), 8.25 (s, 1H), 7.38 (d, J = 1 Hz, 2H), 5.76 (s, 2H), 1.24 (s, 18H) ppm

### Example 20

### N*3*-(4-Methanesulfonyl-phenyl)-1H-[1,2,4]triazole-3,5-diamine (Compound 20)

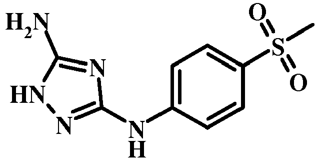

### 1-isothiocyanato-4-(methylsulfonyl)benzene

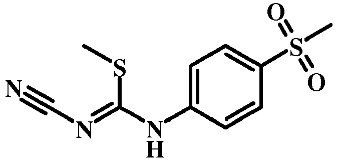

To a suspension of 4-(methylsulfonyl)aniline hydrochloride (1.36 g, 6.55 mmol, Eq: 1.00) in DCM/water, was added at 0, was added calcium carbonate (1.97 g, 19.6 mmol, Eq: 3). After 5 minutes, added thiophosgene (840 mg, 560 µl, 7.31 mmol, Eq: 1.12). The reaction was gradually warmed to room temperature and stirred overnight. Separated organic layer and dried over sodium sulfate to give 1.24 g (89%) of desired product as a white solid.

### (Z)-methyl N'-cyano-N-(4-(methylsulfonyl)phenyl)carbamimidothioate

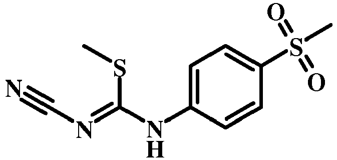

A solution of sodium methoxide (3.5 ml, 1.75 mmol, Eq: 1.24) was added to cyanamide (67 mg, 1.59 mmol, Eq: 1.13) and stirred at room temperature for 15 minutes. 1-isothiocyanato-4-(methylsulfonyl)benzene (300 mg, 1.41 mmol, Eq: 1.00) was added to the reaction mixture and stirred for 1 hr. Iodomethane (454 mg, 200 µl, 3.2 mmol, Eq: 2.27) was added and the pale yellow solution was stirred overnight at room temperature. The resulting suspension was filtered and air dried to give 193 mg (51%) of desired product as a white solid.

### N*3*-(4-Methanesulfonyl-phenyl)-1H-[1,2,4]triazole-3,5-diamine (Compound 20)

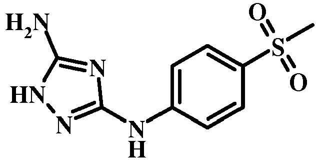

A solution of (Z)-methyl N'-cyano-N-(4-(methylsulfonyl)phenyl)carbamimidothioate (193 mg, 717 µmol, Eq: 1.00) and hydrazine (230 mg, 225 µl, 7.17 mmol, Eq: 10) in ethanol (5 mL) was heated at 65°C o/n. The resulting suspension was filtered and rinsed with cold methanol to give 165 mg (91%) of desired product as a white solid.
¹H NMR (300MHz, DMSO) δ: 11.36 (s, 1H), 9.40 (s, 1H), 7.67 (m, 4H), 6.00 (s, 2H), 3.09 (s, 3H) ppm

### Example 21

### N*3*-(3-Methanesulfonyl-phenyl)-1H-[1,2,4]triazole-3,5-diamine (Compound 21)

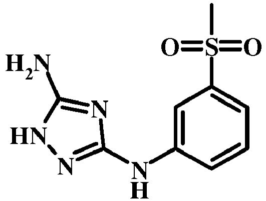

### 1-isothiocyanato-3-(methylsulfonyl)benzene

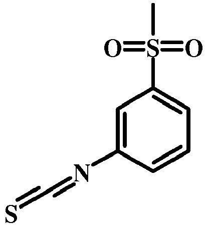

To a suspension of 3-(methylsulfonyl)aniline hydrochloride (1.36 g, 6.55 mmol, Eq: 1.00) in DCM/water, was added at 0, was added calcium carbonate (1.97 g, 19.6 mmol, Eq: 3). After 5 minutes, added thiophosgene (825 mg, 550 µl, 7.18 mmol, Eq: 1.1). The reaction was gradually warmed to room temperature and stirred overnight. Separated organic layer and dried over sodium sulfate to give 1.265 g (91%) of desired product as a light yellow solid.

### (Z)-methyl N'-cyano-N-(3-(methylsulfonyl)phenyl)carbamimidothioate

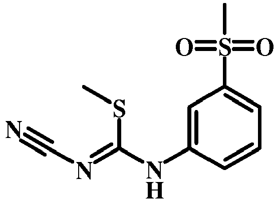

A solution of sodium methoxide (3.5 ml, 1.75 mmol, Eq: 1.24) was added to cyanamide (67.0 mg, 1.59 mmol, Eq: 1.13) and stirred at room temperature for 15 minutes. 1-isothiocyanato-3-(methylsulfonyl)benzene (300 mg, 1.41 mmol, Eq: 1.00) was added to the reaction mixture and stirred for 1 hr. Iodomethane (454 mg, 200 µl, 3.2 mmol, Eq: 2.27) was added and the pale yellow solution was stirred overnight at room temperature. The resulting suspension was filtered and air dried to give 94 mg (25%) of desired product as a white solid.

### N*3*-(3-Methanesulfonyl-phenyl)-1H-[1,2,4]triazole-3,5-diamine (Compound 21)

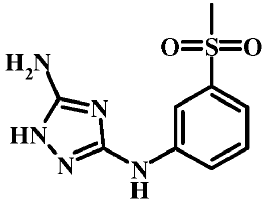

A solution of (Z)-methyl N'-cyano-N-(3-(methylsulfonyl)phenyl)carbamimidothioate (94 mg, 349 µmol, Eq: 1.00) and hydrazine (112 mg, 110 µl, 3.49 mmol, Eq: 10) in ethanol (3 mL) was heated at 65°C. The resulting suspension was filtered and rinsed with cold methanol to give 73 mg (83%) of desired product as a white solid.
¹H NMR (300MHz, DMSO) δ: 11.29 (s, 1H), 9.17 (s, 1H), 8.18 (m, 1H), 7.71 (m, 1H), 7.43 (t, J = 8 Hz, 1H), 7.24 (m, 1H), 5.96 (s, 2H), 3.13 (s, 3H) ppm

### Example 22

### 4-(5-Amino-1H-[1,2,4]triazol-3-ylamino)-N-methyl-benzenesulfonamide (Compound 22)

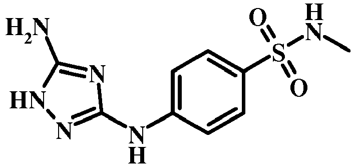

### 4-isothiocyanato-N-methylbenzenesulfonamide

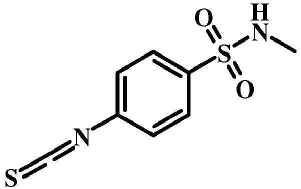

To a suspension of calcium carbonate (1.34 g, 13.4 mmol, Eq: 2.5) and thiophosgene (679 mg, 453 µl, 5.91 mmol, Eq: 1.1) in dichloromethane (13.2 g, 10.0 ml, 155 mmol, Eq: 28.9)/water (10.0 g, 10.0 ml, 555 mmol, Eq: 103) at 0, was added 4-amino-N-methylbenzenesulfonamide (1 g, 5.37 mmol, Eq: 1.00). The reaction was gradually warmed to room temperature. Added IN HCl slowly. Separated organic layer and dried over sodium sulfate to give 1.064 g (87%) of desired product as a white solid.

### (Z)-methyl N'-cyano-N-(4-(N-methylsulfamoyl)phenyl)carbamimidothioate

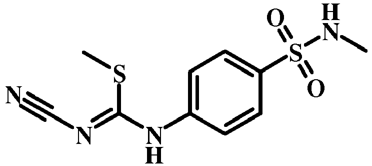

A solution of sodium methoxide (2.63 ml, 1.31 mmol, Eq: 1) was added to cyanamide (55.2 mg, 1.31 mmol, Eq: 1) and stirred at room temperature for 15 minutes. 4-isothiocyanato-N-methylbenzenesulfonamide (300 mg, 1.31 mmol, Eq: 1.00) was added to the reaction mixture and stirred for 1 hr. Iodomethane (188 mg, 83 µl, 1.33 mmol, Eq: 1.01) was added and the solution was stirred overnight at room temperature. The resulting suspension was filtered and air dried to give 238 mg (64%) of desired product as a white solid.

### 4-(5-Amino-1H-[1,2,4]triazol-3-ylamino)-N-methyl-benzenesulfonamide (Compound 22)

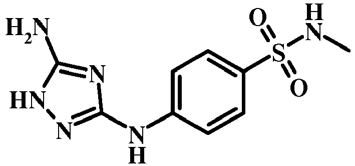

A solution of (Z)-methyl N'-cyano-N-(4-(N-methylsulfamoyl)phenyl)carbamimidothioate (238 mg, 837 µmol, Eq: 1.00) and hydrazine (276 mg, 270 µl, 8.6 mmol, Eq: 10.3) in ethanol (5 mL) was heated at 65°C for 3 hr. The reaction was cooled to rt and stirred overnight. The resulting suspension was filtered to give solid 137 mg (61%) of desired product as a white solid.
¹H NMR (300MHz, DMSO) δ: 11.33 (s, 1H), 9.27 (s, 1H), 7.58 (dd, J = 20 Hz, 11 Hz, 4H), 7.08 (q, J = 5 Hz, 1H), 5.97 (s, 2H), 2.50 (d, J= 2 Hz, 3H) ppm

### Example 23

### [4-(5-Amino-1H-[1,2,4]triazol-3-ylamino)-phenyl]-morpholin-4-yl-methanone (Compound 23)

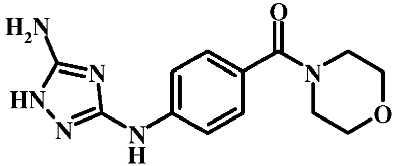

### (4-isothiocyanatophenyl)(morpholino)methanone

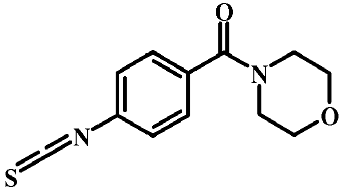

To a suspension of calcium carbonate (1.21 g, 12.1 mmol, Eq: 2.5) and thiophosgene (615 mg, 410 µL, 5.35 mmol, Eq: 1.1) in dichloromethane (13.2 g, 10 mL, 155 mmol, Eq: 32.1)/water (10.0 g, 10 mL, 555 mmol, Eq: 114) at 0, was added (4-aminophenyl)(morpholino)methanone (1 g, 4.85 mmol, Eq: 1.00). The reaction was gradually warmed to room temperature and stirred overnight. Separated organic layer and dried over sodium sulfate. Chromatography (40 g Analogix, 50% ethyl acetate/hexane) gave 1.014 g (84%) of desired product as a white solid.

### (Z)-methyl N'-cyano-N-(4-(morpholine-4-carbonyl)phenyl)carbamimidothioate

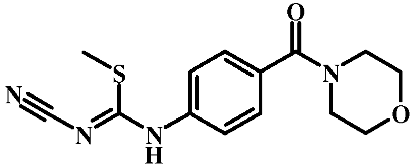

A solution of sodium methoxide (2.9 ml, 1.45 mmol, Eq: 1.2) was added to cyanamide (57 mg, 1.36 mmol, Eq: 1.12) and stirred at room temperature for 15 minutes. (4-isothiocyanatophenyl)(morpholino)methanone (300 mg, 1.21 mmol, Eq: 1.00) was added to the reaction mixture and stirred for 1 hr. Iodomethane (386 mg, 170 µl, 2.72 mmol, Eq: 2.25) was added and the solution was stirred overnight at room temperature. The resulting suspension was filtered and dried to give 201 mg (55%) of desired product as a white solid.

### [4-(5-Amino-1H-[1,2,4]triazol-3-ylamino)-phenyl]-morpholin-4-yl-methanone (Compound 23)

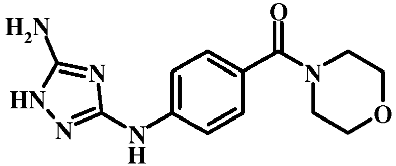

A suspension of (Z)-methyl N'-cyano-N-(4-(morpholine-4-carbonyl)phenyl)carbamimidothioate (201 mg, 660 µmol, Eq: 1.00) and hydrazine (214 mg, 210 µl, 6.69 mmol, Eq: 10.1) in ethanol (10 mL) was heated at 65°C o/n. The resulting suspension was filtered to give 164 mg (86%) of desired product as a white solid.
¹H NMR (300MHz, DMSO) δ: 11.23 (s, 1H), 8.96 (s, 1H), 7.39 (dd, J = 80 Hz, 9 Hz, 4H), 5.91 (s, 2H), 3.58-3.49 (m, 8H) ppm

### Example 24

### 4-(5-Amino-1H-[1,2,4]triazol-3-ylamino)-N,N-dimethyl-benzamide (Compound 24)

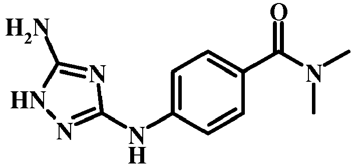

### 4-isothiocyanato-N,N-dimethylbenzamide

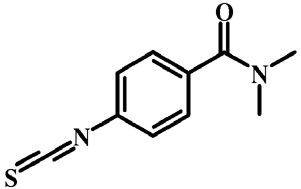

To a suspension of calcium carbonate (1.53 g, 15.3 mmol, Eq: 2.49) and thiophosgene (787 mg, 525 µl, 6.85 mmol, Eq: 1.11) in dichloromethane (13.2 g, 10.0 ml, 155 mmol, Eq: 25.3)/water (10.0 g, 10.0 ml, 555 mmol, Eq: 90.2) at 0, was added 4-amino-N,N-dimethylbenzamide (1.01 g, 6.15 mmol, Eq: 1.00). The reaction was gradually warmed to room temperature and stirred overnight. Added 15 mL IN HCl slowly. Separated organic layer and dried over sodium sulfate. Chromatography (40 g Analogix, 50% ethyl acetate/hexane) gave 1.125 g (89%) of desired product as a colorless oil.

### (Z)-methyl N'-cyano-N-(4-(dimethylcarbamoyl)phenyl)carbamimidothioate

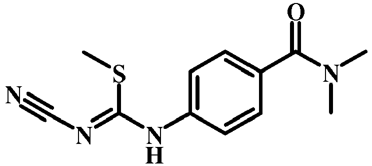

A solution of sodium methoxide (3.5 ml, 1.75 mmol, Eq: 1.2) was added to cyanamide (67 mg, 1.59 mmol, Eq: 1.1) and stirred at room temperature for 15 minutes. 4-isothiocyanato-N,N-dimethylbenzamide (300 mg, 1.45 mmol, Eq: 1.00) was added to the reaction mixture and stirred for 1 hr. Iodomethane (420 mg, 185 µl, 2.96 mmol, Eq: 2.03) was added and the suspension was stirred overnight at room temperature. The resulting suspension was concentrated and chromatographed (50% EtOAc/hex to 100% EtOAc) to give 179 mg (47%) of desired product as a white solid.

### 4-(5-Amino-1H-[1,2,4]triazol-3-ylamino)-N,N-dimethyl-benzamide (Compound 24)

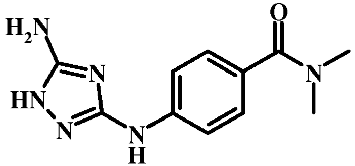

A suspension of (Z)-methyl N'-cyano-N-(4-(dimethylcarbamoyl)phenyl)carbamimidothioate (179 mg, 682 µmol, Eq: 1.00) and hydrazine (225 mg, 220 µl, 7.01 mmol, Eq: 10.3) in ethanol (5 mL) was heated at 65°C o/n. The resulting suspension was filtered to give 108 mg (64%) of desired product as a white solid.
¹H NMR (300MHz, DMSO) δ: 11.22 (s, 1H), 8.93 (s, 1H), 7.38 (dd, J = 75 Hz, 8 Hz, 4H), 5.91 (s, 2H), 2.95 (s, 6H) ppm

### Example 25

### 4-(5-Amino-1H-[1,2,4]triazol-3-ylamino)-benzenesulfonamide (Compound 25)

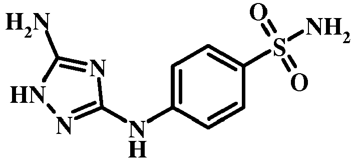

### 4-isothiocyanatobenzenesulfonamide

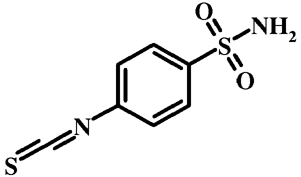

To a suspension of calcium carbonate (1.45 g, 14.5 mmol, Eq: 2.5) and thiophosgene (734 mg, 490 µl, 6.39 mmol, Eq: 1.1) in dichloromethane (13.2 g, 10 ml, 155 mmol, Eq: 26.8)/water (10.0 g, 10 ml, 555 mmol, Eq: 95.6) at 0, was added 4-aminobenzenesulfonamide (1 g, 5.81 mmol, Eq: 1.00). The reaction was gradually warmed to room temperature and stirred overnight. Added 15 mL IN HCl slowly. Separated organic layer, and extracted the aqueous layer with ethyl acetate. Combined organic extracts were dried over sodium sulfate and concentrated to give 1.084 g (87%) of desired product as a white solid.

### (Z)-methyl N'-cyano-N-(4-sulfamoylphenyl)carbamimidothioate

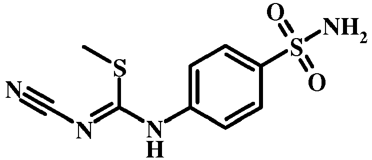

A solution of sodium methoxide (2.8 ml, 1.4 mmol, Eq: 1.00) was added to cyanamide (59 mg, 1.4 mmol, Eq: 1.00) and stirred at room temperature for 15 minutes. 4-isothiocyanatobenzenesulfonamide (300 mg, 1.4 mmol, Eq: 1.00) was added to the reaction mixture and stirred for 1 hr. Iodomethane (204 mg, 90 µl, 1.44 mmol, Eq: 1.03) was added and the suspension was stirred overnight at room temperature. The resulting suspension was filtered and air dried to give 252 mg (67%) of desired product as a white solid.

### 4-(5-Amino-1H-[1,2,4]triazol-3-ylamino)-benzenesulfonamide (Compound 25)

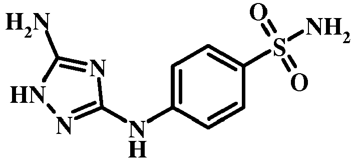

A suspension of (Z)-methyl N'-cyano-N-(4-sulfamoylphenyl)carbamimidothioate (252 mg, 932 µmol, Eq: 1.00) and hydrazine (306 mg, 300 µl, 9.56 mmol, Eq: 10.3) in ethanol (7 mL) was heated at 65°C o/n. The resulting suspension was filtered solid 192 mg (81%) of desired product as a white solid.
¹H NMR (300MHz, DMSO) δ: 11.32 (s, 1H), 9.21 (s, 1H), 7.61 (m, 4H), 7.04 (bs, 1H), 5.95 (s, 2H) ppm

### Example 26

### 4-(5-Amino-1H-[1,2,4]triazol-3-ylamino)-N,N-dimethyl-benzenesulfonamide (Compound 26)

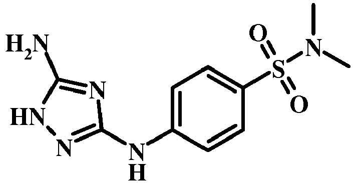

### 4-isothiocyanato-N,N-dimethylbenzenesulfonamide

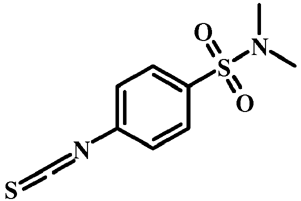

To a suspension of calcium carbonate (960 mg, 9.59 mmol, Eq: 2.5) and thiophosgene (488 mg, 325 µl, 4.24 mmol, Eq: 1.11) in dichloromethane (13.2 g, 10.0 ml, 155 mmol, Eq: 25.3)/water (10.0 g, 10.0 ml, 555 mmol, Eq: 90.2) at 0, was added 4-amino-N,N-dimethylbenzenesulfonamide (767 mg, 3.83 mmol, Eq: 1.00) The reaction was gradually warmed to room temperature and stirred overnight. Added 9 mL IN HCl slowly. Separated organic layer and dried over sodium sulfate to give 807 mg (87%) of desired product as an off-white solid.

### N'-cyano-N-(4-(N,N-dimethylsulfamoyl)phenyl)carbamimidothioate

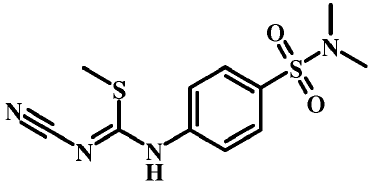

A solution of sodium methoxide (3 ml, 1.5 mmol, Eq: 1.21) was added to cyanamide (58 mg, 1.38 mmol, Eq: 1.11) and stirred at room temperature for 15 minutes. 4-isothiocyanato-N,N-dimethylbenzenesulfonamide (300 mg, 1.24 mmol, Eq: 1.00) was added to the reaction mixture and stirred for 1 hr. Iodomethane (363 mg, 160 µl, 2.56 mmol, Eq: 2.07) was added and the suspension was stirred overnight at room temperature. Filtered white suspension and dried to give 113 mg (31 %) of desired product as a white solid.

### 4-(5-Amino-1H-[1,2,4]triazol-3-ylamino)-N,N-dimethyl-benzenesulfonamide (Compound 26)

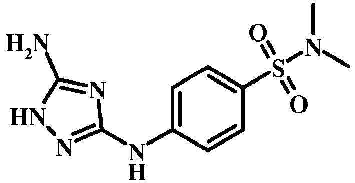

A solution of (Z)-methyl N'-cyano-N-(4-(N,N-dimethylsulfamoyl)phenyl)carbamimidothioate (113 mg, 379 µmol, Eq: 1.00) and hydrazine (121 mg, 119 µl, 3.79 mmol, Eq: 10) in ethanol (5 mL) was heated at 65°C o/n. The reaction mixture was concentrated, triturated with dichloromethane, and filtered to give 94 mg (88%) of desired product as an off-white solid.
¹H NMR (300MHz, DMSO) δ: 11.31 (s, 1H), 9.31 (s, 1H), 7.59 (dd, J = 41 Hz, 9 Hz, 4H), 5.92 (s, 2H), 2.53 (s, 6H) ppm

### Example 27

### N*3*-Methyl-N*3*-(3,4,5-trichloro-phenyl)-1H-[1,2,4]triazole-3,5-diamine (Compound 27)

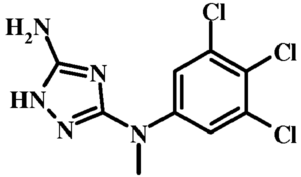

### (Z)-methyl N'-cyano-N-methyl-N-(3,4,5-trichlorophenyl)carbamimidothioate

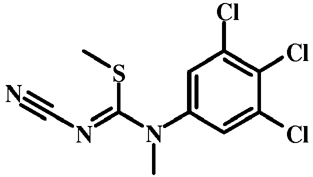

To a solution of (Z)-methyl N'-cyano-N-(3,4,5-trichlorophenyl)carbamimidothioate (107 mg, 363 µmol, Eq: 1.00) in DMF (5 mL) at 0°C, was added NaH (60% 29 mg, 0.725 mmol, Eq: 2.00). After 15 minutes, methyl iodide (114 mg, 50 µL, 800 µmol, Eq: 2.2) was added and the reaction was gradually warmed to room temperature overnight. The reaction mixture was diluted with EtOAc, washed with IN HCl, water, brine and dried over sodium sulfate. Chromatography (24 g Analogix, 10 to 20% EtOAc/hex) gave 38 mg (34%) of desired product as a white solid.

### N*3*-Methyl-N*3*-(3,4,5-trichloro-phenyl)-1H-[1,2,4]triazole-3,5-diamine (Compound 27)

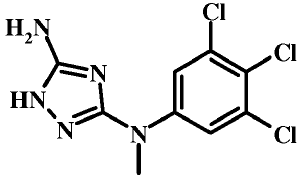

A solution of (Z)-methyl N'-cyano-N-methyl-N-(3,4,5-trichlorophenyl)carbamimidothioate (38 mg, 123 µmol, Eq: 1.00) and hydrazine (40.8 mg, 40 µl, 1.27 mmol, Eq: 10.4) in ethanol (3 mL) was heated at 70o o/n. The reaction mixture was concentrated and chromatographed (11 g Supelco, 100% DCM to 10% MeOH/DCM) to give 26 mg (72%) of desired product as a white solid. MS m/z 292, 294 [M+H]

### Example 28

### N*3*-(3,5-Dichloro-phenyl)-N*3*-methyl-1H-[1,2,4]triazole-3,5-diamine (Compound 28)

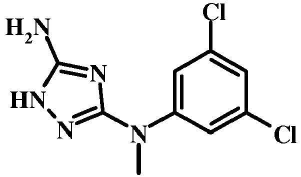

### (Z)-methyl N'-cyano-N-(3,5-dichlorophenyl)carbamimidothioate

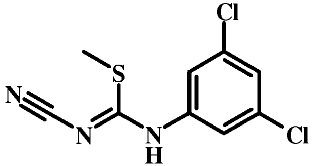

Sodium methoxide (0.5M in methanol) (23.5 ml, 11.8 mmol, Eq: 1.2) was added to cyanamide (459 mg, 10.9 mmol, Eq: 1.11). After 15 minutes, the solution was added to a solution of 1,3-dichloro-5-isothiocyanatobenzene (2 g, 9.8 mmol, Eq: 1.00) in methanol (5 mL). After 1 hr, methyl iodide (2.78 g, 1.23 ml, 19.6 mmol, Eq: 2) was added and the reaction was stirred overnight at room temperature. The resulting suspension was filtered and dried to give 1.193 g of desired product as a white solid. The filtrate was concentrated and chromatographed (115 g Analogix, 20% EtOAc/hex to 40% EtOAc/hex) to give an additional 512 mg of desired product as a white solid. The solids were combined to give a total of 1.705 g (67%) of desired product.

### (Z)-methyl N'-cyano-N-(3,5-dichlorophenyl)-N-methylcarbamimidothioate

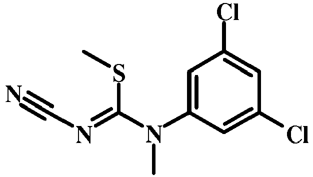

To a solution of (Z)-methyl N'-cyano-N-(3,5-dichlorophenyl)carbamimidothioate (249 mg, 957 µmol, Eq: 1.00) in DMF (5 mL) at 0oC, was added NaH (60%, 80 mg, 2.00 mmol, Eq: 2.09). After 15 minutes, methyl iodide (272 mg, 120 µl, 1.91 mmol, Eq: 2) was added and the reaction was gradually warmed to room temperature overnight. The reaction mixture was diluted with EtOAc, washed with IN HCl, brine (twice) and dried over sodium sulfate. Chromatography (40 g Analogix, 10 to 20% EtOAc/hex) gave 93 mg (35%) of desired product as a white solid.

### N*3*-(3,5-Dichloro-phenyl)-N*3*-methyl-1H-[1,2,4]triazole-3,5-diamine (Compound 28)

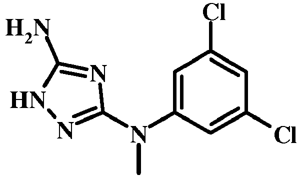

A solution of (Z)-methyl N'-cyano-N-(3,5-dichlorophenyl)-N-methylcarbamimidothioate (93 mg, 339 µmol, Eq: 1.00) and hydrazine (112 mg, 110 µl, 3.5 mmol, Eq: 10.3) in ethanol (5 mL) was heated at 65° o/n. The reaction mixture was concentrated and chromatographed (11 g Supelco, 100% DCM to 10% MeOH/DCM) to give 66 mg (75%) of desired product as a white solid.
MS m/z 258, 260 [M+H]

### Example 29

### N*3*-Benzyl-N*3*-(3,5-dichloro-phenyl)-1H-[1,2,4]triazole-3,5-diamine (Compound 29)

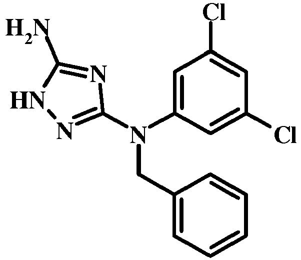

### (Z)-methyl N-benzyl-N'-cyano-N-(3,5-dichlorophenyl)carbamimidothioate

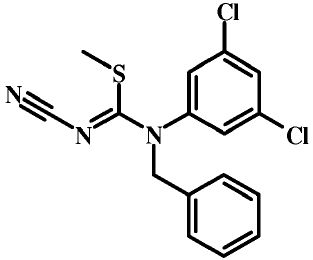

To a solution of (Z)-methyl N'-cyano-N-(3,5-dichlorophenyl)carbamimidothioate (250 mg, 961 µmol, Eq: 1.00) in DMF (5 mL) at 0oC, was added NaH (60%, 77 mg, 1.93 mmol, Eq: 2.00). After 15 minutes, (bromomethyl)benzene (331 mg, 230 µl, 1.94 mmol, Eq: 2.02) was added and the reaction was gradually warmed to room temperature overnight. The reaction mixture was diluted with EtOAc, washed with IN HCl, water, brine and dried over sodium sulfate. Chromatography (40 g Analogix, 5 to 20% EtOAc/hex) gave 120 mg (36%) of desired product as a colorless oil.

### N*3*-Benzyl-N*3*-(3,5-dichloro-phenyl)-1H-[1,2,4]triazole-3,5-diamine (Compound 29)

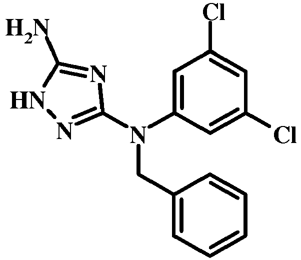

A solution of (Z)-methyl N-benzyl-N'-cyano-N-(3,5-dichlorophenyl)carbamimidothioate (120 mg, 343 µmol, Eq: 1.00) and hydrazine (112 mg, 110 µl, 3.5 mmol, Eq: 10.2) in ethanol (5 mL) was heated at 65° o/n. The reaction mixture was concentrated and chromatographed (11 g Supelco, 100% DCM to 10% MeOH/DCM) to give 82 mg (72%) of desired product as an off-white solid.
MS m/z 334, 336 [M+H]

### Example 30

### [(5-Amino-1H-[1,2,4]triazol-3-yl)-(3,5-dichloro-phenyl)-amino]-acetonitrile (Compound 30)

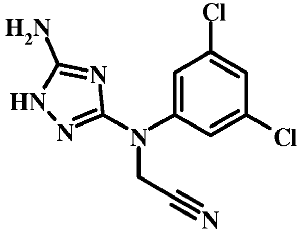

### (Z)-methyl N'-cyano-N-(cyanomethyl)-N-(3,5-dichlorophenyl)carbamimidothioate

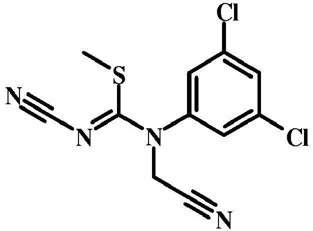

To a solution of (Z)-methyl N'-cyano-N-(3,5-dichlorophenyl)carbamimidothioate (250 mg, 961 µmol, Eq: 1.00) in DMF (5 mL) at 0oC, was added NaH (60% 82 mg, 2.05 mmol, Eq: 2.13). After 15 minutes, 2-bromoacetonitrile (232 mg, 135 µl, 1.94 mmol, Eq: 2.02) was added and the reaction was gradually warmed to room temperature overnight. The reaction mixture was diluted with EtOAc, washed with IN HCl, brine x 2 and dried over sodium sulfate. Chromatography (40 g Analogix, 10 to 20% EtOAc/hex) gave 32 mg (11%) of desired product as a colorless oil.

### [(5-Amino-1H-[1,2,4]triazol-3-yl)-(3,5-dichloro-phenyl)-amino]-acetonitrile (Compound 30)

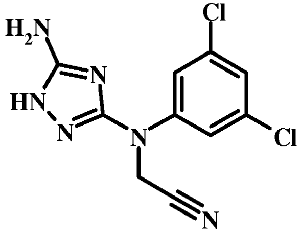

A solution of (Z)-methyl N'-cyano-N-(cyanomethyl)-N-(3,5-dichlorophenyl)carbamimidothioate (32 mg, 107 µmol, Eq: 1.00) and hydrazine (4.08 mg, 4 µl, 127 µmol, Eq: 1.19) in ethanol (3 mL) was heated at 65° o/n. The reaction mixture was concentrated and chromatographed (11 g Supelco, 100% DCM to 10% MeOH/DCM) to give 61 mg (33%) of desired product as a white solid.
MS m/z 283, 285 [M+H]

### Example 31

### 3-(5-Amino-1H-[1,2,4]triazol-3-ylamino)-benzoic acid (Compound 31)

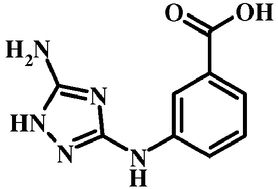

### methyl 3-(3-((3,5-dimethyl-1H-pyrazol-1-yl)(imino)methyl)thioureido)benzoate

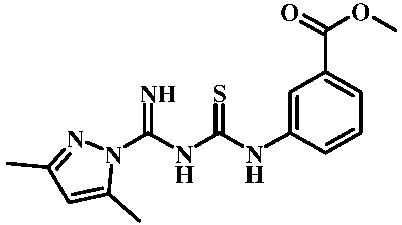

To a suspension of methyl 3-isothiocyanatobenzoate (300 mg, 1.55 mmol, Eq: 1.00) and 3,5-dimethyl-1H-pyrazole-1-carboxamidine nitrate (321 mg, 1.6 mmol, Eq: 1.03) in DMF (5 mL) at 0°, was added powdered potassium hydroxide (100 mg, 1.78 mmol, Eq: 1.15). The reaction mixture was heated at 60 deg o/n. The reaction mixture was diluted with ethyl acetate, washed with IN HCl, brine, and dried over sodium sulfate. Chromatography (60g Analogix, 40 to 60% EtOAc/hex) gave 312 mg (61%) of desired product as a light yellow solid.

### methyl 3-(5-amino-1H-1,2,4-triazol-3-ylamino)benzoate

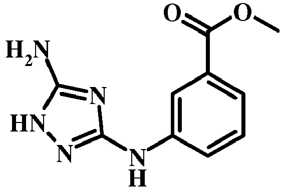

To a solution of methyl 3-(3-((3,5-dimethyl-1H-pyrazol-1-yl)(imino)methyl)thioureido)benzoate (312 mg, 941 µmol, Eq: 1.00) in methanol (10 mL) was added hydrazine (306 mg, 300 µL, 9.56 mmol, Eq: 10.2) . The reaction mixture was heated at 60 deg o/n. The reaction mixture was concentrated and chromatographed (23 Supelco,10% MeOH/DCM) to give 131 mg (60%) of desired product as a white foamy solid.

### 3-(5-Amino-1H-[1,2,4]triazol-3-ylamino)-benzoic acid (Compound 31)

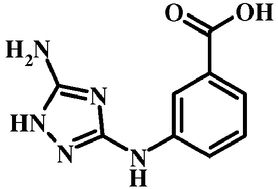

To a solution of methyl 3-(5-amino-1H-1,2,4-triazol-3-ylamino)benzoate (131 mg, 562 µmol, Eq: 1.00) in methanol/THF/water (1:1:1, 6 mL) was added lithium hydroxide (45 mg, 1.88 mmol, Eq: 3.35) . The reaction mixture was heated at 60 deg o/n. The reaction was concentrated to dryness. Added 2 mL IN HCl to give gray suspension, which was filtered to give 106 mg (86%) of desired product as a gray solid.
MS m/z 220 [M+H]

### Example 32

### N*3*-[3-tert-Butylsulfanyl-5-(2-methyl-propane-2-sulfinyl)-phenyl]-1H-[1,2,4]triazole-3,5-diamine (Compound 32)

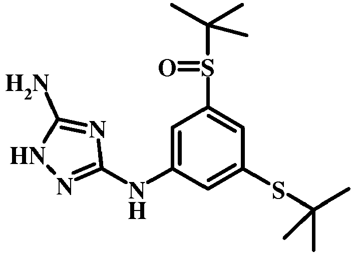

### 1-tert-Butylsulfanyl-3-(2-methyl-propane-2-sulfinyl)-5-nitro-benzene

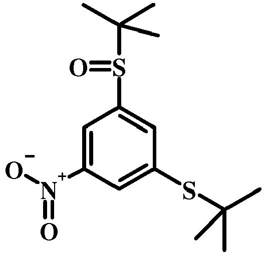

To a solution of (5-nitro-1,3-phenylene)bis((trifluoromethyl)sulfane) prepare by Synthesis( 1975), (11), 721-723 (912 mg, 2.82 mmol, Eq: 1.00) in DCM (20 mL) at 0°C, was added mCPBA (1.9 g, 8.46 mmol, Eq: 3). The orange reaction mixture was gradually warmed to room temperature and stirred overnight. Diluted with DCM, washed with IN NaOH solution, dried org extract over sodium sulfate, and chromatographed (80 g Analogix 100% hex to 10% EtOAc/hex) to give 250 mg (26%) of desired product as a light yellow solid.

### 3-tert-Butylsulfanyl-5-(2-methyl-propane-2-sulfinyl)-phenylamine

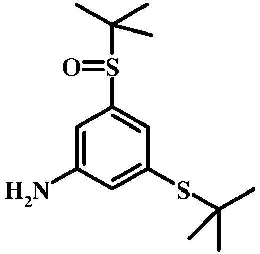

A solution of 1-tert-Butylsulfanyl-3-(2-methyl-propane-2-sulfinyl)-5-nitro-benzene (250 mg, 737 µmol, Eq: 1.00), iron (180 mg, 3.23 mmol, Eq: 4.4) and ammonium chloride (351 mg, 6.56 mmol, Eq: 8.9) in methanol (10 mL) / water (5 mL) was heated at 70°C o/n. The reaction mixture was filtered over Celite, concentrated, diluted with ethyl acetate, washed with brine 3x, and dried over sodium sulfate to give 235 mg of desired product as an orange oil.

### 1-tert-Butylsulfanyl-3-isothiocyanato-5-(2-methyl-propane-2-sulfinyl)-benzene

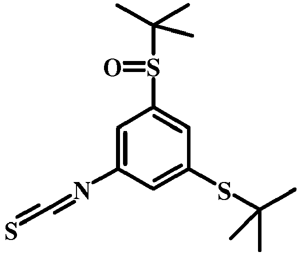

10/18 6:45 p.m. To a suspension of calcium carbonate (165 mg, 1.64 mmol, Eq: 2.16) and thiophosgene (105 mg, 70 (µl, 913 µmol, Eq: 1.20) in dichloromethane (13.2 g, 10.0 ml, 155 mmol, Eq: 25.3)/water (10.0 g, 10.0 ml, 555 mmol, Eq: 90.2) at 0, was added 3-tert-Butylsulfanyl-5-(2-methyl-propane-2-sulfinyl)-phenylamine (235 mg, 761 µmol, Eq: 1.00) The reaction was gradually warmed to room temperature and stirred overnight. Added 2 mL IN HCl. Separated organic layer and dried over sodium sulfate to give 223 mg (83%) of desired product as a pale yellow oil.

### (Z)-methyl N'-cyano-N-(cyanomethyl)-N-(3-tert-Butylsulfanyl-5-(2-methyl-propane-2-sulfinyl)-phenyl)carbamimidothioate

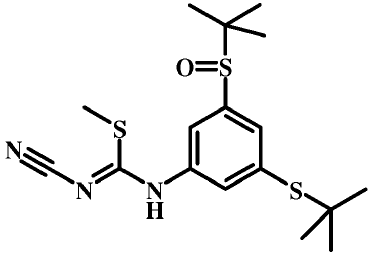

Sodium methoxide (0.5M in methanol) (1.5 ml, 750 µmol, Eq: 1.34) was added to cyanamide (28 mg, 666 µmol, Eq: 1.19). After 15 minutes, the solution was added to a solution of 1-tert-Butylsulfanyl-3-isothiocyanato-5-(2-methyl-propane-2-sulfinyl)-benzene (223 mg, 635 µmol, Eq: 1.00) in methanol (5 mL). After 1 hr, methyl iodide (170 mg, 75 µl, 1.2 mmol, Eq: 1.89) was added and the reaction was stirred overnight at room temperature. The reaction mixture was concentrated and chromatographed (24g Analogix, 10 to 25% EtOAc/hex) to give 108 mg (42%) of desired product as an off-white solid.

### N*3*-[3-tert-Butylsulfanyl-5-(2-methyl-propane-2-sulfinyl)-phenyl]-1H-[1,2,4]triazole-3,5-diamine (Compound 32)

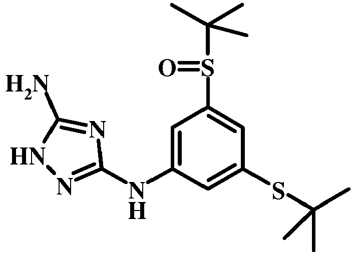

To a solution of [Reactants] (108 mg,0.264 mmol, Eq: 1.0) in methanol (5 mL) was added hydrazine (81.7 mg, 80 µl, 2.55 mmol, Eq: 9.6). The reaction mixture was heated at 60 deg o/n. The reaction was cooled to room temp and poured into ice water. Diluted with ethyl acetate and washed with IN HCl, brine. Dried over sodium sulfate and chromatographed (60g Analogix, 40 to 60% EtOAc/hex) to give 103 mg (88%) of desired product as an off-white foamy solid.
MS m/z 392 [M+H]

### Example 33

### N-[3-(5-Amino-1H-[1,2,4]triazol-3-ylamino)-phenyl]-methanesulfonamide (Compound 33)

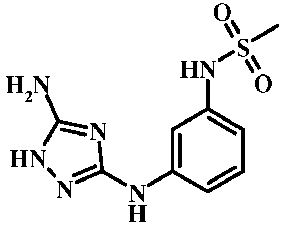

### N-(3-nitrophenyl)methane sulfonamide

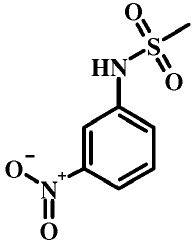

To a solution of 3-nitroaniline (2 g, 14.5 mmol, Eq: 1.00) and pyridine (1.76 g, 1.8 mL, 22.3 mmol, Eq: 1.54) in dichloromethane (30 mL) at 0oC was added methanesulfonyl chloride (1.91 g, 1.3 mL, 16.7 mmol, Eq: 1.15). The reaction mixture was gradually warmed to room temperature overnight. The reaction mixture was diluted with DCM. Added IN HCl to give an off-white precipitate. The organic layer was separated and discarded. The aqueous layer was extracted with ethyl acetate and dried over sodium sulfate to give 2.6 g (83%) of desired product as an off-white solid.

### N-(3-aminophenyl)methanesulfonamide

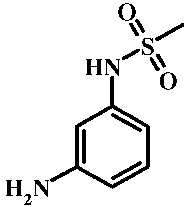

A solution of N-(3-nitrophenyl)methanesulfonamide (2.6 g, 12.0 mmol, Eq: 1.00), iron (3.38 g, 60.5 mmol, Eq: 5.03) and ammonium chloride (6.48 g, 121 mmol, Eq: 10.1) in methanol (40 mL)/water (20 mL) was heated at 70°C o/n. The reaction mixture was filtered over Celite. The filtrate was concentrated, diluted with ethyl acetate, washed with brine 2x, dried over sodium sulfate, and chromatographed (115g Analogix, 20 to 40% EtOAc/hex) to give 1.55 g (69%) of desired product as an orange solid.

### N-(3-isothiocyanatophenyl)methanesulfonamide

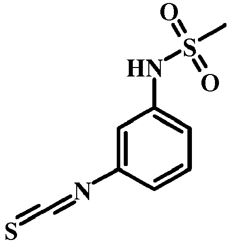

To a solution of N-(3-aminophenyl)methanesulfonamide (.5 g, 2.68 mmol, Eq: 1.00) in DCM (10 mL) at 0oC, was added di(1H-imidazol-1-yl)methanethione (574 mg, 3.22 mmol, Eq: 1.2). The reaction mixture was gradually warmed to room temperature overnight. The reaction mixture was concentrated and chromatographed (40g Analogix, 10% to 30% EtOAc/hex) to give 557 mg (91%) of desired product as a white solid.

### N-(3-(cyanamido(methylthio)methylamino)phenyl)methanesulfonamide

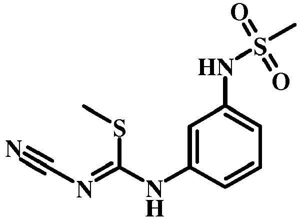

Sodium methoxide (0.5M in methanol) (3 ml, 1.5 mmol, Eq: 1.24) was added to cyanamide (55.9 mg, 1.33 mmol, Eq: 1.1). After 15 minutes, the solution was added to a solution of N-(3-isothiocyanatophenyl)methanesulfonamide (276 mg, 1.21 mmol, Eq: 1.00) in methanol (5 mL). After 1 hr, methyl iodide (182 mg, 80 µl, 1.28 mmol, Eq: 1.06) was added and the reaction was stirred overnight at room temperature. The reaction mixture was concentrated and chromatographed (40g Analogix, 50 to 75% EtOAc/hex) to give 197 mg(57%) of desired product as a pale yellow oil.

### N-[3-(5-Amino-1H-[1,2,4]triazol-3-ylamino)-phenyl]-methanesulfonamide (Compound 33)

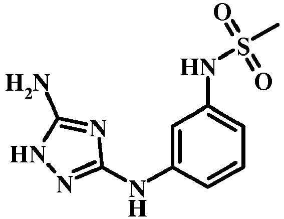

To a solution of N-(3-(cyanamido(methylthio)methylamino)phenyl)methanesulfonamide (197 mg, 688 µmol, Eq: 1.00) in methanol (5 mL) was added hydrazine (225 mg, 220 µl, 7.01 mmol, Eq: 10.2) . The reaction mixture was heated at 60 deg o/n. The reaction mixture was concentrated and chromatographed (11g Supelco, 10% MeOH/DCM) to give 95 mg (52%) of desired product as an off-white solid. MS m/z 269 [M+H]

### Example 34

### 4-(5-Amino-1H-[1,2,4]triazol-3-ylamino)-2-trifluoromethanesulfonyl-benzonitrile (Compound 34)

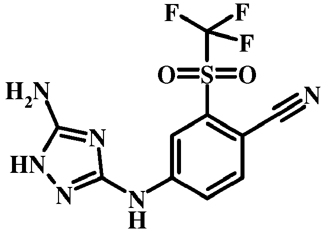

### 4-Bromo-3-trifluoromethanesulfonyl-phenylamine; 2-Bromo-3-trifluoromethanesulfonyl-phenylamine

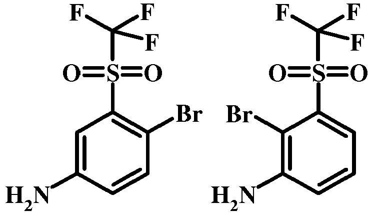

To a solution of 3-(trifluoromethylsulfonyl)aniline (2 g, 8.88 mmol, Eq: 1.00) in DMF (40 mL), was added NBS (1.72 g, 9.66 mmol, Eq: 1.09). The reaction was gradually warmed to room temp and stirred overnight. The reaction was diluted with ethyl acetate and washed with brine 3x. The organic extract was dried over sodium sulfate and chromatographed (80g Redisep, 10 to 30% EtOAc/hex) to give 1.963 g (73%) orange solid, containing a 1:1 mixture of region-isomeric aniline products. The mixture was carried onto the subsequent step.

### N-(4-bromo-3-(trifluoromethylsulfonyl)phenyl)acetamide

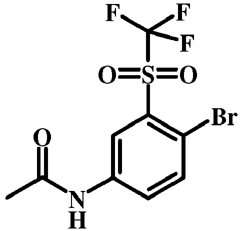

To a solution containing a 1:1 mixture of 4-Bromo-3-trifluoromethanesulfonyl-phenylamine and 2-bromo-3-(trifluoromethylsulfonyl)aniline (1.96 g, 6.45 mmol, Eq: 1) in EtOH (10 mL), was added Ac2O (812 mg, 750 µl, 7.95 mmol, Eq: 1.23). The reaction was stirred at room temp overnight. The reaction mixture was concentrated and chromatographed (120 Silicycle, 10 to 40% EtOAc/hex) to give 1.08 g (48%) of desired product as a yellow solid, and 763 mg of the unreacted region-isomeric aniline starting material.

### N-(4-cyano-3-(trifluoromethylsulfonyl)phenyl)acetamide

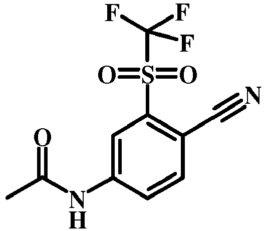

A solution of N-(4-bromo-3-(trifluoromethylsulfonyl)phenyl)acetamide (500 mg, 1.44 mmol, Eq: 1.00) and copper (I) cyanide (200 mg, 2.23 mmol, Eq: 1.55) in NMP (7.5 mL) was heated at 200 with microwave for 1 hr. The reaction was cooled to room temp and poured into ice water. The suspension was extracted with ethyl acetate. The organic extract was washed with brine 2x, dried over sodium sulfate, and chromatographed (40g Redisep, 10 to 50% EtOAc/hex) to give 307 mg (73%) of desired product as a pale yellow foamy oil.

### 4-amino-2-(trifluoromethylsulfonyl)benzonitrile

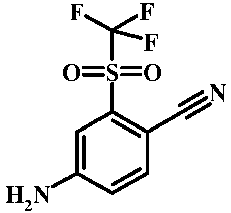

A solution of N-(4-cyano-3-(trifluoromethylsulfonyl)phenyl)acetamide (367 mg, 1.26 mmol, Eq: 1.00) and HCl (5.04 g, 4.2 ml, 25.2 mmol, Eq: 20.1) in ethanol (5 mL) was heated at 65 deg o/n. The reaction mixture was concentrated, dissolved in EtOAc, washed with 25 mL IN NaOH and dried over sodium sulfate to give 271 mg (86%) of desired product as a yellow solid.

### 4-isothiocyanato-2-(trifluoromethylsulfonyl)benzonitrile

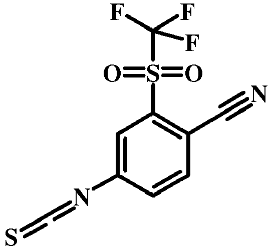

A suspension of 4-amino-2-(trifluoromethylsulfonyl)benzonitrile (271 mg, 1.08 mmol, Eq: 1.00), thiophosgene (495 mg, 330 µl, 4.31 mmol, Eq: 3.97), triethylamine (436 mg, 600 µl, 4.3 mmol, Eq: 3.97) in benzene (84.6 mg, 10.0 ml, 1.08 mmol, Eq: 1.00) was heated at reflux overnight. The brown reaction mixture was concentrated, diluted with DCM, washed with IN HCl (5mL) and brine, dried over sodium sulfate. 0.5g crude was chromatographed (40 g Redisep, 0 to 10 ethyl acetate/hexane) to give 211mg (67%) of desired product as a light yellow oil.

### N-((4-cyano-3-(trifluoromethylsulfonyl)phenylamino)(methylthio)methyl)cyanamide

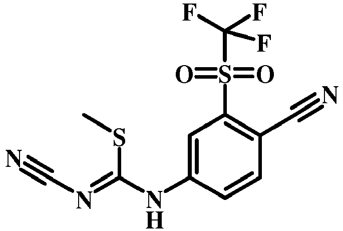

To a solution of 4-isothiocyanato-2-(trifluoromethylsulfonyl)benzonitrile (211 mg, 722 µmol, Eq: 1.00) in MeOH (4 mL) was added to sodium hydrogen cyanamide (52 mg, 812 µmol, Eq: 1.13). After 30 minutes, methyl iodide (216 mg, 95 µl, 1.52 mmol, Eq: 2.1) was added and the reaction was stirred overnight at room temperature. The reaction mixture was concentrated and chromatographed (24g Redisep, 25% to 75% EtOAc/hex) to give 138 mg (55%) of desired product as a yellow solid.

### 4-(5-Amino-1H-[1,2,4]triazol-3-ylamino)-2-trifluoromethanesulfonyl-benzonitrile (Compound 34)

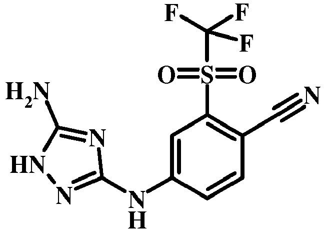

To a solution of N-((4-cyano-3-(trifluoromethylsulfonyl)phenylamino)(methylthio)methyl)cyanamide (138 mg, 394 µmol, Eq: 1.00) in ethanol (5 mL) was added hydrazine (126 mg, 124 µl, 3.94 mmol, Eq: 10). The reaction mixture was heated at 60 deg o/n. The reaction mixture was concentrated and chromatographed (11g Supelco, 0 to 10% MeOH/DCM) to give 106 mg (81%) of desired product as a yellow solid. MS m/z 333 [M+H]

### Example 35

### 4-(5-Amino-1H-1,2,4-triazol-3-ylamino)-2-cyclopropylbenzonitrile (Compound 35)

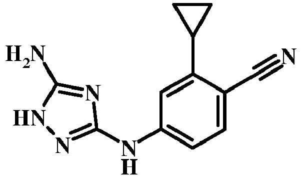

### 2-Cyclopropyl-4-nitrobenzonitrile

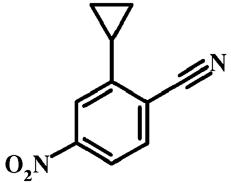

In a Teflon-capped reaction tube, 2-chloro-4-nitrobenzonitrile (450 mg, 2.46 mmol), 2-dicyclohexylphosphino-2',4',6'-triisopropylbiphenyl (X-phos, 118 mg, 0.246 mmol), cyclopropylboronic acid (800 mg, 9.31 mmol), potassium carbonate (1.02 g, 7.39 mmol) and palladium (II) acetate (28 mg, 0.125 mmol) were combined with THF (10 mL) and water (1 mL) to give a light brown suspension. The reaction tube was sealed, and the reaction mixture was heated at 75 °C for 16 hours. After this time, reverse-phase HPLC shows approximately a 2:1 ratio of starting material to desired product. The reaction mixture was cooled to room temperature and partitioned between ethyl acetate and water. The organic phase was dried over Na₂SO₄, filtered, and concentrated. The crude product was combined with crude fractions from small scale (60 mg) versions of the same reaction. This combined crude product was dissolved in CH₂Cl₂ and this mixture was concentrated over celite. The celite supported crude product was loaded onto a 80 gram SiliCycle column. Flash chromatography (10% ethyl acetate-hexanes) was used to isolate 2-cyclopropyl-4-nitrobenzonitrile.

### 4-Amino-2-cyclopropylbenzonitrile

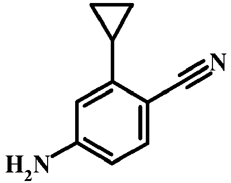

In a 250 mL round-bottomed flask, 2-cyclopropyl-4-nitrobenzonitrile (220 mg, 1.17 mmol, Eq: 1.00), iron (326 mg, 5.85 mmol, Eq: 5.00) and ammonium chloride (625 mg, 11.7 mmol, Eq: 10.00) were combined with methanol (10 mL) to give a grey suspension. Water (2.0 mL) was added. The reaction mixture was heated at 50 °C for 8 hours. After this time, the reaction mixture was cooled to room temperature and filtered to remove the iron. The filtrate was concentrated to remove the methanol. The resulting mixture was partitioned between water and ethyl acetate, and the organic layer was dried (Na₂SO₄), filtered, and concentrated to an oily yellow solid. ¹H NMR indicates the presence of more than one product, and reverse-phase HPLC shows three clear bands (one band was starting material). The crude product was again dissolved in 10 mL of methanol and 5 mL of water. Iron (326 mg, 5.85 mmol, Eq: 5.00) was added followed by ammonium chloride (625 mg, 11.7 mmol). The reaction mixture was heated at 75 °C for another 5 hours. After this time, reverse-phase HPLC indicated one main product, with no remaining starting material. The reaction mixture was worked-up again as described above to provide 4-amino-2-cyclopropylbenzonitrile (59 mg, 29%) as an off-white oily solid isolated at 90% purity. The crude product was used in the subsequent step without further purification.

### 2-Cyclopropyl-4-isothiocyanatobenzonitrile

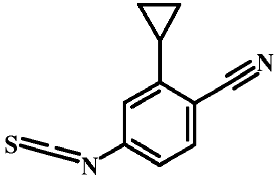

In a 50 mL round-bottomed flask, 4-amino-2-cyclopropylbenzonitrile (115 mg, 727 µmol, Eq: 1.00) and 1,1'-thiocarbonyldiimidazole (259 mg, 1.45 mmol, Eq: 2.0) were combined with CH₂Cl₂ (2.5 mL) to give a light brown solution. The reaction mixture was stirred at room temperature over 12 hours. After this time, the reaction mixture was concentrated over silica gel, and the silica-gel supported crude product was loaded onto a 40 g Analogix column. Flash chromatography (5% ethyl acetate-hexanes) afforded 2-cyclopropyl-4-isothiocyanatobenzonitrile (20 mg, 14%).

### (Z)-methyl N'-cyano-N-(4-cyano-3-cyclopropylphenyl)carbamimidothioate

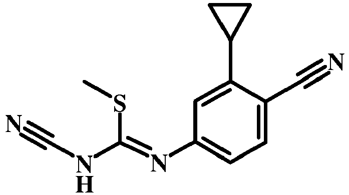

In a 25 mL round-bottomed flask, cyanamide (12.6 mg, 300 µmol, Eq: 3.0) and a 0.5 M solution of sodium methoxide in methanol (300 µL, 150 µmol, Eq: 1.5) were combined to give a colorless solution. After stirring at room temperature for 15 minutes, this mixture was added to a solution of 2-cyclopropyl-4-isothiocyanatobenzonitrile (20 mg, 99.9 µmol, Eq: 1.00) in methanol. This solution was stirred at room temperature for 1 hour, then methyl iodide (9.37 µL, 150 µmol, Eq: 1.5) was added. The reaction mixture was stirred at room temperature overnight. In the morning, the product had precipitated out of solution as a white solid. The product was collected via vacuum filtration to provide (Z)-methyl N'-cyano-N-(4-cyano-3-cyclopropylphenyl)carbamimidothioate (12 mg, 47%).

### 4-(5-Amino-1H-1,2,4-triazol-3-ylamino)-2-cyclopropylbenzonitrile (Compound 35)

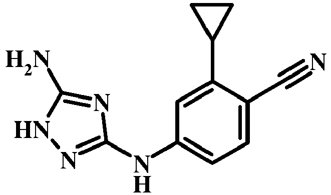

In a 25 mL round-bottomed flask, hydrazine (1.5 mg, 46.8 µmol, Eq: 1.00) and (Z)-methyl N'-cyano-N-(4-cyano-3-cyclopropylphenyl)carbamimidothioate (12 mg, 46.8 µmol, Eq: 1.00) were combined with ethanol (1 mL) to give a colorless solution. The reaction mixture was refluxed for 6 hours, and then cooled to room temperature. The mixture was stirred at room temperature overnight. In the morning, the reaction mixture was concentrated on the rotary evaporator to yield 4-(5-amino-1H-1,2,4-triazol-3-ylamino)-2-cyclopropylbenzonitrile (11 mg, 98%) as a white powder. ¹H NMR (300 MHz, DMSO-*d*₆) δ ppm 11.35 (br. s., 1 H), 9.23 (s, 1 H), 7.49 (d, *J*=8.60 Hz, 1 H), 7.40 (dd, *J*=8.60, 2.40 Hz, 1 H), 7.22 (d, *J*=1.51 Hz, 1 H), 5.97 (br. s., 2 H), 1.01 - 1.12 (m, 3 H), 0.69 (dd, *J*=4.71, 1.88 Hz, 2 H).

### Example 36

### N*3*-(3-Chloro-phenyl)-1H-[1,2,4]triazole-3,5-diamine (Compound 36)

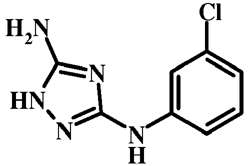

### (Z)-methyl N-3-chlorophenyl-N'-cyanocarbamimidothioate

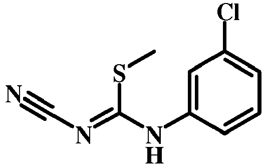

Charged cyanamide (145.9 mg, 3.47 mmol, Eq: 1.18) into a 50-mL round-bottomed flask while purging with argon. Added 0.5 M sodium methoxide in methanol (7.0 mL, 3.5 mmol, Eq: 1.19) at room temperature. Stirred four ~15 min, then added 3-chlorophenyl isothiocyanate (500 mg, 0.390 mL, 2.95 mmol, Eq: 1.00; from Aldrich) at room temperature. Stirred for 25 min and monitored by HPLC. Added iodomethane (613 mg, 0.270 mL, 4.32 mmol, Eq: 1.47) at room temperature. After 20 min white solids crashed out. After 3 h, cooled the reaction mixture in an ice bath, then filtered off the solids and rinsed with a small amount of cold methanol. Obtained 390 mg (58.6%) of product as a white solid. MS calcd. for C₉H₈ClN₃S [(M+H)⁺] 226, obsd. 226.2

### N*3*-(3-Chloro-phenyl)-1H-[1,2,4]triazole-3,5-diamine (Compound 36)

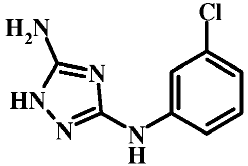

Charged (Z)-methyl N-3-chlorophenyl-N'-cyanocarbamimidothioate (390 mg, 1.73 mmol, Eq: 1.00) to a 2-neck, 50-mL round-bottomed flask fitted with a vigroux column. Added ethanol (15 mL) and stirred to make a slurry. After adding hydrazine (551 mg, 0.54 mL, 17.2 mmol, Eq: 9.96) the solids all dissolved at room temperature. Heated the mixture to 65 °C. After 1 h the reaction was complete according LC/MS. Cooled reaction mixture to room temperature, then removed the solvent *in vacuo*. Obtained a semi-solid material, which contained a large amount of ethanol according to ¹H NMR. Using methanol and dichloromethane, solids crashed out from the semi-solids. Dried in vacuum oven at 60 °C overnight. Obtained 325.6 mg (90%) of N*3*-(3-Chloro-phenyl)-1H-[1,2,4]triazole-3,5-diamine as a white solid. MS calcd. for C₈H-₈ClN₅ [(M+H)⁺] 210.1, obsd. 210.3

### Example 37

### N*3*-(4-Benzyloxy-3-chloro-phenyl)-1H-[1,2,4]triazole-3,5-diamine (Compound 37)

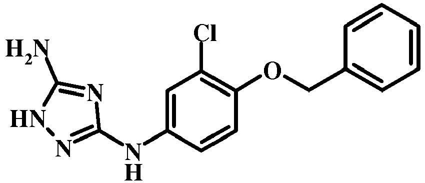

### 1-(benzyloxy)-2-chloro-4-isothiocyanatobenzene

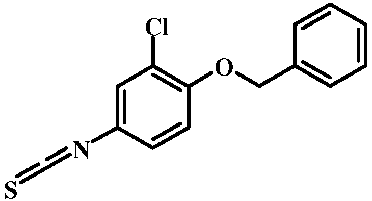

Purged a 50-mL round-bottomed flask with argon. Added 4-(benzyloxy)-3-chloroaniline (603.3 mg, 2.58 mmol, Eq: 1.00; from Aldrich) and dichloromethane (30 mL). Cooled the mixture to 0 °C in an ice bath, then added 1,1'-thiocarbonyldiimidazole (690 mg, 3.87 mmol, Eq: 1.50) in one portion. Removed the ice bath an allowed the reaction mixture to warm to room temperature. Stirred for 1 h, then diluted with dichloromethane and concentrated onto silica. Purified using a 120 g silica gel column on an Intelliflash 280; collected peaks only in 28 mL fractions at 53 mL/min; equilibrated wtih hexanes; dry loaded; eluted for 3 min with hexanes; increased from 0 - 25% dichloromethane/hexanes over 15 min; Held at 25% dichloromethane/hexanes for 4 min. Obtained 620 mg (81%) of 1-(benzyloxy)-2-chloro-4-isothiocyanatobenzene as a white solid.

### (Z)-methyl N-4-(benzyloxy)-3-chlorophenyl-N'-cyanocarbamimidothioate

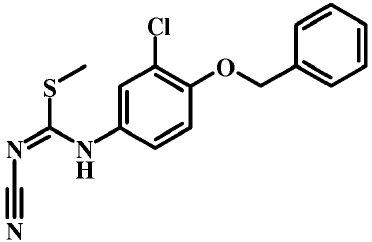

Charged cyanamide (146 mg, 3.48 mmol, Eq: 1.2) into a 50-mL round-bottomed flask while purging with argon. Added 0.5 M sodium methoxide in methanol (6.96 mL, 3.48 mmol, Eq: 1.2) at room temperature, then stirred for 15 min. Meanwhile, purged the 100-mL round-bottomed flask containing 1-(benzyloxy)-2-chloro-4-isothiocyanatobenzene (800 mg, 2.9 mmol, Eq: 1.00) with argon. Added methanol (15 mL) and began stirring. Transferred the cyanamide mixture to the reaction mixture via syringe. The solids dissolved after a short time of stirring. Stirred for a total of 1 h, then added iodomethane (617 mg, 0.272 mL, 4.35 mmol, Eq: 1.5) and stirred overnight at room temperature. Solids crashed out overnight. After 21 h, removed an aliquot and took an HPLC: no starting material remained. Tried to dissolve the solids in various solvents, but nothing worked. Removed all solvents *in vacuo*, and then added cold methanol and filtered off the solids. Obtained 627.5 mg (60.6%) of product as a white solid.

### N*3*-(4-Benzyloxy-3-chloro-phenyl)-1H-[1,2,4]triazole-3,5-diamine (Compound 37)

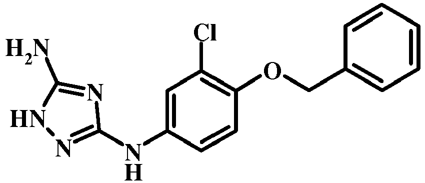

Purged the 2-neck, 100-mL round-bottomed flask, fitted with a vigroux column, containing (Z)-methyl N-4-(benzyloxy)-3-chlorophenyl-N'-cyanocarbamimidothioate (620 mg, 1.87 mmol, Eq: 1.00) with argon. Added ethanol (25 mL) and hydrazine (510 mg, 0.50 mL, 15.9 mmol, Eq: 8.53) and heated the mixture at 75 °C. After ~1 h, removed an aliquot and took an HPLC: no starting material remained. Cooled the reaction to room temperature. LC/MS showed the product mass. Removed ethanol *in vacuo*. dichloromethane was added to the resulting solids and heated briefly. Placed the flask in the freezer for ~2 h, then removed and filtered off the solids. Obtained 485 mg (82%) of N*3*-(4-Benzyloxy-3-chloro-phenyl)-1H-[1,2,4]triazole-3,5-diamine as a white solid. MS calcd. for C₁₅H₁₄ClN₅O [(M+H)⁺] 316.1, obsd. 316.3

### Example 38

### N*3*-(3,5-Dichloro-4-methyl-phenyl)-1H-[1,2,4]triazole-3,5-diamine (Compound 38)

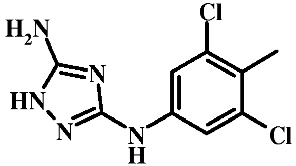

### 3,5-dichloro-4-methylaniline

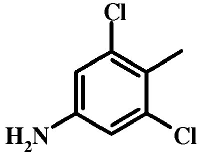

Purged a 50-mL round-bottomed flask with argon. Charged 1,3-dichloro-2-methyl-5-nitrobenzene (500 mg, 2.43 mmol, Eq: 1.00; from Maybridge), iron (678 mg, 12.1 mmol, Eq: 5.0), ammonium chloride (1.3 g, 24.3 mmol, Eq: 10.0), methanol (10 mL), and water (5 mL) while purging with argon. Fitted the reaction flask with a condenser, then sealed under a positive pressure of argon. Heated at reflux for 2 h, then cooled to room temperature. Stirred over weekend at room temperature. HPLC showed the reaction was complete. Filtered the reaction mixture through a bed of celite, rinsing with methanol. Concentrated filtrate. Added water and ethyl acetate, then added solid sodium bicarbonate. Transferred the mixture to a separatory funnel and added more ethyl acetate. Split layers and washed the organic layer with saturated sodium chloride solution. Dried over magnesium sulfate, filtered, and concentrated. Obtained 0.28 g (65.5%) of 3,5-dichloro-4-methylaniline as a yellow solid. The ¹H NMR showed a second, unknown component. Carried to the isothiocyanate.

### 1,3-dichloro-5-isothiocyanato-2-methylbenzene

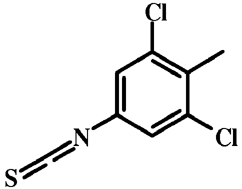

Transferred 3,5-dichloro-4-methylaniline (0.28 g, 1.59 mmol, Eq: 1.00) to a 50-mL round-bottomed flask using dichoromethane (10 mL). Added 1,1'-thiocarbonyldiimidazole (429 mg, 2.41 mmol, Eq: 1.51) and stirred overnight at room temperature. HPLC showed no starting material remaining. Concentrated the reaction mixture onto silica. Purified using a 12 g silica column on an Intelliflash 280; collected peaks only in 9 mL fraction at 32 mL/min; equilibrated with heptane; dry loaded; eluted 1 min with heptane; increased from 0 - 10% dichloromethane/heptane over 5 min. Obtained 226 mg (65%) of 1,3-dichloro-5-isothiocyanato-2-methylbenzene as a yellow solid.

### (Z)-methyl N'-cyano-N-(3,5-dichloro-4-methylphenyl)carbamimidothioate

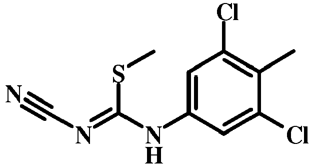

Purged a 50-mL round-bottomed flask with argon. Charged cyanamide (89.0 mg, 2.12 mmol, Eq: 2.03) to the flask while still purging. Added 0.5 M sodium methoxide in methanol (2.72 mL, 1.36 mmol, Eq: 1.3) at room temperature and stirred for ~30 min. Meanwhile, attempted to dissolve the 1,3-dichloro-5-isothiocyanato-2-methylbenzene (228 mg, 1.05 mmol, Eq: 1.00) in methanol (5 mL), but the material appeared to be too non-polar. Added toluene (1 mL) and sonicated. The result was a suspension, which was subsequently added to the cyanamide mixture via syringe. Rinsed the flask containing the starting with methanol (5 mL) and toluene (1 mL) and added it to the reaction mixture. After about 20 min, the solids were still present. Added N,N-dimethylformamide (5 mL) and stirred at room temperature. After 2 h, HPLC confirmed all the starting material was consumed. Added iodomethane (227 mg, 0.10 mL, 1.6 mmol, Eq: 1.53) and stirred at room temperature for 3 d. Concentrated the reaction mixture onto silica. Purified using a 24 g silica gel column on an Intelliflash 280; collected peaks only in 9 mL fractions at 32 mL/min; equilibrated with heptane; dry loaded; eluted 2 min with heptane; increased from 0 - 25% ethyl acetate/heptane over 12 min; held at 25% for 3 min; increased from 25 - 50% ethyl acetate/heptane over 13 min and held at 50% for 11 min. Obtained 377 mg (85.8% yield at 65.2 wt. % purity) of a mixture of (Z)-methyl N'-cyano-N-(3,5-dichloro-4-methylphenyl)carbamimidothioate with a 2:1 N,N-dimethylformamide/product molar ratio.

### N*3*-(3,5-Dichloro-4-methyl-phenyl)-1H-[1,2,4]triazole-3,5-diamine (Compound 38)

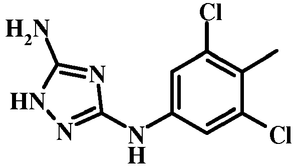

Added ethanol (20 mL) and hydrazine (321 mg, 0.314 mL, 10.0 mmol, Eq: 10.0) to the 250-mL round-bottomed flask containing (Z)-methyl N'-cyano-N-(3,5-dichloro-4-methylphenyl)carbamimidothioate (274 mg, 1.0 mmol, Eq: 1.00). Refluxed for ~1.5 h. HPLC showed all the starting material had been consumed. Cooled to room temperature overnight. Concentrated the reaction mixture. Obtained a waxy solid, which began to slowly solidify. HPLC showed purification was necessary. Tried to dissolve in dichloromethane, but needed methanol to full dissolve. Absorbed onto silica gel for purification: used a 24 g silica gel column on an Intelliflash 280; collected peaks only in 9 mL fractions at 32 mL/min (1 min/CV); equilibrated with 3% methanol/ dichloromethane with 1% ammonium hydroxide; dry loaded; eluted 2 min with 3% methanol/ dichloromethane with 1% ammonium hydroxide; increased from 3 - 10% methanol/ dichloromethane with 1% ammonium hydroxide over 13 min; held at 10% methanol/ dichloromethane with 1% ammonium hydroxide for 4 min. Obtained 147.2 mg (57% yield) of N*3*-(3,5-Dichloro-4-methyl-phenyl)-1H-[1,2,4]triazole-3,5-diamine as a white solid. MS calcd. for C₉H₉Cl₂N₅ [(M+H)⁺] 258.0, obsd. 258.0.

### Example 39

### N*3*-(3,5-Dichloro-4-methyl-phenyl)-1H-[1,2,4]triazole-3,5-diamine (Compound 39)

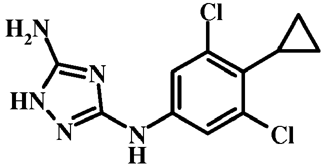

### 1,3-dichloro-2-cyclopropyl-5-nitrobenzene

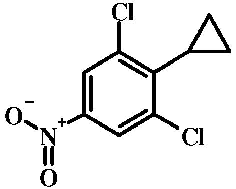

While purging a 50-mL seal tube with argon, added 1,3-dichloro-2-iodo-5-nitrobenzene (1.0 g, 3.15 mmol, Eq: 1.00), potassium cyclopropyltrifluoroborate (584.3 mg, 3.95 mmol, Eq: 1.26), potassium carbonate (1.0 g, 7.24 mmol, Eq: 2.3), bis(triphenylphosphine)palladium(II) chloride (442 mg, 630 µmol, Eq: 0.200), tetrahydrofuran (18 mL), and water (2.0 mL). Sealed the reaction mixture under argon and heated to 105 °C. After 16 h, cooled and carefully removed an aliquot while purging with argon. The HPLC showed starting material remaining. Added more potassium cyclopropyltrifluoroborate (100.0 mg, 0.676 mmol, Eq: 0.215), bis(triphenylphosphine)palladium(II) chloride (220.5 mg, 0.314 mmol, Eq: 0.10), and potassium carbonate (1.00 g, 7.24 mmol, Eq: 2.3) while purging with argon. Heated over weekend at 105 °C. HPLC after 64 h more showed all the starting material had been consumed. Diluted with ethyl acetate and washed with 1 N hydrochloric acid and saturated sodium chloride solution. Dried over magnesium sulfate, filtered, and concentrated onto silica gel. Purified using a 120 g silica gel column on an Intelliflash 280; collected peaks only in 28 mL fractions at 76 mL/min; equilibrated with heptane; dry loaded; eluted 4 min with heptane; increased from 0 - 15% dichlormethane/heptane over 20 min. Obtained 505 mg (69%) of 1,3-dichloro-2-cyclopropyl-5-nitrobenzene as a white waxy solid.

### 3,5-dichloro-4-cyclopropylaniline

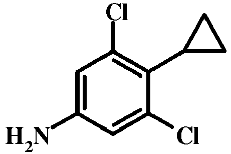

Purged the 50-mL round-bottomed flask containing 1,3-dichloro-2-cyclopropyl-5-nitrobenzene (505 mg, 2.18 mmol, Eq: 1.00) with argon. Added methanol (10 mL), water (5.0 mL), iron (608 mg, 10.9 mmol, Eq: 5.00), and ammonium chloride (1.16 g, 21.8 mmol, Eq: 10.00). Stirred for several minutes at room temperature, then heated at reflux for 2 h; at which point the reaction was complete according to HPLC. Cooled to room temperature and diluted with methanol. Filtered through celite, rinsing with methanol. Concentrated the filtrate and obtained a light yellow solid. Added water and ethyl acetate, then slowly added solid sodium carbonate until pH 8. Split layers and back extracted aqueous layer with ethyl acetate. Combined organics and washed with water and saturated sodium chloride solution. Dried over sodium sulfate, filtered, and concentrated. Obtained 415 mg (91%) of 3,5-dichloro-4-cyclopropylaniline as a yellow solid.

### 1,3-dichloro-2-cyclopropyl-5-isothiocyanatobenzene

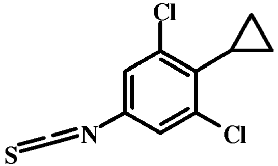

Purged the 50-mL round-bottomed flask containing 3,5-dichloro-4-cyclopropylaniline (415 mg, 2.05 mmol, Eq: 1.00) with argon. Added 1,1'-thiocarbonyldiimidazole (549 mg, 3.08 mmol, Eq: 1.5) and dichloromethane (10 mL) and stirred over weekend at room temperature. HPLC showed all the starting material had been consumed. Concentrated the reaction mixture onto silica gel, then purified using flash chromatography. Used a 24 g silica gel column on an Intelliflash 280; collected peaks only in 9 mL fractions at 32 mL/min; equal with heptane; dry loaded; eluted 2 min with heptane; increased from 0 - 25% dichloromethane/heptane over 10 min. Obtained 252.5 mg (50%) of 1,3-dichloro-2-cyclopropyl-5-isothiocyanatobenzene as an orangish oil.

### (Z)-methyl N'-cyano-N-(3,5-dichloro-4-cyclopropylphenyl)carbamimidothioate

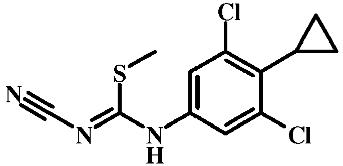

Purged a 50-mL round-bottomed flask with argon. Charged cyanamide (64.6 mg, 1.54 mmol, Eq: 1.5) into the flask, then added 0.5 M sodium methoxide in methanol (2.7 mL, 1.35 mmol, Eq: 1.32) at room temperature. Stirred for ~30 min, then transferred the cyanamide mixture to a mixture of the 1,3-dichloro-2-cyclopropyl-5-isothiocyanatobenzene (250 mg, 1.02 mmol, Eq: 1.00) in methanol (10 mL) and toluene (5.0 mL). After ~2 h, took HPLC which showed the starting material had been consumed. Added iodomethane (295 mg, 0.13 mL, 2.08 mmol, Eq: 2.03) and stirred overnight at room temperature. After 18 h observed solids crashing out. Placed in the freezer for 4 h, then filtered off the solids, rinsing with cold methanol. Obtained 121 mg (39%) clean (Z)-methyl N'-cyano-N-(3,5-dichloro-4-cyclopropylphenyl)carbamimidothioate as a white solid.

### N*3*-(3,5-Dichloro-4-methyl-phenyl)-1H-[1,2,4]triazole-3,5-diamine (Compound 39)

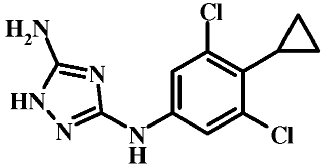

Added ethanol (10 mL) and hydrazine (123 mg, 0.120 mL, 3.82 mmol, Eq: 9.98) to the round-bottomed flask containing (Z)-methyl N'-cyano-N-(3,5-dichloro-4-cyclopropylphenyl)carbamimidothioate (115 mg, 383 µmol, Eq: 1.00). Heated at reflux for ~2 h, then removed an aliquot and took a HPLC: no starting material remained. Cooled the mixture to room temperature. Diluted with methanol and absorbed onto silica gel. Purified using a 12 g silica gel column on an Intelliflash 280; collected peaks only in 9 mL fractions at 32 mL/min (0.5 min/CV); equilibrated with 3% methanol/ dichloromethane with 1% ammonium hydroxide; dry loaded; eluted 1 min with 3% methanol/ dichloromethane with 1% ammonium hydroxide; increased from 3 - 10% methanol/ dichloromethane with 1% ammonium hydroxide over 7.5 min; held at 10% methanol/ dichloromethane with 1% ammonium hydroxide for 2.5 min. Obtained 99.6 mg (91.5%) of N*3*-(3,5-Dichloro-4-methyl-phenyl)-1H-[1,2,4]triazole-3,5-diamine as a light pink solid. MS calcd. for C₁₁H₁₁Cl₂N₅ [(M+H)⁺] 284.0, obsd. 284.0.

### Example 40

### N*3*-(4-Bromo-3-chloro-phenyl)-1H-[1,2,4]triazole-3,5-diamine (Compound 40)

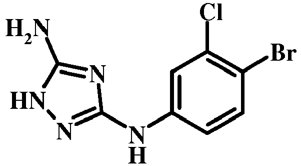

### 1-bromo-2-chloro-4-isothiocyanatobenzene

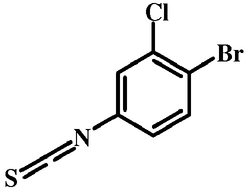

Purged a 100-mL round-bottomed flask with argon. Charged 4-bromo-3-chloroaniline (1.0 g, 4.84 mmol, Eq: 1.00; from Oakwood), dichloromethane (20 mL), and 1,1'-thiocarbonyldiimidazole (1.29 g, 7.27 mmol, Eq: 1.5) to the reaction flask and stirred at room temperature. After 1.5 h, the starting material was consumed according to HPLC. Diluted the mixture with dichloromethane and concentrated onto Celite. Purified using a 40 g silica gel column on an Intelliflash 280; collected peaks only in 28 mL fractions at 53 mL/min; equilibrated with hexanes; dry loaded on Celite; eluted 2 min with hexanes; increased from 0 - 15% dichloromethane/hexanes over 10 min. Obtained 1.096 g (91%) of 1-bromo-2-chloro-4-isothiocyanatobenzene as a clear, colorless oil that solidified to white crystals.

### (Z)-methyl N-4-bromo-3-chlorophenyl-N'-cyanocarbamimidothioate

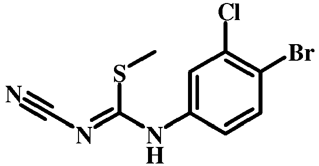

Purged a 25-mL round-bottomed flask with argon. Charged cyanamide (565.4 mg, 13.4 mmol, Eq: 3.05) to the flask, then added 0.5 M sodium methoxide in methanol (13.2 mL, 6.61 mmol, Eq: 1.5). Stirred at room temperature for 15 min. Meanwhile, added methanol (20 mL) to the 100-mL round-bottomed flask containing 1-bromo-2-chloro-4-isothiocyanatobenzene (1.096 g, 4.41 mmol, Eq: 1.00) while purging with argon. Added the cyanamide mixture to the isothiocyanate mixture via syringe. Stirred at room temperature for 1 h. HPLC at this time showed all the starting material consumed. Added iodomethane (1.25 g, 0.551 mL, 8.81 mmol, Eq: 2.00) and stirred overnight at room temperature. In the morning, observed that white solids had precipitated. Filtered off the solids and concentrated the filtrate onto Celite. The solids were pure product according to HPLC; obtained 688 mg (51% yield). The filtrate was purified using a 60 g silica gel column on an Intelliflash 280; collected peaks only in 28 mL fractions at 40 mL/min; equilibrated with 10% ethyl acetate/hexanes; dry loaded on Celite; eluted 2 min with 10% ethyl acetate/hexanes; increased to 60% ethyl acetate/hexanes over 20 min; held at 60% for 21 min. Obtained 324 mg of product according HPLC. Combined with the solids from the filtration for a total of 1.012 g (75% yield) of (Z)-methyl N-4-bromo-3-chlorophenyl-N'-cyanocarbamimidothioate as a white solid.

### N*3*-(4-Bromo-3-chloro-phenyl)-1H-[1,2,4]triazole-3,5-diamine (Compound 40)

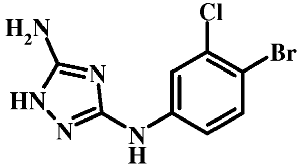

Added ethanol (30 mL) and hydrazine (1.05 g, 1.03 mL, 32.8 mmol, Eq: 10.0) to the 100-mL round-bottomed flask containing (Z)-methyl N-4-bromo-3-chlorophenyl-N'-cyanocarbamimidothioate (1.0 g, 3.28 mmol, Eq: 1.00). Refluxed the mixture for ~2 h. HPLC showed all the starting material was consumed. Cooled to room temperature. Transferred to a larger flask and concentrated onto Celite. Purified using a 60 g silica gel column on an Intelliflash 280; collected peaks only in 28 mL fractions at 40 mL/min (2 min/CV); equilibrated with 5% methanol/ dichloromethane with 1% ammonium hydroxide; dry loaded on Celite; eluted 2 min with 5% methanol/ dichloromethane with 1% ammonium hydroxide; increased from 5 - 10% methanol/ dichloromethane with 1% ammonium hydroxide over 22 min; held at 10% for 6 min. Obtained 807.5 mg (85.2 % yield) of N*3*-(4-Bromo-3-chloro-phenyl)-1H-[1,2,4]triazole-3,5-diamine (Joe Intermediate A) as an off-white solid after removing all the methanol in a 70 °C drying pistol overnight. MS calcd. for C8H7BrC1N5 [(M+H)+] 288.0, obsd. 287.9.

### Example 41

### 3-Amino-5-(3,5-Dichloroanilino)-S-triazole (Compound41)

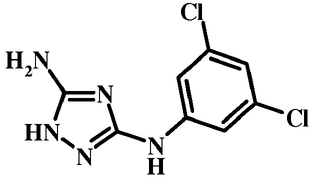

### 1-Cyano-3-(3,5-dichlorophenyl)-2-methyl-2-thiopseudourea

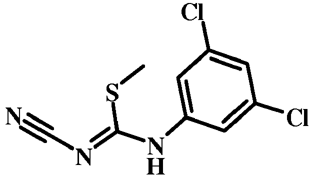

To a solution of 1,3-dichloro-5-isothiocyanatobenzene (10 g, 49.0 mmol, Adrich) in acetonitrile (90 ml) and MeOH (100 ml) was added a suspension of sodium hydrogencyanamide (3.45 g, 53.9 mmol) in MeOH (30 ml). The reaction was stirred at room temperature for 1 hour and methyl iodide (13.9 g, 6.13 ml, 98.0 mmol) was added. Continued stirring for 4 hours, and the reaction mixture was filtered, and washed with acetonitrile to give a white solid as desired product (8.04g, 63% yield).

### 3-Amino-5-(3,5-dichloroanilino)-s-triazole (Compound 41)

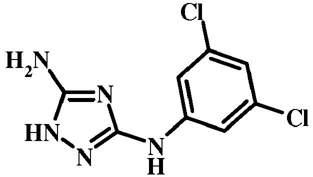

A solution of (Z)-methyl N'-cyano-N-(3,5-dichlorophenyl)carbamimidothioate (8 g, 30.8 mmol) and hydrazine (9.85 g, 9.65 ml, 308 mmol) in Ethanol (120 ml) was stirred at 70 °C for 3 h. It was concentrated to a small volume and water was added to give a suspension. The suspension was filtered, washed with water to give a white solid as desired product (7.44g, 99% yield). MS +m/z: 244.0 (M+H)⁺

### Example 42

### N3-(4-bromo-3-chloro-5-(trifluoromethyl)phenyl)-1H-1,2,4-triazole-3,5-diamine (Compound 42)

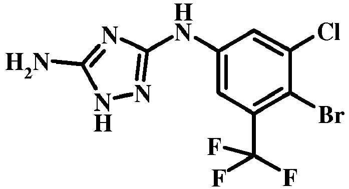

### 2-bromo-1-chloro-5-isothiocyanato-3-trifluoromethyl-benzene

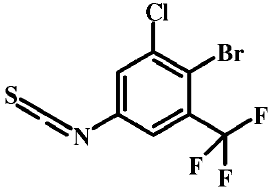

To a suspension of 4-bromo-3-chloro-5-(trifluoromethyl)aniline (15 g, 54.7 mmol, APAC) in CH2Cl2 (200 ml) was added 1,1'-thiocarbonyldiimidazole(11.7 g, 65.6 mmol) at 0 °C. The reaction was stirred cold for 30 minutes and then at RT overnight. The reaction mixture was concentrated and the crude material was purified by flash chromatography (silica gel, 220g, 5% to 15% DCM/Hexanes) to give a yellow oil as desired product (12.3g, 71% yield).

### (Z)-methyl N-4-bromo-3-chloro-5-(trifluoromethyl)phenyl-N'-cyanocarbamimidothioate

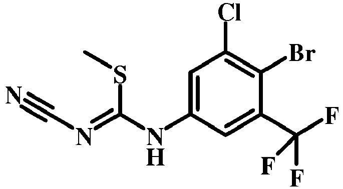

To a solution of 2-bromo-1-chloro-5-isothiocyanato-3-(trifluoromethyl)benzene (11.9 g, 37.6 mmol) in DME (100 ml) was added sodium hydrogencyanamide (3.61 g, 56.4 mmol) followed by MeOH (30 ml). The reaction was stirred at room temperature for 1.5 hours, methyl iodide (10.7 g, 75.2 mmol) was added and stirring was continued for 2 hours. The reaction mixture was concentrated to a small volume, added acetonitrile (20 ml) and water (100 ml) to give a suspension. The suspension was filtered to give a white solid as desired product (12.54g, 85% yield).

### N3-(4-bromo-3-chloro-5-(trifluoromethyl)phenyl)-1H-1,2,4-triazole-3,5-diamine

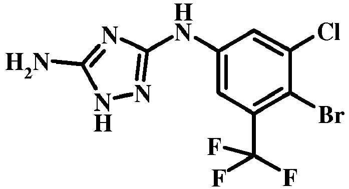

A solution of (Z)-methyl N'-cyano-N-(3,5-dichlorophenyl)carbamimidothioate (8 g, 30.8 mmol) and hydrazine (9.85 g, 9.65 ml, 308 mmol) in Ethanol (120 ml) was heated to 70 °C and stirred for 3 h.

The reaction mixture was concentrated to a small volume and water was added to give a suspension. The suspension was filtered and washed with water to give a white solid as desired product (7.44g, 99% yield).

### Example 43

### 4-(5-amino-1H-1,2,4-triazol-3-ylamino)-2-chloro-6-(trifluoromethyl)benzonitrile (Compound 43)

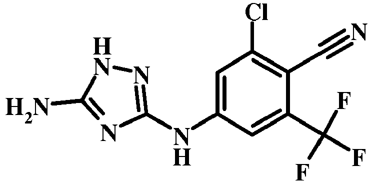

### 2-chloro-4-isothiocyanato-6-(trifluoromethyl)benzonitrile

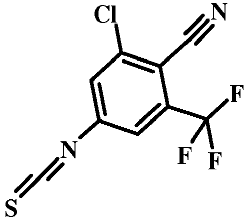

To a suspension of 4-amino-2-chloro-6-(trifluoromethyl)benzonitrile (200 mg, 907 µmol, WO 2009/003077) and calcium carbonate (318 mg, 3.17 mmol) in CH2Cl2 (2 ml) and water (2 ml) was added thiophosgene (115 mg, 76.5 µl, 997 µmol) at 0 °C. The reaction mixture was stirred cold for 30 minutes and then let to warm up to room temperature and stirred overnight. Added more CaCO3 (100 mg, 907 µmol) and thiophosgene (110 mg, 907 µmol) two more times and stirred for 18 hours. The reaction was neutralized with IN HCl to pH3 and was partitioned between water and DCM (5 ml/5 ml). The layers were separated and the aqueous layer was extracted with DCM (3x5 ml). The combined organic solution was washed with water (1x6 ml), dried over MgSO4 and concentrated to a residue. The crude material was purified by flash chromatography (silica gel, 24g, 5% to 30% DCM/Hexanes) to give a colorless oil as desired product (207mg, 87% yield).

### (Z)-methyl N-3-chloro-4-cyano-5-(trifluoromethyl)phenyl-N'-cyanocarbamimidothiolate

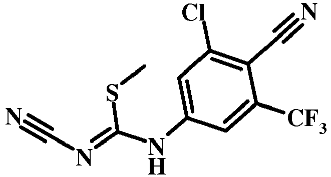

To a solution of 2-chloro-4-isothiocyanato-6-(trifluoromethyl)benzonitrile (200 mg, 762 µmol ) in MeOH (4 ml) was added sodium hydrogencyanamide (53.6 mg, 838 µmol) was added. The reaction was stirred at room temperature for 1 hour, added methyl iodide (216 mg, 95.2 µl, 1.52 mmol) and continued stirring for 18 hours. The reaction mixture was concentrated and the crude material was absorbed onto silica gel and concentrated to dryness. This was purified by flash chromatography (silica gel, 24g, 0% to 30% EtOAc in DCM) to give a white solid as desired product (83 mg, 34% yield).

### 4-(5-amino-1H-1,2,4-triazol-3-ylamino)-2-chloro-6-(trifluoromethyl)benzonitrile (Compound 43)

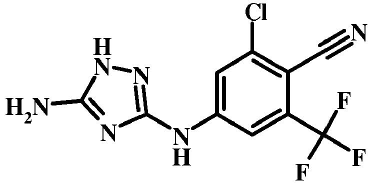

A solution of (Z)-methyl N-3-chloro-4-cyano-5-(trifluoromethyl)phenyl-N'-cyanocarbamimidothiolate (85 mg, 267 µmol) and hydrazine (85.5 mg, 83.7 µl, 2.67 mmol) in Ethanol (5 ml) was stirred at 70 °C for 3 h, and then concentrated to a small volume. Water was added and the reaction mixture was let stand for overnight to give a suspension. The suspension was filtered to afford a white solid as desired product (80mg, 99% yield). MS +m/z: 303 (M+H)⁺

### Example 44

### N3-(3,5-dichloro-4-(morpholinosulfonyl)phenyl)-1H-1,2,4-triazole-3,5-diamine (Compound 44)

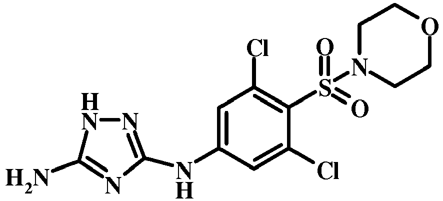

### 4-(2,6-dichloro-4-isothiocyanatophenylsulfonyl)morpholine

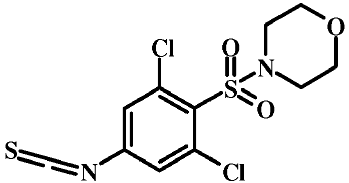

To a solution of 3,5-dichloro-4-(morpholinosulfonyl)aniline (200 mg, 643 µmol, J. Med. Chem. 1980, 23, 1083) in CH2Cl2 (5 ml) was added 1,1'-thiocarbonyldiimidazole (126 mg, 707 µmol) at 0 °C. The reaction was stirred cold for 30 minutes and then at RT overnight. The reaction was concentrated and the crude material was purified by flash chromatography (silica gel, 40 g, 10% to 100% DCM/Hexanes) to give a yellow oil as desired product (0.10g, 44% yield).

### 1-Cyano-3-(2,6-dichloro-4-isothiocyanatophenylsulfonyl)-2-methyl-2-thiopseudourea

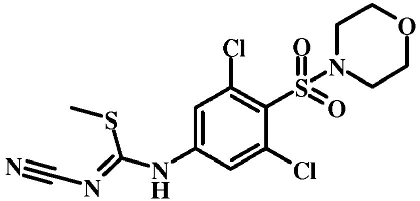

To a solution of 4-(2,6-dichloro-4-isothiocyanatophenylsulfonyl)morpholine (99 mg, 280 µmol) in acetonitrile (1.5 ml) and MeOH (1 ml) was added a mixture of sodium hydrogencyanamide (19.7 mg, 308 µmol) in MeOH (0.5ml). The reaction was stirred at room temperature for 1 hour and methyl iodide (79.6 mg, 35.0 µl, 561 µmol) was added. The reaction was stirred overnight. Water (5 ml) was added and the mixture was freeze dried and was used for the next step.

### N3-(3,5-dichloro-4-(morpholinosulfonyl)phenyl)-1H-1,2,4-triazole-3,5-diamine (Compound 44)

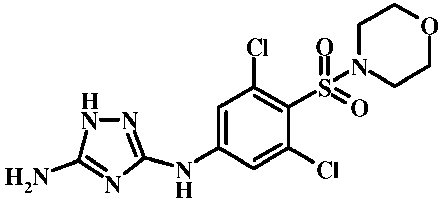

A solution of (Z)-methyl N'-cyano-N-(3,5-dichloro-4-(morpholinosulfonyl)phenyl)carbamimidothioate (115 mg, 281 µmol) and hydrazine (90.9 mg, 2.81 mmol) in Ethanol (3 ml) was heated to 80 °C and stirred for 3 h and then concentrated to a small volume. Water was added and the resulting suspension was filtered to give a solid. Purification by super fluid chromatography afforded a white foam as desired product (36.6 mg, 33% yield two steps). MS +m/z: 393 (M+H)⁺

### Example 45

### N⁵-(4-bromo-3-fluoro-5-trifluoromethylphenyl)-1H-[1,2,4]-triazole-3,5-diamine (Compound 45, Intermediate 3)

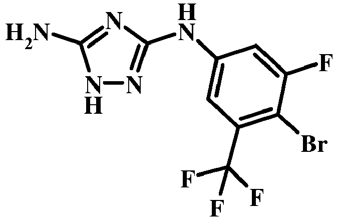

### 2-bromo-1-fluoro-5-isothiocyanato-3-trifluoromethylbenzene

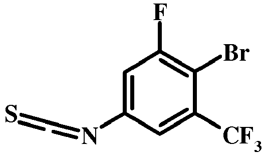

4-bromo-3-fluoro-5-trifluoromethylaniline (4.22 g, 16.4 mmol, Eq: 1.00) and calcium carbonate (3.44 g, 1.17 ml, 34.3 mmol, Eq: 2.1) were suspended in 50% aqueous dichlormethane (20ml) mixture. The thick suspension was stirred vigorously at 0°C. Thiophosgene (2.07 g, 1.38 ml, 18.0 mmol, Eq: 1.1) was added slowly dropwise to the mixture. After the addition the mixture was stirred at 0°C for 1.5hr then stirred overnight at room temperature. The solids were filtered and the filtrate was extracted with dichloromethane. The combined organic phases were washed with water, brine, dried over sodium sulfate and concentrated in vacuo to afford 4.71 g (96%) of the desired material as a light brown solid.
¹H NMR (300 MHz, DMSO-*d₆*) δ ppm 7.84 (s, 1 H) 7.96 (dd, J=9.06, 2.27 Hz, 1 H)

### (4-Bromo-3-fluoro-5-trifluoromethyl-phenylamino)-(methyl-λ⁴sulfanylidene)-methyl-cyanamide

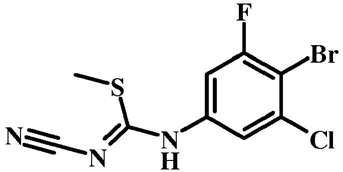

2-bromo-1-fluoro-5-isothiocyanato-3-trifluoromethylbenzene (4.71 g, 15.7 mmol, Eq: 1.00) was dissolved in anhydrous methanol (30 ml). Sodium hydrogencyanamide (1.00 g, 15.7 mmol, Eq: 1) was added and the reaction was stirred for 1hr at ambient temperature. Methyl iodide (4.46 g, 1.96 ml, 31.4 mmol, Eq: 2) was added dropwise and the reaction was stirred overnight at ambient temperature. The light brown suspension was filtered to afford 1.91 g (34%) of the desired product as a pink solid. MS +m/z: 357.7. (M+1)
¹H NMR (300 MHz, DMSO-*d*₆) δ ppm 2.78 (s, 3 H) 7.87 (s, 1 H) 7.97 (dd, *J*=1.00 Hz, 1 H) 10.38 (br. s, 1 H)

### Prepared of N⁵-(4-bromo-3-fluoro-5-trifluoromethylphenyl)-1H-[1,2,4]-triazole-3,5-diamine (Compound 45, Intermediate 3)

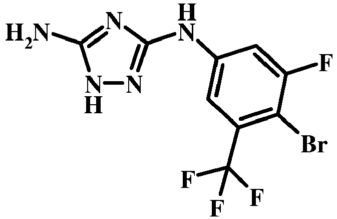

Hydrazine (1.71 g, 53.4 mmol, Eq: 10) was added to a stirred suspension of (4-Bromo-3-fluoro-5-trifluoromethyl-phenylamino)-(methyl-λ⁴sulfanylidene)-methyl-cyanamide (1.9 g, 5.34 mmol, Eq: 1.00) in ethanol (30 ml). The mixture was heated to 70°C for 1hr. The reaction mixture was concentrated to a reduced volume (~5ml) and water (~10ml) was added dropwise while stirring. The suspension was stirred for 30min. The precipitate was filtered and washed with water (~50ml), then dried under high vacuum at 70°C for two hours to filtered to afford 1.73 g (95%) of the desired product as a light pink solid. MS +m/z: 339.9. (M+1)
¹H NMR (400 MHz, DMSO-*d*₆) δ ppm 6.03 (s, 2 H) 7.81 (s, 1 H) 7.86 (d, *J*=12.13 Hz, 1 H) 9.52 (s, 1 H) 11.40 (s, 1 H)

### Example 46

### N*5*-(4-Bromo-3,5-difluoro-phenyl)-1H-[1,2,4]triazole-3,5-diamine (Compound 46, Intermediate 4)

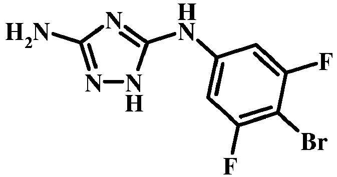

### 2-bromo-1,3-difluoro-5-isothiocyanatobenzene

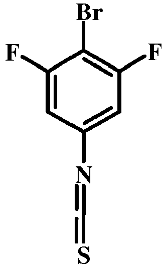

4-bromo-3,5-difluoroaniline (5 g, 24.0 mmol, Eq: 1.00) and calcium carbonate (5.05 g, 1.72 ml, 50.5 mmol, Eq: 2.1) were suspended in a 50% aqueous dichlormethane (24ml) mixture. The thick suspension was stirred vigorously at 0°C. Thiophosgene (3.04 g, 2.03 ml, 26.4 mmol, Eq: 1.1) was added slowly dropwise to the mixture. After the addition the mixture was stirred at 0°C for 1hr, then stirred overnight at room temperature. The precipitate was filtered and the filter cake was washed with dichloromethane. The phases were separated and the aqueous was extracted with dichloromethane. The combined organic phases were washed with water and brine, dried over sodium sulfate and concentrated in vacuo to afford 5.18 g (86%) of the desired product as an off-white solid which was used without further purification.

### (4-Bromo-3,5-difluoro-phenylamino)-(methyl-λ⁴-sulfanylidene)-methyl-cyanamide

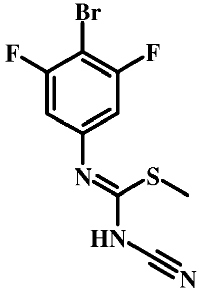

2-bromo-1,3-difluoro-5-isothiocyanatobenzene (5.18 g, 20.7 mmol, Eq: 1.00) was dissolved in anhydrous methanol (30.0 ml) and dichloromethane (10 ml). Sodium hydrogencyanamide (1.33 g, 20.7 mmol, Eq: 1) was added slowly and the reaction was stirred for 1hr at room temperature. The reaction was cooled to 0°C and methyl iodide (5.88 g, 2.59 ml, 41.4 mmol, Eq: 2) was added dropwise. The reaction was stirred overnight at room temperature. The white suspension was filtered and the filter cake was washed with methanol and dried under high vacuum to afford 4.62 g (73%) of the desired product as a white solid. MS +m/z: 307. (M+1)
¹H NMR (300 MHz, DMSO-*d*₆) δ ppm 2.74 (s, 3 H) 7.47 (d, *J*=8.69 Hz, 2 H) 10.35 (s, 1 H)

### N⁵-(4-bromo-3,5-difluorophenyl)-1H-[1,2,4]-triazole-3,5-diamine (Compound 46, Intermediate 4)

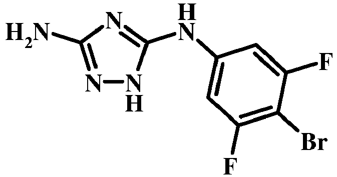

Hydrazine (4.84 g, 151 mmol, Eq: 10) was added to a stirred suspension of (4-Bromo-3,5-difluoro-phenylamino)-(methyl-□⁴-sulfanylidene)-methyl-cyanamide (4.62 g, 15.1 mmol, Eq: 1.00) in ethanol (78.9 ml). During the addition of hydrazine the reaction went into solution. The mixture was heated to 70°C for 45 minutes. The reaction mixture was concentrated (~10ml). Water (~80ml) was added dropwise and the suspension was stirred for 30min. The precipitate was filtered, washed with water (~100ml) and dried under high vacuum at 70°C to afford 4.28 g (97%) of the desired product as a white solid. MS +m/z: 291.9. (M+1)
¹H NMR (300 MHz, DMSO-*d*₆) δ ppm 6.00 (br. s., 2 H) 7.36 (d, *J*=10.58 Hz, 2 H) 9.37 (s, 1 H) 11.35 (br. s., 1 H)

### Example 47

### 4-(5-Amino-1H-[1,2,4]triazol-3-ylamino)-benzonitrile (Compound 47)

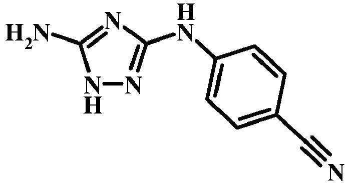

### 5-Bromo-1-(4-methoxy-benzyl)-3-nitro-1H-[1,2,4]triazole

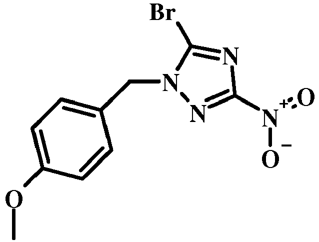

In a 250 mL round-bottomed flask, 5-bromo-3-nitro-1H-1,2,4-triazole (12 g, 62.2 mmol, Eq: 1.00), 1-(chloromethyl)-4-methoxybenzene (9.74 g, 62.2 mmol, Eq: 1) and N-ethyl-N-isopropylpropan-2-amine (16.1 g, 124 mmol, Eq: 2) were combined with acetonitrile (100 ml) to give a light yellow solution. Potassium iodide (5.16 g, 31.1 mmol, Eq: 0.5) was added. The reaction mixture was heated to reflux for 2 hours. The reaction mixture was cooled and diluted with EtOAc (100 mL), washed with H₂O (50 mL) and brine (50 mL). The organic layer was dried over anhydrous MgSO₄, filtered and volatiles were removed under reduced pressure to yield an oil from which the compound was isolated by column chromatography (Hexanes/EtOAc = 70/30) to product 7.8 g (40%).

### Bis-(4-methoxy-benzyl)-[2-(4-methoxy-benzyl)-5-nitro-2H-[1,2,4]triazol-3-yl]-amine

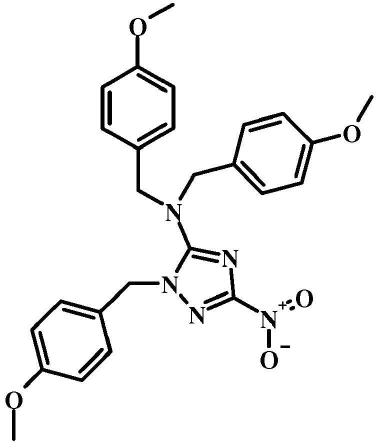

In a 10 mL sealed tube, 5-bromo-1-(4-methoxybenzyl)-3-nitro-1H-1,2,4-triazole (5.48 g, 17.5 mmol, Eq: 1.00) and bis(4-methoxybenzyl)amine (4.5 g, 17.5 mmol, Eq: 1.00) were combined, the mixture was heated to 150°C for overnight. Cool the reaction down, added CH₂Cl₂ (50 mL) washed with H₂O (50 mL) and brine (50 mL), the organic layer was dried over anhydrous MgSO₄, filtered and volatiles were removed under reduced pressure to afford the crude product 8.3 g (97% crude).MH+ 490.3

### 1,N*5*,N*5*-Tris-(4-methoxy-benzyl)-1H-[1,2,4]triazole-3,5-diamine

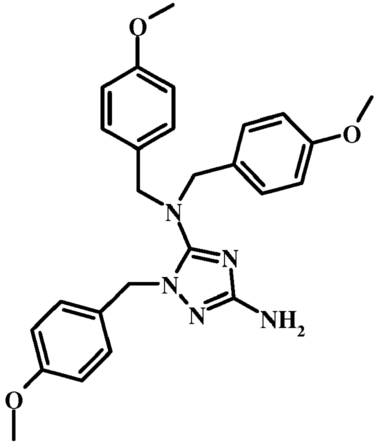

In a 100 mL round bottle, bis-(4-methoxy-benzyl)-[2-(4-methoxy-benzyl)-5-nitro-2H-[1,2,4]triazol-3-yl]-amine (530 mg, 1.08 mmol, Eq: 1.00) and zinc (354 mg, 5.41 mmol, Eq: 5.00) were combined with the solution of saturated NH4Cl aqueous solution / THF (1:1) (60.0 ml), the mixture was stirred at room temperature for 1 hour. Filter out the solid, extracted the mixture with CH₂Cl₂ (50 mLx2), the organic layer was dried over anhydrous MgSO₄, filtered and volatiles were removed under reduced pressure. The compound was isolated by column chromatography to give a light yellow solid 420 mg (84%). MH+ 460.3

### 4-[5-[Bis-(4-methoxy-benzyl)-amino]-1-(4-methoxy-benzyl)-1H-[1,2,4]triazol-3-ylamino]-benzonitrile

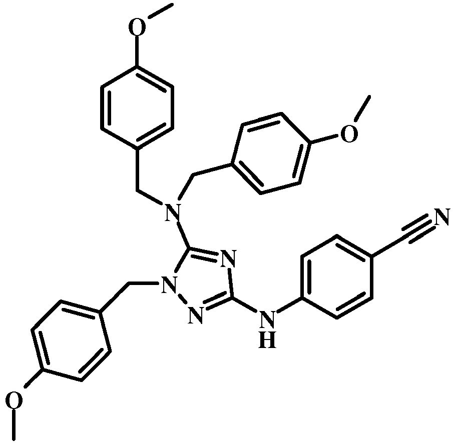

In a 25 mL sealed tube, sodium 2-methylpropan-2-olate (50.2 mg, 522 µmol, Eq: 1.20), bis(dibenzylideneacetone)palladium (25.0 mg, 43.5 µmol, Eq: 0.1) and 2-di-tert-butylphosphino-2',4',6'-triisopropylbiphenyl (18.5 mg, 43.5 µmol, Eq: 0.1) were combined with toluene (5.00 ml) to give a dark brown suspension. 1,N*5*,N*5*-Tris-(4-methoxy-benzyl)-lH-[1,2,4]triazole-3,5-diamine (200 mg, 435 µmol, Eq: 1.00) and 4-bromobenzonitrile (95.1 mg, 522 µmol, Eq: 1.20) were added. The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 60 mg (25%). MH+ 561.4

### 4-(5-Amino-1H-[1,2,4]triazol-3-ylamino)-benzonitrile (Compound 47)

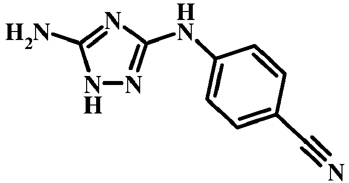

In a 10 mL round bottle, 4-(5-(bis(4-methoxybenzyl)amino)-1-(4-methoxybenzyl)-1H-1,2,4-triazol-3-ylamino)benzonitrile (60 mg, 107 µmol, Eq: 1.00) was combined with TFA (5.00 ml) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 10 mg (47%). MH+ 200.8

### Example 48

### 3-(5-Amino-1H-[1,2,4]triazol-3-ylamino)-5-chloro-benzonitrile (Compound 48)

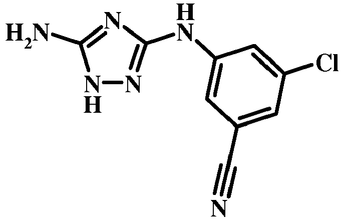

### 3-[5-[Bis-(4-methoxy-benzyl)-amino]-1-(4-methoxy-benzyl)-1H-[1,2,4]triazol-3-ylamino]-5-chloro-benzonitrile

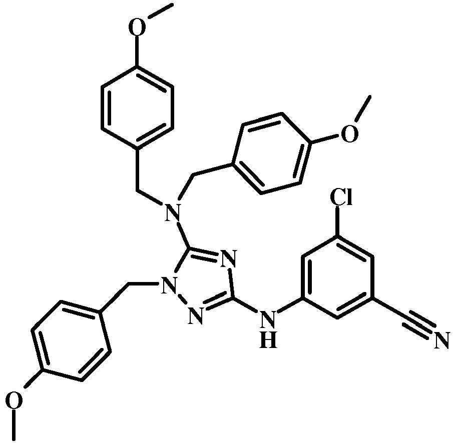

In a 25 mL sealed tube, sodium 2-methylpropan-2-olate (45.2 mg, 470 µmol, Eq: 1.20), bis(dibenzylideneacetone)palladium (22.5 mg, 39.2 µmol, Eq: 0.1) and 2-di-tert-butyl(2',4',6'-triisopropylbiphenyl-2-yl)phosphine (16.6 mg, 39.2 µmol, Eq: 0.1) were combined with toluene (5 ml) to give a dark brown suspension. N5,N5,1-tris(4-methoxybenzyl)-1H-1,2,4-triazole-3,5-diamine (180 mg, 392 µmol, Eq: 1.00) and 3-bromo-5-chlorobenzonitrile (84.8 mg, 392 µmol, Eq: 1.00) were added. The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 55 mg (24%). MH+ 595.3

### 3-(5-Amino-1H-[1,2,4]triazol-3-ylamino)-5-chloro-benzonitrile (Compound 48)

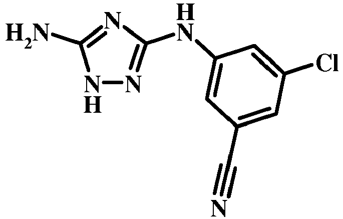

In a 10 mL round bottle, 3-[5-[bis-(4-methoxy-benzyl)-amino]-1-(4-methoxy-benzyl)-1H-[1,2,4]triazol-3-ylamino]-5-chloro-benzonitrile (55 mg, 92.4 µmol, Eq: 1.00) was combined with TFA (5.00 ml) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 9 mg (42%). MH+ 234.9

### Example 49

### N*3*-(2,3-Dichloro-phenyl)-1H-[1,2,4]triazole-3,5-diamine (Compound 49)

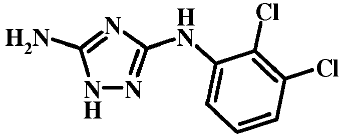

### N*3*-(2,3-Dichloro-phenyl)-1,N*5*,N*5*-tris-(4-methoxy-benzyl)-1H-[1,2,4]triazole-3,5-diamine

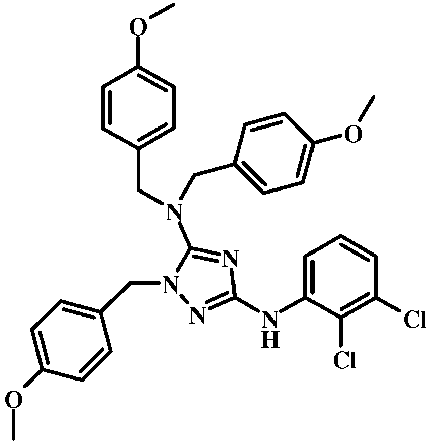

In a 25 mL sealed tube, sodium 2-methylpropan-2-olate (45.2 mg, 470 µmol, Eq: 1.20), bis(dibenzylideneacetone)palladium (22.5 mg, 39.2 µmol, Eq: 0.1) and 2-di-tert-butyl(2',4',6'-triisopropylbiphenyl-2-yl)phosphine (16.6 mg, 39.2 µmol, Eq: 0.1) were combined with toluene (5 mL) to give a dark brown suspension. N5,N5,1-tris(4-methoxybenzyl)-1H-1,2,4-triazole-3,5-diamine (180 mg, 392 µmol, Eq: 1.00) and 1-bromo-2,3-dichlorobenzene (88.5 mg, 392 µmol, Eq: 1.00) were added.

The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 80 mg (34%). MH+ 604.3

### N*3*-(2,3-Dichloro-phenyl)-1H-[1,2,4]triazole-3,5-diamine (Compound 49)

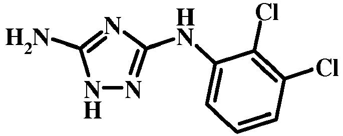

In a 10 mL round bottle, N*3*-(2,3-dichloro-phenyl)-1,N*5*,N*5*-tris-(4-methoxy-benzyl)-1H-[1,2,4]triazole-3,5-diamine (80 mg, 132 µmol, Eq: 1.00) was combined with TFA (5.00 mL) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 21 mg (65%). MH+ 243.8

### Example 50

### N*3*-(3-Chloro-5-trifluoromethyl-phenyl)-1H-[1,2,4]triazole-3,5-diamine (Compound 50)

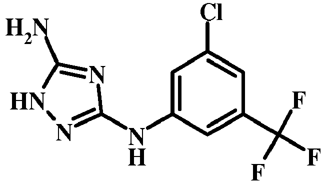

### N*3*-(3-Chloro-5-trifluoromethyl-phenyl)-1,N*5*,N*5*-tris-(4-methoxy-benzyl)-1H-[1,2,4]triazole-3,5-diamine

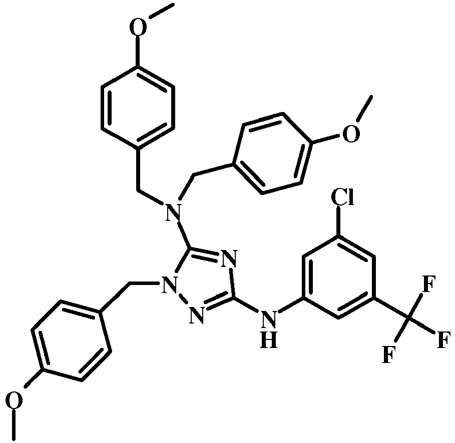

In a 25 mL sealed tube, sodium 2-methylpropan-2-olate (62.7 mg, 653 µmol, Eq: 1.20), bis(dibenzylideneacetone)palladium (31.3 mg, 54.4 µmol, Eq: 0.1) and 2-di-tert-butyl(2',4',6'-triisopropylbiphenyl-2-yl)phosphine (23.1 mg, 54.4 µmol, Eq: 0.1) were combined with toluene (5.00 mL) to give a dark brown suspension. N5,N5,1-tris(4-methoxybenzyl)-1H-1,2,4-triazole-3,5-diamine (250 mg, 544 µmol, Eq: 1.00) and 1-bromo-3-chloro-5-(trifluoromethyl)benzene (141 mg, 544 µmol, Eq: 1.00) were added. The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 40 mg (12%). MH+ 638.4

### N*3*-(3-Chloro-5-trifluoromethyl-phenyl)-1H-[1,2,4]triazole-3,5-diamine (Compound 50)

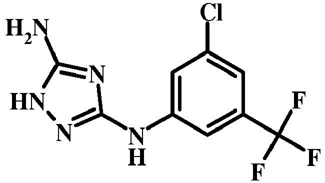

In a 10 mL round bottle, N*3*-(3-Chloro-5-trifluoromethyl-phenyl)-1,N*5*,N*5*-tris-(4-methoxy-benzyl)-1H-[1,2,4]triazole-3,5-diamine (40 mg, 62.7 µmol, Eq: 1.00) was combined with TFA (5.00 ml) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 6 mg (35%). MH+ 277.9

### Example 51

### N*3*-(4-Chloro-3-trifluoromethyl-phenyl)-1H-[1,2,4]triazole-3,5-diamine (Compound 51)

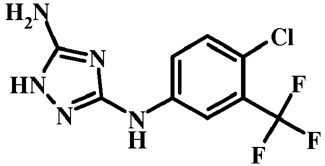

### N*3*-(4-Chloro-3-trifluoromethyl-phenyl)-1,N*5*,N*5*-tris-(4-methoxy-benzyl)-1H-[1,2,4]triazole-3,5-diamine

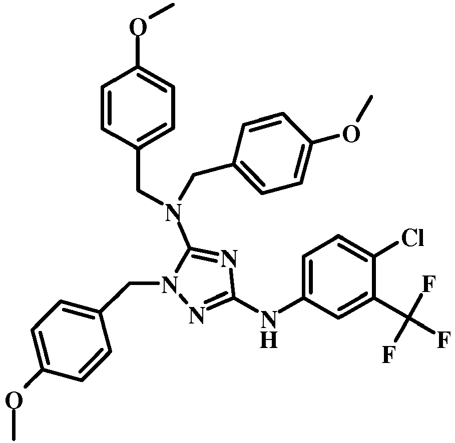

In a 25 mL sealed tube, sodium 2-methylpropan-2-olate (62.7 mg, 653 µmol, Eq: 1.20), bis(dibenzylideneacetone)palladium (31.3 mg, 54.4 µmol, Eq: 0.1) and 2-di-tert-butyl(2',4',6'-triisopropylbiphenyl-2-yl)phosphine (23.1 mg, 54.4 µmol, Eq: 0.1) were combined with toluene

(5.00 ml) to give a dark brown suspension. N5,N5,1-tris(4-methoxybenzyl)-1H-1,2,4-triazole-3,5-diamine (250 mg, 544 µmol, Eq: 1.00) and 4-bromo-1-chloro-2-(trifluoromethyl)benzene (141 mg, 544 µmol, Eq: 1.00) were added. The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 80 mg (23%). MH+ 638.4

### N*3*-(4-Chloro-3-trifluoromethyl-phenyl)-1H-[1,2,4]triazole-3,5-diamine (Compound 51)

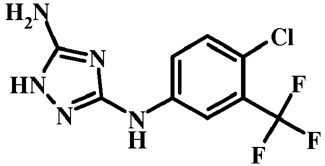

In a 10 mL round bottle, N*3*-(4-Chloro-5-trifluoromethyl-phenyl)-1,N*5*,N*5*-tris-(4-methoxy-benzyl)-1H-[1,2,4]triazole-3,5-diamine (80 mg, 125 µmol, Eq: 1.00) was combined with TFA (5.00 mL) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 9 mg (26%). MH+ 277.9

### Example 52

### N*3*-(3-Trifluoromethoxy-phenyl)-1H-[1,2,4]triazole-3,5-diamine (Compound 52)

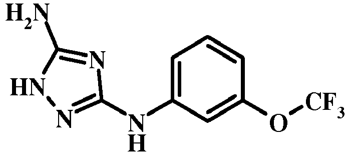

### 1,N*5*,N*5*-Tris-(4-methoxy-benzyl)-N*3*-(3-trifluoromethoxy-phenyl)-1H-[1,2,4]triazole-3,5-diamine

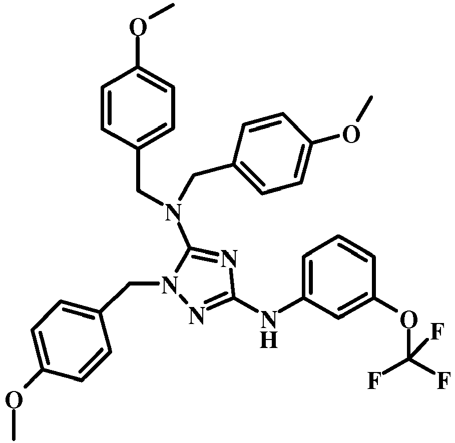

In a 25 mL sealed tube, sodium 2-methylpropan-2-olate (62.7 mg, 653 µmol, Eq: 1.20), bis(dibenzylideneacetone)palladium (31.3 mg, 54.4 µmol, Eq: 0.1) and 2-di-tert-butyl(2',4',6'-triisopropylbiphenyl-2-yl)phosphine (23.1 mg, 54.4 µmol, Eq: 0.1) were combined with toluene (5.00 ml) to give a dark brown suspension. N5,N5,1-tris(4-methoxybenzyl)-1H-1,2,4-triazole-3,5-diamine (250 mg, 544 µmol, Eq: 1.00) and 1-bromo-3-(trifluoromethoxy)benzene (131 mg, 544 µmol, Eq: 1.00) were added. The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 85 mg (25%). MH+ 620.4

### N*3*-(3-Trifluoromethoxy-phenyl)-1H-[1,2,4]triazole-3,5-diamine (Compound 52)

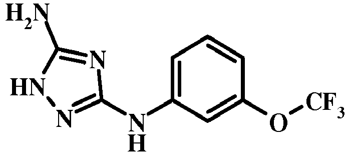

In a 10 mL round bottle, 1,N*5*,N*5*-Tris-(4-methoxy-benzyl)-N*3*-(3-trifluoromethoxy-phenyl)-1H-[1,2,4]triazole-3,5-diamine (85 mg, 137 µmol, Eq: 1.00) was combined with TFA (5.00 mL) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 2 mg (6%). MH+ 260.0

### Example 53

### N*3*-(2-Fluoro-3-trifluoromethyl-phenyl)-1H-[1,2,4]triazole-3,5-diamine (Compound 53)

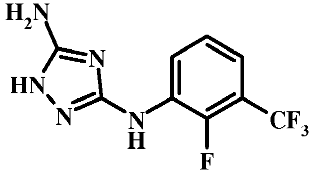

### N*3*-(2-Fluoro-3-trifluoromethyl-phenyl)-1,N*5*,N*5*-tris-(4-methoxy-benzyl)-1H-[1,2,4]triazole-3,5-diamine

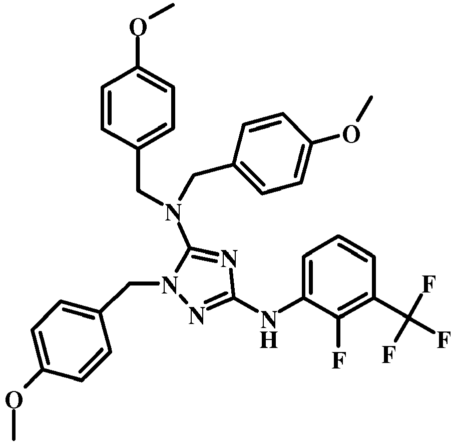

In a 25 mL sealed tube, sodium 2-methylpropan-2-olate (62.7 mg, 653 µmol, Eq: 1.20), bis(dibenzylideneacetone)palladium (31.3 mg, 54.4 µmol, Eq: 0.1) 2-di-tert-butyl(2',4',6'-triisopropylbiphenyl-2-yl)phosphine (23.1 mg, 54.4 µmol, Eq: 0.1) were combined with toluene

(5.00 mL) to give a dark brown suspension. N5,N5,1-tris(4-methoxybenzyl)-1H-1,2,4-triazole-3,5-diamine (250 mg, 544 µmol, Eq: 1.00) and 1-bromo-2-fluoro-3-(trifluoromethyl)benzene (132 mg, 544 µmol, Eq: 1.00) were added. The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 90 mg (27%). MH+ 622.4

### N*3*-(2-Fluoro-3-trifluoromethyl-phenyl)-1H-[1,2,4]triazole-3,5-diamine (Compound 53)

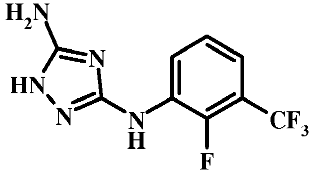

In a 10 mL round bottle, N*3*-(2-fluoro-3-trifluoromethyl-phenyl)-1,N*5*,N*5*-tris-(4-methoxy-benzyl)-1H-[1,2,4]triazole-3,5-diamine (90 mg, 145 µmol, Eq: 1.00) was combined with TFA (5.00 mL) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 14 mg (37%). MH+ 261.9

### Example 54

### N*3*-(2-Fluoro-5-trifluoromethyl-phenyl)-1H-[1,2,4]triazole-3,5-diamine (Compound 54)

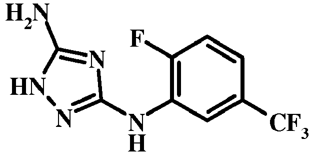

### N*3*-(2-Fluoro-5-trifluoromethyl-phenyl)-1,N*5*,N*5*-tris-(4-methoxy-benzyl)-1H-[1,2,4]triazole-3,5-diamine

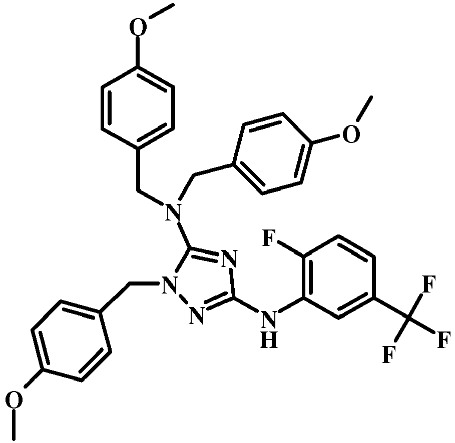

In a 25 mL sealed tube, sodium 2-methylpropan-2-olate (62.7 mg, 653 µmol, Eq: 1.20), bis(dibenzylideneacetone)palladium (31.3 mg, 54.4 µmol, Eq: 0.1) and 2-di-tert-butyl(2',4',6'-triisopropylbiphenyl-2-yl)phosphine (23.1 mg, 54.4 µmol, Eq: 0.1) were combined with toluene (5.00 ml) to give a dark brown suspension. N5,N5,1-tris(4-methoxybenzyl)-1H-1,2,4-triazole-3,5-diamine (250 mg, 544 µmol, Eq: 1.00) and 2-bromo-1-fluoro-4-(trifluoromethyl)benzene (132 mg, 544 µmol, Eq: 1.00) were added. The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 95 mg (28%). MH+ 622.4

### N*3*-(2-Fluoro-5-trifluoromethyl-phenyl)-1H-[1,2,4]triazole-3,5-diamine (Compound 54)

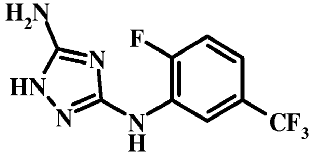

In a 10 mL round bottle, N*3*-(2-Fluoro-5-trifluoromethyl-phenyl)-1,N*5*,N*5*-tris-(4-methoxy-benzyl)-1H-[1,2,4]triazole-3,5-diamine (95 mg, 153 µmol, Eq: 1.00) was combined with TFA (5.00 mL) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 7 mg (18%). MH+ 261.9

### Example 55

### N*3*-[3-(2,2,2-Trifluoro-ethyl)-phenyl]-1H-[1,2,4]triazole-3,5-diamine (Compound 55)

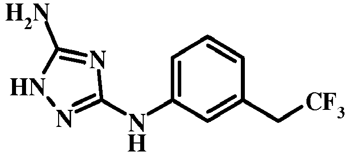

### 1,N*5*,N*5*-Tris-(4-methoxy-benzyl)-N*3*-[3-(2,2,2-trifluoro-ethyl)-phenyl]-1H-[1,2,4]triazole-3,5-diamine

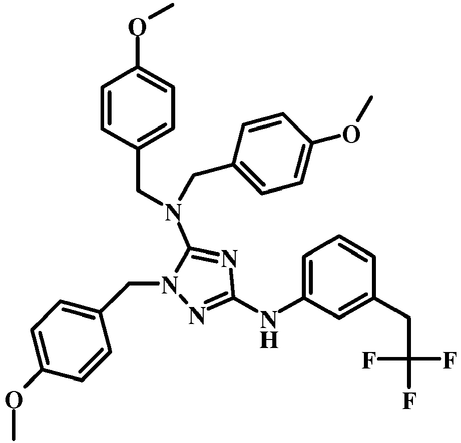

In a 25 mL sealed tube, sodium 2-methylpropan-2-olate (62.7 mg, 653 µmol, Eq: 1.20), bis(dibenzylideneacetone)palladium (31.3 mg, 54.4 µmol, Eq: 0.1) and 2-di-tert-butyl(2',4',6'-triisopropylbiphenyl-2-yl)phosphine (23.1 mg, 54.4 µmol, Eq: 0.1) were combined with toluene (5.00 mL) to give a dark brown suspension. N5,N5,1-tris(4-methoxybenzyl)-1H-1,2,4-triazole-3,5-diamine (250 mg, 544 µmol, Eq: 1.00) and 1-bromo-3-(2,2,2-trifluoroethyl)benzene (130 mg, 544 µmol, Eq: 1.00)were added. The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 90 mg (27%). MH+ 618.4

### N*3*-[3-(2,2,2-Trifluoro-ethyl)-phenyl]-1H-[1,2,4]triazole-3,5-diamine (Compound 55)

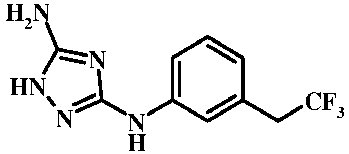

In a 10 mL round bottle, 1,N*5*,N*5*-Tris-(4-methoxy-benzyl)-N*3*-[3-(2,2,2-trifluoro-ethyl)-phenyl]-1H-[1,2,4]triazole-3,5-diamine (90 mg, 146 µmol, Eq: 1.00) was combined with TFA (5.00 mL) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 8 mg (21%). MH+ 257.9

### Example 56

### N*3*-(3-Isopropyl-phenyl)-1H-[1,2,4]triazole-3,5-diamine (Compound 56)

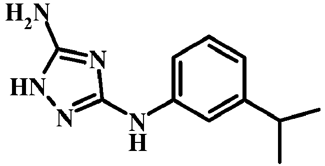

### 3,5-Dibromo-1-(4-methoxy-benzyl)-1H-[1,2,4]triazole

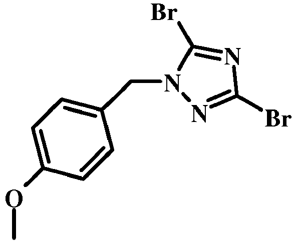

In a 100 mL round-bottomed flask, 3,5-dibromo-1H-1,2,4-triazole (5g, 22.0 mmol, Eq: 1.00), 1-(chloromethyl)-4-methoxybenzene (3.45 g, 22.0 mmol, Eq: 1) and N-ethyl-N-isopropylpropan-2-amine (5.7 g, 44.1 mmol, Eq: 2) were combined with acetonitrile (101 ml) to give a light yellow solution. Potassium iodide (1.83 g, 11.0 mmol, Eq: 0.5) was added. The mixture was heated to reflux for 2 hours. The reaction mixture was cooled and diluted with EtOAc (100 mL), washed with H₂O (50 mL) and brine (50 mL). The organic layer was dried over anhydrous MgSO₄, filtered and volatiles were removed under reduced pressure to yield an oil from which the compound was isolated by column chromatography (Hexanes/EtOAc = 70/30) to product 7.3 g (95%).

### 5-Bromo-2-(4-methoxy-benzyl)-2H-[1,2,4]triazol-3-yl]-bis-(4-methoxy-benzyl)-amine

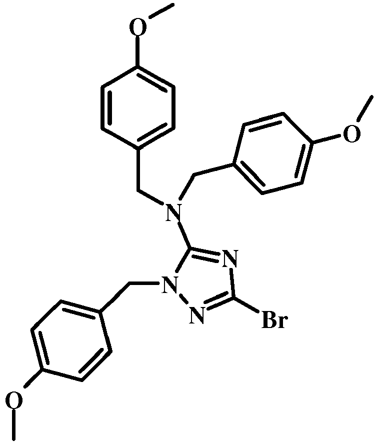

In a 10 mL sealed tube, 3,5-dibromo-1-(4-methoxybenzyl)-1H-1,2,4-triazole (400 mg, 1.15 mmol, Eq: 1.00) and bis(4-methoxybenzyl)amine (356 mg, 1.38 mmol, Eq: 1.20) were combined, the mixture was heated to 140°C for overnight. The reaction mixture was cooled to room temperature, added CH₂Cl₂ (50 mL) washed with H₂O (50 mL) and brine (50 mL). The organic layer was dried over anhydrous MgSO₄, filtered and volatiles were removed under reduced pressure. The compound was isolated by column chromatography to give a light yellow solid 300 mg (50%). MH+ 525.1

### N*3*-(3-Isopropyl-phenyl)-1,N*5*,N*5*-tris-(4-methoxy-benzyl)-1H-[1,2,4]triazole-3,5-diamine

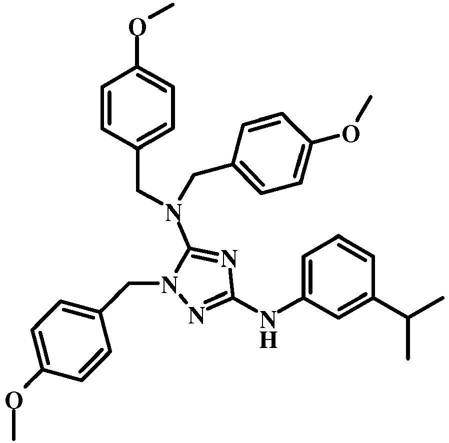

In a 25 mL sealed tube, sodium 2-methylpropan-2-olate (55.1 mg, 573 µmol, Eq: 1.20), bis(dibenzylideneacetone)palladium (27.5 mg, 47.8 µmol, Eq: 0.1) and 2-di-tert-butyl(2',4',6'-triisopropylbiphenyl-2-yl)phosphine (20.3 mg, 47.8 µmol, Eq: 0.1) were combined with toluene (5.00 mL) to give a dark brown suspension. [5-bromo-2-(4-methoxy-benzyl)-2H-[1,2,4]triazol-3-yl]-bis-(4-methoxy-benzyl)-amine (250 mg, 478 µmol, Eq: 1.00) and 3-isopropylaniline (64.6 mg, 478 µmol, Eq: 1.00) were added. The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 95 mg (34%). MH+ 578.5

### N*3*-(3-Isopropyl-phenyl)-1H-[1,2,4]triazole-3,5-diamine (Compound 56)

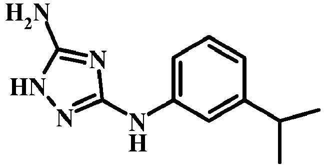

In a 10 mL round bottle, N*3*-(3-Isopropyl-phenyl)-1,N*5*,N*5*-tris-(4-methoxybenzyl)-1H-[1,2,4]triazole-3,5-diamine (80 mg, 138 µmol, Eq: 1.00) was combined with TFA (5.00 mL) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 19 mg (65%). MH+ 217.9

### Example 57

### N*3*-(3-Fluoro-5-trifluoromethyl-phenyl)-1H-[1,2,4]triazole-3,5-diamine (Compound 57)

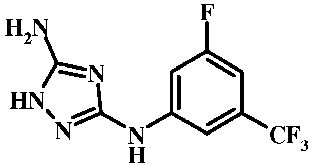

### N*3*-(3-Fluoro-5-trifluoromethyl-phenyl)-1,N*5*,N*5*-tris-(4-methoxy-benzyl)-1H-[1,2,4]triazole-3,5-diamine

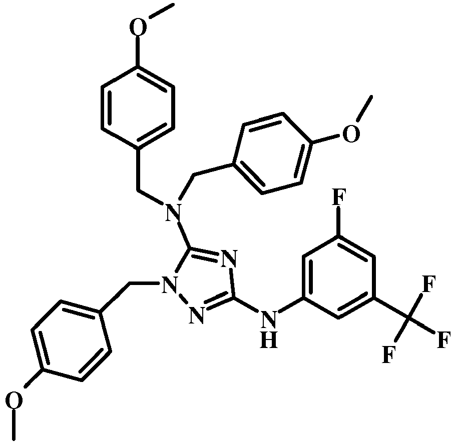

In a 25 mL sealed tube, sodium 2-methylpropan-2-olate (60.2 mg, 626 µmol, Eq: 1.20), bis(dibenzylideneacetone)palladium (30.0 mg, 52.2 µmol, Eq: 0.1) and 2-di-tert-butyl(2',4',6'-triisopropylbiphenyl-2-yl)phosphine (22.2 mg, 52.2 µmol, Eq: 0.1) were combined with toluene (5.00 mL) to give a dark brown suspension. [5- bromo -2-(4-methoxy-benzyl)-2H-[1,2,4]triazol-3-yl]-bis-(4-methoxy-benzyl)-amine (250 mg, 522 µmol, Eq: 1.00) and 3-fluoro-5-(trifluoromethyl)aniline (93.5 mg, 522 µmol, Eq: 1.00) were added. The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 100 mg (31%). MH+ 622.4

### N*3*-(3-Fluoro-5-trifluoromethyl-phenyl)-1H-[1,2,4]triazole-3,5-diamine (Compound 57)

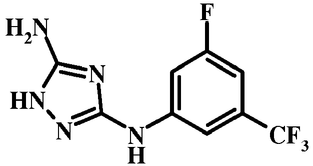

In a 10 mL round bottle, N*3*-(3-fluoro-5-trifluoromethyl-phenyl)-1,N*5*,N*5*-tris-(4-methoxy-benzyl)-1H-[1,2,4]triazole-3,5-diamine (100 mg, 161 µmol, Eq: 1.00) was combined with TFA (5.00 mL) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 8 mg (19%). MH+ 262.0

### Example 58

### [3-(5-Amino-1H-[1,2,4]triazol-3-ylamino)-phenyll-phenyl-methanone (Compound 58)

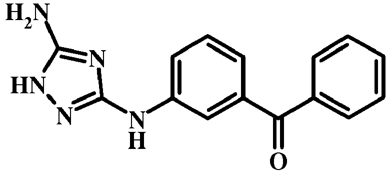

### {3-[5-[Bis-(4-methoxy-benzyl)-amino]-1-(4-methoxy-benzyl)-1H-[1,2,4]triazol-3-ylamino]-phenyl}-phenyl-methanone

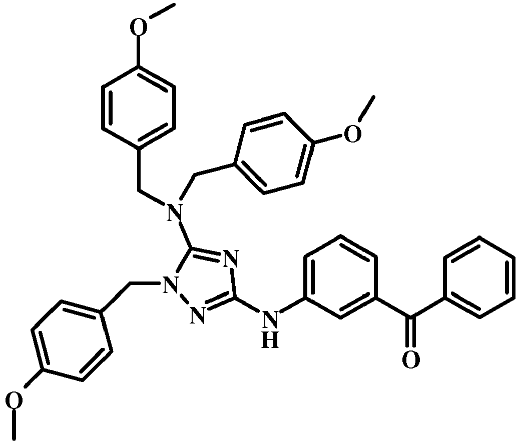

In a 25 mL sealed tube, sodium 2-methylpropan-2-olate (60.2 mg, 626 µmol, Eq: 1.20), bis(dibenzylideneacetone)palladium (30.0 mg, 52.2 µmol, Eq: 0.1) and 2-di-tert-butyl(2',4',6'-triisopropylbiphenyl-2-yl)phosphine (22.2 mg, 52.2 µmol, Eq: 0.1) were combined with toluene (5.00 mL) to give a dark brown suspension. 5- bromo -2-(4-methoxy-benzyl)-2H-[1,2,4]triazol-3-yl]-bis-(4-methoxy-benzyl)-amine (250 mg, 522 µmol, Eq: 1.00) and (3-aminophenyl)(phenyl)methanone (103 mg, 522 µmol, Eq: 1.00) were added. The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 105 mg (31%). MH+ 640.2

### [3-(5-Amino-1H-[1,2,4]triazol-3-ylamino)-phenyl]-phenyl-methanone (Compound 58)

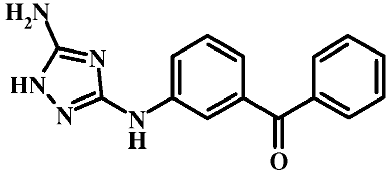

In a 10 mL round bottle, {3-[5-[bis-(4-methoxy-benzyl)-amino]-1-(4-methoxy-benzyl)-1H-[1,2,4]triazol-3-ylamino]-phenyl}-phenyl-methanone (105 mg, 164 µmol, Eq: 1.00) was combined with TFA (5.00 mL) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 9 mg (20%). MH+ 280.0

### Example 59

### 1-[3-(5-Amino-1H-[1,2,4]triazol-3-ylamino)-phenyl]-cyclopropanecarbonitrile (Compound 59)

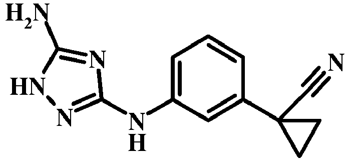

### 1-{3-[5-[Bis-(4-methoxy-benzyl)-amino]-1-(4-methoxy-benzyl)-1H-[1,2,4]triazol-3-ylamino]-phenyl}-cyclopropanecarbonitrile

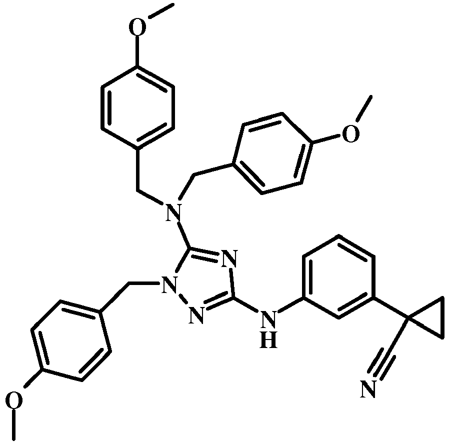

In a 25 mL sealed tube, sodium 2-methylpropan-2-olate (62.7 mg, 653 µmol, Eq: 1.20), bis(dibenzylideneacetone)palladium (31.3 mg, 54.4 µmol, Eq: 0.1) and 2-di-tert-butyl(2',4',6'-triisopropylbiphenyl-2-yl)phosphine (23.1 mg, 54.4 µmol, Eq: 0.1) were combined with toluene (5.00 mL) to give a dark brown suspension. N5,N5,1-tris(4-methoxybenzyl)-1H-1,2,4-triazole-3,5-diamine (250 mg, 544 µmol, Eq: 1.00) and 1-(3-bromophenyl)cyclopropanecarbonitrile (121 mg, 544 µmol, Eq: 1.00) were added. The reaction mixture was degassed with argon for 15 min, and then heated to 110°C for 3 hours. The reaction mixture was cooled and diluted with EtOAc (50 mL), washed with H₂O (25 mL) and brine (25 mL). The organic layer was dried over anhydrous MgSO₄, filtered and volatiles were removed under reduced pressure to yield an oil from which the compound was isolated by column chromatography (Hexanes/EtOAc = 70/30) to give an off-white solid 105 mg (32%). MH+ 601.4

### 1-[3-(5-Amino-1H-[1,2,4]triazol-3-ylamino)-phenyl]-cyclopropanecarbonitrile (Compound 59)

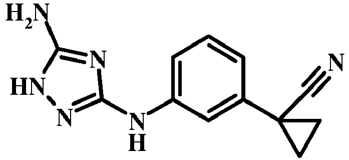

In a 10 mL round bottle, 1-{3-[5-[bis-(4-methoxy-benzyl)-amino]-1-(4-methoxy-benzyl)-1H-[1,2,4]triazol-3-ylamino]-phenyl}-cyclopropanecarbonitrile (105 mg, 175 µmol, Eq: 1.00) was combined with TFA (5.00 ml) to give a colorless solution. The resulting solution was heated to 65 °C overnight, the reaction mixture was concentrated, and then diluted with EtOAc (30mL). The solution was washed with saturated NaHCO₃, organic layer was dried over anhydrous MgSO₄, filtered and volatiles were removed under reduced pressure. The compound was isolated by preparative TLC to give an off-white solid 10 mg (24%). MH+ 241.0

### Example 60

### N*3*-(3-Chloro-4-fluoro-5-trifluoromethyl-phenyl)-1H-[1,2,4]triazole-3,5-diamine (Compound 60)

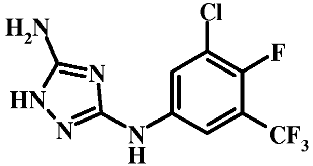

### 1-Chloro-2-fluoro-5-isothiocyanato-3-trifluoromethyl-benzene

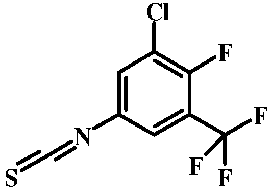

In a 100 mL round-bottomed flask, di(1H-imidazol-1-yl)methanethione (1.25 g, 7.02 mmol, Eq: 1.5) was combined with CH₂Cl₂ (30 mL) to give a colorless solution. 3-chloro-4-fluoro-5-(trifluoromethyl)aniline (1g, 4.68 mmol, Eq: 1.00) in CH₂Cl₂ (20 mL) was added dropwise at 0°C. The reaction was allowed to warm to room temperature, and allowed to stir overnight. Concentrate the solution, the compound was isolated by column chromatography (CH2Cl2/Hexanes = 80/20) to give the product 0.8 g (67%).

### (3-Chloro-4-fluoro-5-trifluoromethyl-phenylamino)-methylsulfanyl-methyl-cyanamide

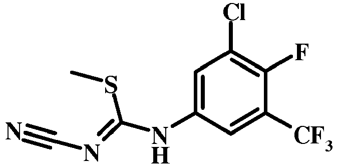

In a 100 mL round-bottomed flask, 1-chloro-2-fluoro-5-isothiocyanato-3-(trifluoromethyl)benzene (0.8 g, 3.13 mmol, Eq: 1.00) in MeOH (20 mL), sodium hydrogencyanamide (226 mg, 3.54 mmol, Eq: 1.13) was added. The suspension turned to clear after a few minutes, the reaction was allowed to stir at room temperature for 1 hour, iodomethane (888 mg, 6.26 mmol, Eq: 2) was added to the reaction, the reaction mixture was allowed to stir at room temperature overnight. Concentrate the solution, the compound was isolated by column chromatography (Hexanes/EtOAc = 70/30) to give an off-white solid 300 mg (31%). MH+ 313.8

### N*3*-(3-Chloro-4-fluoro-5-trifluoromethyl-phenyl)-1H-[1,2,4]triazole-3,5-diamine (Compound 60)

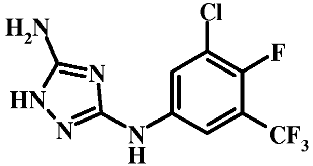

In a 100 mL round-bottomed flask, (3-chloro-4-fluoro-5-trifluoromethyl-phenylamino)-methylsulfanyl-methyl-cyanamide (300 mg, 956 µmol, Eq: 1.00) in EtOH (30 mL), hydrazine (306 mg, 9.56 mmol, Eq: 10.00) was added. The reaction was heated to 65 °C for 3 hours. The reaction mixture was concentrated, added H₂O (20 mL) to the residue, filter out the solid and wash the solid with H₂O (30 mL) and CH₂Cl₂ (10 mL), air-dried the solid overnight to give an off-white solid 235 mg (83%). MH+ 295.9

### Example 61

### N*3*-(3-Chloro-5-fluoro-phenyl)-1H-[1,2,4]triazole-3,5-diamine (Compound 61)

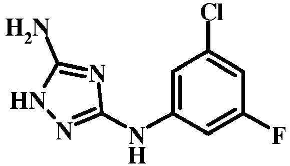

### 1-Chloro-3-fluoro-5-isothiocyanato- benzene

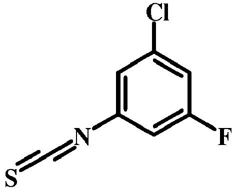

In a 100 mL round-bottomed flask, di(1H-imidazol-1-yl)methanethione (1.84 g, 10.3 mmol, Eq: 1.5) was combined with CH₂Cl₂ (30 mL) to give a colorless solution. 3-chloro-5-fluoroaniline (1 g, 6.87 mmol, Eq: 1.00) in CH₂Cl₂ (20 mL) was added dropwise at 0°C. The reaction was allowed to warm to room temperature, and allowed to stir overnight. Concentrate the solution, the compound was isolated by column chromatography (Hexanes/EtOAc = 80/20) to give the product 1.2 g (93%).

### (3-Chloro-4-fluoro-5-trifluoromethyl-phenylamino)-methylsulfanyl-methyl-cyanamide

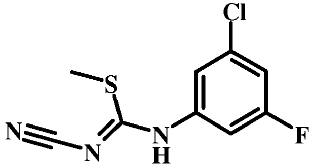

In a 100 mL round-bottomed flask, 1-chloro-3-fluoro-5-isothiocyanatobenzene (1.2 g, 6.4 mmol, Eq: 1.00) in MeOH (20 mL), sodium hydrogencyanamide (463 mg, 7.23 mmol, Eq: 1.13) was added. The suspension turned to clear after a few minutes, the reaction was allowed to stir at room temperature for 1 hour, iodomethane (1.82 g, 12.8 mmol, Eq: 2) was added, the reaction mixture was allowed to stir at room temperature overnight. Concentrate the solution, the compound was isolated by column chromatography (Hexanes/EtOAc = 70/30) to give an off-white solid 0.6 g (38%). MH+ 245.8

### N*3*-(3-Chloro-5-fluoro-phenyl)-1H-[1,2,4]triazole-3,5-diamine (Compound 61)

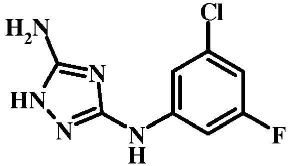

In a 100 mL round-bottomed flask, (3-Chloro-4-fluoro-5-trifluoromethyl-phenylamino)-methylsulfanyl-methyl-cyanamide (0.6 g, 2.44 mmol, Eq: 1.00) in EtOH (30 mL), hydrazine (783 mg, 24.4 mmol, Eq: 10.00) was added. The reaction was heated to 65°C for 3 hours. The reaction mixture was concentrated, added H₂O (20 mL) to the residue, filtered out the solid and washed the solid with H₂O (30 mL) and CH₂Cl₂ (10 mL), air-dried the solid overnight to give an off-white solid 185 mg (33%). MH+ 228.0

### Example 62

### 1-[4-(5-Amino-1H-[1,2,4]triazol-3-ylamino)-2-chloro-phenyl]-3-methyl-urea (Compound 62)

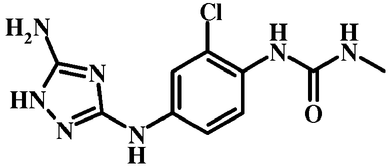

### 1-(2-Chloro-4-nitro-phenyl)-3-methyl-1H-pyrimidine-2,4-dione

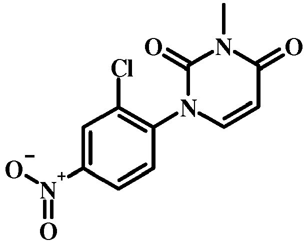

In a 50 mL round-bottomed flask, potassium 2-methylpropan-2-olate (445 mg, 3.96 mmol, Eq: 1.00) and 3-methylpyrimidine-2,4(1H,3H)-dione (0.5 g, 3.96 mmol, Eq: 1.00) were combined with DMF (5.0 ml) to give a light brown suspension at 0°C under nitrogen. 2-Chloro-1-fluoro-4-nitrobenzene (696 mg, 3.96 mmol, Eq: 1.00) was added. The reaction was stirred overnight at room temperature. The reaction mixture was concentrated under reduced pressure, the compound was isolated by column chromatography (Hexanes / EtOAc = 50/50) to give the product 0.79 g (71%). MH+ 282.0

### 1-(4-Amino-2-chloro-phenyl)-3-methyl-1H-pyrimidine-2,4-dione

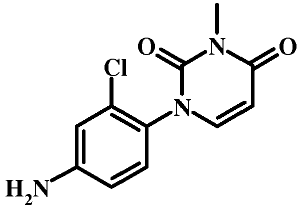

In a 100 mL round bottle, 1-(2-chloro-4-nitro-phenyl)-3-methyl-1H-pyrimidine-2,4-dione
(0.79 g, 2.8 mmol, Eq: 1.00) and zinc (917 mg, 14.0 mmol, Eq: 5.00) were combined with saturated solution of NH₄Cl aqueous solution/ THF (1:1) (11.6 ml), the mixture was stirred at room temperature overnight. Filter out the solid, extracted the solution with CH₂Cl₂ (50 mLx2), dried the organic layer over anhydrous Na₂SO₄, concentrate the solution, get the crude product 0.69 g (98%) MH+ 251.9

### 1-(2-Chloro-4-isothiocyanato-phenyl)-3-methyl-1H-pyrimidine-2,4-dione

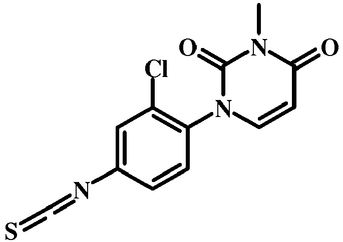

In a 100 mL round-bottomed flask, di(1H-imidazol-1-yl)methanethione (733 mg, 4.11 mmol, Eq: 1.5) was combined with CH₂Cl₂ to give a colorless solution. 1-(4-amino-2-chlorophenyl)-3-methylpyrimidine-2,4(1H,3H)-dione (690 mg, 2.74 mmol, Eq: 1.00) in CH₂Cl₂ (20 mL) was added dropwise at 0°C. The reaction was allowed to warm to room temperature, and allowed to stir overnight. Concentrate the solution, the compound was isolated by column chromatography (Hexanes/EtOAc = 80/20) to give the product 0.69 g (86%).

### [3-Chloro-4-(3-methyl-2,4-dioxo-3,4-dihydro-2H-pyrimidin-1-yl)-phenylamino]-methylsulfanyl-methyl-cyanamide

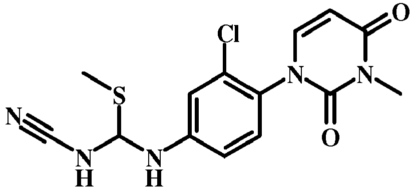

In a 100 mL round-bottomed flask, 1-(2-chloro-4-isothiocyanatophenyl)-3-methylpyrimidine-2,4(1H,3H)-dione (690 mg, 2.35 mmol, Eq: 1.00) in MeOH (20 mL), sodium hydrogencyanamide (170 mg, 2.65 mmol, Eq: 1.13) was added. The suspension turned to clear after a few minutes, the reaction was allowed to stir at room temperature for 1 hour, iodomethane (667 mg, 4.7 mmol, Eq: 2) was added, the reaction mixture was allowed to stir at room temperature overnight. Concentrate the solution, the compound was isolated by column chromatography (Hexanes/EtOAc = 70/30) to give an off-white solid 0.65 g (79%). MH+ 351.9

### 1-[4-(5-Amino-1H-[1,2,4]triazol-3-ylamino)-2-chloro-phenyl]-3-methyl-urea (Compound 62)

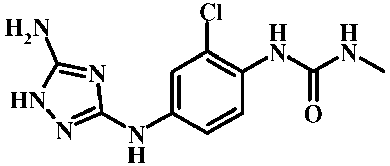

In a 100 mL round-bottomed flask, [3-chloro-4-(3-methyl-2,4-dioxo-3,4-dihydro-2H-pyrimidin-1-yl)-phenylamino]-methylsulfanyl-methyl-cyanamide (650 mg, 1.85 mmol, Eq: 1.00) in EtOH (30 mL), hydrazine (592 mg, 18.5 mmol, Eq: 10.00) was added. The reaction was heated to 65°C for 3 hours. The reaction mixture was concentrated, added H₂O (20 mL) to the residue, filtered out the solid and washed the solid with H₂O (30 mL) and (10 mL), air-dried the solid overnight to give an off-white solid 274 mg (53%). MH+ 282.0

### Example 63

### 4-(5-Amino-1H-[1,2,4]triazol-3-ylamino)-2,6-dichloro-benzoic acid methyl ester (Compound 63)

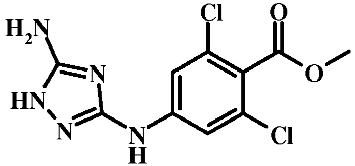

### 2,6-Dichloro-4-isothiocyanato-benzoic acid methyl ester

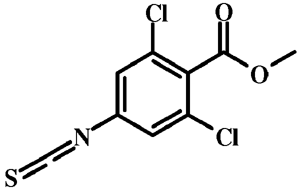

In a 100 mL round-bottomed flask, di(1H-imidazol-1-yl)methanethione (607 mg, 3.41 mmol, Eq: 1.5) was combined with to give a colorless solution. Methyl 4-amino-2,6-dichlorobenzoate (500 mg, 2.27 mmol, Eq: 1.00) in (20 mL) was added dropwise at 0°C. The reaction was allowed to warm to room temperature, and allowed to stir overnight. Concentrate the solution, the compound was isolated by column chromatography (Hexanes/EtOAc = 80/20) to give the product 480 mg (81%).

### 2,6-Dichloro-4-[(cyanoamino-methylsulfanyl-methyl)-amino]-benzoic acid methyl ester

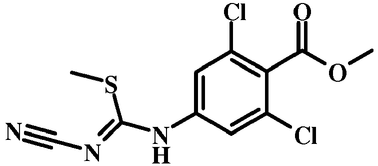

In a 100 mL round-bottomed flask, methyl 2,6-dichloro-4-isothiocyanatobenzoate (480 mg, 1.83 mmol, Eq: 1.00) in MeOH (20 mL), sodium hydrogencyanamide (132 mg, 2.07 mmol, Eq: 1.13) was added. The suspension turned to clear after a few minutes, the reaction was allowed to stir at room temperature for 1 hour, iodomethane (520 mg, 3.66 mmol, Eq: 2) was added, the reaction mixture was allowed to stir at room temperature overnight. Concentrate the solution, the compound was isolated by column chromatography (Hexanes/EtOAc = 70/30) to give an off-white solid 150 mg (26%).

### 4-(5-Amino-1H-[1,2,4]triazol-3-ylamino)-2,6-dichloro-benzoic acid methyl ester (Compound 63)

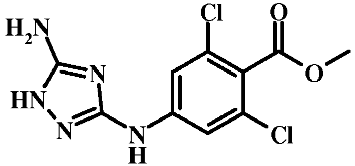

In a 100 mL round-bottomed flask, 2,6-dichloro-4-[(cyanoamino-methylsulfanyl-methyl)-amino]-benzoic acid methyl ester (100 mg, 312 µmol, Eq: 1.00) in EtOH (30 mL), hydrazine (100 mg, 3.12 mmol, Eq: 10.00) was added. The reaction was heated to 65 °C for 3 hours. The reaction mixture was concentrated, added H₂O (20 mL) to the residue, filtered out the solid and washed the solid with H₂O (30 mL) and (10 mL), air-dried the solid overnight to give an off-white solid 85 mg (90%). MH+ 301.9

### Example 64

### 4-(5-Amino-1H-[1,2,4]triazol-3-ylamino)-2,6-dichloro-N-phenyl-benzamide (Compound 64)

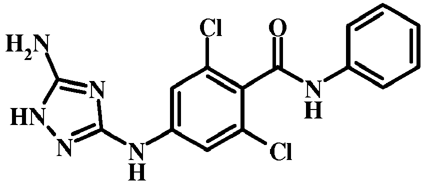

### 2,6-Dichloro-4-nitro-N-phenyl-benzamide

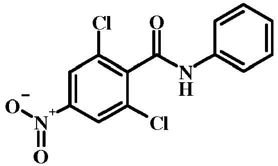

In a 25 mL round-bottomed flask, 2,6-dichloro-4-nitrobenzoic acid (300 mg, 1.27 mmol, Eq: 1.00), N-ethyl-N-isopropylpropan-2-amine (493 mg, 3.81 mmol, Eq: 3.00) and 2-(3H-[1,2,3]triazolo[4,5-b]pyridin-3-yl)-1,1,3,3-tetramethylisouroniumhexafluorophosphate(V) (483 mg, 1.27 mmol, Eq: 1.00) were combined with DMF (5.00 ml) to give a light brown solution. Let the reaction stir for 15 min, aniline (118 mg, 1.27 mmol, Eq: 1.00) was added. The reaction was stirred at room temperature overnight. (30 mL) was added to the reaction mixture, washed with H₂O (20 mL) and Brine (20 mL), the organic layer was dried over anhydrous Na₂SO₄. The compound was isolated by column chromatography (Hexanes/EtOAc = 50/50) to give the product 380 mg (96%). MH+ 310.9

### 4-Amino-2,6-dichloro-N-phenyl-benzamide

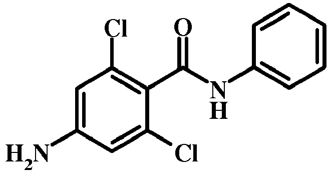

In a 100 mL round bottle, 2,6-dichloro-4-nitro-N-phenylbenzamide (380 mg, 1.22 mmol, Eq: 1.00) and zinc (399 mg, 6.11 mmol, Eq: 5.00) were combined with a solution of saturated NH₄Cl aqueous solution/ THF (1:1) (50.0 ml), the mixture was stirred at for overnight. Filter out the solid, extracted the reaction mixture with (50 mLx2), dried the organic layer over anhydrous Na₂SO₄, concentrate the solution under reduced vacuum, the compound was isolated by column chromatography (Hexanes/EtOAc = 50/50) to give the product 150 mg (44%). MH+ 280.8

### 2,6-Dichloro-4-isothiocyanato-N-phenyl-benzamide

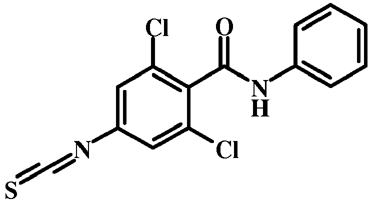

In a 100 mL round-bottomed flask, di(1H-imidazol-1-yl)methanethione (143 mg, 800 µmol, Eq: 1.5) was combined with to give a colorless solution. 4-amino-2,6-dichloro-N-phenylbenzamide (150 mg, 534 µmol, Eq: 1.00) in (20 mL) was added dropwise at 0°C. The reaction was allowed to warm to room temperature, and allowed to stir overnight. Concentrate the solution, the compound was isolated by column chromatography (Hexanes/EtOAc = 80/20) to give the product 100 mg (58%). MH+322.8

### 2,6-Dichloro-4-[(cyanoamino-methylsulfanyl-methyl)-amino]-N-phenyl-benzamide

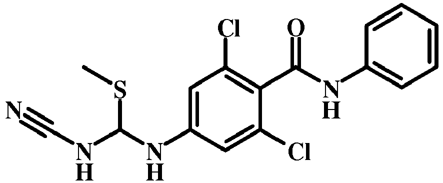

In a 100 mL round-bottomed flask, 2,6-dichloro-4-isothiocyanato-N-phenylbenzamide (100 mg, 309 µmol, Eq: 1.00) in MeOH (20 mL), sodium hydrogencyanamide (22.4 mg, 350 µmol, Eq: 1.13) was added. The suspension turned to clear after a few minutes, the reaction was allowed to stir at room temperature for 1 hour, iodomethane (87.8 mg, 619 µmol, Eq: 2) was added, the reaction mixture was allowed to stir at room temperature overnight. Concentrate the solution, the compound was isolated by column chromatography (Hexanes/EtOAc = 70/30) to give an off-white solid 25 mg (21%). MH+380.8 **4-(5-Amino-1H-[1,2,4]triazol-3-ylamino)-2,6-dichloro-N-phenyl-benzamide (Compound 64)**

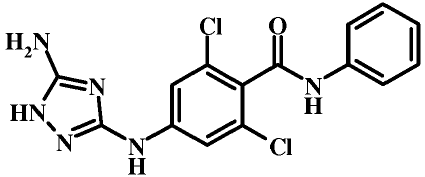

In a 100 mL round-bottomed flask, 2,6-dichloro-4-(cyanamido(methylthio)methylamino)-N-phenylbenzamide (25 mg, 65.6 µmol, Eq: 1.00) in EtOH (30 mL), hydrazine (21.0 mg, 656 µmol, Eq: 10.00) was added. The reaction was heated to 65 °C for 3 hours. The reaction mixture was concentrated, added H₂O (20 mL) to the residue, filtered out the solid and washed the solid with H₂O (30 mL) and (10 mL), air-dried the solid overnight to give an off-white solid 19 mg (80%). MH+ 362.9

### Example 65

### [4-(5-Amino-1H-[1,2,4]triazol-3-ylamino)-2,6-dichloro-phenyl]-pyrrolidin-1-yl-methanone (Compound 65)

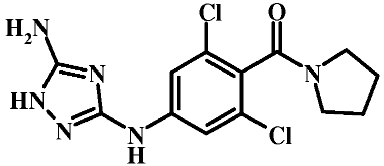

### 2,6-Dichloro-4-nitro-benzoic acid

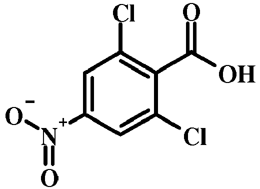

In a 100 mL round-bottomed flask, cetyltrimethylammonium (80.0 mg, 220 µmol, Eq: 0.00452), 1,3-dichloro-2-methyl-5-nitrobenzene (10 g, 48.5 mmol, Eq: 1.00) and were combined with H₂O (200 ml) to give a light yellow suspension. Potassium permanganate (30.7 g, 194 mmol, Eq: 4.00) was added portionwise. The reaction was stir and heated to reflux for 2 days, filtered out the solid, acidify the solution with concentrated HCl to PH=1, added (100 mL x3) to extract the product, concentrate the organic layer to afford the product as an off-white solid 1.1 g (9.6%)

### (2,6-Dichloro-4-nitro-phenyl)-pyrrolidin-1-yl-methanone

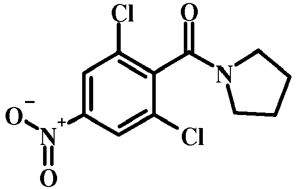

In a 25 mL round-bottomed flask, 2,6-dichloro-4-nitrobenzoic acid (300 mg, 1.27 mmol, Eq: 1.00), N-ethyl-N-isopropylpropan-2-amine (493 mg, 3.81 mmol, Eq: 3.00) and 2-(3H-[1,2,3]triazolo[4,5-b]pyridin-3-yl)-1,1,3,3-tetramethylisouroniumhexafluorophosphate(V) (483 mg, 1.27 mmol, Eq: 1.00) were combined with DMF (5.00 ml) to give a light brown solution. Let the reaction stir for 15 min, pyrrolidine (90.4 mg, 1.27 mmol, Eq: 1.00) was added. The reaction was stirred at room temperature overnight. (30 mL) was added to the reaction mixture, washed with H₂O (20 mL) and Brine (20 mL), the organic layer was dried over anhydrous Na₂SO₄. The compound was isolated by column chromatography (Hexanes/EtOAc = 50/50) to give the product 300 mg (82%). MH+ 288.9

### (4-Amino-2,6-dichloro-phenyl)-pyrrolidin-1-yl-methanone

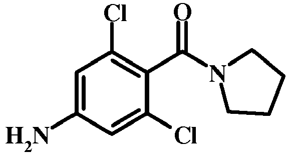

In a 100 mL round bottle, (2,6-dichloro-4-nitrophenyl)(pyrrolidin-1-yl)methanone (300 mg, 1.04 mmol, Eq: 1.00) and zinc (339 mg, 5.19 mmol, Eq: 5.00) were combined with a solution of saturated NH₄Cl aqueous solution/ THF (1:1) (50.0 ml). The mixture was stirred at for overnight. Filter out the solid, extracted the reaction mixture with (50 mLx2), dried the organic layer over anhydrous Na₂SO₄, concentrate the solution under reduced vacuum, the compound was isolated by column chromatography (Hexanes/EtOAc = 50/50) to give the product 250 mg (93%). MH+ 258.9

### (2,6-Dichloro-4-isothiocyanato-phenyl)-pyrrolidin-1-yl-methanone

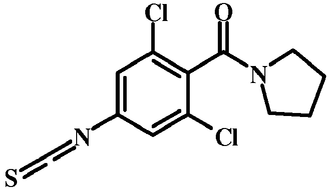

In a 100 mL round-bottomed flask, di(1H-imidazol-1-yl)methanethione (258 mg, 1.45 mmol, Eq: 1.5) was combined with to give a colorless solution. (4-amino-2,6-dichlorophenyl)(pyrrolidin-1-yl)methanone (250 mg, 965 µmol, Eq: 1.00) in (20 mL) was added dropwise at 0°C. The reaction was allowed to warm to room temperature, and allowed to stir overnight. Concentrate the solution, the compound was isolated by column chromatography (Hexanes/EtOAc = 80/20) to give the product 200 mg (69%). MH+300.9

### [3,5-Dichloro-4-(pyrrolidine-1-carbonyl)-phenylamino]-methylsulfanyl-methyl-cyanamide

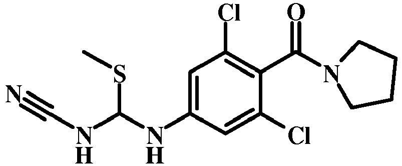

In a 100 mL round-bottomed flask, (2,6-dichloro-4-isothiocyanatophenyl)(pyrrolidin-1-yl)methanone (200 mg, 664 µmol, Eq: 1.00) in MeOH (20 mL), sodium hydrogencyanamide (48.0 mg, 750 µmol, Eq: 1.13) was added. The suspension turned to clear after a few minutes, the reaction was allowed to stir at room temperature for 1 hour, iodomethane (189 mg, 1.33 mmol, Eq: 2) was added, the reaction mixture was allowed to stir at room temperature overnight. Concentrate the solution, the compound was isolated by column chromatography (Hexanes/EtOAc = 70/30) to give an off-white solid 90 mg (38%). MH+358.8

### [4-(5-Amino-1H-[1,2,4]triazol-3-ylamino)-2,6-dichloro-phenyl]-pyrrolidin-1-yl-methanone (Compound 65)

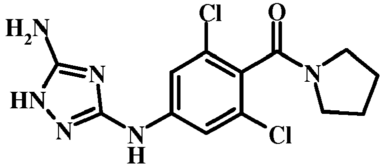

In a 100 mL round-bottomed flask, [3,5-dichloro-4-(pyrrolidine-1-carbonyl)-phenylamino]-methylsulfanyl-methyl-cyanamide (90 mg, 251 µmol, Eq: 1.00) in EtOH (30 mL), hydrazine (80.3 mg, 2.51 mmol, Eq: 10.00) was added. The reaction was heated to 65 °C for 3 hours. The reaction mixture was concentrated, added H₂O (20 mL) to the residue, filtered out the solid and washed the solid with H₂O (30 mL) and (10 mL), air-dried the solid overnight to give an off-white solid 47 mg (55%). MH+ 340.9

### Example 66

### 4-(5-Amino-1H-[1,2,4]triazol-3-ylamino)-2,6-dichloro-N-(3-methanesulfonyl-phenyl)-benzamide (Compound 66)

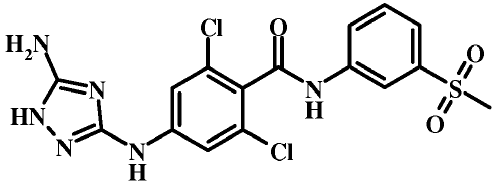

### 2,6-Dichloro-N-(3-methanesulfonyl-phenyl)-4-nitro-benzamide

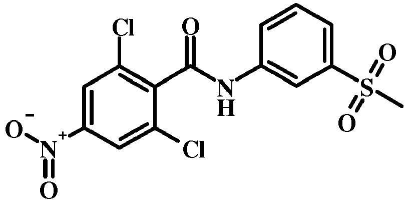

In a 25 mL round-bottomed flask, 2,6-dichloro-4-nitrobenzoic acid (300 mg, 1.27 mmol, Eq: 1.00), N-ethyl-N-isopropylpropan-2-amine (493 mg, 3.81 mmol, Eq: 3.00) and 2-(3H-[1,2,3]triazolo[4,5-b]pyridin-3-yl)-1,1,3,3-tetramethylisouroniumhexafluorophosphate(V) (483 mg, 1.27 mmol, Eq: 1.00) were combined with DMF (5.00 ml) to give a light brown solution. Let the reaction stir for 15 min, 3-(methylsulfonyl)aniline hydrochloride (264 mg, 1.27 mmol, Eq: 1.00) was added. The reaction was stirred at room temperature overnight. (30 mL) was added to the reaction mixture, washed with H₂O (20 mL) and Brine (20 mL), the organic layer was dried over anhydrous Na₂SO₄. The compound was isolated by column chromatography (Hexanes/EtOAc = 50/50) to give the product 200 mg (40%). MH+ 386.9

### 4-Amino-2,6-dichloro-N-(3-methanesulfonyl-phenyl)-benzamide

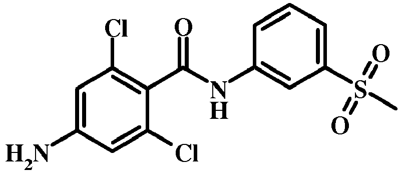

In a 50 mL round-bottomed flask, 2,6-dichloro-N-(3-(methylsulfonyl)phenyl)-4-nitrobenzamide (200 mg, 514 µmol, Eq: 1.00), zinc (168 mg, 2.57 mmol, Eq: 5.00) were combined with a solution of saturated NH₄Cl aqueous solution/ THF (1:1) (10.00 ml). The mixture was stirred at for overnight. Filter out the solid, extracted the reaction mixture with (50 mLx2), dried the organic layer over anhydrous Na₂SO₄, concentrate the solution under reduced vacuum, the compound was isolated by column chromatography (Hexanes/EtOAc = 50/50) to give the product 150 mg (81%). MH+ 358.8

### 2,6-Dichloro-4-isothiocyanato-N-(3-methanesulfonyl-phenyl)-benzamide

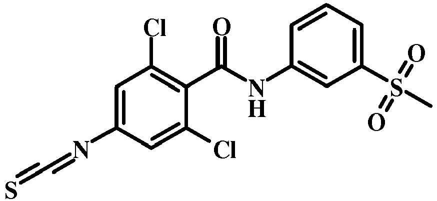

In a 100 mL round-bottomed flask, di(1H-imidazol-1-yl)methanethione (112 mg, 626 µmol, Eq: 1.5) was combined with to give a colorless solution. 4-Amino-2,6-dichloro-N-(3-(methylsulfonyl-)phenyl)benzamide (150 mg, 418 µmol, Eq: 1.00) in (20 mL) was added dropwise at 0°C. The reaction was allowed to warm to room temperature, and allowed to stir overnight. Concentrate the solution, the compound was isolated by column chromatography (Hexanes/EtOAc = 60/40) to give the product 70 mg (42%). MH+400.8

### 2,6-Dichloro-4-[(cyanoamino-methylsulfanyl-methyl)-amino]-N-(3-methanesulfonylphenyl)-benzamide

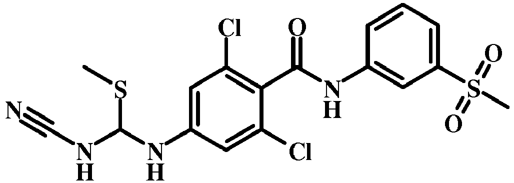

In a 100 mL round-bottomed flask, 2,6-dichloro-4-isothiocyanato-N-(3-(methylsulfonyl)phenyl)-benzamide (70 mg, 174 µmol, Eq: 1.00) in MeOH (20 mL), sodium hydrogencyanamide (12.6 mg, 197 µmol, Eq: 1.13) was added. The suspension turned to clear after a few minutes the reaction was allowed to stir at room temperature for 1 hour, iodomethane (49.5 mg, 349 µmol, Eq: 2) was added, the reaction mixture was allowed to stir at room temperature overnight. Concentrate the solution, the compound was isolated by column chromatography (Hexanes/EtOAc = 70/30) to give an off-white solid 15 mg (19%). MH+458.8

### 4-(5-Amino-1H-[1,2,4]triazol-3-ylamino)-2,6-dichloro-N-(3-methanesulfonyl-phenyl)-benzamide (Compound 66)

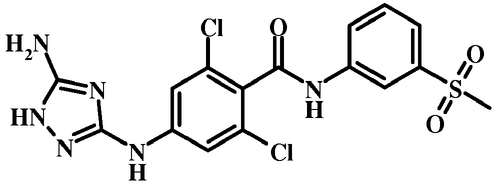

In a 100 mL round-bottomed flask, 2,6-dichloro-4-(cyanamido(methylthio)methylamino)-N-(3-(methylsulfonyl)phenyl)benzamide (15 mg, 32.7 µmol, Eq: 1.00) in EtOH (30 mL), hydrazine (10.5 mg, 327 µmol, Eq: 10.00) was added. The reaction was heated to 65 °C for 3 hours. The reaction mixture was concentrated, added H₂O (20 mL) to the residue, filtered out the solid and washed the solid with H₂O (30 mL) and CH₂Cl₂ (10 mL), air-dried the solid overnight to give an off-white solid 5.5 mg (38%). MH+ 440.8

### Example 67

### N*3*-(3,5-Dichloro-4-vinyl-phenyl)-1H-[1,2,4]triazole-3,5-diamine (Compound 67)

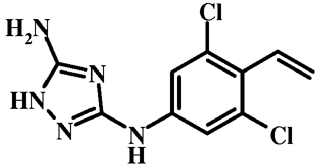

In a 15 mL sealed tube, **compound 1** (1.5 g, 4.64 mmol, Eq: 1.00), tetrakis(triphenylphosphine)-palladium (0) (537 mg, 464 µmol, Eq: 0.1) and potassium trifluoro(vinyl)borate (933 mg, 6.97 mmol, Eq:1.5) were combined with 1,4-dioxane/H₂O = 4/1 (25.0 ml) to give a light yellow suspension. Potassium carbonate (2.57 g, 18.6 mmol, Eq: 4) was added. The reaction mixture was degassed with argon for 15 min, and then heated to 120°C for overnight. The reaction mixture was cooled and diluted with CH₂Cl₂ (50 mL), washed with H₂O (25 mL) and brine (25 mL). The organic layer was dried over anhydrous MgSO₄, filtered and volatiles were removed under reduced pressure to yield an oil from which the compound was isolated by column chromatography (CH₂Cl₂/MeOH = 95/5) to give an off-white solid (340 mg, 27%). MH+271.8

### Example 68

### N⁵-(4-Cyano-3-fluoro-5-trifluoromethylphenyl)-1H-[1,2,4]-triazole-3,5-diamine (Compound 68)

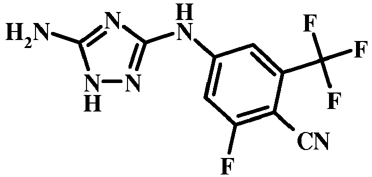

To a 5 mL microwave vial was added **compound 45** (100 mg, 294 µmol, Eq: 1.00), Pd(PPh₃)₄ (408 mg, 353 µmol, Eq: 1.2) and zinc cyanide (41.4 mg, 353 µmol, Eq: 1.2) in DMF (2 ml). The vial was capped and heated in the microwave under argon at 120°C for 30 minutes. The reaction was diluted in ethyl acetate (10ml) and filtered. The filtrate was washed with water, dried over sodium sulfate and concentrated. The crude material was purified by preparative HPLC (0.1%TFA in water/0.1% TFA in AcCN) 95% to 10% over 16 minutes to afford 27 mg (25%) of the desired product as a white solid. MS +m/z: 286.9. (M+1)

### Example 69

### N⁵-(4-Cyano-3,5-difluorophenyl)-1H-[1,2,4]-triazole-3,5-diamine (Compound 69)

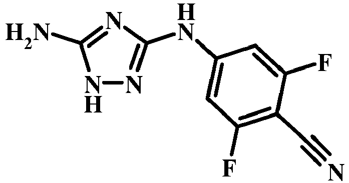

To a 5 mL microwave vial was added **compound 46** (100 mg, 345 µmol, Eq: 1.00), Pd(PPh₃)₄ (478 mg, 414 µmol, Eq: 1.2) and zinc cyanide (48.6 mg, 414 µmol, Eq: 1.2) in DMF (2.00 ml). The vial was capped and heated in the microwave under argon at 125°C for 30 minutes. The reaction mixture was filtered over a plug of silica gel and washed with dioxane. The filtrate was concentrated and purified by preparative HPLC (0.1 %TFA in water/0.1 % TFA in AcCN) 95% to 10% over 16 minutes to afford 10 mg (8%) of the desired product as a white solid. MS +m/z: 237.0. (M+1)
¹H NMR (300 MHz, DMSO-d₆) δ ppm 6.11 (s, 2 H) 7.38 (d, *J*=12.47 Hz, 2 H) 10.07 (br. s., 1 H) 11.57 (br. s., 1 H)

### Example 70

### N*3*-(3-pentafluorosulfur-phenyl)-1H-[1,2,4]triazole-3,5-diamine (Compound 70)

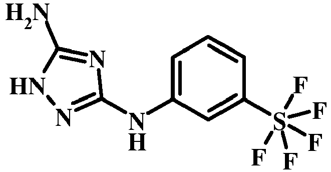

### 3-Isothiocyanato-phenylsulfur pentafluoride

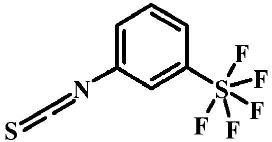

To a cold (0°C) suspension of 3-aminophenylsulfur pentafluoride (1.5 g, 6.84 mmol, Eq: 1.00) and calcium carbonate (1.37 g, 13.7 mmol, Eq: 2) in water (15 ml) and dichloromethane (15 ml) was added thiophosgene (881 mg, 584 µl, 7.67 mmol, Eq: 1.12). The reaction mixture was allowed to warm to room temperature and was vigorously stirred for 24h. IN HCl (14 mL) was added to adjust the pH of the aqueous layer to 6-7. The organic layer was collected and the aqueous layer was extracted one time with dichloromethane. Combined organic layers were concentrated, leading to 1.8 g of a brown-orange liquid. This liquid was purified on silicagel (silica 40g, Hexanes 100% for 5 min then Hexane/ethyl acetate 1:0 to 4:1). One fraction was isolated and dried in vacuo, leading to 1.64 g (92%) of a non-viscous colorless oil.

### (Z)-methyl-N-3-pentafluorosulfurbenz-1-yl-N'-cyanocarbamimidothioate

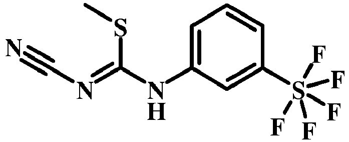

To a sodium methoxide solution in methanol (0.5 M, 15.1 ml, 7.53 mmol, Eq: 1.2) was added at room temperature cyanamide (298 mg, 7.09 mmol, Eq: 1.13). The mixture was stirred 15 min at room temperature then a solution of 3-isothiocyanato-phenylsulfur pentafluoride (1.64g, 6.28 mmol, Eq: 1.00) in dry methanol (18 ml) was added to the reaction media.
After 1h, iodomethane (1.82 g, 799 µl, 12.8 mmol, Eq: 2.04) was added and the reaction mixture was stirred overnight at room temperature. The reaction mixture was adsorbed unto silica (9g), concentrated and purified on silica gel (silica 120g, hexane/ethyl acetate 90:10 to 40:60 within 30 min). One fraction was isolated and dried in vacuo to afford 1.5 g (73%) of the desired product as a white solid. MS -m/z: 308.2 (M-H)⁻

### N*3*-(3-pentafluorosulfur-phenyl)-1H-[1,2,4]triazole-3,5-diamine (Compound 70)

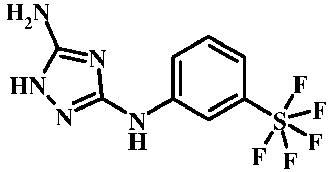

A solution of (Z)-methyl-N-3-pentafluorosulfurbenz-1-yl-N'-cyanocarbamimidothioate (0.4 g, 1.26 mmol, Eq: 1.00) and hydrazine (408 mg, 400 µl, 12.7 mmol, Eq: 10.1) in dry ethanol (10 ml) was stirred overnight at 70°C. The reaction mixture was adsorbed on silica (2g) then purified on silica gel (column 40 g, dichloromethane / methanol 95:5 to 70:30 within 30 min). One fraction was isolated and dried in vacuo to afford 348 mg (92%) of the desired product as a white solid.
MS +m/z: 301.9 (M+H)⁺

### Example 71

### 1-Methyl-N*5*-(3-pentanuorosulfur-phenyl)-1H-[1,2,4]triazole-3,5-diamine (Compound 71)

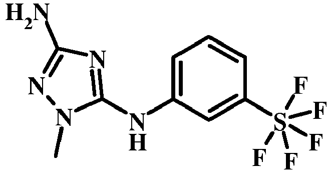

A solution of (Z)-methyl-N-3-pentafluorosulfurbenz-1-yl-N'-cyanocarbamimidothioate (400mg, 1.26 mmol, Eq: 1.00) and methylhydrazine (581 mg, 664 µl, 12.6 mmol, Eq: 10) was stirred overnight at 70°C. The reaction mixture was concentrated and purified using silica gel (adsorption on silica 2 g, silica 40 g, dichloromethane / methanol 95:5 to 70:30). One fraction was isolated and dried in vacuo to afford 394 mg (99%) of the desired product as a white solid. NMR (300 MHz, DMSO d⁶): 9.08 (1H, s); 8.11 (1H, t, J= 1.2 Hz); 7.88 (1H, broad d, J=8 Hz); 7.49 (1H, broad t, J= 8 Hz); 7.34 (1H, dd, J= 8, 1 Hz); 5.11 (2H, s); 3.51 (3H, s). Position of the methyl on the triazole ring was identified after NMR NOE experiment (irradiation at δ 3.24 ppm leading to a NOE effect at δ 9.08 ppm). MS +m/z: 316.0 (M+H)⁺

### Example 72

### 1-Cyclohexyl-N*5*-(3-pentafluorosulfur-phenyl)-1H-[1,2,4]triazole-3,5-diamine (Compound 72)

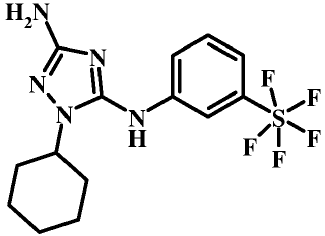

A solution of (Z)-methyl-N-3-pentafluorosulfurbenz-1-yl-N'-cyanocarbamimidothioate (40 mg, 126 µmol, Eq: 1.00), ethyldiisopropylamine (179 mg, 230 µl, 1.39 mmol, Eq: 11) and cyclohexylhydrazine hydrochloride (190 mg, 1.26 mmol, Eq: 10) in Ethanol dry (2 ml) was stirred overnight at 70°C. The reaction mixture was concentrated in vacuo and purified on silica gel (silica 8 g, dichloromethane /methanol 100:0 to 70:30). One fraction was isolated and dried in vacuo to afford 40 mg (83%) of the desired product as a colorless semisolid.
NMR (300 MHz, DMSO d⁶): 9.20 (1H, s); 8.21 (1H, broad s); 7.64 (1H, broad d, J=8 Hz); 7.37 (1H, broad t, J= 8 Hz); 7.18 (1H, broad d, J= 8 Hz); 6.11 (2H, s); 4.03-3.88 (1H, m); 3.68-3.52 (1H, m); 3.20-3.07 (1H, m); 1.90-1.59 (6H, m); 1.45-1.20 (2H, m).
MS +m/z: 384.1 (M+H)⁺

### Example 73

### N*3*-(3-Fluoro-5-pentafluorosulfur-phenyl)-1H-[1,2,4]triazole-3,5-diamine (Compound 73)

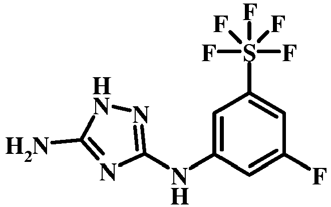

### 3-amino-5-fluoro-phenylsulfurpentafluoride

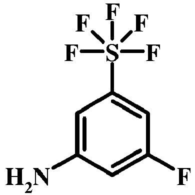

A flask containing 3-nitro-5-fluoro-phenylsulfurpentafluoride (2.56 g, 9.58 mmol, Eq: 1.00), iron (2.61 g, 46.7 mmol, Eq: 4.88) and ammonium chloride (5.0 g, 93.5 mmol, Eq: 9.75) was flushed with nitrogen (cycle nitrogen/vacuum) then methanol (40 ml) and water (20 ml) were added. The reaction mixture was refluxed 1.5h (oil bath at 90°C) then cooled down to room temperature and filtered on a patch of Celite. The patch was washed with methanol. The filtrate was dried in vacuo and the residue was dissolved in ethyl acetate.

The organic layer was washed with aqueous sat. sodium hydrogenocarbonate, water and brine then dried over sodium sulfate, filtered and dried in vacuo to afford 1.42 (63%) of the desired product as a light yellow oil. This oil was used in the next step without further purification.

### 5-fluoro-3-isothiocyanato-phenylsulfur-pentafluoride

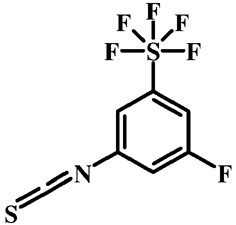

To a cold (0°C) suspension of 3-amino-5-fluoro-phenylsulfurpentafluoride (1.42 g, 5.99 mmol, Eq: 1.00) and calcium carbonate (1.2 g, 12.0 mmol, Eq: 2) in water (13 ml) and dichloromethane (13.0 ml) was added thiophosgene (771 mg, 511 µl, 6.71 mmol, Eq: 1.12). The reaction mixture was allowed to warm up to room temperature and was vigorously stirred for 24h. IN HCl (12 mL) was added to adjust the pH to ca. 6. The organic layer was collected and the aqueous layer was extracted one time with dichloromethane. Combined organic layers were washed with brine, adsorbed unto silica (5g) and purified on silica gel (column 80 g, hexane/ethyl acetate 1:0 to 4:1). One fraction was isolated and dried in vacuo to afford 1.34 g (80%) of the desired product as a colorless oil.

### (Z)-methyl-N-(3-pentafluorosulfur-5-fluoro)-benz-1-yl-N'-cyanocarbamimidothioate

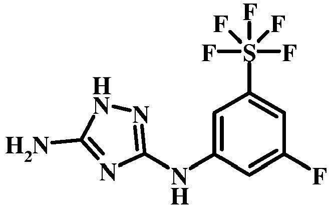

To a solution of 5-fluoro-3-isothiocyanato-phenylsulfur-pentafluoride (1.34 g, 4.8 mmol, Eq: 1.00) in dry methanol (14 ml) was added sodium hydrogencyanamide (347 mg, 5.42 mmol, Eq: 1.13). The light yellow reaction solution was stirred 1h at room temperature then iodomethane (1.36 g, 9.6 mmol, Eq: 2) was added and the reaction mixture was stirred overnight at room temperature. The reaction mixture was adsorbed unto silica (5g) and purified on silica gel (silica 80g, Hexane/Ethyl acetate 95:5 to 40:60). One fraction was isolated and dried in vacuo to afford 1.11 g (69%) of the desired product as a white solid.
MS +m/z: 335.9 (M+H)⁺

### N*3*-(3-Fluoro-5-pentafluorosulfur-phenyl)-1H-[1,2,4]triazole-3,5-diamine (Compound 73)

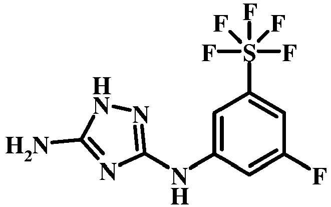

A solution of (Z)-methyl-N-(3-pentafluorosulfur-5-fluoro)-benz-1-yl-N'-cyanocarbamimidothioate (200 mg, 596 µmol, Eq: 1.00) and hydrazine (191 mg, 187 µl, 5.96 mmol, Eq: 10) was stirred overnight at 70°C. The reaction mixture was adsorbed unto silica (0.8g) and purified on silica gel (column 8 g, dichloromethane/methanol 95:5 to 70:30). One fraction was isolated and dried in vacuo to afford 190 mg (quant.) of the desired product as a white solid. MS +m/z: 318.0 (M+H)⁺

### Example 74

### N*5*-(3-Fluoro-5-pentafluorosulfur-phenyl)-1-methyl-1H-[1,2,4]triazole-3,5-diamine (Compound 74)

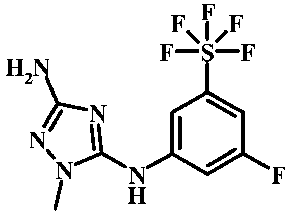

A solution of (Z)-methyl-N-(3-pentafluorosulfur-5-fluoro)-benz-1-yl-N'-cyanocarbamimidothioate (200 mg, 596 µmol, Eq: 1.00) and methylhydrazine (275 mg, 314 µl, 5.96 mmol, Eq: 10) in ethanol dry (6 ml) was stirred overnight at 70°C.
The reaction mixture was purified on silica gel (column 8 g, dichloromethane/methanol 100:0 to 75:25). One fraction was isolated and dried in vacuo to afford 216 mg (quant.) of the desired product as a colorless viscous oil.
NMR (300 MHz, DMSO d⁶): 9.40 (1H, s); 7.98 (1H, broad d, J= 8 Hz); 7.85 (1H, broad s); 7.32 (1H, broad d, J= 8 Hz); 5.2 (2H, s); 3.52 (3H, s).
MS +m/z: 334.0 (M+H)⁺

### Example 75

### N*3*-(4-pentafluorosulfur-phenyl)-1H-[1,2,4]triazole-3,5-diamine (Compound 75)

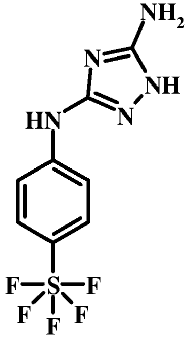

### 4-isothiocyanato-phenylsulfur-pentafluoride

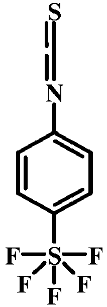

To a cold (0°C) suspension of 4-pentafluorosulfur-phenylamine (1.31 g, 5.99 mmol, Eq: 1.00) and calcium carbonate (1.22 g, 12.2 mmol, Eq: 2.03) in water (13 ml) and dichloromethane (13.0 ml) was added thiophosgene (771 mg, 511 µl, 6.7 mmol, Eq: 1.12). The reaction mixture was allowed to warm to room temperature and was vigorously stirred 4h (biphasic, 1000 rpm). IN HCl (12 mL) was added to adjust the pH to ca. 6. The organic layer was collected and the aqueous layer was extracted one time. Combined organic layers were washed with brine, adsorbed unto silica (5g), and purified on silicagel (column 80 g, Hexane/Ethyl acetate 100:0 to 90:10). One fraction was isolated and dried in vacuo to afford 1.41 g (90%) of the desired product as a white solid.

### (Z)-methyl-N-(4-pentafluorosulfur)-benz-1-yl-N'-cyanocarbamimidothioate

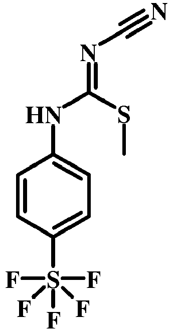

To a solution of 4-isothiocyanato-phenylsulfur-pentafluoride (1.4 g, 5.36 mmol, Eq: 1.00) in dry methanol (15 ml) was added sodium hydrogencyanamide (360 mg, 5.63 mmol, Eq: 1.05). The light yellow reaction solution was stirred 1h at room temperature then iodomethane (1.52 g, 746 µl, 10.7 mmol, Eq: 2) was added and the reaction mixture was stirred overnight at room temperature. The reaction mixture was adsorbed unto silica (5g) and purified on silica gel (silica 80g, Hexane/Ethyl acetate 90:10 to 0:100). 1 Fraction was isolated and dried in vacuo to afford 1.055 g (62%) of the desired product as a white solid.

### N*3*-(4-pentafluorosulfur-phenyl)-lH-[1,2,4]triazole-3,5-diamine (Compound 75)

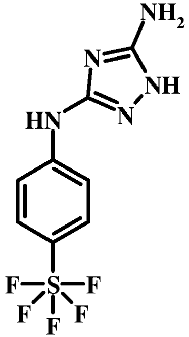

A solution of (Z)-methyl-N-(4-pentafluorosulfur)-benz-1-yl-N'-cyanocarbamimidothioate (200 mg, 630 µmol, Eq: 1.00) and hydrazine (202 mg, 198 µl, 6.3 mmol, Eq: 10) in ethanol dry (6 ml) was stirred overnight at 70°C. The reaction mixture was adsorbed unto silica (1.5g) and purified on silicagel (column 20 g, dichloromethane/ methanol 95:5 to 70:30). One fraction was isolated and dried in vacuo to afford 170 mg (90%) of the desired product as a white solid.
MS +m/z: 301.9 (M+H)⁺

### Example 76

### N3-(3-chloro-4-methylphenyl)-1H-1,2,4-triazole-3,5-diamine (Compound 76)

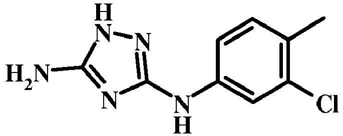

### (Z)-methyl N-3-chloro-4-methylphenyl-N'-cyanocarbamimidothioate

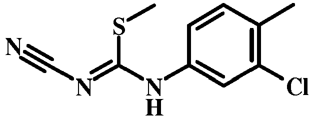

To a solution of 2-chloro-4-isothiocyanato-1-methylbenzene (0.5 g, 2.72 mmol, Eq: 1.00) in dry methanol (8 ml) was added sodium hydrogencyanamide (209 mg, 3.27 mmol, Eq: 1.2). The reaction mixture was stirred 1h at room temperature then iodomethane (773 mg, 5.44 mmol, Eq: 2) was added and the reaction mixture was stirred 4h at room temperature. The strong precipitate was filtered and the solid was washed with dry methanol and dried in vacuo to afford 360 mg (55%) of the desired product as a white solid.

### N3-(3-chloro-4-methylphenyl)-1H-1,2,4-triazole-3,5-diamine (Compound 76)

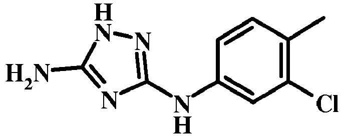

A mixture of (Z)-methyl N-3-chloro-4-methylphenyl-N'-cyanocarbamimidothioate (350 mg, 1.46 mmol, Eq: 1.00), hydrazine (468 mg, 14.6 mmol, Eq: 10) in dry ethanol (4 ml) was stirred overnight at 70°C (oil bath temperature). The reaction mixture was adsorbed unto silica (2 g) then purified silicagel (column 24 g, dichloromethane/methanol 95:5 to 70:30).
One fraction was isolated and dried in vacuo to afford 300 mg (92%) of the desired product as an off-white solid. MS +m/z: 223.9 (M+H)⁺

### Example 7

### N3-(3-chloro-4-(methylsulfonyl)phenyl)-lH-l,2,4-triazole-3,5-diamine (Compound 77)

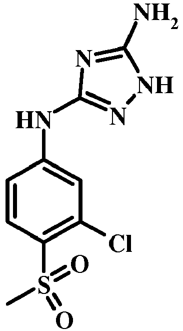

### 2-chloro-4-isothiocyanato-1-(methylsulfonyl)benzene

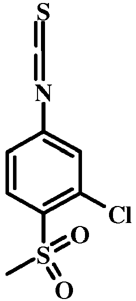

To a cold (0°C) suspension of 3-chloro-4-(methylsulfonyl)aniline (1.03 g, 5 mmol, Eq: 1.00) and calcium carbonate (1.00 g, 10.0 mmol, Eq: 2) in water (10 ml) and dichloromethane (10.0 ml) was added thiophosgene (690 mg, 457 µl, 6.00 mmol, Eq: 1.2). The reaction mixture was allowed to warm to room temperature and was vigorously stirred for 24h. IN HCl (12 mL) was added to adjust the pH at ca. 4-5. The organic layer was collected and the aqueous layer was extracted one time with dichloromethane. Combined organic layers were washed with brine, adsorbed unto silica (3g) and purified on silicagel (column 40 g, Hexane/ethyl acetate 90:10 to 70:30). One fraction was isolated and dried in vacuo, leading to 1.09g (88%) of the desired product as a light yellow solid.

### (Z)-methyl N-3-chloro-4-(methylsulfonyl)phenyl-N'-cyanocarbamimidothioate

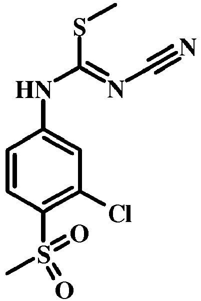

To a solution of 2-chloro-4-isothiocyanato-1-(methylsulfonyl)benzene (1.08 g, 4.36 mmol, Eq: 1.00) in methanol dry (12 ml) was added sodium hydrogencyanamide (293 mg, 4.58 mmol, Eq: 1.05). The light yellow reaction solution was stirred 1h at room temperature then iodomethane (1.24 g, 607 µl, 8.72 mmol, Eq: 2) was added and the reaction mixture was stirred overnight at room temperature. The reaction mixture was partitioned between HCl 1M and ethyl acetate. The aqueous layer was extracted 4 times with ethyl acetate. Combined organic layers were adsorbed unto silica (5g), concentrated and purified on silicagel (silica 80g, dichloromethane/methanol 100:0 to 80:20). One fraction was isolated and dried in vacuo to afford 180 mg (14%) of the desired product.

### N3-(3-chloro-4-(methylsulfonyl)phenyl)-1H-1,2,4-triazole-3,5-diamine (Compound 77)

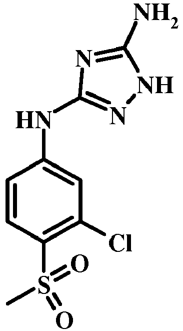

A mixture of (Z)-methyl N-3-chloro-4-(methylsulfonyl)phenyl-N'-cyanocarbamimidothioate (180 mg, 593 µmol, Eq: 1.00), hydrazine (190 mg, 187 µl, 5.93 mmol, Eq: 10) in dry ethanol (2 ml) was stirred overnight at 70°C. After 3h at 70°C, the reaction mixture was cooled down, filtered and the solid was washed with dry ethanol, then dried in vacuo, to afford 27 mg (16%) of the desired product as a white solid.
MS +m/z: 287.9 (M+H)⁺

### Example 78

### 4-(5-amino-1H-1,2,4-triazol-3-ylamino)-2-chloro-N,N-dimethylbenzamide (Compound 78)

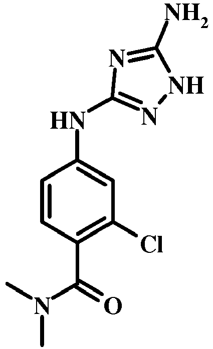

### 2-chloro-4-isothiocyanato-N,N-dimethylbenzamide

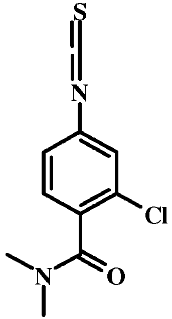

To a cold (0°C) suspension of 4-amino-2-chloro-N,N-dimethylbenzamide (993 mg, 5 mmol, Eq: 1.00) and calcium carbonate (1.00 g, 10.0 mmol, Eq: 2) in water (10 ml) and dichlormethane (10.0 ml) was added thiophosgene (690 mg, 457 µl, 6.00 mmol, Eq: 1.2).
The reaction mixture was allowed to warm to room temperature and was vigorously stirred for 24h. IN HCl (14 mL) was added to adjust the pH to ca.4- 5. The organic layer was collected and the aqueous layer was extracted one time with dichloromethane. Combined organic layers were washed with brine, adsorbed unto silica (3g) and purified on silicagel (column 40 g, dichloromethane/ethyl acetate 100:0 to 80:20). One fraction was isolated and dried in vacuo to afford 1.067g (89%) of the desired product as a white solid.

### (Z)-methyl N-3-chloro-4-(dimethylcarbamoyl)phenyl-N'-cyanocarbamimidothioate

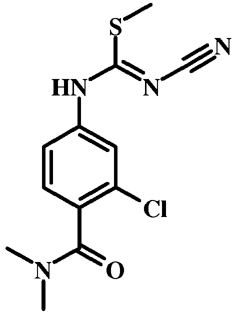

To a solution of 2-chloro-4-isothiocyanato-N,N-dimethylbenzamide (1.06 g, 4.4 mmol, Eq: 1.00) in methanol dry (12 ml) was added sodium hydrogencyanamide (296 mg, 4.62 mmol, Eq: 1.05). The light yellow reaction solution was stirred 1h at room temperature then iodomethane (1.25 g, 613 µl, 8.81 mmol, Eq: 2) was added and the reaction mixture was stirred overnight at room temperature. The reaction mixture was adsorbed unto silica (5g) and purified on silica gel (silica 80g, dichloromethane/methanol 100:0 to 80:20). One Fraction was isolated and dried in vacuo to afford 1.06 g (81%) of the desired product as a white solid.

### 4-(5-amino-1H-1,2,4-triazol-3-ylamino)-2-chloro-N,N-dimethylbenzamide (Compound 78)

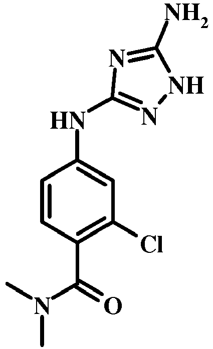

A mixture of (Z)-methyl N-3-chloro-4-(dimethylcarbamoyl)phenyl-N'-cyanocarbamimidothioate (1 g, 3.37 mmol, Eq: 1.00), hydrazine (1.08 g, 1.07 ml, 33.7 mmol, Eq: 10) in dry ethanol (10 ml) was stirred overnight at 70°C (oil bath temperature). The reaction mixture was adsorbed unto silica (2 g) then purified on silicagel (column 40 g, dichloromethane/methanol 95:5 to 70:30). One fraction was isolated and dried in vacuo to afford 570 mg (60%) of the desired product as a white solid. MS +m/z: 281.0 (M+H)⁺

### Example 79

### N3-(3,5-dichloro-4-(3,3-dimethylbut-1-ynyl)phenyl)-1H-1,2,4-triazole-3,5-diamine (Compound 79)

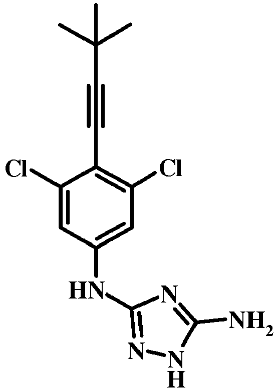

### 3,5-dichloro-4-(3,3-dimethylbut-1-ynyl)nitrobenzene

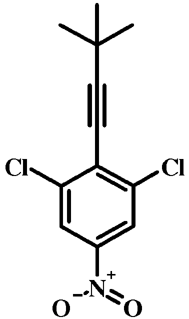

A mixture of 1,3-dichloro-2-iodo-5-nitrobenzene (1.12 g, 3.53 mmol, Eq: 1.00), bis(triphenylphosphine)palladium chloride (247 mg, 353 µmol, Eq: 0.1) and copper iodide (134 mg, 706 µmol, Eq: 0.2) were degassed (cycles vacuum/nitrogen) then degassed (nitrogen bubbling with sonication) dry THF (9 ml) and diisopropylamine (1.43 g, 1.99 ml, 14.1 mmol, Eq: 4) were added as a mixture. After 15 min stirring at room temperature, degassed (nitrogen bubbling with sonication) 3,3-dimethylbut-1-yne (1.16 g, 1.74 ml, 14.1 mmol, Eq: 4) was added. The reaction mixture was stirred at 40 C for 4 days then was adsorbed unto silica (3g) and purified on silicagel (column 50 g, Hexane/ethyl acetate 100:0 to 80:20) to afford 820 mg (84%) of the desired product as a yellow semi solid.

### 3,5-dichloro-4-(3,3-dimethylbut-l-ynyl)aniline

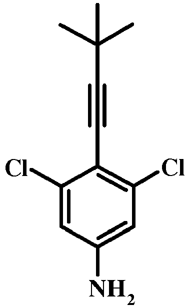

A mixture of 1,3-dichloro-2-(3,3-dimethylbut-1-ynyl)-5-nitrobenzene (850 mg, 3.12 mmol, Eq: 1.00),iron (872 mg, 15.6 mmol, Eq: 5) and ammonium chloride (1.67 g, 31.2 mmol, Eq: 10) in methanol (10 ml) and water (5 ml) was refluxed for 2h. The resulting suspension was filtered over a Celite pad and the pad was washed with ethyl acetate, then the organic layer was concentrated in vacuo. The residue was partitioned between ethyl acetate and water. The aqueous layer was extracted 3 times with ethyl acetate then combined organic layers were washed with brine, adsorbed unto silica (2.5g) and purified on silicagel (column 40 g, Hexane/ethyl acetate 95:5 to 70:30). One fraction was isolated and dried in vacuo, to afford 588 mg (70%) of the desired product as a yellow oil.

### 1,3-dichloro-2-(3,3-dimethylbut-1-ynyl)-5-isothiocyanatobenzene

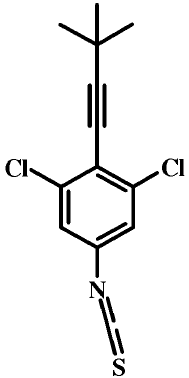

To a cold (0°C) suspension of 3,5-dichloro-4-(3,3-dimethylbut-1-ynyl)aniline (580 mg, 2.4 mmol, Eq: 1.00) and calcium carbonate (479 mg, 4.79 mmol, Eq: 2) in water (5 ml) and dichloromethane (5.00 ml) was added thiophosgene (303 mg, 201 µl, 2.63 mmol, Eq: 1.1). The reaction mixture was allowed to warm to room temperature and was vigorously stirred for 24h. IN HCl (5.5 mL) was added to adjust the pH to ca. 3. The organic layer was collected and the aqueous layer was extracted 2 times with dichloromethane. Combined organic layers were washed with brine, adsorbed unto silica (2g), and purified on silicagel (column 25 g, Hexane/ethyl acetate 1:0 to 90:10). One fraction was isolated and dried in vacuo, leading to 521 mg (77%) of a yellow oil.

### (Z)-methyl N'-cyano-N-(3,5-dichloro-4-(3,3-dimethylbutynyl)phenyl)carbamimidothioate

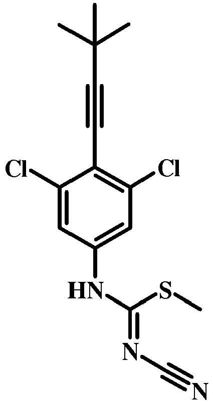

To a solution of 1,3-dichloro-2-(3,3-dimethylbut-1-ynyl)-5-isothiocyanatobenzene (510 mg, 1.79 mmol, Eq: 1.00) in dry methanol (5 ml) was added sodium hydrogen cyanamide (121 mg, 1.88 mmol, Eq: 1.05). The solution was stirred 1h at room temperature then iodomethane (509 mg, 250 µl, 3.59 mmol, Eq: 2) was added and the reaction mixture was stirred overnight at room temperature. The yellow suspension adsorbed unto silica (2g) and purified on silica gel (silica 25g, dichloromethane/ethyl acetate 100:0 to 85:15). One fraction was isolated and dried in vacuo, to afford 405 mg (63%) of the desired compound as a white solid.

### N3-(3,5-dichloro-4-(3,3-dimethylbut-l-ynyl)phenyl)-1H-1,2,4-triazole-3,5-diamine (Compound 79)

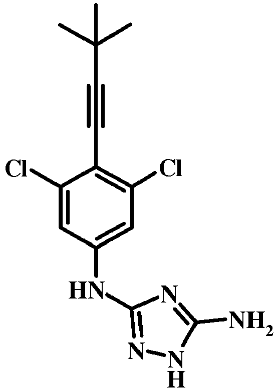

A solution of (Z)-methyl N'-cyano-N-(3,5-dichloro-4-(3,3-dimethylbut-1-ynyl)phenyl)carbamimidothioate (400 mg, 1.18 mmol, Eq: 1.00) and hydrazine (377 mg, 369 µl, 11.8 mmol, Eq: 10) in dry ethanol (11 ml) was stirred overnight at 70°C. The reaction mixture was adsorbed unto silica (1.5 g) and purified on silica gel (column 25 g, dichloromethane/methanol 100:0 to 70:30). One fraction was isolated and dried in vacuo to afford 336 mg of a white solid. This solid was triturated in dichloromethane then filtered. The residue was washed with several portions of dichloromethane then dried in vacuo to afford 247 mg (65%) of a white solid.
MS +m/z: 326.0 (M+H)⁺

### Example 80

### N3-(3-chloro-4-(3,3-dimethylbut-1-ynyl)phenyl)-1H-1,2,4-triazole-3,5-diamine (Compound 80)

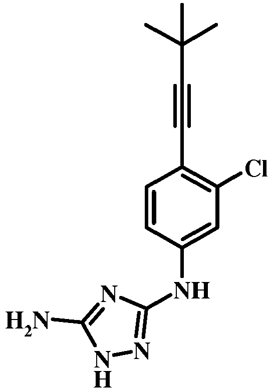

### 2-chloro-1-(3,3-dimethylbut-1-ynyl)-4-nitrobenzene

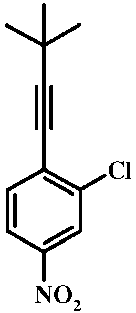

A mixture of 2-chloro-1-iodo-4-nitrobenzene (1 g, 3.53 mmol, Eq: 1.00), bis(triphenylphosphine) palladium chloride (247 mg, 353 µmol, Eq: 0.1) and copper(I) iodide (134 mg, 706 µmol, Eq: 0.2) were degassed (cycles vacuum/nitrogen) then degassed (nitrogen bubbling with sonication) dry THF (9 ml) and diisoproylamine (1.43 g, 1.99 ml, 14.1 mmol, Eq: 4) were added as a mixture. After 15 min stirring at room temperature, degazed (nitrogen bubbling with sonication) 3,3-dimethylbut-1-yne (1.16 g, 1.74 ml, 14.1 mmol, Eq: 4) was added. The black reaction mixture was stirred at 40°C overnight. The reaction mixture was adsorbed unto silica (3g) and purified on silica gel (column 50 g, Hexane/ethyl acetate 100:0 to 80:20). One fraction was isolated and dried in vacuo to afford 693 mg (83%) of a yellow semi solid

### 3-chloro-4-(3,3-dimethylbut-1-ynyl)aniline

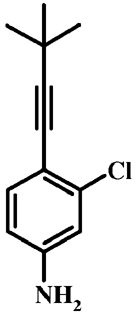

A mixture of 2-chloro-1-(3,3-dimethylbut-1-ynyl)-4-nitrobenzene (690 mg, 2.9 mmol, Eq: 1.00), iron (811 mg, 14.5 mmol, Eq: 5) and ammonium chloride (1.55 g, 29.0 mmol, Eq: 10) in methanol (7 ml) and Water (4 ml) was refluxed for 2h. The resulting suspension was filtered over a Celite pad and the pad was washed with ethyl acetate, then concentrated in vacuo. The residue was partitioned between ethyl acetate and water. The aqueous layer was extracted 3 times with ethyl acetate then combined organic layers were washed with brine. The aqueous layer was adsorbed on silica (1.5g), then purified on silica gel (column 24 g, Hexane/ethyl acetate 100:0 to 65:35). One fraction was isolated and dried in vacuo to afford 450 mg (75%) of the desired product as a light brown oil.

### 2-chloro-1-(3,3-dimethylbut-l-ynyl)-4-isothiocyanatobenzene

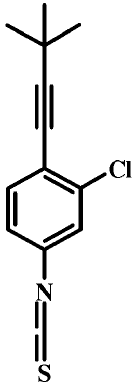

To a cold (0°C) suspension of 3-chloro-4-(3,3-dimethylbut-1-ynyl)aniline (440 mg, 2.12 mmol, Eq: 1.00) and calcium carbonate (424 mg, 4.24 mmol, Eq: 2) in water (5 ml) and dichloromethane (5 ml) was added thiophosgene (268 mg, 178 µl, 2.33 mmol, Eq: 1.1).
The reaction mixture was allowed to warm to room temperature and was vigorously stirred for 24h. IN HCl (5 mL) was added to adjust the pH to ca. 3. The organic layer was collected and the aqueous layer was extracted 2 times with dichloromethane. Combined organic layers were washed with brine, adsorbed unto silica (2g), and purified on silica gel (column 25 g, Hexane/ethyl acetate 1:0 to 85:15). One fraction was isolated and dried in vacuo to afford 451 mg (85%) of the desired product as a colorless oil.

### (Z)-methyl N-3-chloro-4-(3,3-dimethylbut-1-ynyl)phenyl-N'-cyanocarbamimidothioate

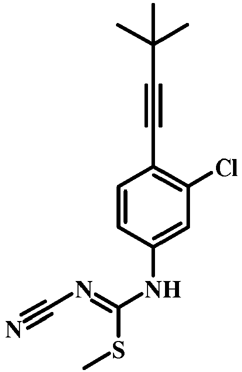

To a solution of 2-chloro-1-(3,3-dimethylbut-1-ynyl)-4-isothiocyanatobenzene (440 mg, 1.76 mmol, Eq: 1.00) in dry methanol (5 ml) was added sodiumhydrogencyanamide (118 mg, 1.85 mmol, Eq: 1.05). The reaction mixture was stirred 1h at room temperature then iodomethane (500 mg, 245 µl, 3.52 mmol, Eq: 2) was added and the reaction mixture was stirred overnight at room temperature. The clear reaction mixture was adsorbed unto silica (2g) and purified on silica gel (silica 25g, dichloromethane/ethyl acetate 100:0 to 85:15). One Fraction was isolated and dried in vacuo to afford 440 mg (82%) of the desired product as a white solid

### N3-(3-chloro-4-(3,3-dimethylbut-1-ynyl)phenyl)-1H-1,2,4-triazole-3,5-diamine (Compound 80)

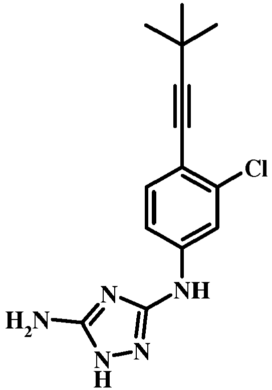

A solution of (Z)-methyl N-3-chloro-4-(3,3-dimethylbut-1-ynyl)phenyl-N'-cyanocarbamimidothioate (415 mg, 1.36 mmol, Eq: 1.00) and hydrazine (435 mg, 426 µl, 13.6 mmol, Eq: 10) in dry ethanol (13 ml) was stirred overnight at 70°C. The reaction mixture was adsorbed unto silica (1.5 g) and purified on silica gel (column 25 g, dichloromethane/methanol 100:0 to 70:30). One fraction was isolated and dried in vacuo to afford 367 mg of a white solid. This solid was triturated in dichloromethane then filtered. The residue was washed with several portions of dichloromethane then dried in vacuo to afford 131 mg (33%) of a white solid.
MS +m/z: 290.0 (M+H)⁺

### Example 81

### N*3*-(4-Bromo-3-pentafluorosulfur-phenyl)-1H-[1,2,4]triazole-3,5-diamine (Compound 81)

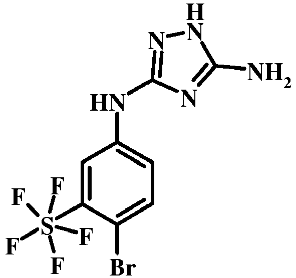

### 3-pentafluorosulfur-4-bromo-aniline

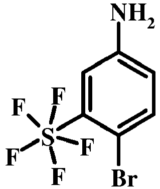

To a mixture of 3-pentafluorosulfuraniline (0.3 g, 1.37 mmol, Eq: 1.00) in dimethyl sulfoxide (3.5 ml) was added N-bromosuccinimide (270 mg, 1.52 mmol, Eq: 1.11) in 3 portions over 3h. After 1h, the reaction mixture was partitioned between 10% aqueous sodium sulfite and ethyl acetate. The organic layer was washed with aqueous sat. sodium hydrogenocarbonate, water (3 times) and brine then adsorbed unto silica (1.8g) and purified on silicagel (column 25 g, Hexane/ethyl acetate 95:5 to 60:40). One fraction was isolated and dried in vacuo to afford 209 mg (51%) of the desired product as a yellow oil.

### 3-pentafluorsulfur-4-bromo-isothiocyanatobenzene

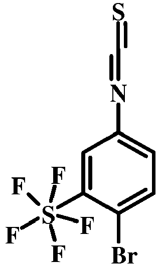

To a cold (0°C) suspension of 3-pentafluorosulfur-4-bromo-aniline (260 mg, 872 µmol, Eq: 1.00) and calcium carbonate (175 mg, 1.74 mmol, Eq: 2) in water (2 ml) and dichloromethane (2.00 ml) was added thiophosgene (110 mg, 73.3 µl, 960 µmol, Eq: 1.1). The reaction mixture was allowed to warm to room temperature and was vigorously stirred for 24h. IN HCl (2.0 mL) was added to adjust the pH to ca. 3 and the reaction mixture was partitioned between water and ethyl acetate. The organic layer was washed with water and brine, adsorbed unto silica (0.8g), and purified on silicagel (column 11 g, Hexane/ethyl acetate 1:0 to 85:15). One fraction was isolated and dried in vacuo to afford 276 mg (93%) of the desired product as a colorless oil.

### (Z)-methyl N-3-pentafluorosulphur-4-bromo-phenyl-N'-cyanocarbamimidothioate

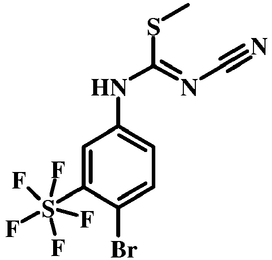

To a solution of 3-pentafluorsulfur-4-bromo-isothiocyanatobenzene (270 mg, 794 µmol, Eq: 1.00) in dry methanol (3 ml) was added sodium hydrogencyanamide (53.4 mg, 834 µmol, Eq: 1.05). The light yellow reaction solution was stirred 1h at room temperature then iodomethane (225 mg, 110 µl, 1.59 mmol, Eq: 2) was added and the reaction mixture was stirred overnight at room temperature. The clear reaction mixture was adsorbed unto silica (0.8g) and purified on silica gel (silica 11g, dichloromethane /ethyl acetate 100:0 to 85:15). One Fraction was isolated and dried in vacuo to afford 207 mg (66%) of the desired product as a white solid.

### N*3*-(4-Bromo-3-pentafluorosulfur-phenyl)-1H-[1,2,4]triazole-3,5-diamine (Compound 81)

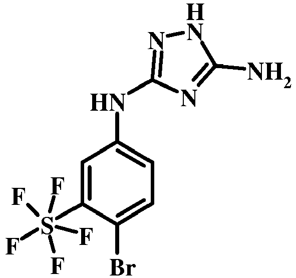

A solution of (Z)-methyl N-3-pentafluorosulphur-4-bromo-phenyl-N'-cyanocarbamimidothioate (205 mg, 517 µmol, Eq: 1.00) and hydrazine (166 mg, 162 µl, 5.17 mmol, Eq: 10) in dry ethanol (5 ml) was stirred overnight at 70°C. The reaction mixture was adsorbed unto silica (0.8 g) and purified on silicagel (column 12 g, dichloromethane/methanol 100:0 to 70:30). One fraction was isolated and dried in vacuo to afford 176 mg (90%) of the desired product as a white solid.
MS +m/z: 380.0 (M+H)⁺

### Example 82

### N*3*-(3-Bromo-5-pentafluorosulfur-phenyl)-1H-[1,2,4]triazole-3,5-diamine (Compound 82)

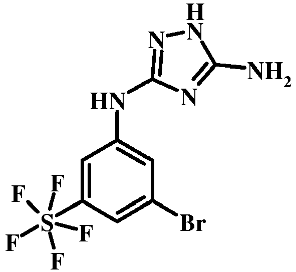

### 3-bromo-5-nitro-phenylsulfurpentafluoride

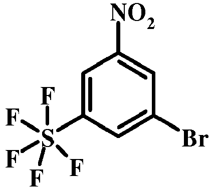

To a warm (40°C) mixture of 3-nitro-phenylsulfurpentafluoride (2.33 g, 9.35 mmol, Eq: 1.00) in trifluoroacetic acid (11.1 g, 7.5 ml, 96.4 mmol, Eq: 10.3) and concentrated sulfuric acid (4.6 g, 2.5 ml, 45.5 mmol, Eq: 4.87) was added N-bromosuccinimide (2.52 g, 14.2 mmol, Eq: 1.51) in 9 portions over 8h. The reaction mixture was stirred overnight. The reaction mixture was partitioned between ice water and ethyl acetate then potassium carbonate (20 g) was carefully added. The organic layer was washed with sat. aqueous sodium hydrogenocarbonate, 10% aqueous sodium sulfite, water and brine then adsorbed unto silica (5g) and purified on silica (column 80g, Hexane/ethyl acetate 95:5 to 70:30). One fraction was isolated and dried in vacuo to afford 0.93 g (30 %) of the desired product as a light brown oil.

### 3-pentafluorosulfur-5-bromo-aniline

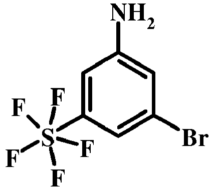

A flask containing 3-bromo-5-nitro-phenylsulfurpentafluoride (0.5 g, 1.52 mmol, Eq: 1.00), iron (426 mg, 7.62 mmol, Eq: 5) and ammonium chloride (815 mg, 15.2 mmol, Eq: 10) was flushed with nitrogen (cycle nitrogen/vacuum). Methanol (2 ml) and water (1 ml) were added and the reaction mixture was degassed again (cycle nitrogen/vacuum). The reaction mixture was refluxed 1.5h (oil bath at 90°C) then cooled down to room temperature. The reaction mixture was filtered on a fritted glass filter. The solid was washed with methanol. The filtrate was dried in vacuo and the residue was partitioned between ethyl acetate and water. The organic layer was washed with water and brine then adsorbed unto silica (2 g) and purified on silica gel (column 25 g, Hexane/ethyl acetate 90:10 to 60:40). One fraction was isolated and dried in vacuo to afford 431 mg (95%) of the desired product as a light yellow oil.

### 3-pentafluorsulfur-5-bromo-isothiocyanatobenzene

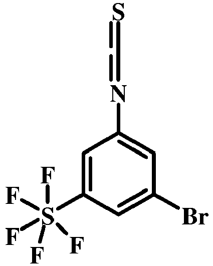

To a cold (0°C) suspension of 3-pentafluorosulfur-5-bromo-aniline (430 mg, 1.44 mmol, Eq: 1.00) and calcium carbonate (289 mg, 2.89 mmol, Eq: 2) in water (4 ml) and dichloromethane (4.00 ml) was added thiophosgene (182 mg, 121 µl, 1.59 mmol, Eq: 1.1). The reaction mixture was allowed to warm to room temperature and was vigorously stirred for 24h. IN HCl (3.5 mL) was added to adjust the pH to ca. 3 and the reaction mixture was partitioned between water and ethyl acetate. The organic layer was washed with water and brine, adsorbed unto silica (1.6g), and purified on silica gel (column 23 g, Hexane/ethyl acetate 1:0 to 85:15). One fraction was isolated and dried in vacuo to afford 430 mg (88%) of the desired product as a yellow oil.

### (Z)-methyl N-3-pentafluorosulphur-5-bromo-phenyl-N'-cyanocarbamimidothioate

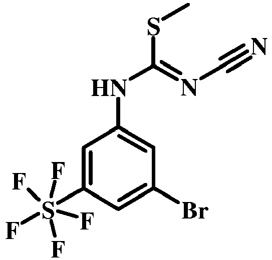

To a solution of 3-pentafluorsulfur-5-bromo-isothiocyanatobenzene (425 mg, 1.25 mmol, Eq: 1.00) in methanol dry (4 ml) was added sodium hydrogencyanamide (84.0 mg, 1.31 mmol, Eq: 1.05). The reaction mixture was stirred 1h at room temperature then iodomethane (355 mg, 174 µl, 2.5 mmol, Eq: 2) was added and the reaction mixture was stirred overnight at room temperature. The reaction mixture was adsorbed unto silica (1.5g) and purified on silica gel (silica 23g, dichloromethane/ethyl acetate 100:0 to 85:15). One fraction was isolated and dried in vacuo to afford 296 mg (60%) of the desired product as a white solid.

### N*3*-(3-Bromo-5-pentafluorosulfur-phenyl)-1H-[1,2,4]triazole-3,5-diamine (Compound 82)

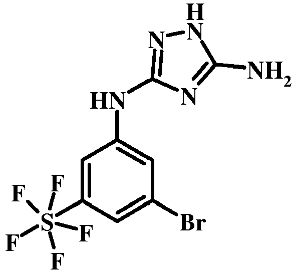

A solution of (Z)-methyl N-3-pentafluorosulphur-5-bromo-phenyl-N'-cyanocarbamimidothioate (296 mg, 747 µmol, Eq: 1.00) and hydrazine (239 mg, 234 µl, 7.47 mmol, Eq: 10) in dry ethanol (7 ml) was stirred overnight at 70°C. The reaction mixture was adsorbed unto silica (1.5 g) and purified on silicagel (column 23 g, dichloromethane/methanol 100:0 to 70:30). One fraction was isolated and dried in vacuo to afford 277 mg (98%) of the desired product as a white solid.
MS +m/z: 380.0 (M+H)⁺

### Example 83

### N*3*-(4-Bromo-3-fluoro-5-pentafluorosulfur-phenyl)-1H-[1,2,4]triazole-3,5-diamine (Compound 83)

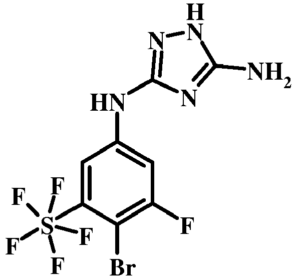

### 3-bromo-5-amino-phenylsulfurpentafluoride

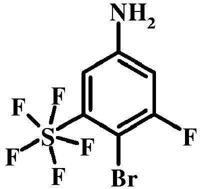

A flask containing 3-nitro-5-fluoro-phenylsulfurpentafluoride (3.5 g, 13.1 mmol, Eq: 1.00), iron (3.57 g, 63.9 mmol, Eq: 4.88) and ammonium chloride (6.83 g, 128 mmol, Eq: 9.75) was flushed with nitrogen (cycle nitrogen/vacuum) then methanol (50 ml) and water (25 ml) were added. The reaction mixture was refluxed 1.5h (oil bath at 90°C) then cooled down to room temperature and filtered on a Celite pad. The pad was washed with methanol. The filtrate was dried in vacuo and the residue was dissolved in ethyl acetate. The organic layer was washed with water (2 times) and brine then dried in vacuo to afford 2.72 g of a yellow oil. This oil was adsorbed unto silica (8g) and purified on silicagel (column 120 g, Hexane/ethyl acetate 90:10 to 60:40). One fraction was isolated and dried in vacuo to afford 2.31 g (74%) of the desired product as a light yellow oil.

### 3-pentafluorosulfur-4-bromo-5-fluoro-aniline

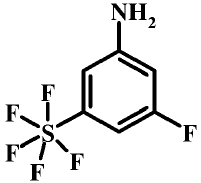

To a mixture of 3-bromo-5-amino-phenylsulfurpentafluoride (1 g, 4.22 mmol, Eq: 1.00) in dimethylsulfoxide (11 ml) was added N-bromosuccinimide (800 mg, 4.49 mmol, Eq: 1.07) in 4 portions over 2h15 (200 mg each 45 min). 2h after the last addition, the reaction was partitioned between 10% aqueous sodium sulfite and ethyl acetate. The organic layer was washed with aqueous sat. sodium carbonate, water (3 times) and brine then adsorbed unto silica (5g) and purified on silicagel (column 80 g, Hexane/ethyl acetate 95:5 to 70:30). One fraction was isolated and dried in vacuo to afford 833 mg (63%) of the desired product and a light yellow oil.

### 3-pentafluorosulfur-4-bromo-5-fluoro-isothiocyanatobenzene

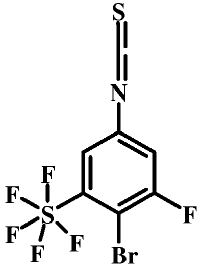

To a cold (0°C) suspension of 3-pentafluorosulfur-4-bromo-5-fluoro-aniline (455 mg, 1.44 mmol, Eq: 1.00) and calcium carbonate (288 mg, 2.88 mmol, Eq: 2) in water (4 ml) and dichloromethane (4.00 ml) was added thiophosgene (182 mg, 121 µl, 1.58 mmol, Eq: 1.1). The reaction mixture was allowed to warm to room temperature and was vigorously stirred for 24h.
IN HCl (3.5 mL) was added to adjust the pH to ca. 3 then the reaction mixture was partitioned between water and ethyl acetate. The organic layer was washed with water and brine, adsorbed unto silica (1.6g), and purified on silicagel (column 23 g, Hexane/ethyl acetate 1:0 to 85:15). One fraction was isolated and dried in vacuo to afford 486 mg (94%) of the desired product as a colorless oil.

### (Z)-methyl N-3-pentafluorosulphur-5-bromo-phenyl-N'-cyanocarbamimidothioate

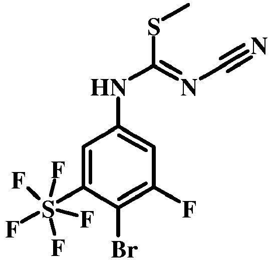

To a solution of 3-pentafluorosulfur-4-bromo-5-fluoro-isothiocyanatobenzene (480 mg, 1.34 mmol, Eq: 1.00) in dry methanol (4 ml) was added sodium hydrogencyanamide (90.1 mg, 1.41 mmol, Eq: 1.05). The light yellow reaction solution was stirred 1h at room temperature then iodomethane (380 mg, 187 µl, 2.68 mmol, Eq: 2) was added and the reaction mixture was stirred overnight at room temperature. The reaction mixture was dried in vacuo and purified on silica gel (silica 23g, dichloromethane/ethyl acetate 100:0 to 85:15). One fraction was isolated and dried in vacuo to afford 285 mg (51%) of the desired product as a white solid.

### N*3*-(4-Bromo-3-fluoro-5-pentafluorosulfur-phenyl)-1H-[1,2,4]triazole-3,5-diamine (Compound 83)

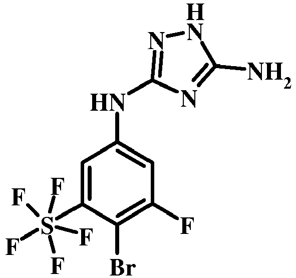

A solution of (Z)-methyl N-3-pentafluorosulphur-5-bromo-phenyl-N'-cyanocarbamimidothioate (296 mg, 747 µmol, Eq: 1.00) and hydrazine (239 mg, 234 µl, 7.47 mmol, Eq: 10) in dry ethanol (7 ml) was stirred overnight at 70°C. The reaction mixture was adsorbed unto silica (1.5 g) and purified on silicagel (column 23 g, dichloromethane/methanol 100:0 to 70:30). One fraction was isolated and dried in vacuo to afford 249 mg (77%) of the desired product as a white solid.
MS +m/z: 400.0 (M+H)⁺

### Example 84

### 2-(4-(5-amino-1H-1,2,4-triazol-3-ylamino)-2-chloro-6-(trifluoromethyl)phenyl)acetonitrile (Compound 84)

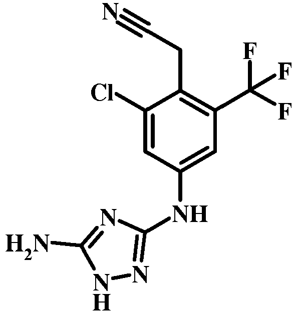

### 2-(2-chloro-4-nitro-6-(trifluoromethyl)phenyl)acetonitrile

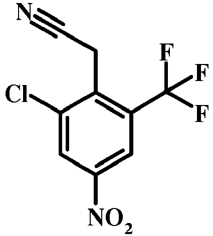

To a cold (ice bath) suspension of sodium hydride dispersion in oil (601 mg, 15.0 mmol, Eq: 3.05) in dry dimethylsulfoxide (8 ml) was slowly added ethyl 2-cyanoacetate (1.7 g, 1.6 ml, 15.0 mmol, Eq: 3.05). The reaction was stirred for 30 min at room temperature then 1-chloro-2-fluoro-5-nitro-3-(trifluoromethyl)benzene (1.2 g, 750 µl, 4.93 mmol, Eq: 1.00) was added and the reaction mixture was stirred at 100°C overnight. Water (200 mL) was added, followed by HCl 1N (16 mL) to adjust the pH to ca. 1. The aqueous layer was extracted with ethyl acetate (3 times) then dichloromethane (3 times). Combined organic layers were washed with brine then dried in vacuo to afford 3 g of a black oil. This black oil was dissolved in dry dimethyl sulfoxide (3 ml) and water (1.2 ml) then lithium chloride (209 mg, 4.93 mmol, Eq: 1.00) was added and the solution was stirred at 165°C for 30 min in a preheated oil bath. The reaction mixture was poured unto water and extracted with diethyl ether and dichloromethane. Combined organic layer were washed with brine (4 times) then adsorbed unto silica (5 g) and purified on silica gel (column 80 g, Hexane/ethyl acetate 90:10 to 50:50). One fraction was isolated and dried in vacuo to afford 390 mg (30 %) of the desired product.

### 2-(4-amino-2-chloro-6-(trifluoromethyl)phenyl)acetonitrile

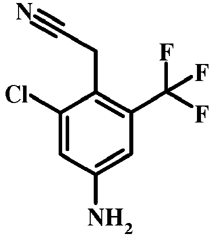

A flask containing 2-(2-chloro-4-nitro-6-(trifluoromethyl)phenyl)acetonitrile (385 mg, 1.46 mmol, Eq: 1.00), iron (406 mg, 7.28 mmol, Eq: 5) and ammonium chloride (778 mg, 14.6 mmol, Eq: 10) was flushed with nitrogen (cycle nitrogen/vacuum). Methanol (2 ml) and water (1 ml) were added and the reaction mixture was again degassed (cycle nitrogen/vacuum). The reaction mixture was refluxed 1.5h (oil bath at 90°C) then cooled down to room temperature and filtered on a fritted glass filter. The filtrate was dried in vacuo, adsorbed unto silica (1.5 g) and purified on silica gel (column 24 g, Hexane/ethyl acetate 85:15 to 50:50). One fraction was isolated and dried in vacuo to afford 329 mg (96%) of the desired product as a light yellow oil.

### 2-(2-chloro-4-isothiocyanato-6-(trifluoromethyl)phenyl)acetonitrile

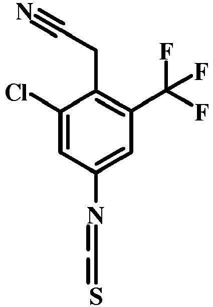

To a cold (0°C) suspension of 2-(4-amino-2-chloro-6-(trifluoromethyl)phenyl)acetonitrile (325 mg, 1.39 mmol, Eq: 1.00) and calcium carbonate (277 mg, 2.77 mmol, Eq: 2) in water (3 ml) and dichloromethane (3 ml) was added thiophosgene (175 mg, 116 µl, 1.52 mmol, Eq: 1.1). The reaction mixture was allowed to warm to room temperature and was vigorously stirred for 24h. IN HCl (3 mL) was added to adjust the pH to ca. 3 and the reaction mixture was partitioned between water and ethyl acetate. The organic layer was washed with water and brine, adsorbed unto silica (0.8g), and purified on silica gel (column 11 g, Hexane/ethyl acetate 1:0 to 85:15). One fraction was isolated and dried in vacuo to afford 275 mg (72%) of the desired product as a colorless oil.

### (Z)-methyl N-3-chloro-4-(cyanomethyl)-5-(trifluoromethyl)phenyl-N'-cyanocarbamimidothioate

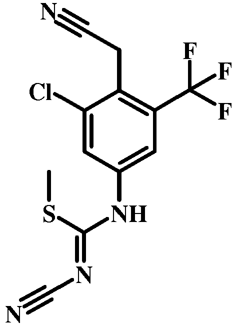

To a solution of 2-(2-chloro-4-isothiocyanato-6-(trifluoromethyl)phenyl)acetonitrile (275 mg, 994 µmol, Eq: 1.00) in dry ethanol (3 ml) was added sodium hydrogen cyanamide (66.8 mg, 1.04 mmol, Eq: 1.05). The reaction mixture was stirred 30 min at room temperature then iodomethane (282 mg, 138 µl, 1.99 mmol, Eq: 2) was added and the reaction mixture was stirred 3h at room temperature. The reaction mixture was adsorbed unto silica (1.5g) and purified on silica gel (silica 24g, dichloromethane/ethyl acetate 100:0 to 80:20). One fraction was isolated and dried in vacuo to afford 237 mg (72%) of the desired product.

### 2-(4-(5-amino-1H-1,2,4-triazol-3-ylamino)-2-chloro-6-(trifluoromethyl)phenyl)acetonitrile (Compound 84)

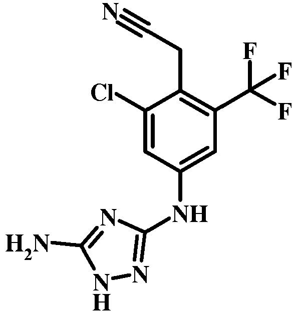

To a suspension of (Z)-methyl N-3-chloro-4-(cyanomethyl)-5-(trifluoromethyl)phenyl-N'-cyanocarbamimidothioate (235 mg, 706 µmol, Eq: 1.00) in dry ethanol (5 ml) was added hydrazine (226 mg, 222 µl, 7.06 mmol, Eq: 10). The reaction mixture was stirred 2h at 70°C then was adsorbed unto silica (1 g) and purified on silicagel (column 24 g, dichloromethane/methanol 95:5 to 60:40). One fraction was isolated and dried in vacuo to afford 200 mg of an off white solid. This solid was triturated with diethylether then filtered and the solid was washed with diethyl ether, leading to 159 mg (71%) of the desired product as an off-white solid.
NMR (300 MHz, DMSO d⁶): 11.40 (1H, s); 9.50 (1H, s); 8.06 (1H, s); 7.91 (1H, s); 6.06 (2H,); 3.96 (2H,s).

### Example 85

### 4-(5-Amino-1H-[1,2,4]triazol-3-ylamino)-2-pentafluorosulfur-benzonitrile (Compound 85)

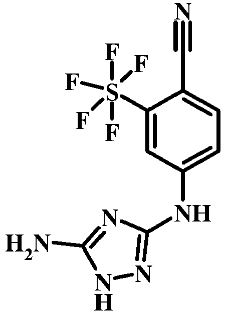

### 4-amino-2-pentafluorosulfur-benzonitrile

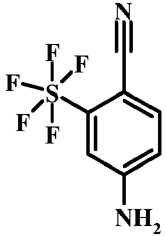

A mixture of 4-bromo-3-pentafluorosulfur-phenylamine (200 mg, 671 µmol, Eq: 1.00, *cf*. **example 82, step 1**) and copper cyanide (120 mg, 1.34 mmol, Eq: 2) in N-methyl-pyrrolidinone (2 ml) was degassed (vacuum/nitrogen cycles) then placed in a preheated oil bath (180°C) for 4h. The reaction was cooled to room temp and partitioned between ice water and ethyl acetate. The organic layer was washed with water (3 times) then brine. The reaction mixture was adsorbed unto silica (0.8 g) and purified on silicagel (column 12 g, Hexane /ethyl acetate 90:10 to 60:40). One fraction was isolated and dried in vacuo to afford 123 mg (75%) of the desired product as an off-white solid.

### 4-Isothiocyanato-2-pentafluorosulfur-benzonitrile

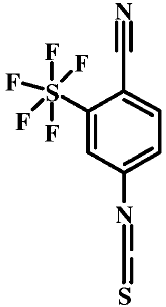

To a cold (0°C) suspension of 4-amino-2-pentafluorosulfur-benzonitrile (120 mg, 491 µmol, Eq: 1.00) and calcium carbonate (98.4 mg, 983 µmol, Eq: 2) in water (1.2 ml) and dichloromethane (1.2 ml) was added thiophosgene (62.2 mg, 41.3 µl, 541 µmol, Eq: 1.1). The reaction mixture was allowed to warm to room temperature and was vigorously stirred for 24h. IN HCl (1 mL) was added to adjust the pH to ca. 3 and the reaction mixture was partitioned between water and ethyl acetate. The organic layer was washed with water and brine, adsorbed unto silica (0.8g), and purified on silicagel (column 12 g, Hexane/ethyl acetate 1:0 to 80:20). One fraction was isolated and dried in vacuo to afford 36 mg (26%) of the desired product as a light yellow oil.

### (Z)-methyl N-3-pentafluorosulfur-4-cyano-phenyl-N'-cyanocarbamimidothioate

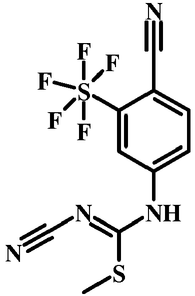

To a solution of 4-Isothiocyanato-2-pentafluorosulfur-benzonitrile (36 mg, 126 µmol, Eq: 1.00) in dry ethanol (0.8 mL) was added sodium hydrogen cyanamide (10 mg, 156 µmol, Eq: 1.24). The reaction mixture was stirred 30 min at room temperature then methyl iodide (61.2 mg, 30 µL, 431 µmol, Eq: 3.43) was added and the reaction mixture was stirred overnight at room temperature. The reaction mixture was adsorbed unto silica (0.2g) and purified on silica gel (silica 4 g, dichloromethane/ethyl acetate 100:0 to 80:20). One Fraction was isolated and dried in vacuo, to afford 19 mg (44%) of the desired product as an off white solid.

### 4-(5-Amino-1H-[1,2,4]triazol-3-ylamino)-2-pentafluorosulfur-benzonitrile

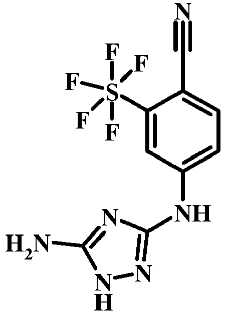

To a suspension of (Z)-methyl N-3-pentafluorosulfur-4-cyano-phenyl-N'-cyanocarbamimidothioate (19 mg, 55.5 µmol, Eq: 1.00) in dry ethanol (500 µl) was added hydrazine (20.4 mg, 20 µl, 637 µmol, Eq: 11.5). The reaction mixture was stirred 16h at 70°C. The reaction mixture was adsorbed unto silica (0.3 g) and purified on silicagel (column 4 g, dichloromethane/methanol 100:0 to 70:30). One fraction was isolated and dried in vacuo to afford 16 mg (88%) of the desired product as an off white solid. MS +m/z: 327.0 (M+H)⁺

### Examples 86 and 87

### 4-(5-Amino-1H-[1,2,4]triazol-3-ylamino)-2-fluoro-6-pentafluorosulfur-benzonitrile (Compound 86) and

### 4-(5-Amino-1H-[1,2,4]triazol-3-ylamino)-2-hydrazino-6-pentafluorosulfur-benzonitrile (Compound 87)

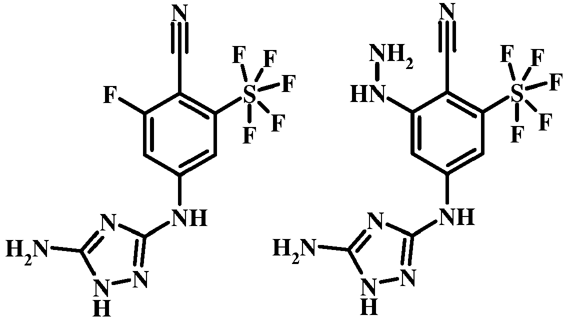

### 4-amino-2-fluoro-6-pentaflurosulfur-benzonitrile

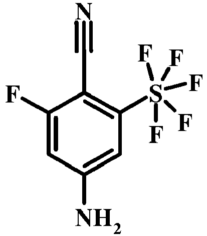

A mixture of 4-bromo-3-fluoro-5-pentafluorosulfur-phenylamine (550 mg, 1.74 mmol, Eq: 1.00, *cf.* **example 84, step 1**) and copper cyanide (216 mg, 2.41 mmol, Eq: 1.39) in N-methyl-pyrrolidinone (5 ml) was degassed (vacuum/nitrogen) then placed in a preheated oil bath (180°C) for 2.5h. The reaction was cooled to room temp and partitioned between ice water and ethyl acetate. The aqueous layer was extracted with ethyl acetate then combined organic layers were washed with water (3 times) and brine. The reaction mixture was adsorbed unto silica (2 g) and purified on silicagel (column 40 g, Hexane/ethyl acetate 90:10 to 40:60). One fraction was isolated and dried in vacuo to afford 288 mg (63%) of the desired product as a yellow oil

### 2-fluoro-4-isothiocyanato-6-pentafluorosulfur-benzonitrile

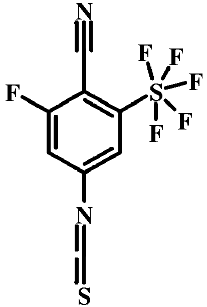

To a mixture of 4-amino-2-fluoro-6-pentaflurosulfur-benzonitrile (288 mg, 1.1 mmol, Eq: 1.00), triethylamine (556 mg, 772 µl, 5.49 mmol, Eq: 5) in benzene (6 ml) was added thiophosgene (379 mg, 251 µl, 3.3 mmol, Eq: 3). The mixture was refluxed for 6h then partitioned between water and HCl 0.2 N. The dark organic layer was washed with water (2 times) then brine, then adsorbed unto silica and purified on silicagel (column 24 g, Hexane/ethyl acetate 1:0 to 85:15). One fraction was isolated and dried in vacuo to afford 88 mg (26%) of the desired compound a a yellow oil.

### (Z)-methyl N-3-pentafluorosulfur-4-cyano-5-fluoro-phenyl-N'-cyanocarbamimidothioate

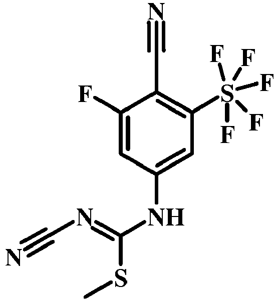

To a solution of 2-fluoro-4-isothiocyanato-6-pentafluorosulfur-benzonitrile (114 mg, 375 µmol, Eq: 1.00) in dry methanol (1.2 ml) was added sodium hydrogencyanamide (26.4 mg, 412 µmol, Eq: 1.1). The reaction mixture was stirred 30 min at room temperature then iodomethane (106 mg, 52.1 µl, 749 µmol, Eq: 2) was added and the reaction mixture was stirred for 6h at room temperature. The reaction mixture was adsorbed unto silica (0.6g), concentrated and purified on silica gel (silica 12 g, dichloromethane/ethyl acetate 100:0 to 80:20). One fraction was isolated and dried in vacuo to afford 74 mg (55%) of the desired product as a light yellow solid.

### 4-(5-Amino-1H-[1,2,4]triazol-3-ylamino)-2-fluoro-6-pentafluorosulfur-benzonitrile (Compound 86) and 4-(5-Amino-1H-[1,2,4]triazol-3-ylamino)-2-hydrazino-6-pentafluorosulfur-benzonitrile (Compound 87)

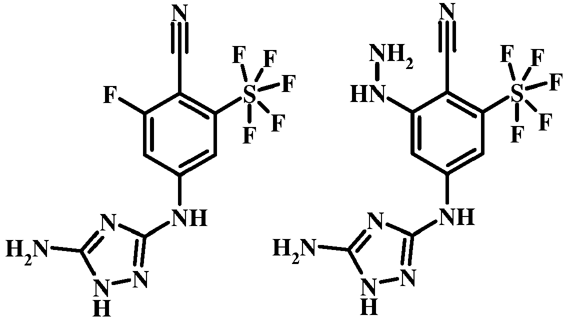

To a suspension of (Z)-methyl N-3-pentafluorosulfur-4-cyano-5-fluoro-phenyl-N'-cyanocarbamimidothioate (74 mg, 205 µmol, Eq: 1.00) in dry ethanol (1.5 ml) was added hydrazine (65.8 mg, 64.5 µl, 2.05 mmol, Eq: 10). The reaction mixture was stirred 4h at 70°C then was adsorbed unto silica (0.3 g) and purified on silicagel (column 4 g, dichloromethane/methanol 100:0 to 70:30). 2 fractions were isolated and dried in vacuo. Fraction 1 (example 26): 60 mg (85%) as a yellow solid. MS +m/z: 345.0 (M+H)⁺
Fraction 2 (example 27): 15 mg (21%) as a yellow solid. MS +m/z: 357.0 (M+H)⁺

### Example 88

### (1-Acetyl-5-amino-1H-[1,2,4]triazol-3-ylamino)-2,6-dichloro-benzonitrile (Compound 88)

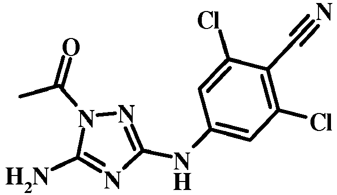

4-(5-amino-1H-1,2,4-triazol-3-ylamino)-2,6-dichlorobenzonitrile (150 mg, 0.557 mmol) in DMF (3 mL) was treated with acetic anhydride (0.63 uL, 0.669 mmol) and triethylamine (93.2 uL, 0.669 mmol) and the mixture was stirred at rt for 2 hrs. The mixture was poured into water (20 ml) and the solid was filtered and dried to give a white solid. 158 mg, yield: 91 %.¹H NMR (300 MHz, DMSO-*d*₆) δ ppm 2.50 (s, 3 H), 7.67 - 7.84 (m, 4 H), 10.41 (s, 1 H)
MS(H+) = 310.85

### Example 89

### 4-[5-Amino-1-(4-trifluoromethyl-benzenesulfonyl)-1H-[1,2,4]triazol-3-ylamino]-2,6-dichloro-benzonitrile (Compound 89)

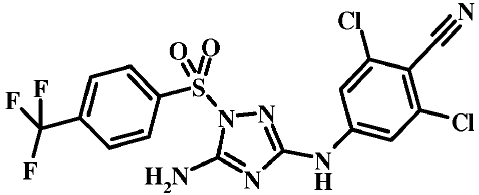

To a stirred mixture of 4-(5-amino-1H-1,2,4-triazol-3-ylamino)-2,6-dichlorobenzonitrile (150 mg, 0.557 mmol) in ethyl acetate (5 mL), 4-(trifluoromethyl)benzene-1-sulfonyl chloride(Aldrich, 136 mg 0.557 mmol) was added followed by Et3N(100 uL, 0.72 mmol). Stir at rt for 2 hrs until lc/mass indicates disappearance of starting material. The solvent was reduced to about 1 mL and 2 mL of methylene chloride was added. The mixture was loaded onto a silica gel column and eluted with 25-40% ethyl acetate in methylene chloride to give a white solid after concentration. 172 mg, 65%. ¹H NMR (300 MHz, DMSO-*d*₆) δ ppm 7.65 (s, 2 H) 7.75 (br. s., 2 H) 8.15 (q, *J*=8.48 Hz, 4 H) 10.43 (s, 1 H). MS(H+) = 476.8

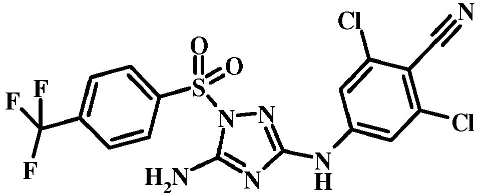

### Example 90

### N3-(4-((1H-tetrazol-5-yl)methyl)-3-chloro-5-(trifluoromethyl)phenyl)-1H-1,2,4-triazole-3,5-diamine (Compound 90)

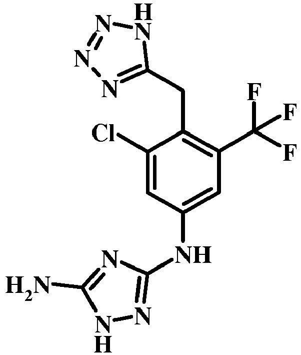

A mixture of 2-(4-(5-amino-1H-1,2,4-triazol-3-ylamino)-2-chloro-6-(trifluoromethyl)phenyl)acetonitrile (60 mg, 189 µmol, Eq: 1.00), sodium azide (61.6 mg, 947 µmol, Eq: 5), and ammonium chloride (50.7 mg, 947 µmol, Eq: 5) in dry dimethylformamide (1 mL) was stirred at 130°C under a nitrogen atmosphere for 16 h. Sodium azide (123 mg, 1894 µmol, Eq: 10), and ammonium chloride (101.4 mg, 1894 µmol, Eq: 10) were added and the reaction was stirred at 130°C for an additional 20h. The reaction mixture was adsorbed on silica and purified on silica gel (column 12 g, dichloromethane/methanol 90:10 to 60:40). One fraction was isolated and dried in vacuo to afford 30 mg (44%) of the desired product as a light brown waxy solid.
MS +m/z: 360.0 (M+H)⁺

### Biological Examples

Determination of compounds HCV GT1b and GT1a entry inhibitory activity using the pseudotyped HCV particle (HCVpp) reporter assay.
Mammalian expression plasmids for the generation of pseudotyped virus particles.

Plasmids expressing HCV E1 and E2 envelope proteins of GT1a H77 strain (Proc Natl Acad Sci USA 1997 94:8738-43) or GT1b Con1 strain (Science 1999 285:110-3) were constructed by cloning the nucleic acids encoding the last 60 amino acids of HCV core protein and all of the HCV E1 and E2 proteins into pcDNA3.1(+) vector. Plasmid pVSV-G expressing the glycoprotein G of the vesicular stomatitis virus (VSV G) is from Clontech (cat # 631530). The HIV packaging construct expressing the firefly luciferase reporter gene was modified based on the envelope defective pNL.4.3.Luc-R⁻.E⁻ vector (Virology 1995 206:935-44) by further deleting part of the HIV envelope protein.
Generation of pseudotyped virus particles in transiently transfected HEK-293T cells.

Pseudotyped HCV GT1a and GT1b particles (HCVpp) and the pseudotyped VSV G particles (VSVpp) were generated from transiently transfected HEK-293T cells (ATCC cat# CRL-573). For generating HCVpp, the HEK-293T cells were transfected with equal amounts of plasmids expressing the HCV envelope proteins and the HIV packaging genome by using polyethylenimine (Polysciences cat# 23966) as transfection reagent. For generating VSVpp, the HEK-293T cells were transfected with equal amounts of plasmids expressing VSV G and the HIV packaging genome by using polyethylenimine. 24 hours after the transfection, the cell culture medium containing the transfection mixture was replaced with fresh Dulbecco's Modified Eagle Medium (DMEM-Glutamax™-I; Invitrogen cat # 10569-010) supplemented with 10% Fetal Bovine Serum (Invitrogen cat # 10082-147) and 2 mM L-glutamine (Invitrogen cat # 25030-081). The supernatant was collected 48 hours after the transfection and filtered through a sterile 0.45 µm filter. Aliquots of the supernatant was frozen and stored at -80°C until use.

Huh7-high CD81 cells with high CD81 expression level were enriched by flow cytometry sorting using FITC-labeled CD81 antibody JS-81 (BD Biosciences cat# 561956) to allow more efficient HCV entry. The Huh7-high CD81 cells were cultured in Dulbecco's Modified Eagle Medium (DMEM-Glutamax™-I; Invitrogen cat # 10569-010). The medium was supplemented with 10% Fetal Bovine Serum (Invitrogen cat # 10082-147) and 1% penicillin/streptomycin (Invitrogen cat # 15070-063). Cells were maintained at 37 °C in a humidified 5% CO₂ atmosphere.
Determination of compound HCVpp entry inhibitory activity in Huh7-high CD81 cells.

Huh7-high CD81 cells were plated at a cell density of 8000 cells per well in 96 well plates (Perkin Elmer, cat # 6005660). Cells were plated in 100 µl of Dulbecco's Modified Eagle Medium (DMEM-Glutamax™-I, Invitrogen Cat # 10569-010) supplemented with 10% Fetal Bovine Serum (Invitrogen Cat # 10082-147) and 1% penicillin/streptomycin (Invitrogen cat # 15070-063). Cells were allowed to equilibrate for 24 hours at 37°C and 5% CO2 at which time compounds and pseudotyped viruses were added. On the day of the assay, HCVpp aliquots were thawed in 37°C water bath and kept at 4°C until use. Compounds (or medium as a control) were diluted in 3 fold dilution series in DMEM-Glutamax™-I with 2% DMSO and 2% penicillin/streptomycin. The 100 µl plating medium in each culture well was removed followed by the addition of 50 µl compound dilutions and 50 µl thawed HCVpp. Firefly luciferase reporter signal was read 72 hours after the addition of compounds and HCVpp using the Steady-Glo luciferase Assay System (Promega, cat # E2520) following the manufacturer's instruction. EC50 values were defined as the compound concentration at which a 50% reduction in the levels of firefly luciferase reporter was observed as compared to control samples in the absence of compound and was determined by non-linear fitting of compound dose-response data.
Determination of compound selectivity in Huh7-high CD81 cells.

Huh7 hCD81 cell assay plates and compound dilutions were set up in the same format as in the HCVpp assay. 24 hours after cell plating, thawed VSVpp was diluted by 800 fold in DMEM-Glutamax^{™}-I supplemented with 10% fetal bovine serum. After removal of the cell plating medium from the culture wells, 50 µl compound dilutions and 50 µl diluted VSVpp were added to the wells. Firefly luciferase reporter signal was read 72 hours after the addition of compounds and VSVpp using the Steady-Glo luciferase Assay System (Promega, cat # E2520). EC50 values were defined as the compound concentration at which a 50% reduction in the levels of firefly luciferase reporter was observed as compared to control samples in the absence of compound and was determined by non-linear fitting of compound dose-response data. The EC50 was approximated if maximum percentage inhibition was less than 90% and more than 70%. Representative assay data can be found in Table II below:

**Table II.**

| **Compound #** | **HCVpp GT-1a (EC₅₀, µM)** | **HCVpp GT-1b (EC₅₀, µM)** | **VSVpp (EC₅₀, µM)** |
|---|---|---|---|
| I-1 | 0.024 | 0.541 | 27.8 |
| I-2 | 0.009 | 0.277 | 64.6 |
| I-3 | 0.012 | 0.386 | 32.7 |
| I-4 | 0.412 | 0.823 | 100 |
| I-5 | | 0.536 | 45.5 |
| I-6 | 0.948 | 1.594 | 100 |
| I-7 | 1.279 | 0.91 | 100 |
| I-8 | 0.141 | 1.147 | 98.1 |
| I-9 | 0.161 | 3.075 | 45.5 |
| I-10 | 0.368 | 5.136 | 100 |
| I-11 | 0.166 | 4.678 | 100 |
| I-12 | 0.335 | 4.194 | 100 |
| I-13 | 0.539 | 4.847 | 100 |
| I-14 | 5.337 | 32.744 | 100 |
| I-15 | 1.406 | 18.142 | 100 |
| I-16 | 5.339 | 16.146 | 100 |
| I-17 | 12.623 | 14.168 | 100 |
| I-18 | 2.95 | 27.455 | 100 |
| I-19 | 44.712 | 56.812 | 87.8 |
| I-20 | 90.585 | 100 | 100 |
| I-21 | 12.621 | 41.354 | 100 |
| I-22 | 100 | 100 | 100 |
| I-23 | 100 | 100 | 100 |
| I-24 | 100 | 100 | 100 |
| I-25 | 86.42 | 100 | 100 |
| I-26 | 100 | 100 | 100 |
| I-27 | 21.14 | | 94.4 |
| I-28 | 82.83 | | 100 |
| I-29 | 6.49 | | 7.5 |
| I-30 | 14.52 | | 100 |
| I-31 | 100 | | 100 |
| I-32 | 11.04 | | 87.2 |
| I-33 | 100 | | 100 |
| I-34 | 0.20 | | >1.0 |
| I-35 | 0.34 | 1.73 | 62.8 |
| I-36 | 1.84 | 28.59 | 100 |
| I-37 | 0.92 | 13.41 | 33.3 |
| I-38 | 0.023 | 0.75 | 79.4 |
| I-39 | 0.015 | 0.574 | 37.9 |
| I-40 | | 3.291 | 45.7 |
| I-41 | 0.138 | 1.613 | |
| I-42 | 0.081 | 0.404 | |
| I-43 | 0.038 | 0.146 | 30.3 |
| I-44 | | 0.545 | 100 |
| I-45 | | 0.095 | 82.5 |
| I-46 | | 1.18 | 100 |
| I-47 | 19.62 | | 100 |
| I-48 | 0.558 | 5.65 | 100 |
| I-49 | 100 | | 100 |
| I-50 | 0.094 | 1.099 | 100 |
| I-51 | 0.475 | 3.437 | 53.7 |
| I-52 | 2.39 | 12.63 | 100 |
| I-53 | 100 | | 100 |
| I-54 | 2.91 | 26.37 | 100 |
| I-55 | 10.26 | | 100 |
| I-56 | 8.358 | 19.61 | 100 |
| I-57 | 0.146 | 0.624 | 100 |
| I-58 | 4.076 | | |
| I-59 | 1.461 | | 100 |
| I-60 | 0.071 | 0.489 | 33.3 |
| I-61 | | 0.331 | >10 |
| I-62 | | 100 | 100 |
| I-63 | | 1.738 | 100 |
| I-64 | | 4.087 | 8.6 |
| I-65 | | 3.673 | 100 |
| I-66 | | 4.994 | 100 |
| I-67 | | 0.685 | 100 |
| I-68 | | 0.161 | 43.3 |
| I-69 | | 0.561 | 100 |
| I-70 | 0.152 | 1.099 | 100 |
| I-71 | 100 | | 100 |
| I-72 | 36.1215 | | 68.322 |
| I-73 | 0.035 | 0.188 | 100 |
| I-74 | 34.3106 | | 100 |
| I-75 | 14.3438 | | 100 |
| I-76 | 0.791 | | 100 |
| I-77 | 1.5874 | 20.352 | |
| I-78 | 32.0655 | | 100 |
| I-79 | 0.051 | 3.309 | 66.01 |
| I-80 | 1.1217 | 11.24 | 91.642 |
| I-81 | | 0.527 | 34.044 |
| I-82 | | 0.918 | 57.4988 |
| I-83 | 0.076 | 0.17 | 32.5218 |
| I-84 | | 0.628 | 42.1108 |
| I-85 | | 0.2869 | >10 |
| I-86 | | 0.104 | >10 |
| I-87 | | 1.652 | >10 |
| I-90 | 24.387 | | 100 |

The foregoing invention has been described in some detail by way of illustration and example, for purposes of clarity and understanding. It will be obvious to one of skill in the art that changes and modifications may be practiced within the scope of the appended claims.

## Claims

1. Compound of formula I 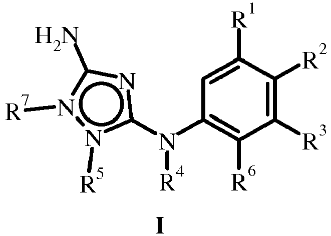 for use in the treatment of a Hepatitis C Virus (HCV) infection wherein:
R¹ is H, halo, C₁-C₆ haloalkyl, lower haloalkyl C₁-C₆ alkyl, trifluoromethyl sulfonyl, C₁-C₆ alkyl, alkoxy, nitro, carboxyl, C₁-C₆ alkyl sulfonamido, trifluoromethyl sulfinyl, cycloalkyl, SF₅,
or -NHNH₂;
R² is H, halo, cyano, nitro, trifluoromethyl sulfonyl, C₁-C₆ halo alkyl, phenyl ethynyl, C₁-C₆ alkyl sulfonyl, C₁-C₆ alkyl amino sulfonyl, C₁-C₆ alkyl amido, amino sulfonyl, sulfonamido, benzyl oxy, cycloalkyl, heterocycloalkyl sulfonyl, aryl carbonyl, C₁-C₆ alkyl urea, C₁-C₆ alkyl ester, phenyl amido, heterocycloalkyl carbonyl, C₁-C₆ alkyl sulfonyl phenyl amido, C₁-C₆ alkyl cyano, cyano C₁-C₆ alkyl, or tetrazolyl C₁-C₆ alkyl;
R³ is H, halo, C₁-C₆ alkyl, C₁-C₆ alkenyl, C₁-C₆ haloalkyl, cyano, C₁-C₆ haloalkyl oxy, cyano cycloalkyl, C₁-C₆ haloalkyl C₁-C₆ alkyl, SF₅, or trifluoromethyl sulfanyl; or R² and R³ together form an aryl ring system;
R⁴ is H, C₁-C₆ alkyl, cyano C₁-C₆ alkyl, or phenyl C₁-C₆ alkyl;
R⁵ is absent, H, C₁-C₆ alkyl, or cycloalkyl;
R⁶ is H or halo;
R⁷ is absent, H, C₁-C₆ alkyl carbonyl, or C₁-C₆ haloalkyl phenyl sulfonyl;
with the proviso that when R⁵ is absent, R⁷ is not absent; and
with the proviso that when R⁷ is absent, R⁵ is not absent;
or a pharmaceutically acceptable salt thereof, wherein carboxyl denotes a group of formula -C (=O)R₂ wherein each R is independently H or C₁-C₆ alkyl;
wherein amido denotes a group of formla -C (=O)NR'R" or -NR'C(=O)R" wherein R' and R" are independently H, alkyl, alkoxy, cycloalkyl, heterocyclo-alkyl, aryl or heteroaryl;
wherein amino denotes a group of forumal -NR'R" wherein R' and R" are independently H, alkyl, alkoxy, cycloakyl, heterocycloalkyl, aryl or heteroaryl and wherein R' and R" optionally form together with the mitrogen to which they are attached a heterocycloalkyl.

2. The compound of claim 1, wherein R⁵ is H.

3. The compound of claim 2, wherein R⁴ is H.

4. The compound of claim 3, wherein R⁶ is H.

5. The compound of any one of claims 1 to 4, wherein R¹ is halo.

6. The compound of any one of claims 1 to 5, wherein R² is halo.

7. The compound of any one of claims 1 to 6, wherein R³ is halo.

8. The compound of any one of claims 1 to 6, wherein R³ is C₁-C₆ haloalkyl.

9. The compound of any one of claims 1 to 3, wherein R¹ is H.

10. The compound of any one of claims 1 to 3, wherein R² is H.

11. The compound of any one of claims 1 to 3, wherein R³ is H.

12. A compound for use in the treatment of a Hepatatis C Virus (HCV) infection, wherein the compound is selected from the group consisting of:
*N*³-Naphthalen-2-yl-1*H*-[1,2,4]triazole-3,5-diamine;
4-(5-Amino-1*H*-[1,2,4]triazol-3-ylamino)-*N*-methyl-benzenesulfonamide;
4-(5-Amino-1*H*-[1,2,4]triazol-3-ylamino)-benzenesulfonamide;
3-(5-Amino-1*H*-[1,2,4]triazol-3-ylamino)-benzoic acid;
*N*³-(3-Chloro-phenyl)-1*H*-[1,2,4]triazole-3,5-diamine;

13. A compound selected from the group consisting of:
*N*³-(4-Bromo-3,5-dichloro-phenyl)-1*H*-[1,2,4]triazole-3,5-diamine;
4-(5-Amino-1*H*-[1,2,4]triazol-3-ylamino)-2,6-dichloro-benzonitrile;
*N*³-(3,5-Dichloro-4-iodo-phenyl)-1*H*-[1,2,4]triazole-3,5-diamine;
*N*³-(3-Trifluoromethanesulfonyl-phenyl)-1*H*-[1,2,4]triazole-3,5-diamine;
*N*³-(4-Bromo-3-chloro-5-fluoro-phenyl)-1*H*-[1,2,4]triazole-3,5-diamine;
*N*³-(3-*tert*-Butyl-phenyl)-1*H*-[1,2,4]triazole-3,5-diamine;
*N*³-(3-Chloro-4-trifluoromethanesulfonyl-phenyl)-1*H*-[1,2,4]triazole-3,5-diamine;
*N*³-(3-Chloro-4-nitro-phenyl)-1*H*-[1,2,4]triazole-3,5-diamine;
*N*³-(3-Chloro-4-ethynyl-phenyl)-1*H*-[1,2,4]triazole-3,5-diamine;
*N*³-(3-Chloro-4-trifluoromethyl-phenyl)-1*H*-[1,2,4]triazole-3,5-diamine;
*N*³-(3,4,5-Trichloro-phenyl)-1*H*-[1,2,4]triazole-3,5-diamine;
4-(5-Amino-1*H*-[1,2,4]triazol-3-ylamino)-2-chloro-benzonitrile;
*N*³-(4-Chloro-3-nitro-phenyl)-1*H*-[1,2,4]triazole-3,5-diamine;
*N*³-(3-Chloro-4-phenylethynyl-phenyl)-1*H*-[1,2,4]triazole-3,5-diamine;
*N*³-(3-Bromo-phenyl)-1*H*-[1,2,4]triazole-3,5-diamine;
4-(5-Amino-1*H*-[1,2,4]triazol-3-ylamino)-2-*tert*-butyl-benzonitrile;
*N*³-(4-Trifluoromethanesulfonyl-phenyl)-1*H*-[1,2,4]triazole-3,5-diamine;
*N*³-(3,5-Di-*tert*-butyl-phenyl)-1*H*-[1,2,4]triazole-3,5-diamine;
*N*³-(4-Methanesulfonyl-phenyl)-1*H*-[1,2,4]triazole-3,5-diamine;
*N*³-(3-Methanesulfonyl-phenyl)-1*H*-[1,2,4]triazole-3,5-diamine;
[4-(5-Amino-1*H*-[1,2,4]triazol-3-ylamino)-phenyl]-morpholin-4-yl-methanone;
4-(5-Amino-1*H*-[1,2,4]triazol-3-ylamino)-*N*,*N*-dimethyl-benzamide;
4-(5-Amino-1*H*-[1,2,4]triazol-3-ylamino)-*N*,*N*-dimethyl-benzenesulfonamide;
*N*³-Methyl-*N*³-(3,4,5-trichloro-phenyl)-1*H*-[1,2,4]triazole-3,5-diamine;
*N*³-(3,5-Dichloro-phenyl)-*N*³-methyl-1*H*-[1,2,4]triazole-3,5-diamine;
*N*³-Benzyl-*N*³-(3,5-dichloro-phenyl)-1*H*-[1,2,4]triazole-3,5-diamine;
[(5-Amino-1*H*-[1,2,4]triazol-3-yl)-(3,5-dichloro-phenyl)-amino]-acetonitrile;
N3-(3-Trifluoromethanesulfinyl-5-trifluoromethylsulfanyl-phenyl)-1H-[1,2,4]triazole-3,5-diamine;
N-[3-(5-Amino-1H-[1,2,4]triazol-3-ylamino)-phenyl]-methanesulfonamide;
4-(5-Amino-1H-[1,2,4]triazol-3-ylamino)-2-trifluoromethanesulfonyl-benzonitrile;
4-(5-Amino-1H-[1,2,4]triazol-3-ylamino)-2-cyclopropyl-benzonitrile;
*N*³-(4-Benzyloxy-3-chloro-phenyl)-1*H*-[1,2,4]triazole-3,5-diamine;
*N*³-(3,5-Dichloro-4-methyl-phenyl)-1*H*-[1,2,4]triazole-3,5-diamine
*N*³ -(3,5-Dichloro-4-cyclopropyl-phenyl)-1*H*-[1,2,4]triazole-3,5-diamine
*N*³-(4-Bromo-3-chloro-phenyl)-1*H*-[1,2,4]triazole-3,5-diamine;
*N*³-(3,5 -Dichloro-phenyl)-1*H*-[1,2,4]triazole-3,5-diamine;
*N*³-(4-Bromo-3-chloro-5-trifluoromethyl-phenyl)-1*H*-[1,2,4]triazole-3,5-diamine;
4-(5-Amino-1*H*-[1,2,4]triazol-3-ylamino)-2-chloro-6-trifluoromethyl-benzonitrile;
*N*³-[3,5-Dichloro-4-(morpholine-4-sulfonyl)-phenyl]-1*H*-[1,2,4]triazole-3,5-diamine;
*N*⁵-(4-Bromo-3-fluoro-5-trifluoromethyl-phenyl)-1*H*-[1,2,4]triazole-3,5-diamine;
*N*⁵-(4-Bromo-3,5-difluoro-phenyl)-1*H*-[1,2,4]triazole-3,5-diamine;
4-(5-Amino-1*H*-[1,2,4]triazol-3-ylamino)-benzonitrile;
3-(5-Amino-1*H*-[1,2,4]triazol-3-ylamino)-5-chloro-benzonitrile;
*N*³-(2,3-Dichloro-phenyl)-1*H*-[1,2,4]triazole-3,5-diamine;
*N*³-(3-Chloro-5-trifluoromethyl-phenyl)-1*H*-[1,2,4]triazole-3,5-diamine;
*N*³-(4-Chloro-3-trifluoromethyl-phenyl)-1*H*-[1,2,4]triazole-3,5-diamine;
*N*³-(3-Trifluoromethoxy-phenyl)-1*H*-[1,2,4]triazole-3,5-diamine;
*N*³-(2-Fluoro-3-trifluoromethyl-phenyl)-1*H*-[1,2,4]triazole-3,5-diamine;
*N*³-(2-Fluoro-5-trifluoromethyl-phenyl)-1H-[1,2,4]triazole-3,5-diamine;
*N*³-[3-(2,2,2-Trifluoro-ethyl)-phenyl]-1*H*-[1,2,4]triazole-3,5-diamine;
*N*³-(3-Isopropyl-phenyl)-1*H*-[1,2,4]triazole-3,5-diamine;
[3-(5-Amino-1*H*-[1,2,4]triazol-3-ylamino)-phenyl]-phenyl-methanone;
1-[3-(5-Amino-1*H*-[1,2,4]triazol-3-ylamino)-phenyl]-cyclopropanecarbonitrile;
*N*³-(3-Chloro-4-fluoro-5-trifluoromethyl-phenyl)-1*H*-[1,2,4]triazole-3,5-diamine;
*N*³-(3-Chloro-5-fluoro-phenyl)-1*H*-[1,2,4]triazole-3,5-diamine;
1-[4-(5-Amino-1*H*-[1,2,4]triazol-3-ylamino)-2-chloro-phenyl]-3-methyl-urea;
4-(5-Amino-1*H*-[1,2,4]triazol-3-ylamino)-2,6-dichloro-benzoic acid methyl ester;
4-(5-Amino-1*H*-[1,2,4]triazol-3-ylamino)-2,6-dichloro-*N*-phenyl-benzamide;
[4-(5-Amino-1*H*-[1,2,4]triazol-3-ylamino)-2,6-dichloro-phenyl]-pyrrolidin-1-yl-methanone;
4-(5-Amino-1*H*-[1,2,4]triazol-3-ylamino)-2,6-dichloro-*N*-(3-methanesulfonyl-phenyl)-benzamide;
*N*³-(3,5-Dichloro-4-vinyl-phenyl)-1*H*-[1,2,4]triazole-3,5-diamine;
4-(5-Amino-2*H*-[1,2,4]triazol-3-ylamino)-2-fluoro-6-trifluoromethyl-benzonitrile;
*N*³-(3-Fluoro-5-trifluoromethyl-phenyl)-1*H*-[1,2,4]triazole-3,5-diamine;
4-(5-Amino-2*H*-[1,2,4]triazol-3-ylamino)-2,6-difluoro-benzonitrile;
N*3*-(3-pentafluorosulfur-phenyl)-1H-[1,2,4]triazole-3,5-diamine;
1-Methyl-N*5*-(3-pentafluorosulfur-phenyl)-1H-[1,2,4]triazole-3,5-diamine;
Cyclohexyl-N*5*-(3-pentafluorosulfur-phenyl)-1H-[1,2,4]triazole-3,5-diamine;
N*3*-(3-Fluoro-5-pentafluorosulfur-phenyl)-1H-[1,2,4]triazole-3,5-diamine;
N*5*-(3-Fluoro-5-pentafluorosulfur-phenyl)-1-methyl-1H-[1,2,4]triazole-3,5-diamine;
N*3*-(4-pentafluorosulfur-phenyl)-1H-[1,2,4]triazole-3,5-diamine;
*N*³-(3-Chloro-4-methyl-phenyl)-1*H*-[1,2,4]triazole-3,5-diamine;
*N*³-(3-Chloro-4-methanesulfonyl-phenyl)-1*H*-[1,2,4]triazole-3,5-diamine;
4-(5-Amino-1*H*-[1,2,4]triazol-3-ylamino)-2-chloro-*N*,*N*-dimethyl-benzamide;
*N*³-[3,5-Dichloro-4-(3,3-dimethyl-but-1-ynyl)-phenyl]-1*H*-[1,2,4]triazole-3,5-diamine;
*N*³-[3-Chloro-4-(3,3-dimethyl-but-1-ynyl)-phenyl]-1*H*-[1,2,4]triazole-3,5-diamine;
N*3*-(4-Bromo-3-pentafluorosulfur-phenyl)-1H-[1,2,4]triazole-3,5-diamine;
N*3*-(3-Bromo-5-pentafluorosulfur-phenyl)-1H-[1,2,4]triazole-3,5-diamine;
N*3*-(4-Bromo-3-fluoro-5-pentafluorosulfur-phenyl)-1H-[1,2,4]triazole-3,5-diamine;
[4-(5-Amino-1*H*-[1,2,4]triazol-3-ylamino)-2-chloro-6-trifluoromethyl-phenyl]-acetonitrile;
4-(5-Amino-1*H*-[1,2,4]triazol-3-ylamino)-2-pentafluorosulfur-benzonitrile;
4-(5-Amino-1*H*-[1,2,4]triazol-3-ylamino)-2-fluoro-6-pentafluorosulfur-benzonitrile;
4-(5-Amino-1*H*-[1,2,4]triazol-3-ylamino)-2-hydrazino-6-pentafluorosulfur-benzonitrile;
4-(1-Acetyl-5-amino-1*H*-[1,2,4]triazol-3-ylamino)-2,6-dichloro-benzonitrile;
4-[5-Amino-1-(4-trifluoromethyl-benzenesulfonyl)-1H-[1,2,4]triazol-3-ylamino]-2,6-dichlorobenzonitrile; and
*N*³-[3-Chloro-4-(1*H*-tetrazol-5-ylmethyl)-5-trifluoromethyl-phenyl]-1*H*-[1,2,4]triazole-3,5-diamine.

14. A composition comprising a compound of claim 13 and a pharmaceutically acceptable excipient.

## Patentansprüche

1. Verbindung der Formel I 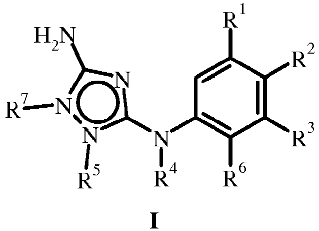 zur Verwendung bei der Behandlung einer Infektion mit einem Hepatitis-C-Virus (HCV), wobei:
R¹ für Wasserstoff, ein Halogen, eine C₁-C₆-Halogenalkylgruppe, eine niedere Halogenalkyl-C₁-C₆-alkyl-Gruppe, eine Trifluormethylsulfonylgruppe, eine C₁-C₆-Alkylgruppe, eine Alkoxygruppe, eine Stickstoffgruppe, eine Carboxylgruppe, eine C₁-C₆-Alkylsulfonamidogruppe, eine Trifluormethylsulfinylgruppe, eine Cycloalkylgruppe, eine SF₅-Gruppe oder eine -NHNH₂-Gruppe steht;
R² für Wasserstoff, ein Halogen, eine Cyanogruppe, eine Stickstoffgruppe, eine Trifluormethylsulfonylgruppe, eine C₁-C₆-Halogenalkylgruppe, eine Phenylethinylgruppe, eine C₁-C₆-Alkylsulfonylgruppe, eine C₁-C₆-Alkylaminosulfonylgruppe, eine C₁-C₆-Alkylamidogruppe, eine Aminosulfonylgruppe, eine Sulfonamidogruppe, eine Benzyloxygruppe, eine Cycloalkylgruppe, eine Heterocycloalkylsulfonylgruppe, eine Arylcarbonylgruppe, eine C₁-C₆-Alkylharnstoffgruppe, einen C₁-C₆-Alkylester, eine Phenylamidogruppe, eine Heterocycloalkylcarbonylgruppe, eine C₁-C₆-Alkylsulfonylphenylamidogruppe, eine C₁-C₆-Alkylcyanogruppe, eine Cyano-C₁-C₆-alkyl-Gruppe oder eine Tetrazolyl-C₁-C₆-alkyl-Gruppe steht;
R³ für Wasserstoff, ein Halogen, eine C₁-C₆-Alkylgruppe, eine C₁-C₆-Alkenylgruppe, eine C₁-C₆-Halogenalkylgruppe, eine Cyanogruppe, eine C₁-C₆-Halogenalkyloxygruppe, eine Cyanocycloalkylgruppe, eine C₁-C₆-Halogenalkyl-C₁-C₆-alkyl-Gruppe, eine SF₅-Gruppe oder eine Trifluormethylsulfanylgruppe steht;
oder R² und R³ zusammen ein Arylringsystem bilden;
R⁴ für Wasserstoff, eine C₁-C₆-Alkylgruppe, eine Cyano-C₁-C₆-alkyl-Gruppe oder eine Phenyl-C₁-C₆-alkylGruppe steht;
R⁵ fehlt oder für Wasserstoff, eine C₁-C₆-Alkyl- oder eine Cycloalkylgruppe steht;
R⁶ für Wasserstoff oder ein Halogen steht;
R⁷ fehlt oder für Wasserstoff, eine C₁-C₆-Alkylcarbonyl- oder eine C₁-C₆-Halogenalkylphenylsulfonylgruppe steht;
mit der Maßgabe, dass wenn R⁵ fehlt, R⁷ nicht fehlt; und
mit der Maßgabe, dass wenn R⁷ fehlt, R⁵ nicht fehlt;
oder ein pharmazeutisch annehmbares Salz davon, wobei Carboxyl eine Gruppe mit der Formel -C(=O)R₂ bezeichnet, wobei jedes R unabhängig für Wasserstoff oder eine C₁-C₆-Alkylgruppe steht;
wobei Amido eine Gruppe der Formel -C(=O)NR'R" oder -NR'C(=O)R" bezeichnet, wobei R' und R" unabhängig für Wasserstoff, eine Alkyl-, Alkoxy-, Cycloalkyl-, Heterocycloalkyl-, Aryl- oder Heteroarylgruppe stehen;
wobei Amino eine Gruppe der Formel -NR'R" bezeichnet, wobei R' und R" unabhängig für Wasserstoff, eine Alkyl-, Alkoxy-, Cycloakyl-, Heterocycloalkyl-, Aryl- oder Heteroarylgruppe stehen und wobei R' und R" gegebenenfalls zusammen mit dem Stickstoff, an den sie gebunden sind, eine Heterocycloalkylgruppe bilden.

2. Verbindung nach Anspruch 1, wobei R⁵ für Wasserstoff steht.

3. Verbindung nach Anspruch 2, wobei R⁴ für Wasserstoff steht.

4. Verbindung nach Anspruch 3, wobei R⁶ für Wasserstoff steht.

5. Verbindung nach einem der Ansprüche 1 bis 4, wobei R¹ für ein Halogen steht.

6. Verbindung nach einem der Ansprüche 1 bis 5, wobei R² für ein Halogen steht.

7. Verbindung nach einem der Ansprüche 1 bis 6, wobei R³ für ein Halogen steht.

8. Verbindung nach einem der Ansprüche 1 bis 6, wobei R³ für eine C₁-C₆-Halogenalkylgruppe steht.

9. Verbindung nach einem der Ansprüche 1 bis 3, wobei R¹ für Wasserstoff steht.

10. Verbindung nach einem der Ansprüche 1 bis 3, wobei R² für Wasserstoff steht.

11. Verbindung nach einem der Ansprüche 1 bis 3, wobei R³ für Wasserstoff steht.

12. Verbindung zur Verwendung bei der Behandlung einer Infektion mit einem Hepatitis-C-Virus (HCV), wobei die Verbindung aus der Gruppe ausgewählt ist, die aus folgenden Verbindungen besteht:
*N*³-Naphthalen-2-yl-1*H*-[1,2,4]triazol-3,5-diamin;
4-(5-Amino-1*H*-[1,2,4]triazol-3-ylamino)-*N*-methylbenzolsulfonamid;
4-(5-Amino-1*H*-[1,2,4]triazol-3-ylamino)-benzolsulfonamid;
3-(5-Amino-1*H*-[1,2,4]triazol-3-ylamino)-benzoesäure;
N³-(3-Chlorphenyl)-1*H*-[1,2,4]triazol-3,5-diamin;

13. Verbindung, ausgewählt aus der Gruppe bestehend aus:
*N*³-(4-Brom-3,5-dichlorphenyl)-1*H*-[1,2,4]triazol-3,5-diamin;
4-(5-Amino-1*H*-[1,2,4|triazol-3-ylamino)-2,6-dichlorbenzonitril;
*N*³-(3,5-Dichlor-4-iodphenyl)-1*H*-[1,2,4]triazol-3,5-diamin;
*N*³-(3-Trifluormethansulfonylphenyl)-1*H*-[1,2,4]triazol-3,5-diamin;
*N*³-(4-Brom-3-chlor-5-fluorphenyl)-1*H*-[1,2,4]triazol-3,5-diamin;
*N*³-(3-tert-Butylphenyl)-1*H*-[1,2,4]triazol-3,5-diamin;
*N*³-(3-Chlor-4-trifluormethansulfonylphenyl)-1*H*-[1,2,4]triazol-3,5-diamin;
*N*³-(3-Chlor-4-nitrophenyl)-1*H*-[1,2,4]triazol-3,5-diamin;
*N*³-(3-Chlor-4-ethinylphenyl)-1*H*-[1,2,4]triazol-3,5-diamin;
*N*³-(3-Chlor-4-trifluormethylphenyl)-1*H*-[1,2,4]triazol-3,5-diamin;
*N*³-(3,4,5-Trichlorphenyl)-1*H*-[1,2,4]triazol-3,5-diamin;
4-(5-Amino-1H-[1,2,4]triazol-3-ylamino)-2-chlorbenzonitril;
*N*³-(4-Chlor-3-nitrophenyl)-1*H*-[1,2,4]triazol-3,5-diamin;
*N*³-(3-Chlor-4-phenylethinylphenyl)-1*H*-[1,2,4]triazol-3,5-diamin;
*N*³-(3-Bromphenyl)-1*H*-[1,2,4]triazol-3,5-diamin;
4-(5-Amino-1*H*-[1,2,4]triazol-3-ylamino)-2-tert-butylbenzonitril;
*N*³-(4-Trifluormethansulfonylphenyl)-1*H*-[1,2,4]triazol-3,5-diamin;
*N*³-(3,5-Di-*tert*-butylphenyl)-1*H*-[1,2,4]triazol-3,5-diamin;
*N*³-(4-Methansulfonylphenyl)-1*H*-[1,2,4]triazol-3,5-diamin;
*N*³-(3-Methansulfonylphenyl)-1*H*-[1,2,4]triazol-3,5-diamin;
[4-(5-Amino-1*H*-[1,2,4]triazol-3-ylamino)-phenyl]-morpholin-4-yl-methanon;
4-(5-Amino-1*H*-[1,2,4]triazol-3-ylamino)-*N*,*N*-dimethylbenzamid;
4-(5-Amino-1*H*-[1,2,4]triazol-3-ylamino)-*N*,*N*-dimethylbenzolsulfonamid;
*N*³-Methyl-*N*³-(3,4,5-trichlorphenyl)-1*H*-[1,2,4]triazol-3,5-diamin;
*N*³-(3,5-Dichlorphenyl)-*N*³-methyl-1*H*-[1,2,4]triazol-3,5-diamin;
*N*³-Benzyl-*N*³-(3,5-dichlorphenyl)-1*H*-[1,2,4]triazol-3,5-diamin;
[(5-Amino-1*H*-[1,2,4]triazol-3-yl)-(3,5-dichlorphenyl)-amino]-acetonitril;
N3-(3-Trifluormethansulfinyl-5-trifluormethylsulfanylphenyl)-1H-[1,2,4] triazol-3,5-diamin;
N-[3-(5-Amino-1H-[1,2,4]triazol-3-ylamino)-phenyl]-methansulfonamid;
4-(5-Amino-1H-[1,2,4]triazol-3-ylamino)-2-trifluormethansulfonylbenzonitril;
4-(5-Amino-1H-[1,2,4]triazol-3-ylamino)-2-cyclopropylbenzonitril;
*N*³-(4-Benzyloxy-3-chlorphenyl)-1*H*-[1,2,4]triazol-3,5-diamin;
*N*³-(3,5-Dichlor-4-methylphenyl)-1*H*-[1,2,4]triazol-3,5-diamin;
*N*³-(3,5-Dichlor-4-cyclopropylphenyl)-1*H*-[1,2,4]triazol-3,5-diamin;
*N*³-(4-Brom-3-chlorphenyl)-1*H*-[1,2,4]triazol-3,5-diamin;
*N*³-(3,5-Dichlorphenyl)-1*H*-[1,2,4]triazol-3,5-diamin;
*N*³-(4-Brom-3-chlor-5-trifluormethylphenyl)-1*H*-[1,2,4]triazol-3,5-diamin;
4-(5-Amino-1*H*-[1,2,4]triazol-3-ylamino)-2-chlor-6-trifluormethylbenzonitril;
*N*³-[3,5-Dichlor-4-(morpholin-4-sulfonyl)-phenyl]-1*H*-[1,2,4]triazol-3,5-diamin;
*N*³-(4-Brom-3-fluor-5-trifluormethylphenyl)-1*H*-[1,2,4]triazol-3,5-diamin;
*N⁵*-(4-Brom-3,5-difluorphenyl)-1*H*-[1,2,4]triazol-3,5-diamin;
4-(5-Amino-1*H*-[1,2,4]triazol-3-ylamino)-benzonitril;
3-(5-Amino-1H-[1,2,4]triazol-3-ylamino)-5-chlorbenzonitril;
*N*³-(2,3-Dichlorphenyl)-1*H*-[1,2,4] triazol-3,5-diamin;
*N*³-(3-Chlor-5-trifluormethylphenyl)-1*H*-[1,2,4]triazol-3,5-diamin;
*N*³-(4-Chlor-3-trifluormethylphenyl)-1*H*-[1,2,4]triazol-3,5-diamin;
*N*³-(3-Trifluormethoxyphenyl)-1*H*-[1,2,4]triazol-3,5-diamin;
*N*³-(2-Fluor-3-trifluormethylphenyl)-1*H*-[1,2,4]triazol-3,5-diamin;
*N*³-(2-Fluor-5-trifluormethylphenyl)-1*H*-[1,2,4]triazol-3,5-diamin;
*N*³-[3-(2,2,2-Trifluorethyl)-phenyl]-1*H*-[1,2,4]triazol-3,5-diamin;
*N*³-(3-Isopropylphenyl)-1*H*-[1,2,4]triazol-3,5-diamin;
[3-(5-Amino-1*H*-[1,2,4]triazol-3-ylamino)-phenyl]-phenylmethanon;
1-[3-(5-Amino-1*H*-[1,2,4]triazol-3-ylamino)-phenyl]-cyclopropancarbonitril;
*N*³-(3-Chlor-4-fluor-5-trifluormethylphenyl)-1*H*-[1,2,4]triazol-3,5-diamin;
*N*³-(3-Chlor-5-fluorphenyl)-1*H*-[1,2,4]triazol-3,5-diamin;
1-[4-(5-Amino-1*H*-[1,2,4]triazol-3-ylamino)-2-chlorphenyl]-3-methylharnstoff;
4-(5-Amino-1*H*-[1,2,4]triazol-3-ylamino)-2,6-dichlorbenzoesäuremethylester;
4-(5-Amino-1*H*-[1,2,4]triazol-3-ylamino)-2,6-dichlor-*N*-phenylbenzamid;
[4-(5-Amino-1*H*-[1,2,4]triazol-3-ylamino)-2,6-dichlorphenyl]-pyrrolidin-1-yl-methanon;
4-(5-Amino-1*H*-[1,2,4]triazol-3-ylamino)-2,6-dichlor-*N*-(3-methansulfonylphenyl)-benzamid;
*N*³-(3,5-Dichlor-4-vinylphenyl)-1*H*-[1,2,4]triazol-3,5-diamin;
4-(5-Amino-2*H*-[1,2,4]triazol-3-ylamino)-2-fluor-6-trifluormethylbenzonitril;
*N*³-(3-Fluor-5-trifluormethylphenyl)-1*H*-[1,2,4]triazol-3,5-diamin;
4-(5-Amino-2*H*-[1,2,4]triazol-3-ylamino)-2,6-difluorbenzonitril;
N*3*-(3-Pentafluorschwefelphenyl)-1*H*-[1,2,4]triazol-3,5-diamin;
1-Methyl-N*5*-(3-pentafluorschwefelphenyl)-1*H*-[1,2,4]triazol-3,5-diamin;
Cyclohexyl-N*5*-(3-pentafluorschwefelphenyl)-1*H*-[1,2,4]triazol-3,5-diamin;
N*3*-(3-Fluor-5-pentafluorschwefelphenyl)-1*H*-[1,2,4]triazol-3,5-diamin;
N*5*-(3-Fluor-5-pentafluorschwefelphenyl)-1-methyl-1*H*-[1,2,4]triazol-3,5-diamin;
N*3*-(4-Pentafluorschwefelphenyl)-1*H*-[1,2,4]triazol-3,5-diamin;
*N*³-(3-Chlor-4-methylphenyl)-1*H*-[1,2,4]triazol-3,5-diamin;
*N*³-(3-Chlor-4-methansulfonylphenyl)-1*H*-[1,2,4]triazol-3,5-diamin;
4-(5-Amino-1*H*-[1,2,4]triazol-3-ylamino)-2-chlor-*N*,*N*-dimethylbenzamid;
*N*³-[3,5-Dichlor-4-(3,3-dimethyl-but-1-inyl)-phenyl]-1*H*-[1,2,4]triazol-3,5-diamin;
*N*³-[3-Chlor-4-(3,3-dimethyl-but-1-inyl)-phenyl]-1*H*-[1,2,4]triazol-3,5-diamin;
N*3*-(4-Brom-3-pentafluorschwefelphenyl)-1H-[1,2,4]triazol-3,5-diamin;
N*3*-(3-Brom-5-pentafluorschwefelphenyl)-1H-[1,2,4]triazol-3,5-diamin;
N*3*-(4-Brom-3-fluor-5-pentafluorschwefelphenyl)-1H-[1,2,4]triazol-3,5-diamin;
[4-(5-Amino-1H-[1,2,4]triazol-3-ylamino)-2-chlor-6-trifluormethylphenyl]-acetonitril;
4-(5-Amino-1H-[1,2,4]triazol-3-ylamino)-2-pentafluorschwefelbenzonitril;
4-(5-Amino-1H-[1,2,4]triazol-3-ylamino)-2-fluor-6-pentafluorschwefelbenzonitril;
4-(5-Amino-1H-[1,2,4]triazol-3-ylamino)-2-hydrazino-6-pentafluorschwefelbenzonitril;
4-(1-Acetyl-5-amino-1H-[1,2,4]triazol-3-ylamino)-2,6-dichlorbenzonitril;
4-[5-Amino-1-(4-trifluormethylbenzolsulfonyl)-1H-[1,2,4]triazol-3-ylamino]-2,6-dichlorbenzonitril; und
*N*³-[3-Chlor-4-(1H-tetrazol-5-ylmethyl)-5-trifluormethyl-phenyl]-1*H*-[1,2,4]triazol-3,5-diamin.

14. Zusammensetzung, umfassend eine Verbindung nach Anspruch 13 und einen pharmazeutisch annehmbaren Exzipienten.

## Revendications

1. Composé de formule I 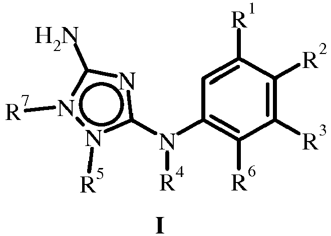 destiné à être utilisé dans le traitement d'une infection par le virus de l'hépatite C (VHC), dans laquelle :
R¹ est H, un atome d'halogène, un groupe halogénoalkyle en C₁ à C₆, (halogénoalkyle inférieur)alkyle en C₁ à C₆, trifluorométhylsulfonyle, alkyle en C₁ à C₆, alcoxy, nitro, carboxyle, (alkyle en C₁ à C₆)sulfonamido, trifluorométhylsulfinyle, cycloalkyle, SF₅, ou -NHNH₂ ;
R² est H, un atome d'halogène, un groupe cyano, nitro, trifluorométhylsulfonyle, halogénoalkyle en C₁ à C₆, phényléthynyle, (alkyle en C₁ à C₆)sulfonyle, (alkyle en C₁ à C₆)aminosulfonyle, (alkyle en C₁ à C₆)amido, aminosulfonyle, sulfonamido, benzyloxy, cycloalkyle, hétérocycloalkylsulfonyle, arylcarbonyle, (alkyle en C₁ à C₆)urée, ester d'alkyle en C₁ à C₆, phénylamido, hétérocycloalkylcarbonyle, (alkyle en C₁ à C₆)sulfonylphénylamido, (alkyle en C₁ à C₆)cyano, cyanoalkyle en C₁ à C₆ ou tétrazolylalkyle en C₁ à C₆ ;
R³ est H, un atome d'halogène, un groupe alkyle en C₁ à C₆, alcényle en C₁ à C₆, halogénoalkyle en C₁ à C₆, cyano, halogénoalkyloxy en C₁ à C₆, cyanocycloalkyle, (halogénoalkyle en C₁ à C₆)alkyle en C₁ à C₆, SF₅ or trifluorométhylsulfanyle ;
ou R² et R³ forment ensemble un système de cycle arylique ;
R⁴ est H, un groupe alkyle en C₁ à C₆, cyanoalkyle en C₁ à C₆ ou phénylalkyle en C₁ à C₆ ;
R⁵ est absent, H, un groupe alkyle en C₁ à C₆ ou cycloalkyle ;
R⁶ est H ou un atome d'halogène ;
R⁷ est absent, H, un groupe (alkyle en C₁ à C₆)carbonyle ou (halogénoalkyle en C₁ à C₆)phénylsulfonyle ;
à condition que lorsque R⁵ est absent, R⁷ ne soit pas absent ; et
à condition que lorsque R⁷ est absent, R⁵ ne soit pas absent ;
ou sel pharmaceutiquement acceptable de celui-ci, le groupe carboxyle représentant un groupe de formule -C (=O)R₂ où chaque R est indépendamment H ou un groupe alkyle en C₁ à C₆ ;
le groupe amido représentant un groupe de formule -C (=O)NR'R" ou -NR'C(=O)R" où R' et R" sont indépendamment H, un groupe alkyle, alcoxy, cycloalkyle, hétérocycloalkyle, aryle ou hétéroaryle ;
le groupe amino représentant un groupe de formule -NR'R" où R' et R" sont indépendamment H, un groupe alkyle, alcoxy, cycloalkyle, hétérocycloalkyle, aryle ou hétéroaryle et où R' et R" forment éventuellement avec l'atome d'azote auquel ils sont liés un groupe hétérocycloalkyle.

2. Composé selon la revendication 1, dans lequel R⁵ est H.

3. Composé selon la revendication 2, dans lequel R⁴ est H.

4. Composé selon la revendication 3, dans lequel R⁶ est H.

5. Composé selon l'une quelconque des revendications 1 à 4, dans lequel R¹ est un atome d'halogène.

6. Composé selon l'une quelconque des revendications 1 à 5, dans lequel R² est un atome d'halogène.

7. Composé selon l'une quelconque des revendications 1 à 6, dans lequel R³ est un atome d'halogène.

8. Composé selon l'une quelconque des revendications 1 à 6, dans lequel R³ est un groupe halogénoalkyle en C₁ à C₆.

9. Composé selon l'une quelconque des revendications 1 à 3, dans lequel R¹ est H.

10. Composé selon l'une quelconque des revendications 1 à 3, dans lequel R² est H.

11. Composé selon l'une quelconque des revendications 1 à 3, dans lequel R³ est H.

12. Composé destiné à être utilisé dans le traitement d'une infection par le virus de l'hépatite C (VHC), le composé étant choisi dans le groupe constitué par :
la *N*³-naphtalén-2-yl-1*H*-[1,2,4]triazole-3,5-diamine ;
le 4-(5-amino-1*H*-[1,2,4]triazol-3-ylamino)-*N*-méthyl-benzènesulfonamide ;
le 4-(5-amino-1*H*-[1,2,4]triazol-3-ylamino)-benzènesulfonamide ;
l'acide 3-(5-amino-1*H*-[1,2,4]triazol-3-ylamino)-benzoïque ;
la *N*³-(3-chloro-phényl)-1*H*-[1,2,4]triazole-3,5-diamine ;

13. Composé choisi dans le groupe constitué par :
la *N*³-(4-bromo-3,5-dichloro-phényl)-1*H*-[1,2,4]triazole-3,5-diamine ;
le 4-(5-amino-1*H*-[1,2,4|triazol-3-ylamino)-2,6-dichloro-benzonitrile ;
la *N*³-(3,5-dichloro-4-iodo-phényl)-1*H*-[1,2,4]triazole-3,5-diamine ;
la *N*³-(3-trifluorométhanesulfonyl-phényl)-1*H*-[1,2,4]triazole-3,5-diamine ;
la *N*³-(4-bromo-3-chloro-5-fluoro-phényl)-1*H*-[1,2,4]triazole-3,5-diamine ;
la *N*³-(S-tert-butyl-phényl)-1*H*-[1,2,4]triazole-3,5-diamine ;
la *N*³-(3-chloro-4-trifluorométhanesulfonyl-phényl)-1*H*-[1,2,4]triazole-3,5-diamine ;
la *N*³-(3-chloro-4-nitro-phényl)-1*H*-[1,2,4]triazole-3,5-diamine ;
la *N*³-(3-chloro-4-éthynyl-phényl)-1*H*-[1,2,4]triazole-3,5-diamine ;
la *N*³-(3-chloro-4-trifluorométhyl-phényl)-1*H*-[1,2,4]triazole-3,5-diamine ;
la *N*³-(3,4,5-trichloro-phényl-1*H*-[1,2,4]triazole-3,5-diamine ;
le 4-(5-amino-1H-[1,2,4]triazol-3-ylamino)-2-chloro-benzonitrile ;
la *N*³-(4-chloro-3-nitro-phényl)-1*H*-[1,2,4]triazole-3,5-diamine ;
la *N*³-(3-chloro-4-phényléthynyl-phényl)-1*H*-[1,2,4]triazole-3,5-diamine ;
la *N*³-(3-bromo-phényl)-1*H*-[1,2,4]triazole-3,5-diamine ;
le 4-(5-amino-1*H*-[1,2,4]triazol-3-ylamino)-2-*tert*-butyl-benzonitrile ;
la *N*³-(4-trifluorométhanesulfonyl-phényl)-1*H*-[1,2,4]triazole-3,5-diamine ;
la *N*³-(3,5-di-*tert*-butyl-phényl)-1*H*-[1,2,4]triazole-3,5-diamine ;
la *N*³-(4-méthanesulfonyl-phényl)-1*H*-[1,2,4]triazole-3,5-diamine ;
la *N*-(3-méthanesulfonyl-phényl)-1*H*-[1,2,4]triazole-3,5-diamine ;
la [4-(5-amino-1*H*-[1,2,4]triazol-3-ylamino)-phényl]-morpholin-4-yl-méthanone ;
le 4-(5-amino-1*H*-[1,2,4]triazol-3-ylamino)-*N*,*N*-diméthyl-benzamide ;
le 4-(5-amino-1*H*-[1,2,4]triazol-3-ylamino)-*N*,*N*-diméthyl-benzènesulfonamide ;
la *N*³-méthyl-*N*³-(3,4,5-trichloro-phényl)-1*H*-[1,2,4]triazole-3,5-diamine ;
la *N*³-(3,5-dichloro-phényl)-*N*³-méthyl-1*H*-[1,2,4]triazole-3,5-diamine ;
la *N*³-benzyl-*N*³-(3,5-dichloro-phényl)-1*H*-[1,2,4]triazole-3,5-diamine ;
le [(5-amino-1*H*-[1,2,4]triazol-3-yl)-(3,5-dichloro-phényl)-amino]-acétonitrile ;
la N3-(3-trifluorométhanesulfinyl-5-trifluorométhylsulfanyl-phényl)-1H-[1,2,4] triazole-3,5-diamine ;
le N-[3-(5-amino-iH-[1,2,4]triazol-3-ylamino)-phényl]-méthanesulfonamide ;
le 4-(5-amino-1H-[1,2,4]triazol-3-ylamino)-2-trifluorométhanesulfonyl-benzonitrile ;
le 4-(5-amino-1H-[1,2,4]triazol-3-ylamino)-2-cyclopropyl-benzonitrile ;
la *N*³-(4-benzyloxy-3-chloro-phényl)-1*H*-[1,2,4]triazole-3,5-diamine ;
la *N*³-(3,5-dichloro-4-méthyl-phényl)-1*H*-[1,2,4]triazole-3,5-diamine ;
la *N*³-(3,5-dichloro-4-cyclopropyl-phényl)-1*H*-[1,2,4]triazole-3,5-diamine ;
la *N*³-(4-bromo-3-chloro-phényl)-1*H*-[1,2,4]triazole-3,5-diamine ;
la *N*³-(3,5-dichloro-phényl)-1*H*-[1,2,4]triazole-3,5-diamine ;
la *N*³-(4-bromo-3-chloro-5-trifluorométhyl-phényl)-1*H*-[1,2,4]triazole-3,5-diamine ;
le 4-(5-amino-1*H*-[1,2,4]triazol-3-ylamino)-2-chloro-6-trifluorométhyl-benzonitrile ;
la *N*³-[3,5-dichloro-4-(morpholine-4-sulfonyl)-phényl]-1*H*-[1,2,4]triazole-3,5-diamine ;
la *N*⁵-(4-bromo-3-fluoro-5-trifluorométhyl-phényl)-1*H*-[1,2,4]triazole-3,5-diamine ;
la *N*⁵-(4-bromo-3,5-difluoro-phényl)-1*H*-[1,2,4]triazole-3,5-diamine ;
le 4-(5-amino-1*H*-[1,2,4]triazol-3-ylamino)-benzonitrile ;
le 3-(5-amino-1H-[1,2,4]triazol-3-ylamino)-5-chloro-benzonitrile ;
la *N*³-(2,3-dichloro-phényl)-1*H*-[1,2,4]triazole-3,5-diamine ;
la *N*³-(3-chloro-5-trifluorométhyl-phényl)-1*H*-[1,2,4]triazole-3,5-diamine ;
la *N*³-(4-chloro-3-trifluorométhyl-phényl)-1*H*-[1,2,4]triazole-3,5-diamine ;
la *N*³-(3-trifluorométhoxy-phényl)-1*H*-[1,2,4]triazole-3,5-diamine ;
la *N*³-(2-fluoro-3-trifluorométhyl-phényl)-1*H*-[1,2,4]triazole-3,5-diamine ;
la *N*³-(2-fluoro-5-trifluorométhyl-phényl)-1*H*-[1,2,4]triazole-3,5-diamine ;
la *N*³-[3-(2,2,2-trifluoro-éthyl)-phényl]-1*H*-[1,2,4]triazole-3,5-diamine ;
la *N*³-(3-isopropyl-phényl-1*H*-[1,2,4]triazole-3,5-diamine ;
la [3-(5-amino-1*H*-[1,2,4]triazol-3-ylamino)-phényl]-phényl-méthanone ;
le 1-[3-(5-amino-1*H*-[1,2,4]triazol-3-ylamino)-phényl]-cyclopropanecarbonitrile ;
la *N*³-(3-chloro-4-fluoro-5-trifluorométhyl-phényl)-1*H*-[1,2,4]triazole-3,5-diamine ;
la *N*³-(3-chloro-5-fluoro-phényl)-1*H*-[1,2,4]triazole-3,5-diamine ;
la 1-[4-(5-amino-1*H*-[1,2,4]triazol-3-ylamino)-2-chloro-phényl]-3-méthyl-urée ;
l'ester de méthyle de l'acide 4-(5-amino-1*H*-[1,2,4]triazol-3-ylamino)-2,6-dichloro-benzoïque ;
le 4-(5-amino-1*H*-[1,2,4]triazol-3-ylamino)-2,6-dichloro-*N*-phényl-benzamide ;
la [4-(5-amino-1*H*-[1,2,4]triazol-3-ylamino)-2,6-dichloro-phényl]-pyrrolidin-1-yl-méthanone ;
le 4-(5-amino-1*H*-[1,2,4]triazol-3-ylamino)-2,6-dichloro-*N*-(3-méthanesulfonyl-phényl)-benzamide ;
la *N*³-(3,5-dichloro-4-vinyl-phényl)-1*H*-[1,2,4]triazole-3,5-diamine ;
le 4-(5-amino-2*H*-[1,2,4]triazol-3-ylamino)-2-fluoro-6-trifluorométhyl-benzonitrile ;
la *N*³-fluoro-5-trifluorométhyl-phényl)-1*H*-[1,2,4]triazole-3,5-diamine ;
le 4-(5-amino-2*H*-[1,2,4]triazol-3-ylamino)-2,6-difluoro-benzonitrile ;
la N*3*-(3-pentafluorosulfuro-phényl)-1*H*-[1,2,4]triazole-3,5-diamine ;
la 1-méthyl-N*5*-(3-pentafluorosulfuro-phényl)-1H-[1,2,4]triazole-3,5-diamine ;
la cyclohexyl-N*5*-(3-pentafluorosulfuro-phényl)-1*H*-[1,2,4]triazole-3,5-diamine ;
la N³-(3-fluoro-5-pentafluorosulfuro-phényl)-1H-[1,2,4]triazole-3,5-diamine ;
la N⁵-(3-fluoro-5-pentafluorosulfuro-phényl)-1-méthyl-1H-[1,2,4]triazole-3,5-diamine ;
la N³-(4-pentafluorosulfuro-phényl)-1*H*-[1,2,4]triazole-3,5-diamine ;
la *N*³-(3-chloro-4-méthyl-phényl)-1*H*-[1,2,4]triazole-3,5-diamine ;
la *N*³-(3-chloro-4-méthanesulfonyl-phényl)-1*H*-[1,2,4]triazole-3,5-diamine ;
le 4-(5-amino-1*H*-[1,2,4]triazol-3-ylamino)-2-chloro-*N*,*N*-diméthyl-benzamide ;
la *N*³-[3,5-dichloro-4-(3,3-diméthyl-but-1-ynyl)-phényl]-1*H*-[1,2,4]triazole-3,5-diamine ;
la *N*³-[3-chloro-4-(3,3-diméthyl-but-1-ynyl)-phényl]-1*H*-[1,2,4]triazole-3,5-diamine ;
la N³-(4-bromo-3-pentafluorosulfuro-phényl)-1H-[1,2,4]triazole-3,5-diamine ;
la N³-(3-bromo-5-pentafluorosulfuro-phényl)-1H-[1,2,4]triazole-3,5-diamine ;
la N³-(4-bromo-3-fluoro-5-pentafluorosulfuro-phényl)-1H-[1,2,4]triazole-3,5-diamine ;
le [4-(5-amino-1H-[1,2,4]triazol-3-ylamino)-2-chloro-6-trifluorométhyl-phényl]-acétonitrile ;
le 4-(5-amino-1H-[1,2,4]triazol-3-ylamino)-2-pentafluorosulfuro-benzonitrile ;
le 4-(5-amino-1H-[1,2,4]triazol-3-ylamino)-2-fluoro-6-pentafluorosulfuro-benzonitrile ;
le 4-(5-amino-1H-[1,2,4]triazol-3-ylamino)-2-hydrazino-6-pentafluorosulfuro-benzonitrile ;
le 4-(1-acétyl-5-amino-1H-[1,2,4]triazol-3-ylamino)-2,6-dichloro-benzonitrile ;
le 4-[5-amino-1-(4-trifluorométhyl-benzènesulfonyl)-1H-[1,2,4]triazol-3-ylamino]-2,6-dichloro-benzonitrile ; et
la *N*³-[3-chloro-4-(1H-tétrazol-5-ylméthyl)-5-trifluorométhyl-phényl]-1*H*-[1,2,4]triazole-3,5-diamine.

14. Composition comprenant un composé selon la revendication 13 et un excipient pharmaceutiquement acceptable.
